# EUROPEAN PATENT SPECIFICATION

(11) **EP 1 289 507 B1**
(45) Date of publication and mention of the grant of the patent: **13.02.2008**
(21) Application number: 00978588.2
(22) Date of filing: 14.11.2000
(51) Int. Cl.: A61K 31/00, A61K 31/585, A61P 43/00, A61P 9/00, A61P 9/12, A61P 9/04, A61P 7/10, A61P 9/10, A61P 13/12

(54) **USE OF AN ALDOSTERONE ANTAGONIST FOR THE TREATMENT OR PROPHYLAXIS OF ALDOSTERONE-MEDIATED PATHOGENIC EFFECTS**
VERWENDUNG VON EINEM ALDOSTERONANTAGONIST ZUR BEHANDLUNG UND VORBEUGUNG VON ALDOSTERON-VERMITTELTEN PATHOGENEN ZUSTÄNDEN
EMPLOI D'UN ANTAGONISTE DE L'ALDOSTERONE POUR LE TRAITEMENT OU LA PROPHYLAXIE D'EFFETS PATHOGENES INDUITS PAR L'ALDOSTERONE

(30) Priority: 13.06.2000 US 211264 P; 13.06.2000 US 211064 P; 13.06.2000 US 211250 P; 13.06.2000 US 211253 P; 13.06.2000 US 211311 P; 13.06.2000 US 211340 P; 13.06.2000 US 211451 P; 13.06.2000 US 211459 P; 27.07.2000 US 221358 P; 27.07.2000 US 221364 P; 14.09.2000 US 233056 P
(43) Date of publication of application: 12.03.2003
(73) Proprietor: Pharmacia Corporation, Peapack, NJ 07977 (US)
(72) Inventor: WILLIAMS, Gordon, H., Belmont, MA 02478 (US); FUNDER, John, W., Prahan, VIC 3181 (AU); GARTHWAITE, Susan, M., Evanston, IL 60202 (US); RONIKER, Barbara, Chicago, IL 60610 (US); FEDDE, Kenton, N., Webster Groves, MO 63119 (US); ROCHA, Ricardo, Skokie, IL 60077 (US)
(74) Representative: Ruddock, Keith Stephen
(86) International application number: PCT/US2000/031155
(87) International publication number: WO 2001/095892

(56) References cited:
- WO-A-95/15166
- WO-A-96/24358
- LUFT F.C.: "Salt and hypertension at the close of the millenium." WIENER KLINISCHE WOCHENSCHRIFT, (31 JUL 1998) 110/13-14 (459-466). , XP001003089
- ICHIKAWA S ET AL: "EFFECT OF SPIRONOLACTONE ON FLUID VOLUMES AND ADRENAL STEROIDS IN PRIMARY ALDOSTERONISM." JPN CIRC J, (1984 (RECD 1985)) 48 (11), 1184-1196. , XP000994737
- HELBER A ET AL: "EVIDENCE FOR A SUBGROUP OF ESSENTIAL HYPERTENSIVES WITH NONSUPPRESSIBLE EXCRETION OF ALDO STERONE DURING SODIUM LOADING." KLIN WOCHENSCHR, (1980) 58 (9), 439-448. , XP000994744
- ULICK S ET AL: "A SYNDROME OF APPARENT MINERALO CORTICOID EXCESS ASSOCIATED WITH DEFECTS IN THE PERIPHERAL METABOLISM OF CORTISOL." J CLIN ENDOCRINOL METAB, (1979) 49 (5), 757-764. , XP000994746
- SHACKLETON C H ET AL: "Hypertension in a four-year-old child: gas chromatographic and mass spectrometric evidence for deficient hepatic metabolism of steroids." JOURNAL OF CLINICAL ENDOCRINOLOGY AND METABOLISM, (1980 APR) 50 (4) 786-02. , XP000994740
- FUNDER, JOHN W.: "Eplerenone, a new mineralocorticoid antagonist: in vitro and in vivo studies" CURR. OPIN. ENDOCRINOL. DIABETES (2000), 7(3), 138-142 , XP001004704
- EPSTEIN, M. (1) ET AL: "Eplerenone, a new selective aldosterone receptor antagonist (SARA): Efficacy in patients with mild to moderate hypertension." JOURNAL OF THE AMERICAN SOCIETY OF NEPHROLOGY, (SEPT., 1998) VOL. 9, NO. PROGRAM AND ABSTR. ISSUE, PP. 322A-323A. MEETING INFO.: 31ST ANNUAL MEETING OF THE AMERICAN SOCIETY OF NEPHROLOGY PHILADELPHIA, PENNSYLVANIA, USA OCTOBER 25-28, 1998 AMERICAN SO, XP001003077
- "VIDAL" 1996 , EDITIONS DU VIDAL , PARIS XP002168546 page 37 -page 38 "ALDACTONE"
- HOSTETTER T.H. ET AL: 'MINERALOCORTICOID RECEPTOR BLOCKADE IN THE REMNANT KIDNEY MODEL' JOURNAL OF THE AMERICAN SOCIETY OF NEPHROLOGY vol. 10, September 1999, WILLIAMS AND WILKINS, BALTIMORE, MD, US, page 75A, XP008054964
- EPSTEIN ET AL J. AM. COLL. CARDIOL. vol. 39, no. SUPA, 2002, page 249A

## Description

### BACKGROUND OF THE INVENTION

### Field of the Invention

The present invention relates to the use of eplerenone for the manufacture of a medicament for the treatment or prophylaxis of renal dysfunction in a human subject suffering from or susceptible to renal dysfunction, wherein said renal dysfunction is diabetic nephropathy or end-stage renal disease.

### Description of the Related Art

Essential hypertension is a major vascular disease throughout the world and represents a significant public health problem. Elevated blood pressure associated with hypertension has been linked to the incidence of coronary heart disease and stroke. For example, Blumenfeld JD and Laragh JH. Congestive heart failure: pathophysiology, diagnosis and management, 1^{st} ed. Caddo (OK): Professional Communications, Inc.; 1994, reported that hypertension triples the risk for developing heart failure. Similarly, MacMahon S et al.: Lancet 1990;335:765-71, reported that a prolonged reduction of only 5 mm Hg in diastolic blood pressure results in a reduction of at least 20 percent of the risk for developing coronary heart disease and at least 33 percent of the risk for developing stroke.

Hypertension also has been linked to end-stage renal disease. End-stage renal disease caused by hypertension and/or diabetic nephropathy is an enormous public health burden, with an incidence and prevalence that is increasing alarmingly in many countries including the United States. (United States Renal Data System. Excerpts from United States Renal Data System 1999 Annual Data Report. Am J Kidney Dis 1999;34 Suppl 1:S1-S176). The incidence of hypertension-induced end-stage renal disease has increased despite the substantive decrease in other complications of hypertension, such as stroke and coronary heart disease, due to improved blood pressure awareness and control. (The sixth report of the Joint National Committee on prevention, detection, evaluation, and treatment of high blood pressure. Arch Intern Med 1997;157:2413-46).

The renin-angiotensin-aldosterone system ("RAAS") plays a major role in the development and progression of hypertension. The RAAS plays a pivotal role in nearly all physiologic responses to deficits in extracellular fluid volume and hypotension. A decrease in blood volume or arterial blood pressure causes a release of renal renin that acts on angiotensinogen to form angiotensin I. Angiotensin converting enzyme then converts angiotensin I into angiotensin II, which causes several peripherally and centrally mediated responses that restore blood volume and pressure. Peripherally, angiotensin II acts on smooth muscle cells resulting in vasocontriction to increase blood pressure. Centrally, angiotensin II acts to increase sympathetic outflow and vasopressin release. Although the conversion of angiotensin I to angiotensin II occurs primarily in the lung with angiotensin II then circulating peripherally and centrally to target tissues, this conversion can also occur in regions of the brain.

Angiotensin II also stimulates the zona glomerulosa of the adrenal cortex, resulting in increased synthesis and secretion of the mineralocorticoid aldosterone. Aldosterone acts on the distal and collecting tubules to cause sodium retention and excretion of potassium and hydrogen. Such sodium retention results in increased blood volume. Aldosterone plays a role in the pathophysiology of heart failure and has been linked to high blood pressure, cardiac hypertrophy, cardiac and vascular fibrosis, and ventricular arrhythmias (Dzau VJ et al.: Circulation 1981; 63:645-651). In patients with heart failure, high aldosterone levels appeared to correspond to increased mortality in those patients. (Gerbe JG, et al.: Antihypertensive agents and the drug therapy of hypertension. In: Goodman LS, Gilman AG, editors. Goodman and Gilman's the pharmacological basis of therapeutics, 8th edition. New York: McGraw-Hill; 1993. p.784-813).

Weber KT et al. (Cir 1987;75(Supp. I):I-40 through I-47) reported that chronic elevations in circulating aldosterone levels resulted in fibrous tissue formation in the heart and vessels, which contributed to progressive heart failure in selected animals. The increase in myocardial collagen production and subsequent left ventricular hypertrophy lead to myocardial stiffness, reduced ventricular and vascular compliance, impaired diastolic filling, diastolic and systolic dysfunction, ischemia, and ultimately heart failure. According to Struthers AD. J Cardiac Failure 1996; 2:47-54, such myocardial fibrosis can also lead to arrhythmias and sudden death. Aldosterone blocks myocardial norepinephrine uptake, increases plasma norepinephrine, and promotes ventricular ectopic activity. Rocha R et al.: Am J Hypertension 1999;12:76A, reported that aldosterone affected baroreceptor function and causes cerebro- and renal- vascular damage as well as endothelial dysfunction in rats. Aldosterone also reportedly increases plasminogen activator inhibitor levels and thereby may impede fibrinolysis.

Many clinicians have assumed that the inhibition of the RAAS by an angiotensin-converting enzyme inhibitor ("ACE inhibitor") will prevent aldosterone formation. Marked plasma aldosterone levels have been detected, however, in a majority of patients receiving chronic treatment with an ACE inhibitor. Increasing evidence suggests that ACE inhibitors only transiently suppress aldosterone levels. (Struthers AD. J Cardiac Failure 1996; 2:47-54.) Plasma aldosterone levels decrease initially with ACE inhibitor treatment, but return to pretreatment levels after three to six months of ACE inhibitor therapy, despite good compliance with continued drug administration. (Staessen J et al.: J Endocr 1981;91:457-465). This phenomenon of "aldosterone escape" occurs because there are other important determinants of aldosterone release, such as serum potassium. (Pitt B. Cardiovascular Drugs and Therapy 1995;9:145-149). Marayev V et al., Presentation at the International Meeting on Heart Failure, 1995, Amsterdam, The Netherlands, has proposed that this escape phenomenon could contribute to the high mortality rate in heart failure patients.

Greene E, et al.: J Clin Invest 1996;98:1063-8, have argued that, although much evidence has accumulated to implicate angiotensin II in mediating renal disease, aldosterone also may be associated with progressive renal disease through both hemodynamic effects and direct cellular actions. Hyperaldosteronism and adrenal hypertrophy have been observed in the remnant kidney model, with plasma levels of aldosterone increased approximately tenfold. In addition, clinical studies have reported a relationship between augmented levels of aldosterone and renal deterioration. (Hene RJ et al.: Kidney Int 1982;21:98-101). Berl T, et al.: Kidney Int 1978;14:228-35, for example, noted that plasma aldosterone levels were elevated in 5 of 8 normokalemic patients with renal failure, and in 5 of 6 patients with a creatinine clearance <15 mL/min. In a subsequent study, Hene RJ, et al.: Kidney Int 1982;21:98-101, noted that plasma aldosterone levels of 28 patients with creatinine clearances <50% of normal were increased, despite normal serum potassium levels and normal plasma renin activity. Ibrahim HN, et al.: Semin Nephrol 1997;17:431-40 have suggested that it is likely that potassium and angiotensin II (both at increased levels in patients with renal failure) act in concert to promote the aldosterone excess that accompanies renal insufficiency and progressive renal disease. Quan ZY, et al.: Kidney Int 1992;41:326-33 reported that hypertension, proteinuria, and structural renal injury were less prevalent in rats that underwent subtotal nephrectomy with adrenalectomy compared with rats that had partial nephrectomy but intact adrenal glands. This result occurred despite large doses of replacement glucocorticoid (aldosterone was not replaced) in the adrenalectomized rats.

In a deoxycorticosterone acetate ("DOCA")-salt hypertensive rat model, exogenous administration of mineralocorticoids to DOCA treated animals induced lesions of malignant nephrosclerosis and stroke. (Gavras H, et al.: Circ Res 1975;36:300-9). Horiuchi et al, reported an increased concentration of aldosterone receptors in the kidneys of a substrain of stroke-prone spontaneously hypertensive rats ("SHRSP"), in which the development of malignant nephrosclerosis occurred without salt-loading. (Horiuchi M, et al.: Am J Physiol 1993;264:286-91). Furthermore, Ullian et al.: reported that Wistar-Furth rats (which are unresponsive to the action of aldosterone) are resistant to developing nephropathy in response to subtotal nephrectomy. (Ullian ME, et aL: Am J Physiol 1997;272:1454-61).

Greene et al evaluated four treatment groups (sham-operated rats, untreated partial-nephrectomized ["remnant"] rats, remnant rats treated with losartan and enalapril, and remnant rats treated with losartan and enalapril followed by an infusion of aldosterone) to distinguish the relative importance of aldosterone in the progression of renal injury. (Greene E, et al.: J Clin Invest 1996;98:1063-8). The reported results indicated that remnant rats had a tenfold elevation in aldosterone levels in comparison with sham-operated rats. In contrast, remnant rats undergoing treatment with losartan and enalapril manifested suppressed aldosterone levels, with a decrease in proteinuria, hypertension, and glomerulosclerosis compared with the remnant rats not given these agents. In the final group, remnant rats receiving losartan and enalapril treatment followed by an infusion of aldosterone, the degree of proteinuria, hypertension, and glomerulosclerosis was similar to that of untreated remnant rats.

Control of blood pressure by treatment with antihypertensive drugs has contributed to dramatic reductions in morbidity and mortality attributed to hypertension. For example, age-adjusted death rates from stroke have declined in the United States by nearly 60 percent and from coronary heart disease by 53 percent. (Hansson L, et al.: Lancet 1988;351:1755-62). According to one report, diastolic blood pressure reduction from 105 to 83 mm Hg could prevent four major cardiovascular events per 1,000 patients treated per year, which would result in 2,764,000 prevented events if the estimated 691 million hypertensive patients received optimal antihypertensive treatment. (Hansson L, et al.: Lancet 1988;351:1755-62). Even in medically developed countries such as the U.S., however, it is estimated that only 29 percent of patients receiving antihypertensive treatment are controlled below 140/90 mm Hg. (Joint National Committee. The sixth report of the Joint National Committee on prevention, detection, evaluation, and treatment of high blood pressure. NIH Publication No. 98-4080 November 1997).

A variety of drugs selected from a number of different drug classes can be used to treat hypertension, heart failure and renal dysfunction. (Joint National Committee. The sixth report of the Joint National Committee on prevention, detection, evaluation, and treatment of high blood pressure. NIH Publication No. 98-4080 November 1997). These drugs include diuretics (such as chlorthalidone, hydrochlorothiazide, metolazone and the like), vasodilators (such as hydrolazine, minoxidil, sodium nitroprusside, dizaoxide and the like), β-adrenergic receptor antagonists (such as propranolol, metoprolol, labetalol, acebutolol and the like), calcium channel blockers (such as verapamil, diltiazem, nifedipine and the like), and angiotensin-II receptor antagonists (such as losartan and the like), as well as ACE inhibitors (such as captopril, enalapril, lisinopril, quinapril and the like). The choice of the initial drug therapy for an individual hypertensive patient typically is based on coexisting factors such as age, race, and concurrent diseases. (Kaplan NM. J Hypertension 1995;13 Suppl 2:S113-S117).

ACE inhibitors are commonly used as standard therapy and have been shown to have a beneficial effect on survival and hospitalization in patients with heart failure. In the CONSENSUS (Cooperative North Scandinavian Enalapril Survival Study) trial, mortality at one year was reduced by 31 % in patients with severe heart failure, New York Heart Association (NYHA) Functional Class IV, treated with enalapril (an ACE inhibitor) plus diuretics having no substantial aldosterone antagonistic activity compared to placebo plus diuretics having no substantial aldosterone antagonistic activity. In CONSENSUS, patients with high baseline plasma aldosterone levels had a higher mortality than patients with low baseline levels. In the group treated with enalapril, mortality was reduced only in the group with baseline aldosterone plasma levels above the median. In the group whose baseline aldosterone plasma levels were below the median, no difference from placebo in mortality was observed. (Swedberg K et al.: Circulation 1990; 82:1730-1736.) Patients who experience acute myocardial infarction often develop heart failure and subsequently die. Blockade of the RAAS by ACE inhibitors has been shown to reduce all cause mortality in such patients. Lancet 1993;342:821-828.

The anti-hypertensive drug spironolactone is less commonly used for therapy than ACE inhibitors. It is an aldosterone receptor antagonist that was developed as a treatment for hyperaldosteronism which can occur with hypertension and edematous conditions associated with congestive heart failure, and liver cirrhosis. (Swedberg K, et al Circulation 1990;82:1730-6). Pitt B., et al.:, The New England J. of Med. 1999;341(10): 709-717, recently reported that addition of spironolactone to standard therapy of ACE inhibitor plus loop diuretic having no substantial aldosterone antagonistic activity reduced morbidity and mortality among patients with severe heart failure. Chronic use of spironolactone, however, is limited in many patients because of its clinical adverse effects, particularly those that are progestational and antiandrogenic in nature resulting in gynecomastia, menstrual abnormalities, and impotence. (The RALES Investigators. Am J Cardiol 1996;78:902-12).

Conventional drug therapies to treat hypertension, heart failure and end-stage renal disease are not always effective or have reduced efficacy, however, for a significant number of patients. For example, increased urinary protein secretion is an independent predictor of cardiovascular morbidity and mortality in hypertensive patients (Kannel WB, Stampfer MJ, Castelli WP, Verter J. Am Heart J 1984;108:1347-52). Anti-hypertensive agents that are ACE inhibitors or angiotensin II receptor antagonists have been observed to consistently reduce proteinuria regardless of the presence of kidney disease or their overall anti-hypertensive activity (Maki DD, et al: Arch Intern Med 1995;155:1073-82). This reduction in proteinuria is reduced or absent in individuals with high sodium intake regardless of the overall anti-hypertensive activity of the drug administered (Heeg JE, et al.: Kidney Int 1989;36:272-80). Moreover, a reduction in dietary sodium enhances the anti-hypertensive and anti-proteinuric effect of ACE inhibitors (MacGregor GA, et al: Exp Hypertens 1983;5:1367-80). The reduced efficacy of ACE inhibitors in patients on a high sodium diet can be partially corrected by the addition of hydrochlorothiazide (Buter H, et al.: Nephrol Dial Transplant 1998;13:1682-5). Such combination therapy, however, has the disadvantage of the presence of additive or even synergistic side effects of the combined drugs.

In another example, selected individuals have low plasma-renin levels or low plasma-renin activity yet manifest hypertension. This form of hypertension can be found, for example, in Blacks, the Japanese and the elderly. Such hypertension often is referred to as "low renin hypertension" (or "sodium and volume dependent low renin hypertension" as sodium down-regulates the renin system). In these individuals, increased sodium intake is followed by an increase in blood pressure despite the fact that renin plasma concentrations are normal or low. Agents active in treating essential hypertension, such as ACE inhibitors or angiotensin II receptor antagonists, are relatively ineffective in treating low renin hypertension (Weir J: Hypertension 1997;11 :17-21). Accordingly, low renin hypertension has been described as one form of salt sensitive hypertension.

Am. J. Hypertens., Vol. 12(4), 1999, page 15A; Rocha et al. discloses that a selective aldosterone antagonist (eplerenone) or adrenalectomy prevents L-NAME/ANG-II-induced renal injury in rats.

Hypertension, Vol. 32(3), 1998, page 598; Rocha et al. discloses that eplerenone treatment prevented proteinuria and renal lesions but not severe hypertension in stroke-prone spontaneously hypertensive rats.

Improved drug therapies for patients who do not satisfactorily respond to conventional drug therapies used to treat hypertension, heart failure, end-stage renal disease and other pathogenic conditions would be desirable. Further, the increasing prevalence of such pathogenic conditions suggests that newer therapeutic interventions and strategies are needed to replace or complement current approaches.

### SUMMARY OF THE INVENTION

The present invention relates to the use of eplerenone for the manufacture of a medicament for the treatment or prophylaxis of renal dysfunction in a human subject suffering from or susceptible to renal dysfunction, wherein said renal dysfunction is diabetic nephropathy or end-stage renal disease and the subject has one or more conditions selected from a sub-normal aldosterone level, salt sensitivity and an elevated dietary sodium intake.

### BRIEF DESCRIPTION OF THE DRAWINGS

Fig. 1-A shows X-ray powder diffraction patterns of Form H eplerenone.
Fig. 1-B shows X-ray powder diffraction patterns of Form L eplerenone.
Fig. 1-C shows X-ray powder diffraction patterns of the methyl ethyl ketone solvate of eplerenone.
Fig. 2-A shows a differential scanning calorimetry (DSC) thermogram of non-milled Form L directly crystallized from methyl ethyl ketone.
Fig. 2-B shows a differential scanning calorimetry (DSC) thermogram of non-milled Form L prepared by desolvation of a solvate obtained by crystallization of a high purity eplerenone from methyl ethyl ketone.
Fig. 2-C shows a differential scanning calorimetry (DSC) thermogram of Form L prepared by crystallizing a solvate from a solution of high purity eplerenone in methyl ethyl ketone, desolvating the solvate to yield Form L, and milling the resulting Form L.
Fig. 2-D shows a differential scanning calorimetry (DSC) thermogram of non-milled Form H prepared by desolvation of a solvate obtained by digestion of low purity eplerenone from appropriate solvents.
Fig. 3-A shows the infrared spectra (diffuse reflectance, DRIFTS) of Form H eplerenone.
Fig. 3-B shows the infrared spectra (diffuse reflectance, DRIFTS) of Form L eplerenone.
Fig. 3-C shows the infrared spectra (diffuse reflectance, DRIFTS) of the methyl ethyl ketone solvate of eplerenone.
Fig. 3-D shows the infrared spectra (diffuse reflectance, DRIFTS) of eplerenone in chloroform solution.
Fig. 4 shows ¹³C NMR spectra for Form H of eplerenone.
Fig. 5 shows ¹³C NMR spectra for Form L of eplerenone.
Fig. 6-A shows the thermogravimetry analysis profile for the methyl ethyl ketone solvate.
Fig. 7 shows an X-ray powder diffraction pattern of a crystalline form of 7-methyl hydrogen 4α,5α:9α,11α-diepoxy-17-hydroxy-3-oxo-17α-pregnane-7α,21-dicarboxylate, γ-lactone isolated from methyl ethyl ketone.
Fig. 8 shows an X-ray powder diffraction pattern of the crystalline form of 7-methyl hydrogen 11α,12α-epoxy-17-hydroxy-3-oxo-17α-pregn-4-ene-7α,21-dicarboxylate, γ-lactone isolated from isopropanol.

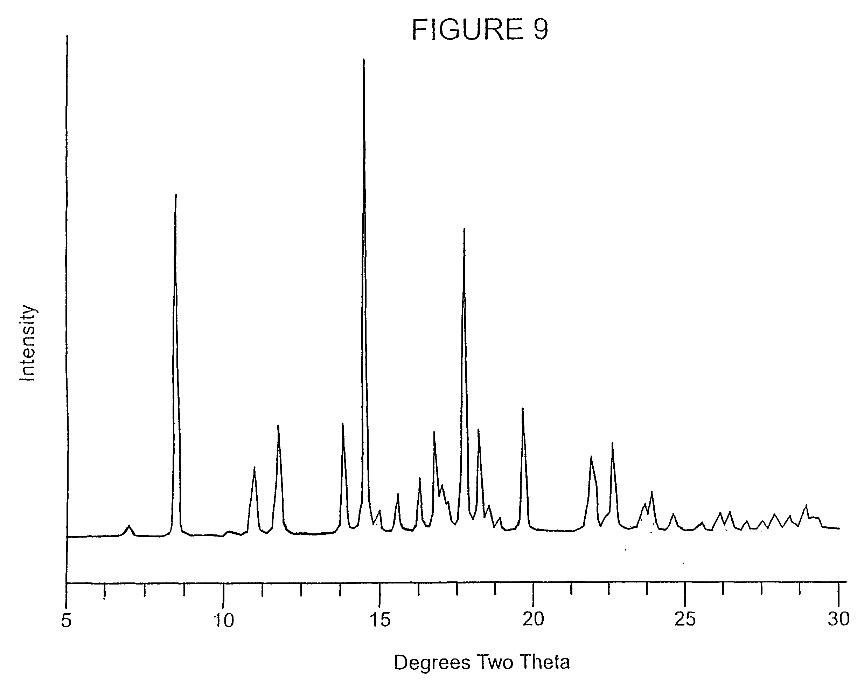

Fig. 9 shows an X-ray powder diffraction pattern of the crystalline form of 7-methyl hydrogen 17-hydroxy-3-oxo-17α-pregna-4,9(11)-diene-7α,21-dicarboxylate, γ-lactone isolated from n-butanol.
Fig. 10 shows the change in systolic blood pressure plotted as a function of the eplerenone dose for a human subject.

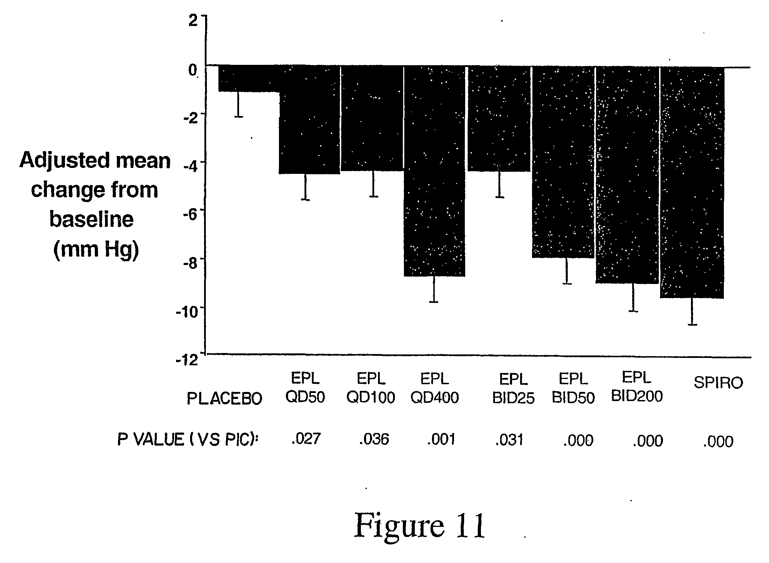

Fig. 11 shows the change in diasytolic blood pressure plotted as a function of the eplerenone dose for a human subject.
Fig. 12 shows the change in plasma renin activity and serum aldosterone plotted as a function of the eplerenone dose for one human subject.
Fig. A-1 shows systolic blood pressure before and after initiation of L-NAME treatment on days 1, 5, 9 and 13.
Fig. A-2 shows plasma renin activity (A) and plasma aldosterone levels (B) determined after sacrifice.
Fig. A-3 shows cardiac histopathology of (A) myocardial necrotic lesions induced by L-NAME/Angiotensin II/NaCI treatment; (B) of myocardium of an animal receiving L-NAME/Angiotensin II/NaCI treatment in the presence of the mineralocorticoid receptor antagonist eplerenone showing no necrotic lesions; C staining of the hearts from figure A with the collagen specific dye; and (D) staining of the hearts from figure 27 B with the collagen specific dye.
Fig. A-4 shows histopathologic scores for myocardial necrosis.
Fig. A-5 shows urinary protein excretion in samples collected on the day of sacrifice (Day 14).
Fig. A-6 show (A) renal histopathology stained with PAS of mid-coronal kidney section from an animal receiving L-NAME/Angiotensin II/NaCl treatment and (B) renal cortex of a rat receiving L-NAME/Angiotensin II/NaCl plus eplerenone.
Fig. A-7 shows histopathologic scores for renal vascular injury.
Fig. A-8 shows inflammatory lesions in coronary arteries of aldosterone/salt uninephrectomized rats.
Fig. A-9 shows inflammatory lesions in eplerenone-treated coronary arteries of aldosterone/salt uninephrectomized rats.
Fig. A-10 shows myocardial injury in aldosterone/salt uninephrectomized rats.
Fig. A-11 shows survival in saline-drinking stroke-prone SHR rats.
Fig. A-12 shows SBP in saline-drinking stroke-prone SHR rats.
Fig. A-13 shows cerebral injury in saline-drinking stroke-prone SHR rats.
Fig. A-14 shows cerebral injury in saline-drinking stroke-prone SHR rats.
Fig. A-15 shows preterminal (A) systolic arterial blood pressure (SBP) and (B) urinary protein excretion (UPE) in stroke-prone spontaneously hypertensive rats receiving chronic treatment with either eplerenone (100 mg/kg/d) or vehicle from 8.4 to 13.1 weeks of age.
Fig. A-16 shows representative photomicrographs of hematoxylin and eosin-stained mid-coronal kidney sections from saline-drinking stroke-prone spontaneously hypertensive rats after 5 weeks of eplerenone or vehicle treatment starting at 8 weeks of age (original magnification, x 130).
Fig. A-17 shows (A) systolic arterial blood pressure and (B) urinary protein excretion in saline-drinking stroke-prone spontaneously hypertensive rats during treatment with captopril plus vehicle (CAP), captopril plus Angiotensin II (CAP + Angiotensin II), or captopril plus Angiotensin II plus eplerenone (CAP + Angiotensin II + EPL).
Fig. A-18 shows plasma aldosterone levels in stroke-prone spontaneously hypertensive rats that were started on captopril treatment (50 mg/kg/d) and 1% NaCl/Stroke-Prone Rodent Diet starting at 8.3 weeks of age.
Fig. A-19 shows representative photomicrographs of hematoxylin and eosin-stained renal cortex from saline-drinking stroke-prone spontaneously hypertensive rats (A) treated with captopril plus vehicle and (B) Captopril-treated animals.
Fig. A-20 shows the population effect on PK profile (Cmax) of eplerenone.
Fig. A-21 shows the population effect on PK profile (AUClqc) of eplerenone.
Fig. A-22 shows the population effect on PK profile (AUC_{inf}) of eplerenone.
Fig. A-23 shows the population effect on PK profile (CL/F) of eplerenone.
Fig. A-24 shows the population effect on PK profile (Vol/F) of eplerenone.
Fig. A-25 shows the population effect on PK profile (Cmax) of the open ring lactone form of eplerenone.
Fig. A-26 shows the population effect on PK profile (AUClqc) of the open ring lactone form of eplerenone.
Fig. A-27 shows the population effect on PK profile (AUC_{inf}) of the open ring lactone form of eplerenone.
Fig. A-28 shows the population effect on PK profile (CL/F) of the open ring lactone form of eplerenone.
Fig. A-29 shows the population effect on PK profile (Vol/F) of the open ring lactone form of eplerenone.
Fig. A-30 shows mean eplerenone plasma concentrations following multiple dose administration.
Fig. C-1 shows the X-ray powder diffraction patterns for the wet cake (methyl ethyl ketone solvate) obtained from (a) 0%, (b) 1%, (c) 3%, and (d) 5% diepoxide-doped methyl ethyl ketone crystallizations.
Fig. C-2 shows the X-ray powder diffraction patterns for the dried solids obtained from (a) 0%, (b) 1%, (c) 3%, and (d) 5% diepoxide-doped methyl ethyl ketone crystallizations.
Fig. C-3 shows the X-ray powder diffraction patterns for the dried solids from the methyl ethyl ketone crystallization with 3% doping of diepoxide (a) without grinding of the solvate prior to drying, and (b) with grinding of the solvate prior to drying.
Fig. C-4 shows the X-ray powder diffraction patterns for the wet cake (methyl ethyl ketone solvate) obtained from (a) 0%, (b) 1%, (c) 5%, and (d) 10% 11,12-epoxide-doped methyl ethyl ketone crystallizations.
Fig. C-5 shows the X-ray powder diffraction patterns for the dried solids obtained from (a) 0%, (b) 1%, (c) 5%, and (d) 10% 11,12-epoxide-doped methyl ethyl ketone crystallizations.
Fig. C-6 shows a cube plot of product purity, starting material purity, cooling rate and endpoint temperature based on the data reported in Table 7A.
Fig. C-7 shows a half normal plot prepared using the cube plot of Fig. 15 to determine those variables having a statistically significant effect on the purity of the final material.
Fig. C-8 is an interaction graph based on the results reported in Table 7A showing the interaction between starting material purity and cooling rate on final material purity.
Fig. C-9 shows a cube plot of Form H weight fraction, starting material purity, cooling rate and endpoint temperature based on the data reported in Table 7A.
Fig. C-10 shows a half normal plot prepared using the cube plot of Fig. 18 to determine those variables having a statistically significant effect on the purity of the final material.
Fig. C-11 is an interaction graph based on the results reported in Table 7A showing the interaction between starting material purity and endpoint temperature on final material purity.
Fig. C-12 shows an X-ray diffraction pattern of amorphous eplerenone.
Fig. C-13 shows a DSC thermogram of amorphous eplerenone.

### DETAILED DESCRIPTION OF THE PREFERRED EMBODIMENTS

### General Statement of the Invention

Disorders mediated by aldosterone conventionally have been considered to be limited to primary and secondary hyperaldosteronism. In hyperaldosteronism, an excessive level of aldosterone is generally believed to be a causative agent leading to hypertension through a mechanism involving high levels of mineralocorticoid receptor occupancy by aldosterone that leads to epithelial sodium channel ("EnaC") activation and increased sodium retention. Individuals affected by hyperaldosteronism are also at increased risk of renal and heart disease. These diseases were previously believed to be a consequence of hypertension.

It has been discovered, however, that endogenous aldosterone at any level (whether elevated, normal or sub-normal) is pathogenic in a human subject. It also has been discovered that the development, rapidity of onset and development, and/or severity of the pathogenic effect meditated by endogenous aldosterone in a human subject is further enhanced when the subject has an elevated level of sodium, regardless of whether the aldosterone is present at an elevated, normal or sub-normal level. The pathogenicity of endogenous aldosterone is particularly notable in a human subject having salt sensitivity and/or an elevated dietary sodium intake.

In addition, novel methods that can reduce or eliminate such aldosterone-mediated pathogenicity in a human subject have been discovered. In general, a therapeutically effective amount of one or more aldosterone antagonists is administered to a human subject in need thereof to treat or prevent one or more pathogenic effects resulting from the action of endogenous aldosterone, including pathogenic effects resulting from the action of a sub-normal level of endogenous aldosterone. The aldosterone antagonist is an epoxy-steroidal compound namely eplerenone. The subject preferably is an individual having salt sensitivity and/or an elevated dietary sodium intake. The pathogenic effect or effects preferably result from the action of endogenous aldosterone in the presence of an elevated sodium level. The elevated sodium level preferably is an elevated level of intracellular sodium, particularly intracellular sodium in one or more of the heart, kidney and brain.

### General Definitions

The term "subject" as used herein includes a mammal, preferably a human, who has been the object of treatment, observation or experiment.

The term "treatment" includes any process, action, application, therapy, procedure or the like, wherein a mammal, particularly a human, is subjected to medical aid with the object of improving the mammal's condition, directly or indirectly.

The terms "prophylaxis" and "prevention" include either preventing the onset of a clinically evident pathogenic effect altogether or preventing the onset of a preclinically evident stage of a pathogenic effect in individuals. These terms encompass the prophylactic treatment of a subject at risk, possibly due to genetic, environmental, social or other factors, of developing a pathogenic effect such as, but not limited to, hypertension and heart failure.

The phrase "therapeutically-effective" qualifies the amount of the aldosterone antagonist that will achieve the goal of improvement in condition or disorder while avoiding adverse side effects typically associated with alternative therapies.

The term "sodium" or "salt" as used herein includes sodium in any form, particularly in the form of sodium chloride.

The terms "aldosterone antagonist" and "aldosterone receptor antagonist" include a compound that inhibits the binding of aldosterone to mineralocorticoid receptors thereby blocking the biological effects of aldosterone.

### Hypothesized Mechanism

Without being held to a particular theory, it is hypothesized that the pathogenicity is mediated, in most cases, by microvascular dysfunction. Such microvascular dysfunction is believed to be the result of microvascular constriction and micro-ischemia. Accordingly, these microvascular changes progress through maladaptive mechanisms to cause various pathogenic effects.

### Pathogenicity of a Sub-Normal Level of Endogenous Aldosterone

As noted above, endogenous aldosterone at any level, including a sub-normal level, is pathogenic in a human subject, particularly in the presence of an elevated level of sodium. This level of endogenous aldosterone can be on a localized level (for example, an intracellular level of endogenous aldosterone in a specific organ) or it can be more widespread or even systemic. Accordingly, the pathogenic effect or effects of endogenous aldosterone can be localized (for example in the brain, heart or kidney), partially but not entirely localized, or even systemic.

The endogenous aldosterone level of a subject can be determined using conventional testing methods. For example, endogenous aldosterone can be measured in a subject as circulating (plasma, serum, or whole blood) aldosterone, urinary aldosterone, and the like. Various commercial kits and/or published standard assays are available for this determination. Plasma aldosterone levels, for example, can be measured by radio-immunoassay. Commercial kits are available for this determination, such as the radio-immunoassay kit marketed by Dinabot Co. (Tokyo, Japan) for measuring plasma aldosterone. Serum aldosterone levels can be determined, for example, by conventional immunoassay methods. Urinary aldosterone levels likewise can be determined, for example, by radio-immunoassay with antiserum from Diagnostic Products Corp. Aldosterone-18-glucuronide typically is hydrolyzed overnight at pH 1 to generate free aldosterone which is then measured by RIA. (Prat JH et al.: Hypertension. 1999;34;315-319). Urinary aldosterone can be expressed as total excretion in a 24 hour sample or it may be normalized to creatinine.

Normal aldosterone values will depend upon the specific assay and population group selected, but these values typically are published in the literature and are readily available to one of ordinary skill in the art. For example, The Merck Manual at pp. 1526-2528 (17^{th} Ed. 1999), reports that normal laboratory values for serum aldosterone as measured by immunoassay typically are less than about 16 ng/dL for subjects in the supine position and between about 4 to about 31 ng/dL for subjects in the upright position (for subjects having a dietary sodium intake of about 100 to about 200 mEq/day). The Merck Manual describes such values as mean values in a healthy population ± two standard deviations. Similarly, Review of Medical Physiology, 6^{th} Edition, Lange Medical Publications, Los Altos, California; William F Ganong, lists normal aldosterone levels as typically ranging between 3 and 10 ng/dL.

Due to variations in specific assay methodologies and population groups, it may sometime be necessary or desirable for a clinician or clinical center to empirically determine its own normative data. Although one skilled in the art is readily able to do this, certain special considerations will apply. For example, genetic and environmental (e.g., dietary) differences may exist in a population in a geographical regions. Accordingly, a mean value in such a region may not be reflective of a world-wide mean. As an example of this, mean plasma aldosterone in a region consisting primarily of Blacks may be lower than a predominantly Caucasian region. Similarly, the mean of a country which consists of a large portion of salt sensitive individuals (e.g., Japan) may be lower than country consisting of a small portion of salt sensitive individuals (e.g. Sweden). Additionally, regional populations may have differences in dietary sodium consumption, which will also result in differences in aldosterone levels. Unless otherwise indicated, when reference is made to a subnormal level of a protein (e.g. aldosterone or renin) herein, it means any value below the mean value believed to represent the world population or the desired sub-population of interest.

The pathogenic effects mediated by aldosterone that can be treated or prevented in accordance with the present invention include, end-stage renal disease and diabetic nephropathy.

The aldosterone antagonist is eplerenone. The subject of the treatment or prophylaxis preferably is an individual having salt sensitivity and/or an elevated dietary sodium intake

### Effect of Sodium On Aldosterone-Mediated Pathologies

As noted above, it has been discovered that the development, rapidity of onset and development, and/or severity of the pathogenic effect meditated by endogenous aldosterone in a human subject is further enhanced in the presence of an elevated level of sodium, regardless of whether the aldosterone is present at an elevated, normal or sub-normal level. The pathogenicity of endogenous aldosterone is particularly notable in a human subject having salt sensitivity and/or an elevated dietary sodium intake. While elevated levels of dietary sodium intake have previously been reported to exacerbate hypertension, especially in salt sensitive individuals, the role of sodium in potentiating endogenous aldosterone pathogenicity was not previously appreciated.

In another embodiment of the present invention, therefore, the subject is an individual having salt sensitivity and/or an elevated dietary sodium intake. When the subject has an elevated dietary sodium intake, the average daily intake of sodium by the subject is at least about 50 milliequivalents, preferably at least about 100 milliequivalents, more preferably at least about 150 milliequivalents, and still more preferably at least about 200 milliequivalents.

Based on results of numerous hypertension studies, the medical community has generally recommended that most individuals lower their dietary sodium intake. For example, The Nutrition Committee of the American Heart Association has recommended a limitation of 3.0 g of salt per day for adults (American Heart Association Nutrition Committee, "Dietary Guidelines for Healthy American Adults." Circulation 77:721-724, 1988). Likewise, the DASH study demonstrated that lowering sodium intake to 1.5 grams decreased blood pressure over a diet of 2.4 grams. (NIH News Release, May 17, 2000 "NHLBI Study Shows Large Blood Pressure Benefit From Reduced Dietary Sodium).

Despite general recognition that a reduction in sodium intake is beneficial, many individuals and populations generally will not or cannot reduce sodium intake. Lack of self-discipline and factors that prevent changes in the diet contribute to such non-compliance with recommended guidelines. Factors preventing a change in diet may include, for example, the following: (1) lack of availability of low sodium chloride foods where geographical or other restrictions prevent or hinder access to low sodium chloride foods; (2) lack of refrigeration requiring use of high sodium chloride levels to preserve food (e.g., pickling); (3) cultural environment, such as the need to honor traditional and religious requirements in food preparation and content, leading to the consumption of foods containing high levels of sodium chloride; and (4) cost restrictions due to poverty, preventing purchase of more expensive foods with lower sodium chloride content (e.g. imported foods). Such individuals, particularly those who are salt sensitive, find themselves with no alternative to the consumption of high sodium chloride foods, except to undergo life-threatening malnutrition or starvation, must succumb to this pathological condition by eating a diet high in sodium chloride. The medicament of the present invention, however, can be used for the treatment or prophylaxis of aldosterone-mediated pathologic effects in such individuals where a reduction in dietary sodium intake cannot be attained.

The effect of sodium is particularly pronounced in salt sensitive individuals. Such individuals are likely to have an impaired pressure-natriuresis response or an increased vascular response to sodium and be at higher risk of becoming hypertensive. A number of studies have indicated that salt sensitive patients have an exacerbated response to increased sodium intake with respect to reduction in renal blood flow, increased glomerular filtration fraction, and proteinuria. (Bakris, GL, et al.: Am J Hypertens 1996;9:200S-6S). Salt sensitivity is also a risk factor for cardiovascular disease. (Campese VM: Hypertension 1992;19;403-18). The medicament of the present invention likewise can be used for the treatment or prophylaxis of aldosterone-mediated pathologic effects in such individuals.

Elevated sodium intake also can adversely affect the response of a subject to pharmaco-therapy. For example, The Treatment of Mild Hypertension Study (TOMHS) examined five classes of drugs used to treat hypertensive Black patients (Neaton JD, et al.: JAMA 1993;270:713-24). The study showed that the patients responded better to chlorthiazide, calcium channel blockers, or beta-adrenergic receptor antagonists with intrinsic sympathomimetic activity than to ACE inhibitors or alpha-adrenergic receptor antagonists (Grim CE, et al.: J Chronic Dis 1980;33:87-94). Black and white patients showed a potentiation of pharmaco-therapies with a reduction of sodium intake. A reduction of sodium intake has other effects on pharmaco-therapy. For example, such reduction results in an improved withdrawal from anti-hypertensive therapy, especially in salt sensitive patients (van Brummelen P, Schalekamp M, de Graeff J. Acta Med Scand 1978;204:151-7). Even a modest reduction of sodium can result in a reduction in effective dose (WeinbergerMH, et al.: JAMA 1988;6:2561-5). The medicament of the present invention, however, can be used either monotherapeutically or in combination therapies, such as in conventional pharmaco-therapies that are not effective for the subject treated due to, for example, salt sensitivity and/or elevated dietary sodium intake.

Salt sensitive hypertension can be easily diagnosed, for example, by measuring the change in blood pressure in a patient under conditions of low sodium chloride intake (such as about 1 to 3 g/day for several days) followed by a high sodium chloride intake (such as about 12-15 g/day). An increase in blood pressure of about 10% or more with increased sodium chloride intake is indicative of salt sensitive hypertension. Alternatively, the change in blood pressure of the subject can be monitored over a period of 24 hours using an ambulatory blood pressure measuring device. Patients with salt sensitive hypertension do not display the circadian decrease (or "dip") in blood pressure that normally occurs during the sleeping period. Instead, the blood pressure of the salt sensitive hypertensive patient remains elevated during the whole 24 hour period. This additional period of time, under which the salt sensitive patient remains maximally hypertensive, adds to the pathological damage to blood vessels and organs, caused by this condition.

Although such a condition might be ameliorated by a reduction in sodium chloride intake, a reduction in sodium intake may not be practical for a variety of reasons as discussed above. In the absence of a dietary remedy, therapeutic intervention has been attempted. Based on the biochemical profile of salt sensitive hypertension, with increased sympathetic nerve activity and catecholamine levels, the use of beta adrenergic blocking drugs is often recommended. This therapy, however, has had little success and can cause severe side effects, providing little or no relief for many patients. Since a diet high in sodium chloride also depresses the renin-angiotensin-aldosterone system, aldosterone levels are usually found to be normal or decreased in salt sensitive hypertensive patients and patients having a high salt intake. As a result, the use of an aldosterone antagonist to treat salt sensitive hypertension has been ignored or discouraged by medical practitioners.

The invention comprises a medicament for the treatment or prophylaxis of renal dysfunction in a human subject in need thereof. A therapeutically effective amount of the aldosterone antagonist eplerenone is administered to a subject having salt sensitivity and/or an elevated dietary sodium intake wherein said subject is suffering from or susceptible to renal dysfunction, wherein said renal dysfunction is diabetic nephropathy or end-stage renal disease.

### Salt Sensitivity

While experimental and clinical testing has shown a relationship between sodium intake and blood pressure, the general population exhibits material heterogeneity with respect to this relationship. Two general population groups exhibiting different responses have been recognized. The first general group is salt insensitive individuals (also referred to as "salt resistant individuals"). In salt insensitive individuals an increase in dietary sodium intake results in increased sodium excretion with no measurable increase, or only a minimal increase in blood pressure. Similarly, reduced sodium intake by a salt insensitive individual does not materially lower blood pressure or the incidence of hypertension. In fact, such sodium intake restrictions may be deleterious to the individual.

The second general group is salt sensitive individuals. In salt sensitive individuals blood pressure generally rises and falls with increased and decreased sodium consumption, respectively. Salt sensitive normotensive individuals may even be more likely to become hypertensive over time when consuming a typical North American diet. (Sullivan JM, 1991; Hypertension 17 (Suppl. I):I61-68). When sodium intake is increased, renal blood flow may either fail to increase or may decrease, such that both vascular resistance and filtration fraction increase, with glomerular filtration rate persisting unchanged or increasing. (Weir MR et al.: Hypertension 1990; 16:235-44).

### Identification of Salt Sensitive Individuals.

Suitable tests for determining salt sensitivity, such as but not limited to the salt challenge test, are generally known to those of ordinary skill in the art. See, for example, Brenner, K. "A method for distinguishing salt-sensitive from non-salt-sensitive forms of human and experimental hypertension", Curr. Opin. Nephrol. Hypertens. 1993 May;2(3):341-349. Such identification can be accomplished through, for example, clinical testing (such as a salt challenge test) or by examination of family history and/or ethnic origin. Clinical testing can be done using uni-directional or bi-directional analyses.

### Salt Challenge Test

One illustrative non-limiting manner of determining salt sensitivity is through a salt challenge test. Uni-directional salt challenge testing can be accomplished by placing an individual on a low sodium diet (e.g. 40 mmol/day for one week; "low salt regimen"). Because individuals with free access to sodium often demonstrate poor compliance, this phase of testing is best performed in an in-patient environment. An in-patient environment is one wherein the individual being tested does not have free access to sodium sources and the diet is determined by a dietician or one skilled in the art to determine the sodium content. Additionally, all dietary sources of sodium ingested are carefully measured. After completion of the low salt regimen, systolic and diastolic blood pressure are determined in resting condition in triplicate. Immediately following completion of the low salt regimen, the individuals are placed again on a controlled dietary regimen wherein total sodium ingested is 220 mmol/day ("high salt regimen"). At the end of one week of the high salt regimen, systolic and diastolic blood pressure are determined as during the low salt regimen. Subjects who demonstrate an increase in either systolic blood pressure or diastolic blood pressure or both, of more than 5 mm Hg are deemed salt sensitive.

Bi-directional salt challenge testing can be accomplished by placing an individual on the low salt regimen and high salt regimen as described above for the uni-directional testing, and then immediately placing the individual on a controlled, low sodium diet similar to the low salt regimen and then determining systolic and diastolic blood pressure as described for the uni-directional testing. Individuals who demonstrate both an increase of 5 mm Hg or more in systolic or diastolic blood pressure or both at the end of the high salt regimen and a decrease of 5 mm Hg or more in systolic or diastolic blood pressure or both at the end of the subsequent controlled, low salt diet are deemed to be salt sensitive.

Another method of identifying salt sensitive individuals is to perform a reverse uni-directional analysis wherein the individual is first placed on a high sodium regimen followed by the low sodium regimen. Individuals who demonstrate a 10% decrease in systolic or diastolic blood pressure or both at the end of the low sodium regimen are deemed to be salt sensitive.

The uni-directional, bi-directional, and reverse uni-directional salt challenge tests described above can be further modified where desirable. For example, instead of an in-patient setting, subjects can be permitted to perform normal activities (i.e. eat, sleep, work, etc.) in their otherwise normal environment. Compliance with the dietary regimen is determined by analysis of 24 hour urine collections. Alternatively, compliance can be monitored by subjective questionnaires and record keeping by the test individuals. Other modifications of the above testing methods include reducing the one week intervals for each regimen of the test to either 3, 4, 5, or 6 days. Still other modifications of the above testing include adjusting the high salt regimen intake of sodium, for example to eight to sixteen grams of sodium per day. Additionally, a lower threshold for change in systolic or diastolic blood pressure (such as 5, 6, 7, 8, or 9%) can be used and, if desired, individuals can be scored as positive for salt sensitivity if meeting such a lowered threshold on two independent uni-directional test determinations.

### Rapid Volume Expansion And Contraction Test

Another illustrative non-limiting manner of determining salt sensitivity is through a rapid volume expansion and contraction test such as, for example, the protocol described in Grim et al;. Hypertension 1979; 1:476-85; as modified by Strazzullo et al.: J Nephrol 2000; 13:46-53. In general, subjects are hospitalized for four days and receive 50 mmol/day of sodium. On Day 1, subjects also receive additional sodium in the form of Slow-Na tablets to achieve a total intake of 150 mmol sodium. On Day 2, sodium intake is again fixed at 150 mmol and the subject is given a constant rate intravenous infusion of 2 L of 0.9% sodium chloride over four hours. Blood pressure determinations (mean of five measurements at three minute intervals) are taken at the start and the end of the sodium chloride infusion using an automatic blood pressure recorder. Urinary sodium excretion is measured during the three hours before and during the saline infusion. Body weight, hematocrit, plasma renin activity, and aldosterone are measured before and after the infusion. On Day 3, sodium and volume depletion are effected by sodium restriction (50 mmol/day) in diet plus three doses of 37.5 mg of furosemide at 10:00 a.m., 02:00 p.m. and 06:00 p.m. The subjects are asked to drink no more than 25 mL of tap water per kg body weight. On Day 4 at 8:00 a.m., blood pressure is measured. The response to the test is calculated by the mean blood pressure measured at the end of the infusion minus the blood pressure after sodium chloride depletion. Subjects are deemed salt sensitive if the response is greater than 5 mm Hg.

### Family History or Ethnic Origin.

Family history and/or ethnic origin also can be helpful in determining subjects at risk of salt sensitivity. Such risk factors include, but are not limited to, a family history consisting of one or more relatives with hypertension or a genealogy traceable to an ethnic group showing a prevalence of salt-sensitivity greater than the general world-wide population. Such genealogy, by way of example only, includes Blacks, American Blacks, American Indians, and Japanese. (Powers DR et al.: Arch Intern Med 1998 158:793-800)

### Molecular Identification.

Molecular identification of the salt sensitivity phenotype through suitable genetic testing methods can also be used to determine salt sensitivity in an individual. For example, Rutledge et al. have described an association between this phenotype and the Hpa11 mutation in the atrial natriuretic peptide in African Americans. (J Hypertens. 1995; 13:953-5). Svetkey et al. have reported concordance between diastolic blood pressure response to sodium loading/volume depletion to the beta 2-adrenergic receptor locus in African Americans. (Hypertension. 1997; 29:918-22). A study of sibling pairs in Italy reported concordance between an alpha-adducin mutation (1460 Trp) and salt sensitivity. (Cusi D et al;. Lancet. 1997; 349:1353-7). An AluI polymorphism in exon 3 of the 11 beta- hydroxysteroid dehydrogenase type 2 (11 betaHSD2) gene was reported to be associated with salt sensitivity in a Swiss population. (Lovati E et al.: J Clin Endocrinol Metab. 1999; 84:3745-9). Furthermore, a CA-repeat allele microsattelite polymorphism in this same gene in unselected patients with essential hypertension was reported to correlate with salt sensitivity. (Ferrari P et al.: Kidney Int. 2000; 57:1374-81). Additionally, the activity of the 11 betaHSD2, as shown by elevated mean ratios of urinary cortisol to cortisone metabolites, was decreased in salt-sensitive compared with salt- resistant subjects. A study of hypertensive individuals in Spain reported an association between the presence of one or two copies of a 287 base pair insertion in the ACE gene with salt sensitivity. (Giner et al.: Hypertension. 2000; 35:512-517.)

### Plasma Immunoreactive Endothelin Levels

Endothelial cells function importantly in the regulation of vascular tone and homeostasis. This function, in part, is mediated through the secretion of vasoactive substances. Endothelins (which are produced by endothelial cells) are potent vasoconstrictors and are peptides coded by three different genes. (Yanagiawa M et al.: Nature 1988 332:411-5). Endothelin-1 ("ET-1") is the predominant member produced by the endothelium and acts in an autocrine and paracrine manner. ET-1 receptors (i.e., Et_{A} and ET_{B}) on smooth muscle cells mediate the contractile, proliferative, and hypertrophy-inducing effects of ET-1 (De Nucci G et al.: Proc Natl Acad Sci USA 1988, 85:9797-800).

In salt resistant hypertensive individuals, endothelin plasma levels are usually normal. (Schiffrin EL et al.: Am J Hypertens 1991 4:303-8). In severely hypertensive individuals and in Blacks, however, plasma immunoreactive ET-1 is often elevated. Salt sensitive individuals respond to an increased salt load by exhibiting elevated plasma ET-1 levels. (Elijovich F et al.: Hypertension 1999; 33:1075). Accordingly, another method of identifying salt sensitive individuals is through testing of plasma immunoreactive endothelin levels. Elevated plasma immunoreactive endothelin levels, particularly elevated plasma immunoreactive endothelin ET-1 levels, often are present in salt sensitive individuals.

### Subjects in Need of Treatment

The pathogenicity of endogenous aldosterone at a sub-normal level in human subjects was not previously appreciated. Similarly, the increased development, rapidity of onset and development, and/or severity of the pathogenic effect meditated by endogenous aldosterone in a human caused by the presence of elevated sodium levels previously was not appreciated. It was conventionally believed that sodium loading typically resulted in a decrease of endogenous aldosterone levels to non-pathogenic levels. Accordingly, subjects who can benefit from treatment or prophylaxis in accordance with the present invention, are generally human subjects who have (i) a sub-normal endogenous aldosterone level, (ii) salt sensitivity regardless of the endogenous aldosterone level, and/or (iii) elevated dietary sodium intake regardless of the endogenous aldosterone level. Within each of these groups of subjects, it can be beneficial to carry out further profiling and/or phenotyping to identify sub-groups of subjects who will benefit from the therapy of the present invention.

Accordingly, subjects who can benefit from treatment or prophylaxis in accordance with the present invention are human subjects generally exhibiting one or more of the following characteristics:
(a) the average daily intake of sodium chloride by the subject is at least about 4 grams, particularly where this condition is satisfied over any one month interval for at least one or more monthly intervals over a given annual period. The average daily intake of sodium by the subj ect preferably is at least about 6 grams, more preferably at least about 8 grams, and still more preferably at least about 12 grams.
(b) the subject exhibits an increase in systolic blood pressure and/or diastolic blood pressure of at least about 5%, preferably at least about 7%, and more preferably at least about 10%, when daily sodium chloride intake by the subject is increased from less than about 3 g/day to at least about 10 g/day.
(c) the activities ratio of plasma aldosterone (ng/dL) to plasma renin (ng/ml/hr) in the subject is greater than about 30, preferably greater than about 40, more preferably greater than about 50; and still more preferably greater than about 60.
(d) the subject has low plasma renin levels; for example, the morning plasma renin activity in the subject is less than about 1.0 ng/dL/hr, and/or the active renin value in the subject is less than about 15 pg/mL.
(e) the subject suffers from or is susceptible to elevated systolic and/or diastolic blood pressure. In general, the systolic blood pressure (measured, for example, by seated cuff mercury sphygmomanometer) of the subject is at least about 130 mm Hg, preferably at least about 140 mm Hg, and more preferably at least about about 150 mm Hg, and the diastolic blood pressure (measured, for example, by seated cuff mercury sphygmomanometer) of the subject is at least about 85 mm Hg, preferably at least about 90 mm Hg, and more preferably at least about 100 mm Hg.
(f) the urinary sodium to potassium ratio (mmol/mmol) of the subject is less than about 6, preferably less than about 5.5, more preferably less than about 5, and still more preferably less than about 4.5.
(g) The urinary sodium level of the subject is at least 60 mmol per day, particularly where this condition is satisfied over any one month interval for at least one or more monthly intervals over a given annual period. The urinary sodium level by the subject preferably is at least about 100 mmol per day, more preferably at least about 150 mmol per day, and still more preferably 200 mmol per day.
(h) the plasma concentration of one or more endothelins, particularly plasma immunoreactive ET-1, in the subject is elevated. Plasma concentration of ET-1 preferably is greater than about 2.0 pmol/L, more preferably greater than about 4.0 pmol/L, and still more preferably greater than about 8.0 pmol/L.
(i) the subject has blood pressure that is substantially refractory to treatment with an ACE inhibitor; particularly a subject whose blood pressure is lowered less than about 8 mm Hg, preferably less than 5 mm Hg, and more preferably less than 3 mm Hg, in response to 10 mg/day enalapril compared to the blood pressure of the subject on no antihypertensive therapy.
(j) the subject has blood volume-expanded hypertension or blood volume-expanded borderline hypertension, that is, hypertension wherein increased blood volume as a result of increased sodium retension contributes to blood pressure.
(k) the subject is a non-modulating individual, that is, the individual demonstrates a blunted positive response in renal blood flow rate and/or in adrenal production of aldosterone to an elevation in sodium intake or to angiotensin II administration, particularly when the response is less than the response of individuals sampled from the general geographical population (for example, individuals sampled from the subject's country of origin or from a country of which the subject is a resident), preferably when the response is less than 40% of the mean of the population, more preferably less than 30%, and more preferably still less than 20%.
(l) the subject has or is susceptible to renal dysfunction, particularly renal dysfunction selected from one or more members of the group consisting of reduced glomerular filtration rate, microalbuminuria, and proteinuria.
(m) the subject has or is susceptible to cardiovascular disease, particularly cardiovascular disease selected from one or more members of the group consisting of heart failure, left ventricular diastolic dysfunction, hypertrophic cardiomyopathy, and diastolic heart failure.
(n) the subject has or is susceptible to liver disease, particularly liver cirrhosis.
(o) the subject has or is susceptible to edema, particularly edema selected from one or more members of the group consisting of peripheral tissue edema, hepatic or splenic congestion, liver ascites, and respiratory or lung congestion.
(p) the subject has or is susceptible to insulin resistance, particularly Type I or Type II diabetes mellitus, and/or glucose resistance.
(q) the subject is at least 55 years of age, preferably at least about 60 years of age, and more preferably at least about 65 years of age.
(r) the subject is, in whole or in part, a member of at least one ethnic group selected from the Japanese ethnic group, the American Indian ethnic group, and the Black ethnic group.
(s) the subject has one or more genetic markers associated with salt sensitivity.
(t) the subject is obese, preferably with greater than 25% body fat, more preferably with greater than 30% body fat, and even more preferably with greater than 35% body fat.
(u) the subject has one or more 1^{st}, 2^{nd}, or 3^{rd} degree relatives who are or were salt sensitive, wherein 1^{st} degree relatives means parents or relatives sharing one or more of the same parents, 2^{nd} degree relatives means grandparents and relatives sharing one or more of the same grandparents, and 3^{rd} degree relatives means great-grandparents and relatives sharing one or more of the same great-grandparents. Preferably, such individuals have four or more salt sensitive 1^{st}, 2^{nd}, or 3^{rd} degree relatives; more preferably, eight or more such relatives; even more preferably, 16 or more such relatives; and even more preferably still, 32 or more such relatives.

Unless otherwise indicated to the contrary, the values listed above preferably represent an average value, more preferably a daily average value based on at least two measurements.

Preferably, the subject in need of treatment satisfies at least two or more of the above-characteristics, more preferably, at least three or more of the above-characteristics, and still more preferably, at least four or more of the above-characteristics.

Accordingly, in one embodiment of the present invention the subject in need of treatment is salt sensitive and satisfies two or more of the following conditions: (i) the average daily intake of sodium chloride by the subject is at least about 4 grams, particularly where this condition is satisfied over any one month interval for at least one or more monthly intervals over a given annual period; and/or (ii) the activities ratio of plasma aldosterone (ng/dL) to plasma renin (ng/ml/hr) in the subject is greater than about 30; (iii) the morning plasma renin activity in the subject is less than about 1.0 ng/dL/hr, and/or the active renin value in the subject is less than about 15 pg/mL; and/or (iv) the systolic blood pressure of the subject is at least about 130 mm Hg and the diastolic blood pressure of the subject is at least about 85 mm Hg; and/or (v) the subject has or is susceptible to cardiovascular disease, particularly cardiovascular disease selected from one or more members of the group consisting of heart failure, left ventricular diastolic dysfunction, hypertrophic cardiomyopathy, and diastolic heart failure.

In another embodiment of the present invention, the subject in need of treatment is salt sensitive and satisfies the following conditions: (i) the activities ratio of plasma aldosterone (ng/dL) to plasma renin (ng/ml/hr) in the subject is greater than about 30; and (ii) the morning plasma renin activity in the subject is less than about 1.0 ng/dL/hr, and/or the active renin value in the subject is less than about 15 pg/mL.

In another embodiment of the present invention, the subject in need of treatment is salt sensitive and satisfies at least two of the following conditions: (i) the average daily intake of sodium chloride by the subject is at least about 4 grams, particularly where this condition is satisfied over any one month interval for at least one or more monthly intervals over a given annual period; and/or (ii) the systolic blood pressure of the subject is at least about 130 mm Hg and the diastolic blood pressure of the subject is at least about 85 mm Hg; and/or (iii) the subject has or is susceptible to cardiovascular disease, particularly cardiovascular disease selected from one or more members of the group consisting of heart failure, left ventricular diastolic dysfunction, hypertrophic cardiomyopathy, ischaemic heart disease, and diastolic heart failure.

### Illustrative Phenotypes

In each of the above embodiments, the subject preferably is a member, in whole or in part, of the Japanese ethnic group or the Black ethnic group. Hypertension in Japan is a significant problem. One recent estimate suggests that around 30 million Japanese adults suffer from hypertension. (Saruta T. J Clin Ther Med 1997;13:4024-9). While blood pressure control status has recently improved in Japan, hypertension management is still considered to be insufficient. (Shimamoto; K. Japanese Cases. Nihon Rinsyo (Clinical Medicine in Japan), 2000;58 (Suppl):593-6). Trends in blood pressure and urinary sodium and potassium excretion in Japan: reinvestigation in the 8th year after the Intersalt Study. Nakagawa H, et al.: Hum Hypertens 1999 Nov;13(11):735-41, recommended that the Japanese population increase dietary potassium and decrease dietary sodium.

Sodium restriction regimens in Japan, however, are confounded by poor compliance. A Japanese study by Kobayashi et. al. prescribed a diet restricted to 5-8 grams/day yet failed also to achieve good compliance. (Kobayashi, Y et al.: Jpn Circ J 1983;47:268-75). The Ministry of Health and Welfare of Japan has recommended that sodium restricted to less than 10 grams/day (Guidelines on treatment of hypertension in the elderly, 1995--a tentative plan for comprehensive research projects on aging and health--Members of the Research Group for "Guidelines on Treatment of Hypertension in the Elderly", Comprehensive Research Projects on Aging and Health, the Ministry of Health and Welfare of Japan). Ogihara T, et al.: Nippon Ronen Igakkai Zasshi. 1996;33(12):945-75). Despite 10 years of initiatives to educate the public, there still remains a high rate of non-compliance (estimated to be greater than about 50%) as measured by urinary sodium levels among normal and hypertensive individuals in Japan. (Kobayashi Y, et al.: Jpn Circ J;47(2):268-75).

Further, the Japanese show two broad groups, salt sensitive and salt insensitive (Preventive nutritional factors in epidemiology: interaction between sodium and calcium. Mizushima S, Clin Exp Pharmacol Physiol 1999;26:573). Many Japanese hypertensives are believed to be salt sensitive. Accordingly, members of the Japanese ethnic group who exhibit the combination of salt sensitivity, high sodium intake and failure to voluntarily limit sodium consumption are particularly benefited by the therapy of the present invention.

In another embodiment of the present invention, therefore, the subject in need of treatment is salt sensitive individual who is, in whole or in part, a member of the Japanese ethnic group, and, *inter alia,* has or is susceptible to hypertension and/or cardiovascular disease, particularly cardiovascular disease selected from one or more members of the group consisting of heart failure, left ventricular diastolic dysfunction, hypertrophic cardiomyopathy, and diastolic heart failure.

In another embodiment of the present invention, the subject in need of treatment is salt sensitive individual who is, in whole or in part, a member of the Japanese ethnic group, and, *inter alia,* the average daily intake of sodium chloride by the subject is at least about 4 grams, particularly where this condition is satisfied over any one month interval for at least one or more monthly intervals over a given annual period.

Hypertension in Blacks similarly is a significant problem. Many hypertensive and normotensive Blacks are salt sensitive (Svetkey, LP et al.: Hypertension. 1996;28:854-8). Accumulated epidemiologic data indicate that the prevalence of hypertension among Blacks is greater than among whites in almost all age- and sex-matched groups. Hypertensive Blacks generally have a higher incidence of left ventricular dysfunction, stroke, and renal damage (but a lower incidence of ischemic heart disease) than do hypertensive whites. (Eisner, GM. Am J Kidney Dis 1990; 16(4 Suppl 1):35-40) The reasons for the epidemic hypertension rates among American Blacks are largely environmental: high sodium and alcohol intake, obesity, physical inactivity, and psychosocial stress have all been identified as causes. (Flack, JM, et al.: J Assoc Acad Minor Phys 1991;2:143-50).

The cause of the problem in both Black and white populations is unclear, but it appears that a difference in sodium handling may contribute to the particular hemodynamic and hormonal profile of Black hypertensives. Intrinsic or hypertension-induced renal abnormalities that limit natriuretic capacity, reduced Na+,K(+)-ATPase pump activity, other membrane ion transport disturbances, differential exposure to psychological stressors, greater insulin resistance, and dietary factors (reduced calcium and potassium intake) have been suggested as possibly playing a role. (Flack, JM et al.: Hypertension; 1991;17(1 Suppl):I115-21). One study has indicated that genetic differences may also underlie the salt sensitivity in Blacks. (Svetkey, LP, et al.: Hypertension 1996; 28:854-8).

Hypertension among Blacks generally is initially managed by restricting sodium intake in the diet. If dietary control is insufficient, administration of an antihypertensive agent with 24-hour efficacy and that lowers vascular peripheral resistance, promotes sodium excretion, and potentially improves renal hemodynamics is recommended. (Eisner, GM. Am J Kidney Dis 1990;16(4 Suppl 1):35-40). Blacks, however, generally respond differently to antihypertensive agents as compared to white. In general, beta-adrenergic receptor antagonists or ACE inhibitors monotherapies are less effective in Blacks than in whites. Black males tend to be even less responsive to ACE inhibitors than Black females (Eisner, GM. Am J Kidney Dis 1990;16(4 Suppl 1):35-40). Accordingly, members of the Black ethnic group who exhibit the combination of salt sensitivity, high sodium intake and failure to voluntarily limit sodium consumption are particularly benefited by the therapy of the present invention.

In another embodiment of the present invention, therefore, the subject in need of treatment is salt sensitive individual who is, in whole or in part, a member of the Black ethnic group, and, *inter alia,* has or is susceptible to hypertension and/or cardiovascular disease, particularly cardiovascular disease selected from one or more members of the group consisting of heart failure, left ventricular diastolic dysfunction, hypertrophic cardiomyopathy, and diastolic heart failure.

In another embodiment of the present invention, the subject in need of treatment is salt sensitive individual who is, in whole or in part, a member of the Black ethnic group, and, *inter alia,* the average daily intake of sodium chloride by the subject is at least about 4 grams, particularly where this condition is satisfied over any one month interval for at least one or more monthly intervals over a given annual period.

In another embodiment of the present invention, the subject in need of treatment is salt sensitive individual who is, in whole or in part, a member of the Black ethnic group, and, *inter alia,* exhibits a diminished incremental decrease in blood pressure in response to standard anti-hypertensive therapy, particularly where such therapy comprises the administration of an ACE inhibitor.

### Non-Modulating Individuals

As noted above, a non-modulating individual demonstrates a blunted positive response in renal blood flow rate and adrenal production of aldosterone to a high sodium intake or angiotensin II administration. Such non-modulating individuals additionally may exhibit increased fasting insulin levels and increased increment in glucose-stimulated insulin levels. (Ferri et al.: Diabetes 1999; 48:1623-30). Insulin resistance is also associated with increased risk of myocardial infarction.

Accordingly, in another embodiment of the present invention the subject in need of treatment is a salt sensitive and non-modulating individual that, *inter alia,* (i) has or is susceptible to insulin resistance, particularly Type I or Type II diabetes mellitus, and/or glucose resistance, and/or (ii) has or is susceptible to cardiovascular disease.

### Aged Individuals

In salt sensitive individuals the incremental blood pressure response to a given increase in dietary intake of sodium increases with age. Similarly, salt sensitivity is more frequently observed in individuals of advanced age. Furthermore, insulin resistance shows a similar increase with age.

Accordingly, in one embodiment of the present invention the subject in need of treatment is.a salt sensitive individual at least 55 years of age, preferably at least about 60 years of age, and more preferably at least about 65 years of age, and, *inter alia*, has or is susceptible to insulin resistance, particularly Type I or Type II diabetes mellitus, and/or glucose resistance.

### Detoxified and Recovering Alcoholics

Detoxified and recovering alcoholics also commonly are salt sensitive (Genaro C et al.: *Hypertension* 2000: 869-874). Accordingly, in another embodiment of the present invention the subject in need of treatment is a salt sensitive individual and, *inter alia,* is a detoxified or recovering alcoholic.

### Obesity

Obese individuals are commonly salt sensitive. A study by Bonner (MMW Fortschr Med 1999; 14:34-6) estimated that 44% of all hypertensive patients are overweight and further associated with salt sensitivity, elevated intracellular calcium, sodium retention, and increased cardiac output. Furthermore, Dimsdale et al. (Am J Hypertens 1990; 3:429-35) reported that obese patients were more likely to increase their systolic pressure in response to salt loading. Additionally, salt sensitive children also have an increased probability of obesity and cardiovascular disease. (Falkner B et al.: Am J Clin Nutr 1997; 65:618S-621 S). Even in normotensive individuals, sodium-sensitive subjects tend to weigh more than sodium-resistant subjects. (Rocchini AP et al.: Am J Med Sci 1994; 307 Suppl 1:S75-80). Accordingly, in another embodiment of the present invention the subject in need of treatment is a salt sensitive individual and, *inter alia,* is obese.

### Treatment of Salt Sensitivity

Salt sensitivity is an independent risk factor for death from cardiovascular disease including stroke, regardless of whether the salt-sensitive person is hypertensive, smokes or has high cholesterol. (Myron H. Weinberger, 54^{th} Annual Conference of the American Heart Association's Council for High Blood Pressure Research, Washington, D.C., 2000).

Accordingly, in one embodiment of the present invention, the subject in need of treatment is a salt sensitive individual who has, more or less, a normal clinical presentation. Normal clinical presentation, as used herein, means an individual who is not markedly hypertensive, and does not have any marked evidence of ongoing disease of the kidney, heart, cardiovasculature, cerebrovasculature, or any indicies of insulinopathies. The use of eplerenone according to the present invention in such an individual will prevent development of aldosterone/salt mediated pathologies.

Typically, an age-related progression of salt sensitivity occurs in most humans. It is believed that this progression in condition is due, in part, to several aldosterone-mediated phenomena. First, in non-modulators on a high salt diet, there is an abnormal angiotensin-mediated control of aldosterone release and renal blood supply leading to hypertension. Second, as the subject grows older vascular compliance is compromised through aldosterone-mediated vessel stiffening, resulting in the loss of the ability of vessels to absorb part of the blood pressure. Third, age-related increases in insulin production exacerbate the aldosterone-mediated increase in ENaC activity.

The administration of the aldosterone antagonist in accordance with the present invention will reduce and/or reverse the progression of the salt sensitivity. The administration of that antagonist will reduce and/or reverse the genetic and dietary impact of salt sensitivity in such individuals on the progression of renal pathologies.

### Sodium Appetite Suppression

Whereas a large number of pathologic consequences of a large number of medical conditions can be reduced by restricting dietary sodium intake, compliance with such restriction is often poor. When such compliance is attained, even only in part, individuals attempting such restriction often experience salt cravings and reduced quality of life. The present invention thus benefits such individuals as an adjunct to other treatments of such medical conditions and further provides improved quality of life benefits.

### Treatment and/or Prophylaxis of Central Aldosterone-Mediated Pathologies

It is hypothesized that salt sensitive individuals experience elevated blood pressure as a result, in part, of the central action of aldosterone. Activation of the RAAS causes vasopressor or sympathetic nerve responses in the intracerebroventricular region of the brain ("ICV"). Aldosterone is hypertensigenic in the brain, particularly in individuals with an elevated level of intracellular sodium in the brain. Such an elevated level of aldosterone is especially likely to occur in salt sensitive individuals. The hypertensigenic action of aldosterone in the brain ("central aldosterone") of an individual is believed to be mediated, in part, to increased neurogenic vasomotor tone and impaired arterial baroflexes. Additionally, it is believed that central aldosterone is pivotal in stress/anxiety induced activation of the hypothalamic-pituitary-adrenal ("HPA") axis. Whereas standard anti-hypertensive therapy in such individuals is confounded by an increase in dietary sodium intake, the present invention provides a treatment that is effective in spite of increased sodium intake and that will result in improved management of hypertension.

### Treatment and/or Prophylaxis of Renal-Aldosterone-Mediated Pathologies

The present invention also relates to the use of the aldosterone antagonists eplerenone to treat hypertensive subjects who have an elevated level of intracellular sodium thereby preventing aberrant renal sodium retention. Accordingly, the present invention encompasses the use said aldosterone antagonist to functionally inactivate aldosterone in the kidney of a human subject, especially a subject having salt sensitivity and/or an elevated dietary sodium intake. "Functionally inactivate" means to partially or completely blockade one or more of the actions meditated by aldosterone.

An increase in sodium intake can result in increased sodium levels and periods of hypertension in salt sensitive individuals. In such individuals the RAAS generally is unstimulated and renin and aldosterone levels are relatively low because, in part, sodium retention is not necessary. Accordingly, inhibitors of the RAAS upstream of aldosterone (e.g., ACE inhibitors and angiotensin II receptor antagonists) have limited efficacy. Although the basic biochemical defect underlying low renin/salt sensitive hypertension is unclear, aberrant renal sodium retention in these individuals contributes to hypertension. Attempts at adequate therapeutic control often involve combination therapies, which can result in increased negative side effects over single therapy. Aldosterone is hypertensigenic in the kidney, especially in individuals having an elevated level of intracellular sodium. Such individuals especially include salt sensitive individuals. Novel medicaments of the present invention, however, effectively block the hypertensive effects of both unstimulated as well as stimulated aldosterone. Administration of the aldosterone antagonist in accordance with present method can correct the defective natriuretic regulation in the kidney and restore blood pressure, despite high sodium intake which confounds standard antihypertensive therapy.

Accordingly, in another embodiment of the invention, the pathogenic effect results, in whole or in part, from the action of aldosterone in the presence of an elevated level of sodium. Preferably, the pathogenic effect results, in whole or in part, from the action of aldosterone in the presence of an elevated level of intracellular sodium.

In another embodiment of the invention, the pathogenic effect is mediated, in whole or in part, by aldosterone present in the kidney. Preferably, the pathogenic effect results, in whole or in part, from the action of aldosterone in the presence of an elevated level of intracellular sodium. More preferably, the pathogenic effect is selected from renal hypertension and nephrosclerosis.

In another embodiment of the invention, the pathogenic effect results, in whole or in part, from the combined action of aldosterone and elevated dietary sodium intake in the subject.

### Aldosterone Antagonists

The aldosterone antagonist used in the present invention is eplerenone.

This epoxy steroid may be prepared by procedures described in Grob et al., U.S. Patent No. 4,559,332. Additional processes for the preparation of 9,11-epoxy steroidal compounds and their salts are disclosed in Ng et al., WO97/21720 and Ng et al., WO98/25948.

**TABLE I: Aldosterone Receptor Antagonist**

| Compound # | Structure | Name |
|---|---|---|
| 1 | 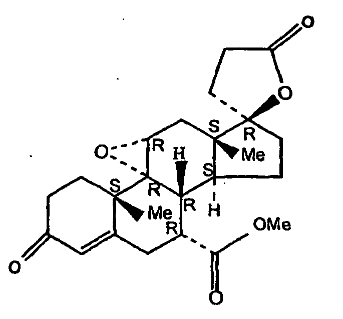 | Pregn-4-ene-7,21-dicarboxylic acid, 9,11-epoxy-17-hydroxy-3-oxo-, γ-lactone, methyl ester, (7α, 11α, 17β)- |

Eplerenone (also known as epoxymexrenone) is compound 1 as shown above. Eplerenone is an aldosterone receptor antagonist and has a higher specificity for aldosterone receptors than does, for example, spironolactone. Selection of eplerenone as the aldosterone antagonist would be beneficial to reduce certain side-effects such as gynecomastia that occur with use of aldosterone antagonists having less specificity.

### Solid State Forms Of Epoxy-Steroidal Aldosterone Receptor Antagonists

The aldosterone antagonist can be administered in any of its solid state forms, either as one or more solid state forms per se or in the form of a pharmaceutical composition comprising one or more solid state forms of the aldosterone antagonist. These novel solid state forms include, but are not limited to, solvated crystalline forms, non-solvated crystalline forms, and the amorphous form. For purposes of illustration, however, the following discussion focuses on solid state forms of epoxy-steroidal compounds.

In one embodiment, the epoxy-steroidal compound administered in accordance with the methods of the present invention is a non-solvated crystalline form of eplerenone having the X-ray powder diffraction pattern set forth in Table 1A below (referred to herein as the "higher melting point polymorph" or "Form H").

In another embodiment of the invention, the eplerenone administered in accordance with the methods of the present invention is a non-solvated crystalline form of eplerenone having the X-ray powder diffraction pattern set forth in Table 1B below (referred to herein as the "lower melting point polymorph" or "Form L").

Unformulated Form H exhibits a faster dissolution rate (approximately 30% higher) at lower temperatures (i.e., temperatures below the enantiotropic transition temperature as later discussed) than, for example, unformulated Form L. Where dissolution of eplerenone in the gastrointestinal tract is the rate-controlling step for delivery of the eplerenone to the target cells, faster dissolution generally results in improved bioavailability. Form H, therefore, can provide an improved bioavailability profile relative to Form L. In addition, selection of a solid state form of eplerenone having a faster dissolution rate likewise provides greater flexibility in the selection of excipients for, and in the formulation of, immediate release pharmaceutical compositions relative to other solid state forms having a slower dissolution rate.

Form L possesses greater physical stability at lower temperatures (i.e., at temperatures below the enantiotropic transition temperature as later discussed) than, for example, Form H. Solid state forms of eplerenone such as Form L that do not require the use of special processing or storage conditions, and that avoid the need for frequent inventory replacement, are desirable. For example, selection of a solid state form of eplerenone that is physically stable during the manufacturing process (such as during milling of eplerenone to obtain a material with reduced particle size and increased surface area) can avoid the need for special processing conditions and the increased costs generally associated with such special processing conditions. Similarly, selection of a solid state form of eplerenone that is physically stable at different conditions of storage (especially considering the different possible storage conditions during the lifetime of the eplerenone product) can help avoid polymorphic or other degradative changes in the eplerenone that can lead to product loss or deterioration of product efficacy. Therefore, the selection of a solid state form of eplerenone such as Form L having greater physical stability provides a meaningful benefit over less stable eplerenone forms.

In another embodiment of the invention, the eplerenone administered in accordance with the present invention is a solvated crystalline form of eplerenone. Preferably, the solvated crystalline forms are substantially exclusive of solvents that are not pharmaceutically-acceptable solvents. Because Form H and Form L typically are more physically stable than the crystalline solvates at room temperature and under atmospheric pressure, the solvated crystalline forms used in such compositions generally comprise a pharmaceutically acceptable higher boiling and/or hydrogen bonding solvent such as, but not limited to, butanol. It is believed that the solvated crystalline forms collectively can offer a range of different dissolution rates and, where dissolution of eplerenone in the gastrointestinal tract is the rate-controlling step for delivery of the eplerenone to the target cells, bioavailabilities relative to Form H and Form L.

In another embodiment of the invention, the eplerenone administered in accordance with the present invention is amorphous eplerenone. It is hypothesized that amorphous eplerenone possesses a different dissolution rate and, where dissolution of eplerenone in the gastrointestinal tract is the rate-controlling step for delivery of the eplerenone to the target cells, bioavailability relative to Form H and Form L.

In another embodiment, the eplerenone administered in accordance the present invention is a combination comprising a first solid state form of eplerenone and a second solid state form of eplerenone. Generally, the first and second solid state forms of eplerenone are selected from Form H, Form L, solvated eplerenone and amorphous eplerenone. Such combinations may further comprise additional solid state forms of eplerenone and are useful, for example, in the preparation of pharmaceutical compositions having differing dissolution profiles, including controlled release compositions. In general, the weight ratio of said first solid state form to said second solid state form preferably is at least about 1:9, more preferably at least about 1:1, still more preferably at least about 2:1, still more preferably at least about 5:1, and still more preferably at least about 9:1.

In another embodiment, the eplerenone is administered in the form of a pharmaceutical composition wherein the entire amount of eplerenone contained in the composition is present as phase pure Form H.

In another embodiment, the eplerenone is administered in the form of a pharmaceutical composition wherein the entire amount of eplerenone contained in the composition is present as phase pure Form L

In another embodiment, the eplerenone is administered in the form of a pharmaceutical composition wherein the entire amount of eplerenone contained in the composition is present as a phase pure solvated crystalline eplerenone.

In another embodiment, the eplerenone is administered in the form of a pharmaceutical composition wherein the entire amount of eplerenone contained in the composition is present as amorphous eplerenone.

In another embodiment, the eplerenone is administered in the form of a pharmaceutical composition wherein the composition comprises a first solid state form of eplerenone and a second solid state form of eplerenone, and the first and second solid state forms of eplerenone are selected from Form H, Form L, solvated eplerenone and amorphous eplerenone. In general, the weight ratio of said first solid state form to said second solid state form preferably is at least about 1:9, preferably about 1:1, more preferably at least about 2:1, more preferably at least about 5:1, and still more preferably at least about 9:1.

In another embodiment, the eplerenone is administered in the form of a pharmaceutical composition wherein the composition comprises both Form H and Form L. The ratio of the amount of Form L to Form H in the composition generally is between about 1:20 to about 20:1. In other embodiments, for example, this ratio is between about 10:1 to about 1:10; about 5:1 to about 1:5; about 2:1 to about 1:2; or about 1:1.

Although each of the above embodiments can embrace the administration of a solid state form of eplerenone over a broad range of eplerenone particle sizes, it has been discovered that coupling the selection of the solid state form of eplerenone with a reduction of the eplerenone particle size can improve the bioavailability of unformulated eplerenone and pharmaceutical compositions comprising that solid state form of eplerenone.

In one such embodiment, the D₉₀ particle size of the unformulated eplerenone or the eplerenone used as a starting material in the pharmaceutical composition generally is less than about 400 microns, preferably less than about 200 microns, more preferably less than about 150 microns, still more preferably less than about 100 microns, and still more preferably less than about 90 microns. In another embodiment, the D₉₀ particle size is between about 40 microns to about 100 microns. In another embodiment, the D₉₀ particle size is between about 30 microns to about 50 microns. In another embodiment, the D₉₀ particle size is between about 50 microns to about 150 microns. In another embodiment, the D₉₀ particle size is between about 75 microns to about 125 microns.

In another such embodiment, the D₉₀ particle size of the unformulated eplerenone or the eplerenone used as a starting material in the pharmaceutical composition generally is less than about 15 microns, preferably less than about 1 micron, more preferably less than about 800 nm, still more preferably less than about 600 nm, and still more preferably less than about 400 nm. In another embodiment, the D₉₀ particle size is between about 10 nm to about 1 micron. In another embodiment, the D₉₀ particle size is between about 100 nm to about 800 nm. In another embodiment, the D₉₀ particle size is between about 200 nm to about 600 nm. In another embodiment, the D₉₀ particle size is between about 400 nm to about 800 nm.

Solid state forms of eplerenone having a particle size less than about 15 microns can be prepared in accordance with applicable particle size reduction techniques known in the art. Such techniques include, those described in U.S. Patents 5,145,684, 5,318,767, 5,384,124 and 5,747,001. U.S. Patents 5,145,684, 5,318,767, 5,384,124 and 5,747,001. In accordance with the method of U.S. Patent 5,145,684, for example, particles of suitable size are prepared by dispersing the eplerenone in a liquid dispersion medium and wet-grinding the mixture in the presence of grinding media to reduce the particles to the desired size. If necessary or advantageous, the particles can be reduced in size in the presence of a surface modifier.

### Solid State Definitions

The term "amorphous" as applied to eplerenone refers to a solid state wherein the eplerenone molecules are present in a disordered arrangement and do not form a distinguishable crystal lattice or unit cell. When subjected to X-ray powder diffraction, amorphous eplerenone does not produce any characteristic crystalline peaks.

Where reference is made in this application to the "boiling point" of a substance or solution, the term "boiling point" means the boiling point of the substance or solution under the applicable process conditions.

The term "crystalline form" as applied to eplerenone refers to a solid state form wherein the eplerenone molecules are arranged to form a distinguishable crystal lattice (i) comprising distinguishable unit cells, and (ii) yielding diffraction peaks when subjected to X-ray radiation.

The term "crystallization" as used throughout this application can refer to crystallization and/or recrystallization depending upon the applicable circumstances relating to the preparation of the eplerenone starting material.

The term "digestion" means a process in which a slurry of solid eplerenone in a solvent or mixture of solvents is heated at the boiling point of the solvent or mixture of solvents under the applicable process conditions.

The term "direct crystallization" as used herein refers to the crystallization of eplerenone directly from a suitable solvent without the formation and desolvation of an intermediate solvated crystalline solid state form of eplerenone.

The term "particle size" as used herein refers to particle size as measured by conventional particle size measuring techniques well known in the art, such as laser light scattering, sedimentation field flow fractionation, photon correlation spectroscopy, or disk centrifugation.

The term "D₉₀ particle size" means the particle size of at least 90% of the particles as measured by such conventional particle size measuring techniques.

The term "purity" means the chemical purity of eplerenone according to conventional HPLC assay. As used herein, "low purity eplerenone" generally means eplerenone that contains an effective amount of a Form H growth promoter and/or a Form L growth inhibitor. As used herein, "high purity eplerenone" generally means eplerenone that does not contain, or contains less than an effective amount of, a Form H growth promoter and/or a Form L growth inhibitor.

The term "phase purity" means the solid state purity of eplerenone with regard to a particular crystalline or amorphous form of the eplerenone as determined by the infrared spectroscopy analytical methods described herein.

The term "XPRD" means X-ray powder diffraction.

The term "Tₘ" means melting temperature.

### Characterization of Solid State Form

### 1. Molecular Conformation

Single crystal X-ray analysis indicates that the eplerenone molecular conformation differs between Form H and Form L, particularly with respect to the orientation of the ester group at the 7-position of the steroid ring. The orientation of the ester group can be defined by the C8-C7-C23-02 torsion angle.

In the Form H crystal lattice, the eplerenone molecule adopts a conformation in which the methoxy group of the ester is approximately aligned with the C-H bond at the 7-position and the carbonyl group is approximately positioned over the center of the B-steroid ring. The C8-C7-C23-02 torsion angle is approximately - 73.0° in this conformation. In this orientation, the carbonyl oxygen atom of the ester group (01) is in close contact with the oxygen atom of the 9,11-epoxide ring (04). The 01-04 distance is about 2.97 Å, which is just below the van der Waal's contact distance of 3.0 Å (assuming van der Waal's radii of 1.5Å for the oxygen).

In the Form L crystal lattice, the eplerenone molecule adopts a conformation in which the ester group is rotated approximately 150° relative to that of Form H and has a C8-C7-C23-02 torsion angle of approximately +76.9°. In this orientation, the methoxy group of the ester is directed toward the 4,5-alkene segment of the A-steroid ring. In this orientation, the distance between either oxygen atom of the ester group (01,02) and the oxygen atom of the 9,11-epoxide ring is increased relative to the distance determined for Form H. The 02-04 distance is approximately 3.04Å, falling just above the van der Waal's contact distance. The 01-04 distance is about 3.45Å.

The eplerenone molecule appears to adopt a conformation characteristic of Form L in the solvated crystalline forms analyzed by single crystal X-ray diffraction to date.

### 2. X-Ray Powder Diffraction

The various crystalline forms of eplerenone were analyzed with either a Siemens D5000 powder diffractometer or an Inel Multipurpose Diffractometer. For the Siemens D500 powder diffractometer, the raw data was measured for 2q values from 2 to 50, with steps of 0.020 and step periods of two seconds. For the Inel Multipurpose Diffractometer, samples were placed in an aluminum sample holder and raw data was collected for 30 minutes at all two theta values simultaneously.

Tables 1A, 1B and 1C set out the significant parameters of the main peaks in terms of 2q values and intensities for the Form H (prepared by desolvation of the ethanol solvate obtained by digestion of low purity eplerenone), Form L (prepared by desolvation of the methyl ethyl ketone solvate obtained by recrystallization of high purity eplerenone), and methyl ethyl ketone solvate (prepared by room temperature slurry conversion of high purity eplerenone in methyl ethyl ketone) crystalline forms of eplerenone, respectively (X-ray radiation at a wavelength of 1.54056 Angstroms).

Minor shifts in peak positioning may be present in the diffraction patterns of Form H and Form L as a result of imperfections in the spacing of the crystal diffraction planes due to the route of manufacture of Form H and Form L (i.e. desolvation of a solvate). In addition, Form H is isolated from a solvate prepared by digestion of crude eplerenone. This method results in a lower overall chemical purity (approximately 90%) of the Form H. Finally, the solvated forms of eplerenone are expected to show some shifting in the positioning of the diffraction peaks due to the increased mobility of the solvent molecules within the solvent channels in the crystal lattice.

**Table 1A: FORM H DATA**

| Angle 2-theta | d-spacing Angstrom | Intensity | Intensity % |
|---|---|---|---|
| 6.994 | 12.628 | 1188 | 7.2 |
| 8.291 | 10.655 | 2137 | 13 |
| 10.012 | 8.827 | 577 | 3.5 |
| 11.264 | 7.849 | 1854 | 11.3 |
| 12.04 | 7.344 | 7707 | 46.8 |
| 14.115 | 6.269 | 3121 | 19 |
| 14.438 | 6.13 | 15935 | 96.8 |
| 15.524 | 5.703 | 637 | 3.9 |
| 16.169 | 5.477 | 1349 | 8.2 |
| 16.699 | 5.305 | 1663 | 10.1 |
| 16.94 | 5.23 | 1692 | 10.3 |

**Table 1A: FORM H DATA**

| Angle 2-theta | d-spacing Angstrom | Intensity | Intensity % |
|---|---|---|---|
| 17.147 | 5.167 | 2139 | 13 |
| 17.66 | 5.018 | 6883 | 41.8 |
| 17.91 | 4.949 | 16455 | 100 |
| 18.379 | 4.823 | 3106 | 18.9 |
| 18.658 | 4.752 | 1216 | 7.4 |
| 19.799 | 4.48 | 1499 | 9.1 |
| 20.235 | 4.385 | 383 | 2.3 |
| 21.707 | 4.091 | 1267 | 7.7 |
| 21.8 | 4.073 | 1260 | 7.7 |
| 21.959 | 4.044 | 1279 | 7.8 |
| 22.461 | 3.955 | 4264 | 25.9 |
| 23.191 | 3.832 | 1026 | 6.2 |
| 23.879 | 3.723 | 1000 | 6.1 |
| 24.599 | 3.616 | 1688 | 10.3 |
| 25.837 | 3.445 | 931 | 5.7 |
| 26.034 | 3.42 | 686 | 4.2 |
| 26.868 | 3.316 | 912 | 5.5 |
| 27.093 | 3.288 | 1322 | 8 |
| 27.782 | 3.209 | 1236 | 7.5 |
| 28.34 | 3.147 | 1845 | 11.2 |
| 28.861 | 3.091 | 957 | 5.8 |
| 29.866 | 2.9892 | 745 | 4.5 |
| 30.627 | 2.9166 | 992 | 6 |
| 31.108 | 2.8726 | 1205 | 7.3 |
| 33.215 | 2.6951 | 1287 | 7.8 |
| 33.718 | 2.656 | 802 | 4.9 |
| 34.434 | 2.6024 | 914 | 5.6 |

**Table 1B: FORM L DATA**

| Angle 2-Theta | d-spacing Angstrom | Intensity Cps | Intensity % |
|---|---|---|---|
| 7.992 | 11.054 | 11596 | 26.6 |
| 10.044 | 8.799 | 12048 | 27.6 |
| 11.206 | 7.889 | 4929 | 11.3 |
| 12.441 | 7.109 | 1747 | 4 |
| 12.752 | 6.936 | 4340 | 9.9 |
| 13.257 | 6.673 | 2444 | 5.6 |
| 14.705 | 6.019 | 43646 | 100 |
| 15.46 | 5.727 | 2670 | 6.1 |
| 15.727 | 5.63 | 7982 | 18.3 |
| 16.016 | 5.529 | 3519 | 8.1 |
| 17.671 | 5.015 | 8897 | 20.4 |
| 17.9 | 4.951 | 2873 | 6.6 |
| 18.352 | 4.83 | 612 | 1.4 |

**Table 1B: FORM L DATA**

| Angle 2-Theta | d-spacing Angstrom | Intensity Cps | Intensity % |
|---|---|---|---|
| 18.703 | 4.74 | 689 | 1.6 |
| 19.524 | 4.543 | 1126 | 2.6 |
| 20.103 | 4.413 | 3753 | 8.6 |
| 20.63 | 4.302 | 1451 | 3.3 |
| 21.067 | 4.214 | 876 | 2 |
| 21.675 | 4.097 | 2760 | 6.3 |
| 22.232 | 3.995 | 1951 | 4.5 |
| 22.652 | 3.922 | 1657 | 3.8 |
| 23.624 | 3.763 | 827 | 1.9 |
| 24.279 | 3.663 | 1242 | 2.8 |
| 25.021 | 3.556 | 5144 | 11.8 |
| 25.485 | 3.492 | 1702 | 3.9 |
| 25.707 | 3.463 | 2493 | 5.7 |
| 26.251 | 3.392 | 1371 | 3.1 |
| 26.85 | 3.318 | 1970 | 4.5 |
| 27.319 | 3.262 | 1029 | 2.4 |
| 27.931 | 3.192 | 440 | 1 |
| 27.969 | 3.187 | 440 | 1 |
| 28.937 | 3.083 | 1128 | 2.6 |
| 29.703 | 3.005 | 1211 | 2.8 |
| 30.173 | 2.9594 | 1506 | 3.5 |
| 30.584 | 2.9206 | 1602 | 3.7 |
| 30.885 | 2.8928 | 1550 | 3.6 |
| 31.217 | 2.8628 | 1068 | 2.4 |
| 31.605 | 2.8285 | 1038 | 2.4 |
| 32.059 | 2.7895 | 1211 | 2.8 |
| 32.64 | 2.7412 | 684 | 1.6 |
| 32.747 | 2.7324 | 758 | 1.7 |
| 33.46 | 2.6759 | 506 | 1.2 |
| 34.194 | 2.6201 | 1085 | 2.5 |
| 34.545 | 2.5943 | 915 | 2.1 |

**Table 1C: METHYL ETHYL KETONE DATA**

| Angle 2-Theta | d-spacing Angstrom | Intensity Cps | Intensity % |
|---|---|---|---|
| 7.584 | 11.648 | 5629 | 32.6 |
| 7.753 | 11.393 | 15929 | 92.3 |
| 10.151 | 8.707 | 2877 | 16.7 |
| 11.31 | 7.817 | 701 | 4.1 |
| 12.646 | 6.994 | 1027 | 5.9 |
| 13.193 | 6.705 | 15188 | 88 |
| 13.556 | 6.526 | 14225 | 82.4 |
| 14.074 | 6.287 | 1966 | 11.4 |
| 14.746 | 6.002 | 2759 | 16 |
| 15.165 | 5.837 | 801 | 4.6 |
| 15.548 | 5.694 | 1896 | 11 |
| 17.031 | 5.202 | 7980 | 46.2 |
| 17.28 | 5.127 | 17267 | 100 |
| 17.706 | 5.005 | 6873 | 39.8 |
| 18.555 | 4.778 | 545 | 3.2 |
| 18.871 | 4.699 | 1112 | 6.4 |
| 19.766 | 4.488 | 1704 | 9.9 |
| 20.158 | 4.401 | 1396 | 8.1 |
| 20.725 | 4.282 | 2644 | 15.3 |
| 21.787 | 4.076 | 1127 | 6.5 |
| 22.06 | 4.026 | 451 | 2.6 |
| 22.864 | 3.886 | 1542 | 8.9 |
| 23.412 | 3.796 | 14185 | 82.2 |
| 23.75 | 3.743 | 1154 | 6.7 |
| 24.288 | 3.662 | 3063 | 17.7 |
| 25.253 | 3.524 | 1318 | 7.6 |
| 25.503 | 3.49 | 1736 | 10.1 |
| 25.761 | 3.455 | 1225 | 7.1 |
| 26.176 | 3.402 | 1346 | 7.8 |
| 26.548 | 3.355 | 1098 | 6.4 |
| 27.357 | 3.257 | 1944 | 11.3 |
| 27.605 | 3.229 | 2116 | 12.3 |
| 27.9 | 3.195 | 858 | 5 |
| 28.378 | 3.142 | 583 | 3.4 |
| 28.749 | 3.103 | 763 | 4.4 |
| 29.3 | 3.046 | 1182 | 6.8 |
| 29.679 | 3.008 | 2606 | 15.1 |
| 30.402 | 2.9377 | 2184 | 12.6 |
| 30.739 | 2.9063 | 648 | 3.8 |

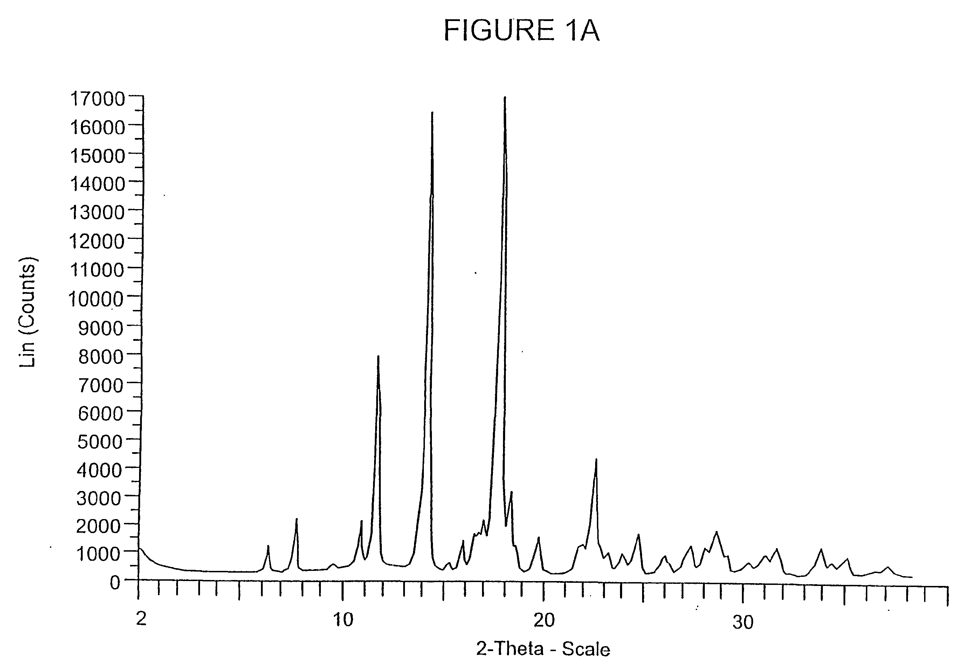

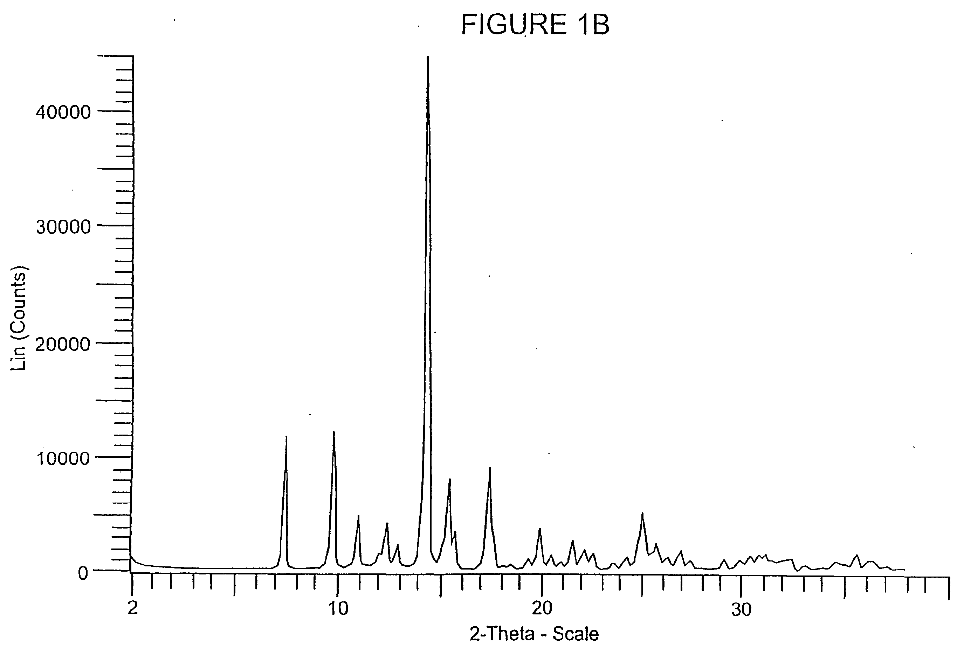

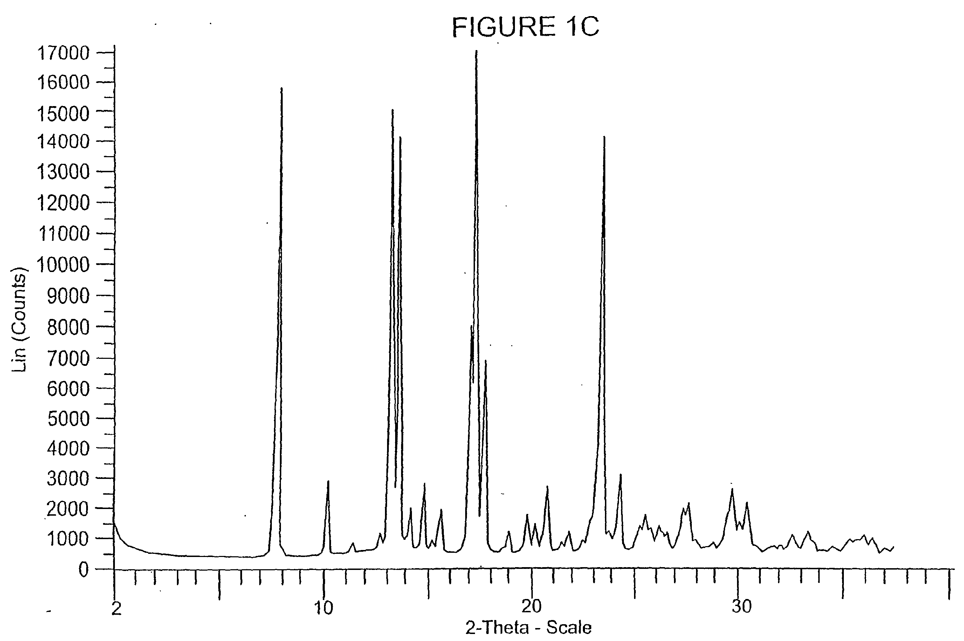

Graphical examples of the x-ray diffraction patterns for Form H, Form L, and the methyl ethyl ketone solvate crystalline forms of eplerenone are shown in Figs. 1-A, 1-B, and 1-C, respectively. Form H shows distinguishing peaks at 7.0 ± 0.2, 8.3 ± 0.2, and 12.0 ± 0.2 degrees two theta. Form L shows distinguishing peaks at 8.0 ± 0.2, 12.4 ± 0.2, 12.8 ± 0.2, and 13.3 ± 0.2 degrees two theta. The methyl ethyl ketone solvated crystalline form shows distinguishing peaks at 7.6 ± 0.2, 7.8 ± 0.2, and 13.6 ± 0.2 degrees two theta.

### 3. Melting/Decomposition Temperature

The temperatures of melting and/or decomposition of non-solvated eplerenone crystalline forms were determined using a TA Instruments 2920 differential scanning calorimeter. Each sample (1-2 mg) was placed in either a sealed or unsealed aluminum pan and heated at 10°C/minute. Melting/decomposition ranges were defined from the extrapolated onset to the maximum of the melting/decomposition endotherm.

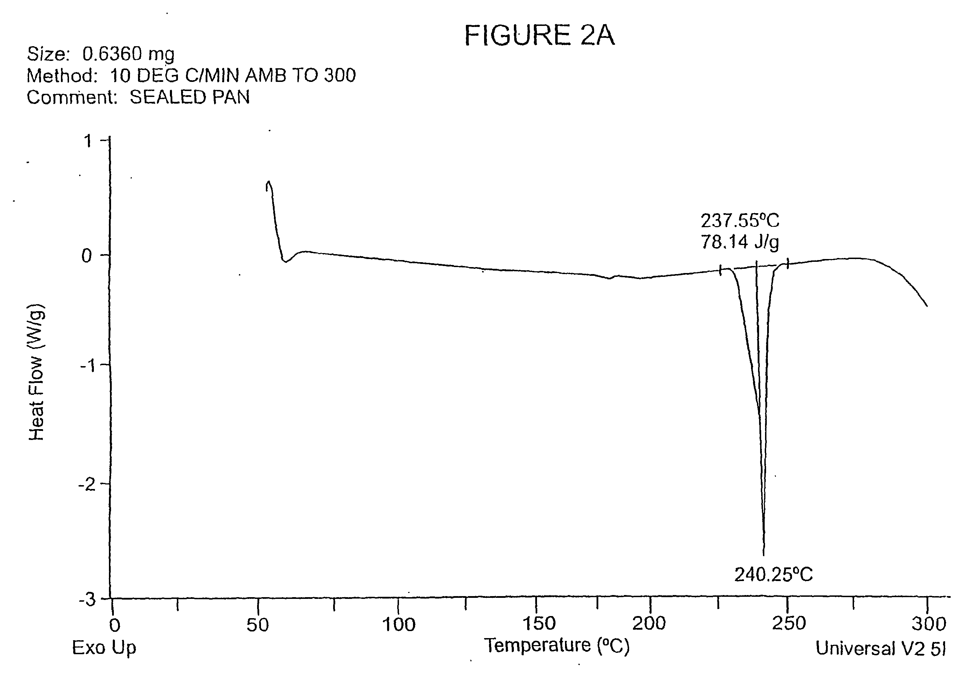

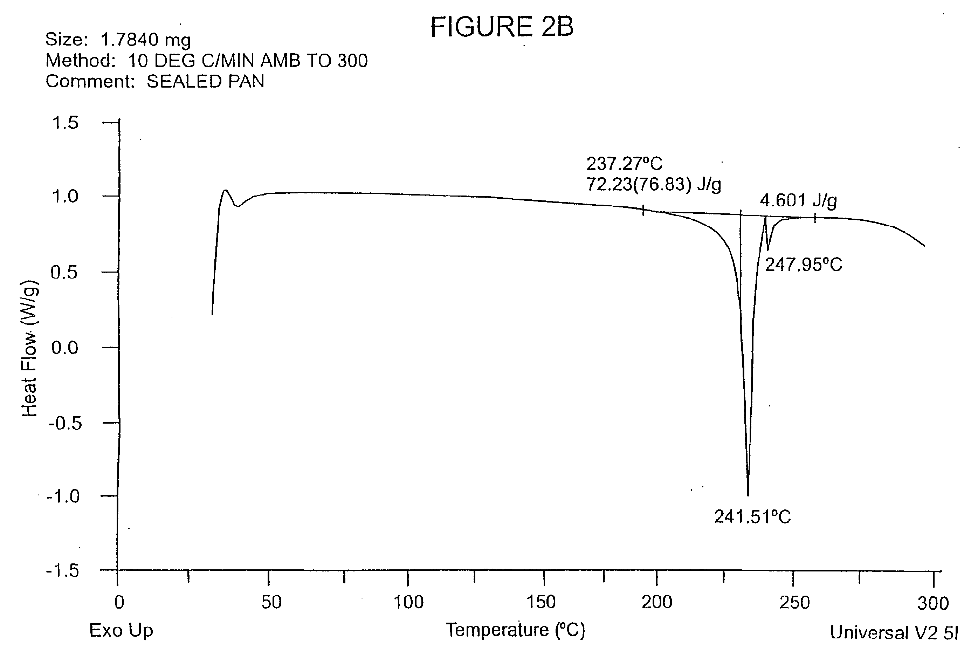

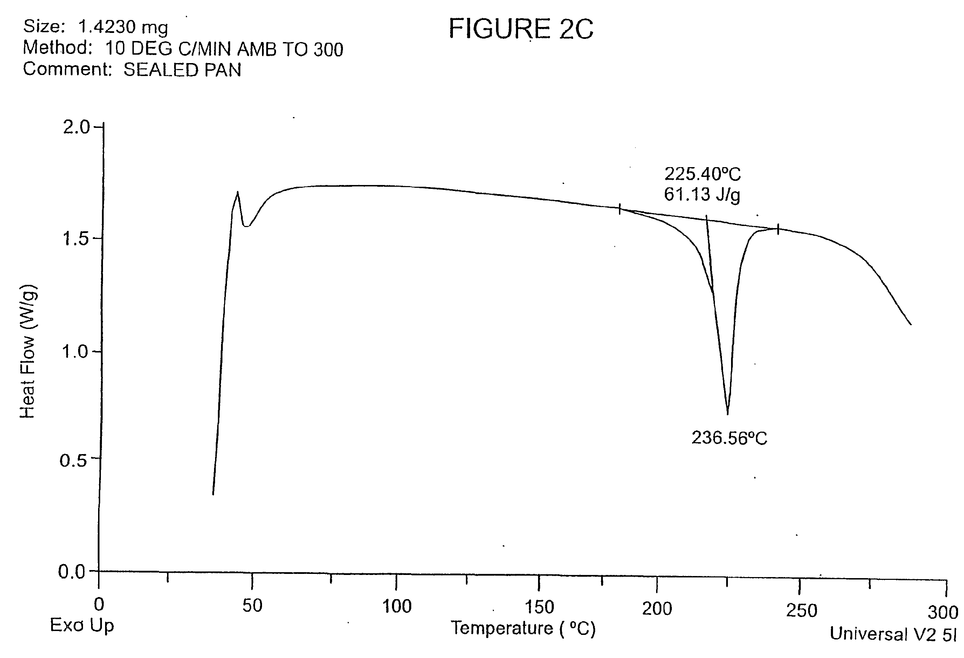

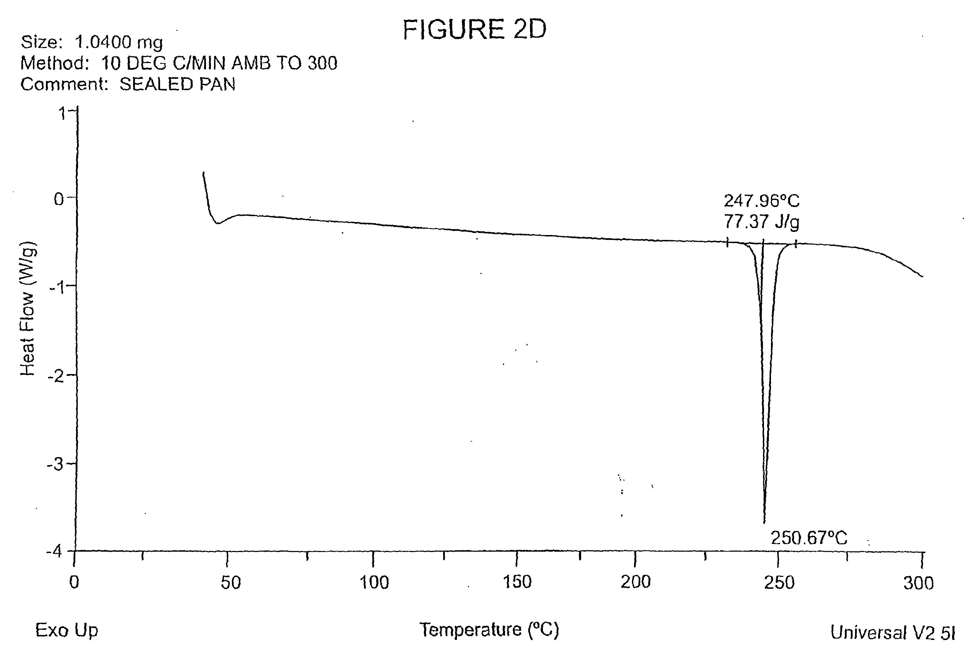

The melting of the non-solvated eplerenone crystals forms (Form H and Form L) was associated with chemical decomposition and loss of trapped solvent from the crystal lattice. The melting/decomposition temperature also was affected by the manipulation of the solid prior to analysis. For example, non-milled Form L (approximate D₉₀ particle size of about 180-450 microns) prepared by direct crystallization from an appropriate solvent or from desolvation of a solvate obtained from crystallization of high purity eplerenone in an appropriate solvent or mixture of solvents generally had a melting range of about 237-242°C. Milled Form L (approximate D₉₀ particle size of about 80-100 microns) (Form L prepared by crystallizing a solvate from a solution of high purity eplerenone in an appropriate solvent or mixture of solvents, desolvating the solvate to yield Form L, and milling the resulting Form L) generally had a lower and broader melting/decomposition range of about 223-234°C. Non-milled Form H (approximate D₉₀ particle size of about 180-450 microns) prepared by desolvation of a solvate obtained by digestion of low purity eplerenone generally had a higher melting/decomposition range of about 247-251°C. Examples of the DSC thermograms of (a) non-milled Form L directly crystallized from methyl ethyl ketone, (b) non-milled Form L prepared by desolvation of a solvate obtained by crystallization of a high purity eplerenone from methyl ethyl ketone, (c) Form L prepared by milling a desolvated solvate obtained by crystallization of high purity eplerenone from methyl ethyl ketone, and (d) non-milled Form H prepared by desolvation of a solvate obtained by digestion of low purity eplerenone from methyl ethyl ketone are given in Figures 2-A, 2-B, 2-C and 2-D, respectively.

DSC thermograms of solvated forms of eplerenone were determined using a Perkin Elmer Pyris 1 differential scanning calorimeter. Each sample (1-10 mg) was placed in an unsealed aluminum pan and heated at 10°C/minute. One or more endothermal events at lower temperatures were associated with enthalpy changes that occurred as solvent was lost from the solvate crystal lattice. The highest temperature endotherm or endotherms were associated with the melting/decomposition of Form L or Form H eplerenone.

### 4. Infrared Absorption Spectroscopy

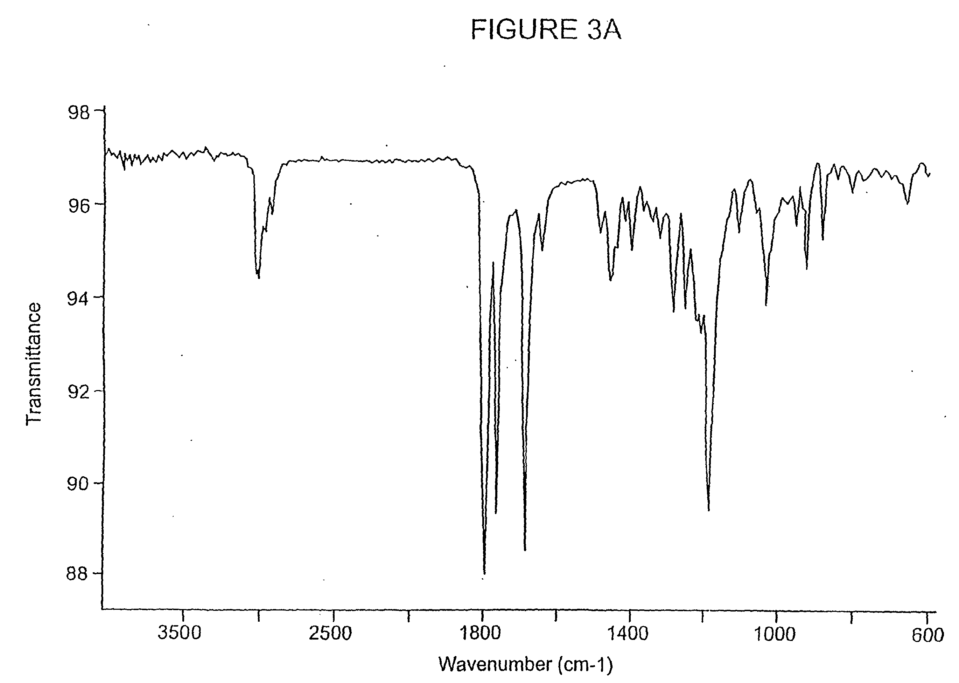

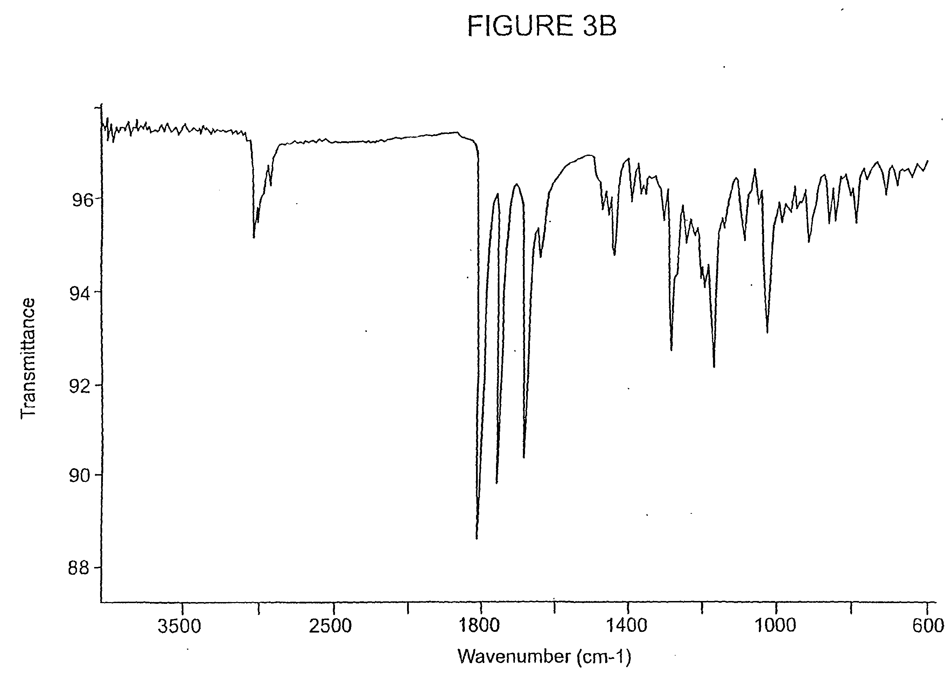

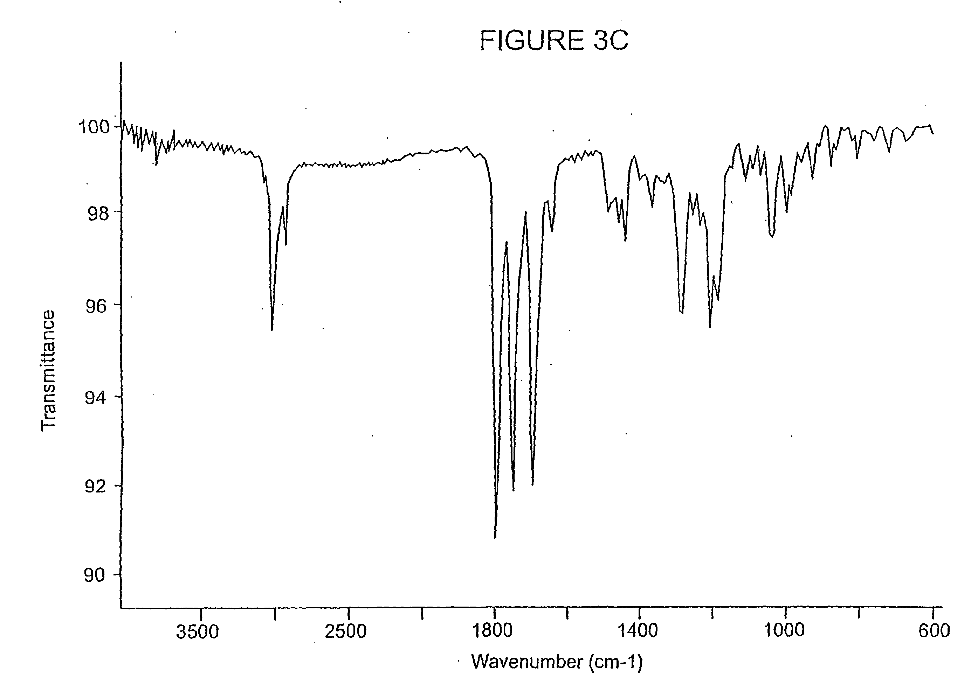

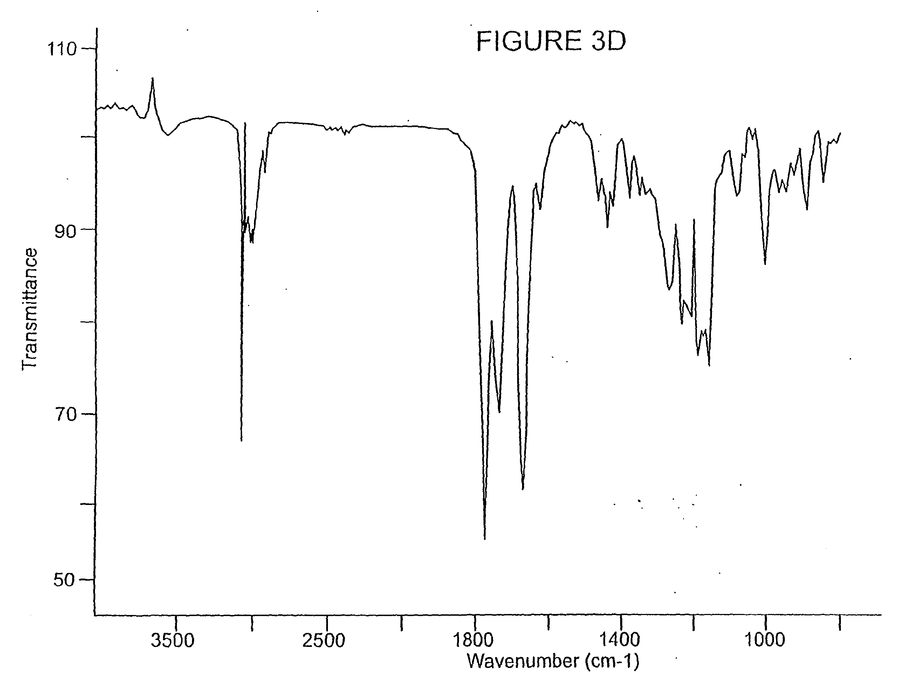

Infrared absorption spectra of the non-solvated forms of eplerenone (Form H and Form L) were obtained with a Nicolet DRIFT (diffuse reflectance infrared fourier transform) Magna System 550 spectrophotometer. A Spectra-Tech Collector system and a microsample cup were used. Samples (5%) were analyzed in potassium bromide and scanned from 400-4000 cm⁻¹. Infrared absorption spectra of eplerenone in dilute chloroform solution (3%) or in the solvated crystal forms were obtained with a Bio-rad FTS-45 spectrophotometer. Chloroform solution samples were analyzed using a solution cell of 0.2 mm path length with sodium chloride salt plates. Solvate FTIR spectra were collected using an IBM micro-MIR (multiple internal reflectance) accessory. Samples were scanned from 400-4000 cm⁻¹. Examples of the infrared absorption spectra of (a) Form H, (b) Form L, (c) the methyl ethyl ketone solvate, and (d) eplerenone in chloroform solution are shown in Figures 3-A, 3-B, 3-C and 3-D, respectively.

Table 2 discloses illustrative absorption bands for eplerenone in the Form H, Form L, and methyl ethyl ketone solvate crystal forms. Illustrative absorption bands for eplerenone in chloroform solution are also disclosed for comparison. Differences between Form H and either Form L or the methyl ethyl ketone solvate were observed, for example, in the carbonyl region of the spectrum. Form H has an ester carbonyl stretch of approximately 1739 cm⁻¹ while both Form L and the methyl ethyl ketone solvate have the corresponding stretch at approximately 1724 and 1722 cm⁻¹, respectively. The ester carbonyl stretch occurs at approximately 1727 cm⁻¹ in the eplerenone in chloroform solution. The change in stretching frequency of the ester carbonyl between Form H and Form L reflects the change in orientation of the ester group between the two crystal forms. In addition, the stretch of the ester of the conjugated ketone in the A-steroid ring shifts from approximately 1664-1667 cm⁻¹ in either Form H or the methyl ethyl ketone solvate to approximately 1655 cm⁻¹ in Form L. The corresponding carbonyl stretch occurs at approximately 1665 cm⁻¹ in dilute solution.

Another difference between Form H and Form L was seen in the C-H bending region. Form H has an absorption at approximately 1399 cm⁻¹ which is not observed in Form L, the methyl ethyl ketone solvate, or the eplerenone in chloroform solution. The 1399 cm⁻¹ stretch occurs in the region of CH₂ scissoring for the C2 and C21 methylene groups adjacent to carbonyl groups.

**Table 2**

| Absorption Region | Form H (cm⁻¹) | Form L (cm⁻¹) | Methyl Ethyl Ketone Solvate (cm⁻¹) | Eplerenone in Chloroform (cm⁻¹) |
|---|---|---|---|---|
| ν C=O(lactone) | 1773 | 1775 | 1767 | 1768 |
| ν C=O(ester) | 1739 | 1724 | 1722 | 1727 |
| ν C=O(3keto) | 1664 | 1655 | 1667 | 1665 |
| ν C=C (3,4-olefin) | 1619 | 1619 | 1622 | 1623 |
| δₐₛCH3,δCH2, δCH2(α to carbonyl) | 1460, 1444, 1426 | 1467, 1438, 1422, 1399 | 1467, 1438, 1422 | 1464, 1438, 1422 |
| δₛCH3 | 1380 | 1381 | ~1380 | 1378 |

### 5. Nuclear Magnetic Resonance

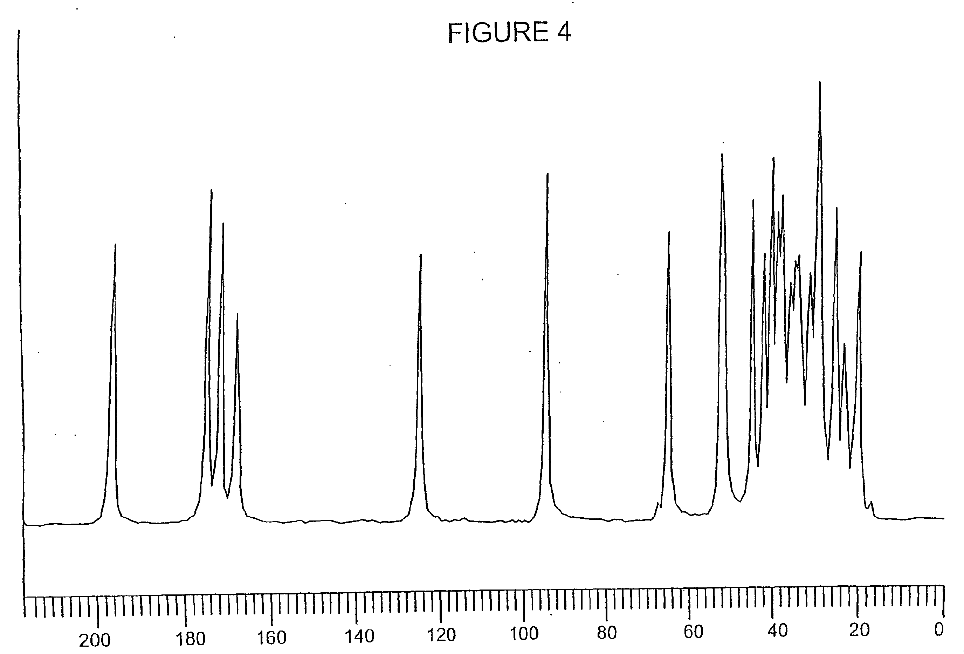

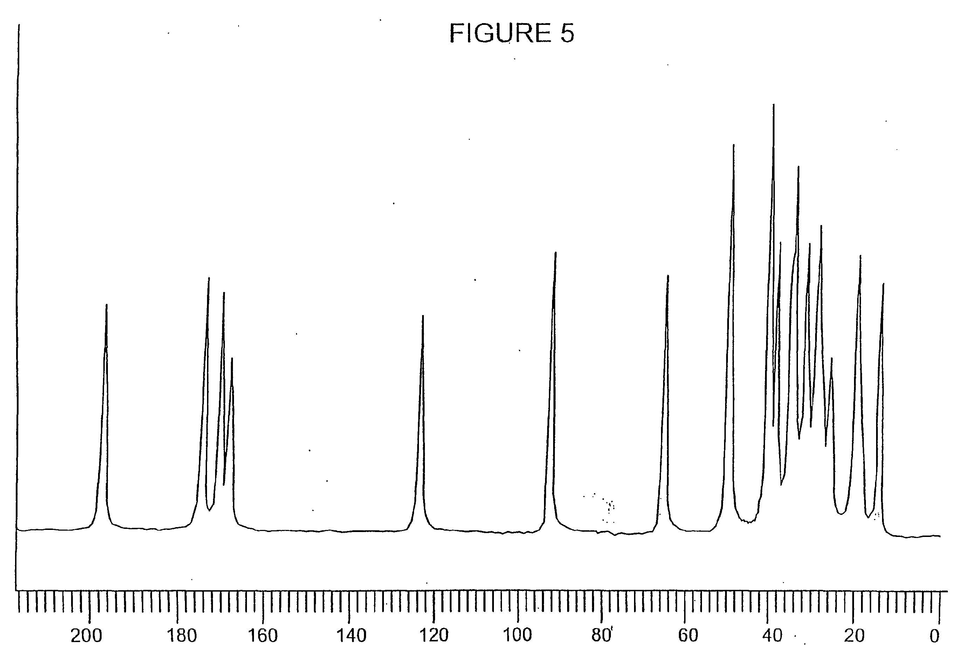

¹³C NMR spectra were obtained at a field of 31.94 MHz. Examples of the ¹³C NMR spectra of Form H and Form L eplerenone are shown in Figs. 4 and 5, respectively. The Form H eplerenone analyzed to obtain the data reflected in Fig. 4 was not phase pure and included a small amount of Form L eplerenone. Form H is most clearly distinguished by the carbon resonances at around 64.8 ppm, 24.7 ppm and 19.2 ppm. Form L is most clearly distinguished by the carbon resonances at around 67.1 ppm and 16.0 ppm.

### 6. Thermogravimetry

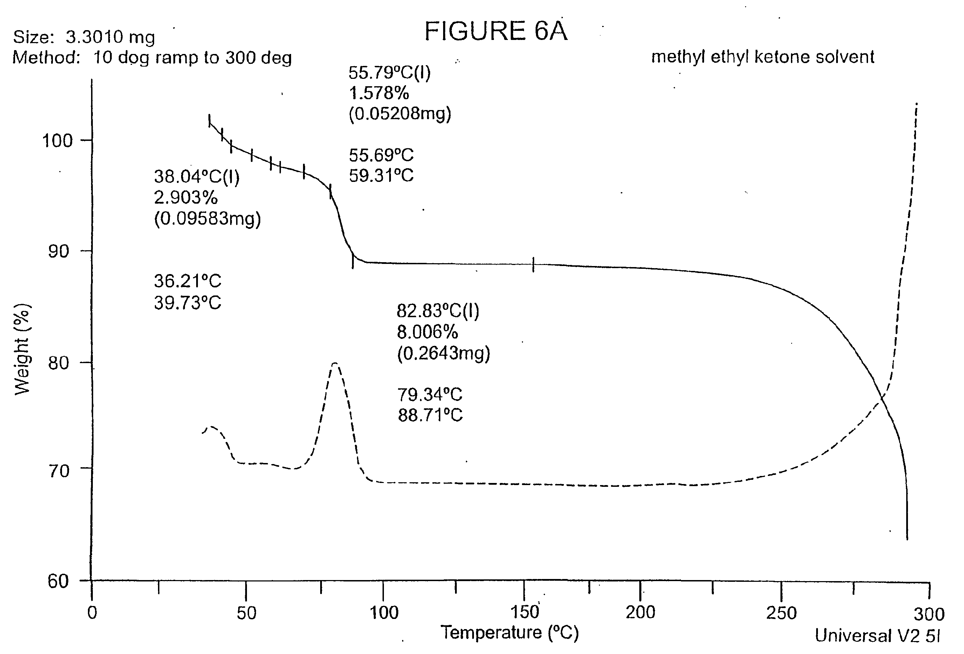

Thermogravimetric analysis of solvates was performed using a TA Instruments TGA 2950 thermogravimetric analyzer. Samples were placed in an unsealed aluminum pan under nitrogen purge. Starting temperature was 25°C with the temperature increased at a rate of about 10°C/minute. An example of the thermogravimetry analysis profile for the methyl ethyl ketone solvate is shown in Fig. 6-A.

### 7. Unit Cell Parameters

Tables 3A, 3B and 3C below summarize the unit cell parameters determined for Form H, Form L, and several solvated crystalline forms.

**Table 3A**

| Parameter | Form H | Form L | Methyl ethyl ketone Solvate |
|---|---|---|---|
| Crystal system | Ortho-rhombic | Monoclinic | Orthorhombic |
| Space group | P2₁2₁2₁ | P2₁ | P2₁2₁2₁ |
| a | 21.22 Å | 8.78 Å | 23.53 Å |
| b | 15.40 Å | 11.14 Å | 8.16 Å |
| c | 6.34 Å | 11.06 Å | 13.08 Å |
| α | 90° | 90° | 90° |
| β | 90° | 93.52° | 90° |
| γ | 90° | 90° | 90° |
| Z | 4 | 2 | 4 |
| Volume (Å) | 2071.3 | 1081.8 | 2511.4 |
| ρ (calculated) | 1.329 g/cm³ | 1.275 g/cm³ | 1.287 g/cm³ |
| R | 0.0667 | 0.062 | 0.088 |

**Table 3B**

| Parameter | Acetone Solvate | Toluene Solvate | Butyl Acetate Solvate¹ |
|---|---|---|---|
| Crystal system | Ortho-rhombic | Ortho-rhombic | Ortho-rhombic |
| Space group | P2₁2₁2₁ | P2₁2₁2₁ | P2₁2₁2₁ |
| a | 23.31 Å | 23.64 Å | 23.07 Å |
| b | 13.13 Å | 13.46 Å | 13.10 Å |
| c | 8.28 Å | 8.16 Å | 8.24 Å |
| α | 90° | 90° | 90° |
| β | 90° | 90° | 90° |
| γ | 90° | 90° | 90° |
| Z | 4 | 4 | 4 |
| Volume (Å) | 2533.7 | 2596.6 | 2490.0 |
| ρ (calculated) | 1.239 g/cm³ | 1.296 g/cm³ | 1.334 g/cm³ |
| R | 0.058 | 0.089 | 0.093 |

| | | | |
|---|---|---|---|
| ¹The solvate molecules were not completely refined due to disorder of the solvent molecules in the channels. | | | |

**Table 3C**

| Parameter | Isobutyl Acetate Solvate¹ | Isopropanol Solvate¹ | Ethanol Solvate¹ |
|---|---|---|---|
| Crystal system | Ortho-rhombic | Ortho-rhombic | Ortho-rhombic |
| Space group | P2₁2₁2₁ | P2₁2₁2₁ | P2₁2₁2₁ |
| a | 23.19 Å | 23.15 Å | 23.51 Å |
| b | 12.95 Å | 12.73 Å | 13.11 Å |
| c | 8.25 Å | 8.25 Å | 8.27 Å |
| α | 90° | 90° | 90° |
| β | 90° | 90° | 90° |
| γ | 90° | 90° | 90° |
| Z | 4 | 4 | 4 |
| Volume (Å) | 2476.4 | 2433.2 | 2548.6 |
| ρ (calculated) | 1.337 g/cm³ | 1.296 g/cm³ | 1.234 g/cm³ |
| R | 0.098 | 0.152 | 0.067 |

| | | | |
|---|---|---|---|
| ¹The solvate molecules were not refined completely due to disorder of the solvent molecules in the channels. | | | |

Additional information on selected solvated crystalline forms of eplerenone is reported in Table 4 below. The unit cell data reported in Table 3A above for the methyl ethyl ketone solvate also are representative of the unit cell parameters for many of these additional eplerenone crystalline solvates. Most of the eplerenone crystalline solvates tested are substantially isostructural to each other. While there may be some minor shifting in the X-ray powder diffraction peaks from one solvated crystalline form to the next due to the size of the incorporated solvent molecule, the overall diffraction patterns are substantially the same and the unit cell parameters and molecular positions are substantially identical for most of the solvates tested.

**Table 4**

| Solvent | Stoichiometry (Solvent: Eplerenone) | Isostructural to Methyl Ethyl Ketone Solvate? | Desolvation Temperature¹ (°C) |
|---|---|---|---|
| Methyl Ethyl Ketone | 1:1 | N/A | 89 |
| 2-Pentanone | --- | --- | --- |
| Acetic Acid | 1:2 | Yes | 203 |
| Acetone | 1:1 | Yes | 117 |
| Butyl Acetate | 1:2 | Yes | 108 |
| Chloroform | --- | Yes | 125 |
| Ethanol | 1:1 | Yes | 166 |
| Isobutanol | --- | --- | --- |
| Isobutyl Acetate | 1:2 | Yes | 112 |
| Isopropanol | 1:1 | Yes | 121 |
| Methyl Acetate | 1:1 | Yes | 103 |
| Ethyl Propionate | 1:1 | Yes | 122 |
| n-Butanol | 1:1 | Yes | 103 |
| n-Octanol | --- | Yes | 116 |
| n-Propanol | 1:1 | Yes | 129 |
| Propyl Acetate | 1:1 | Yes | 130 |
| Propylene Glycol | --- | Yes | 188 |
| t-Butanol | --- | --- | --- |
| Tetrahydrofuran | 1:1 | Yes | 136 |
| Toluene | 1:1 | Yes | 83 |
| t-Butyl Acetate | --- | Yes | 109 |

| | | | |
|---|---|---|---|
| ¹Defined as the extrapolated desolvation temperature from the final solvent weight loss step as determined by thermogravimetric analysis at a heating rate of 10°C/minute under nitrogen purge. Desolvation temperatures, however, can be affected by the method of manufacture of the solvate. Different methods can produce different numbers of nucleation sites capable of initiating desolvation in the solvate at lower temperatures. | | | |

The unit cell of the solvate is composed of four eplerenone molecules. The stoichiometry of the eplerenone molecules and solvent molecules in the unit cell is also reported in Table 4 above for a number of solvates. The unit cell of Form H is composed of four eplerenone molecules. The unit cell of Form L is composed of two eplerenone molecules. The solvate unit cells are converted during desolvation into Form H and/or Form L unit cells when the eplerenone molecules undergo translation and rotation to fill the spaces left by the solvent molecules. Table 4 also reports the desolvation temperatures for a number of different solvates.

### 8. Crystal Properties of Impurity Molecules

Selected impurities in eplerenone can induce the formation of Form H during the desolvation of the solvate. In particular, the effect of the following two impurity molecules was evaluated: 7-methyl hydrogen 4α,5α:9α,11α-diepoxy-17-hydroxy-3-oxo-17a-pregnane-7a,21-dicarboxylate, γ-lactone 3 (the "diepoxide"); and 7-methyl hydrogen 11α,12α-epoxy-17-hydroxy-3-oxo-17α-pregn-4-ene-7α, 21-dicarboxylate, γ-lactone 4 (the "11,12-epoxide").

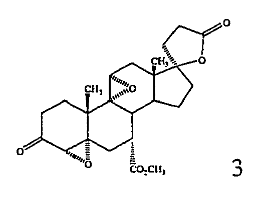

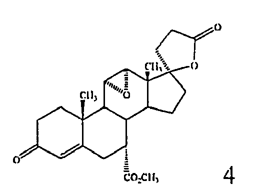

The effect of these impurity molecules on the eplerenone crystalline form resulting from desolvation is described in greater detail in the examples of this application.

Given the similarity in single crystal structure of 7-methyl hydrogen 17-hydroxy-3-oxo-17α-pregna-4,9(11)-diene-7α,21-dicarboxylate, γ-lactone 5 (the "9,11-olefin") and Form H, it is hypothesized that the 9,11-olefin also can induce the formation of Form H during the desolvation of the solvate.

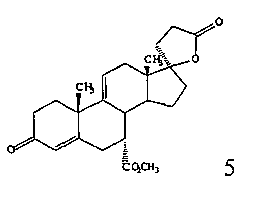

The diepoxide, 11,12-olefin and 9,11-olefin can be prepared as set forth, for example, in Examples 47C, 47B and 37H ofNg et al., WO98/25948, respectively.

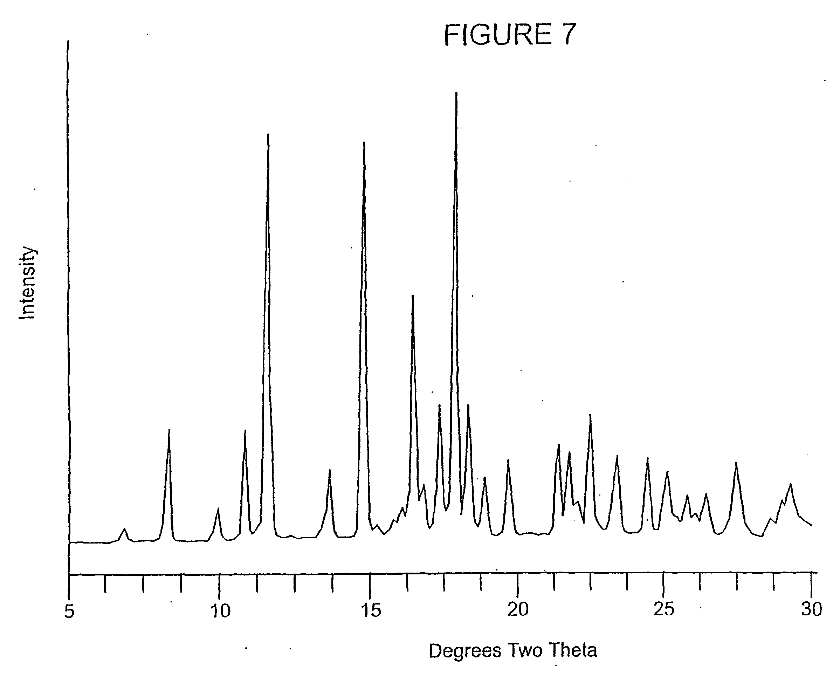

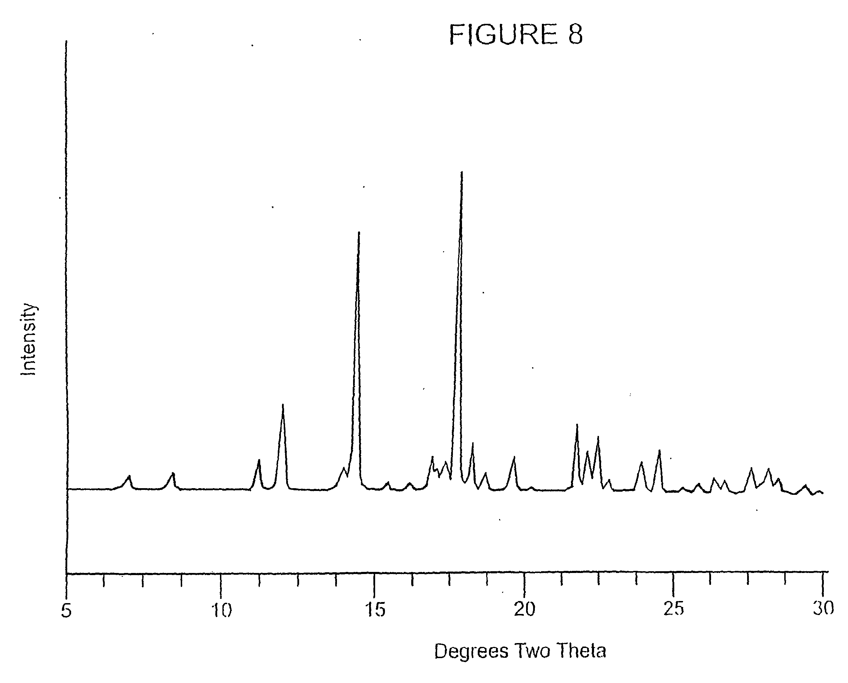

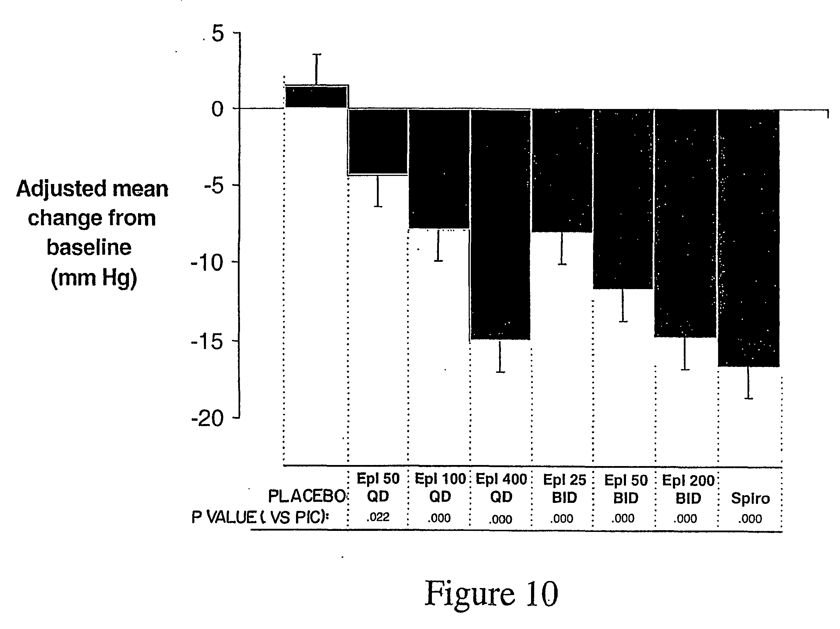

A single crystal form was isolated for each impurity compound. Representative X-ray powder diffraction patterns for the crystal forms isolated for the diepoxide, 11,12-epoxide and 9,11-olefin are given in Figs. 7, 8,and 10, respectively. The X-ray powder diffraction pattern of each impurity molecule is similar to the X-ray powder diffraction pattern of Form H, suggesting that Form H and the three impurity compounds have similar single crystal structures.

Single crystals of each impurity compound also were isolated and subjected to X-ray structure determination to verify that these three compounds adopt single crystal structures similar to that of Form H. Single crystals of the diepoxide were isolated from methyl ethyl ketone. Single crystals of the 11,12-epoxide were isolated from isopropanol. Single crystals of the 9,11-olefin were isolated from n-butanol. Crystal structure data determined for the crystalline form of each impurity compound are given in Table 5. The resulting crystal system and cell parameters were substantially the same for the Form H, diepoxide, 11,12-epoxide, and 9,11-olefin crystalline forms.

**Table 5**

| Parameter | Form H | Diepoxide | 11,12 Epoxide | 9,11 olefin |
|---|---|---|---|---|
| Crystal system | Ortho-rhombic | Ortho-rhombic | Ortho-rhombic | Ortho-rhombic |
| Space group | P2₁2₁2₁ | P2₁2₁2₁ | P2₁2₁2₁ | P2₁2₁2₁ |
| a | 21.22 Å | 21.328 Å | 20.90 Å | 20.90 Å |
| b | 15.40 Å | 16.16 Å | 15.55 Å | 15.74 Å |
| c | 6.34 Å | 6.15 Å | 6.38 Å | 6.29 Å |
| α | 90° | 90° | 90° | 90° |
| β | 90° | 90° | 90° | 90° |
| γ | 90° | 90° | 90° | 90° |
| Z | 4 | 4 | 4 | 4 |
| Volume (Å) | 2071.3 | 2119.0 | 2073.2 | 2069.3 |
| ρ (calculated) | 1.329 g/cm³ | 1.349 g/cm³ | 1.328 g/cm³ | 1.279 g/cm³ |
| R | 0.0667 | 0.0762 | 0.0865 | 0.0764 |

The four compounds reported in Table 5 crystallize into the same space group and have similar cell parameters (i.e., they are isostructural). It is hypothesized that the diepoxide, 11,12-epoxide and 9,11-olefin adopt a Form H conformation. The relative ease of isolation of a Form H packing (directly from solution) for each impurity compound, indicates that the Form H lattice is a stable packing mode for this series of structurally similar compounds.

### Preparation of Eplerenone

The eplerenone starting material used to prepare the novel crystalline forms of the present invention can be prepared using the methods set forth in Ng et al.: WO97/21720; and Ng et al.: WO98/25948, particularly scheme 1 set forth in WO97/21720 and WO98/25948.

### Preparation of Crystalline Forms

### 1. Preparation of Solvated Crystalline Form

The solvated crystalline forms of eplerenone can be prepared by crystallization of eplerenone from a suitable solvent or a mixture of suitable solvents. A suitable solvent or mixture of suitable solvents generally comprises an organic solvent or a mixture of organic solvents that solubilizes the eplerenone together with any impurities at an elevated temperature, but upon cooling, preferentially crystallizes the solvate. The solubility of eplerenone in such solvents or mixtures of solvents generally is about 5 to about 200 mg/mL at room temperature. The solvent or mixtures of solvents preferably are selected from those solvents previously used in the process to prepare the eplerenone starting material, particularly those solvents that would be pharmaceutically acceptable if contained in the final pharmaceutical composition comprising the eplerenone crystalline form. For example, a solvent system comprising methylene chloride that yields a solvate comprising methylene chloride generally is not desirable.

Each solvent used preferably is a pharmaceutically acceptable solvent, particularly a Class 2 or Class 3 solvent as defined in "Impurities: Guideline For Residual Solvents", International Conference On Harmonisation Of Technical Requirements For Registration Of Pharmaceuticals For Human Use (Recommended for Adoption at Step 4 of the ICH Process on July 17, 1997 by the ICH Steering Committee). Still more preferably, the solvent or mixture of solvents is selected from the group consisting of methyl ethyl ketone, 1-propanol, 2-pentanone, acetic acid, acetone, butyl acetate, chloroform, ethanol, isobutanol, isobutyl acetate, methyl acetate, ethyl propionate, n-butanol, n-octanol, isopropanol, propyl acetate, propylene glycol, t-butanol, tetrahydrofuran, toluene, methanol and t-butyl acetate. Still more preferably, the solvent is selected from the group consisting of methyl ethyl ketone and ethanol.

To prepare the solvated crystalline form of eplerenone, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

After the eplerenone starting material has completely solubilized in the solvent, the solution typically is cooled slowly to crystallize the solvated crystalline form of eplerenone. For most of the solvents described herein, for example, the solution is cooled at a rate slower than about 20°C/minute, preferably at a rate of about 10°C/minute or slower, more preferably at a rate of about 5°C/minute or slower, and still more preferably at a rate of about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C. Decreasing the endpoint temperature generally favors the formation of the solvated crystalline form.

Alternatively, other techniques may be used to prepare the solvate. Examples of such techniques include, but are not limited to, (i) dissolving the eplerenone starting material in one solvent and adding a co-solvent to aid in the crystallization of the solvate crystalline form, (ii) vapor diffusion growth of the solvate, (iii) isolation of the solvate by evaporation, such as rotary evaporation, and (iv) slurry converstion.

The crystals of the solvated crystalline form prepared as described above can be separated from the solvent by any suitable conventional means such as by filtration or centrifugation. Increased agitation of the solvent system during crystallization generally results in smaller crystal particle sizes.

### 2. Preparation of Form L From Solvate

Form L eplerenone can be prepared directly from the solvated crystalline form by desolvation. Desolvation can be accomplished by any suitable desolvation means such as, but not limited to, heating the solvate, reducing the ambient pressure surrounding the solvate, or combinations thereof. If the solvate is heated to remove the solvent, such as in an oven, the temperature of the solvate during this process typically does not exceed the enantiotropic transition temperature for Form H and Form L. This temperature preferably does not exceed about 150°C.

The desolvation pressure and time of desolvation are not narrowly critical. The desolvation pressure preferably is about one atmosphere or less. As the desolvation pressure is reduced, however, the temperature at which the desolvation can be carried out and/or the time of desolvation likewise is reduced. Particularly for solvates having higher desolvation temperatures, drying under vacuum will permit the use of lower drying temperatures. The time of desolvation need only be sufficient to allow for the desolvation, and thus the formation of Form L, to reach completion.

To ensure the preparation of a product that comprises substantially all Form L, the eplerenone starting material typically is a high purity eplerenone, preferably substantially pure eplerenone. The eplerenone starting material used to prepare Form L eplerenone generally is at least 90% pure, preferably at least 95% pure, and more preferably at least 99% pure. As discussed in greater detail elsewhere in this application, certain impurities in the eplerenone starting material can adversely affect the yield and Form L content of the product obtained from the process.

The crystallized eplerenone product prepared in this manner from a high purity eplerenone starting material generally comprises at least 10% Form L, preferably at least 50% Form L, more preferably at least 75% Form L, still more preferably at least 90% Form L, still more preferably at least about 95% Form L, and still more preferably substantially phase pure Form L.

### 3. Preparation of Form H From Solvate

A product comprising Form H can be prepared in substantially the same manner as set forth above for the preparation of Form L by (i) using a low purity eplerenone starting material instead of a high purity eplerenone starting material, (ii) seeding the solvent system with phase pure Form H crystals, or (iii) a combination of (i) and (ii).

### A. Use Of Impurities As Growth Promoters and Inhibitors

The presence and amount of selected impurities in the eplerenone starting material, rather than the total amount of all impurities in the eplerenone starting material, affect the potential for Form H crystal formation during the desolvation of the solvate. The selected impurity generally is a Form H growth promoter or Form L growth inhibitor. It may be contained in the eplerenone starting material, contained in the solvent or mixture of solvents before the eplerenone starting material is added, and/or added to the solvent or mixture of solvents after the eplerenone starting material is added. Bonafede et al.: J Amer Chem Soc 1995;117:30 discusses the use of growth promoters and growth inhibitors in polymorph systems and is incorporated by reference herein. For the present invention, the impurity generally comprises a compound having a single crystal structure substantially identical to the single crystal structure of Form H. The impurity preferably is a compound having an X-ray powder diffraction pattern substantially identical to the X-ray powder diffraction pattern of Form H, and more preferably is selected from the group consisting of the diepoxide, the 11,12-epoxide, the 9,11-olefin and combinations thereof.

The amount of impurity needed to prepare Form H crystals typically can depend, in part, upon the solvent or mixture of solvents and the solubility of the impurity relative to eplerenone. In the crystallization of Form H from a methyl ethyl ketone solvent, for example, the weight ratio of diepoxide to low purity eplerenone starting material typically is at least about 1:100, preferably at least about 3:100, more preferably between about 3:100 and about 1:5, and still more preferably between about 3:100 and about 1:10. The 11,12-epoxide has a higher solubility in methyl ethyl ketone than the diepoxide and generally requires a larger amount of the 11,12-epoxide generally is necessary to prepare Form H crystals. Where the impurity comprises the 11,12-epoxide, the weight ratio of the diepoxide to the low purity eplerenone starting material typically is at least about 1:5, more preferably at least about 3:25, and still more preferably between about 3:25 and about 1:5. Where both the diexpoxide and the 11,12-epoxide impurities are used in the preparation of the Form H crystals, the weight ratio of each impurity to the eplerenone starting material may be lower than the corresponding ratio when only that impurity is used in the preparation of the Form H crystals.

A mixture of Form H and Form L is generally obtained when a solvate comprising the selected impurity is desolvated. The weight fraction of Form H in the product resulting from the initial desolvation of the solvate typically is less than about 50%. Further treatment of this product by crystallization or digestion, as discussed below, generally will increase the weight fraction of Form L in the product.

### Seeding

Form H crystals also can be prepared by seeding the solvent system with phase pure Form H crystals (or a Form H growth promoter and/or Form L growth inhibitor as previously discussed above) prior to crystallization of the eplerenone. The eplerenone starting material can be either a low purity eplerenone or a high purity eplerenone. When the resulting solvate prepared from either starting material is desolvated, the weight fraction of Form H in the product typically is at least about 70% and may be as great as about 100%.

The weight ratio of Form H seed crystals added to the solvent system to the eplerenone starting material added to the solvent system generally is at least about 0.75:100, preferably between about 0.75:100 to about 1:20, and more preferably between about 1:100 to about 1:50. The Form H seed crystals can be prepared by any of the methods discussed in this application for the preparation of Form H crystals, particularly the preparation of Form H crystals by digestion as discussed below.

The Form H seed crystals may be added at one time, in multiple additions or substantially continually over a period of time. The addition of the Form H seed crystals, however, generally is completed before the eplerenone begins to crystallize from solution, i.e., the seeding is completed before the cloud point (the lower end of the metastable zone) is reached. Seeding typically is performed when the solution temperature ranges from about 0.5°C above the cloud point to about 10°C above the cloud point, preferably within about 2°C to about 3°C above the cloud point. As the temperature above the cloud point at which the seeds are added increases, the amount of seeding needed for crystallization of Form H crystals generally increases.

The seeding preferably occurs not only above the cloud point, but within the metastable zone. Both the cloud point and the metastable zone are dependent on the eplerenone solubility and concentration in the solvent or mixture of solvents. For a 12 volume dilution of methyl ethyl ketone, for example, the high end of the metastable zone generally is between about 70°C to about 73°C and the lower end of the metastable zone (i.e., the cloud point) is between about 57°C and 63°C. For a concentration of 8 volumes of methyl ethyl ketone, the metastable zone is even narrower because the solution is supersaturated. At this concentration, the cloud point of the solution occurs at about 75°C to about 76°C. Because the boiling point of methyl ethyl ketone is about 80°C under ambient conditions, seeding for this solution typically occurs between about 76.5°C and the boiling point.

An illustrative non-limiting example of seeding with Form H is set forth below in Example C-7.

The crystallized eplerenone product obtained using a Form H growth promoter or Form L growth inhibitor, and/or Form H seeding generally comprises at least 2% Form H, preferably at least 5% Form H, more preferably at least 7% Form H, and still more preferably at least about 10% Form H. The remaining crystallized eplerenone product generally is Form L.

### Form H Prepared By Grinding Eplerenone

In yet another alternative, it has been discovered that a small amount of Form H can be prepared by suitable grinding eplerenone. Concentrations of Form H in ground eplerenone as high as about 3% have been observed.

### 4. Preparation of Form L From Solvate Prepared From Low Purity Eplerenone

As discussed above, crystallization of low purity eplerenone to form a solvate followed by desolvation of the solvate generally yields a product comprising both Form H and Form L. A product having a greater Form L content can be prepared from low purity eplerenone in substantially the same manner as set forth above for the preparation of Form H by seeding the solvent system with phase pure Form L crystals, or by using a Form L growth promoter and/or Form H growth inhibitor. The seeding protocol and the weight ratio of the amount of Form L seed crystals added to the solvent system to the amount of the eplerenone starting material added to the solvent system generally are similar to those ratios previously discussed above for the preparation of Form H eplerenone by seeding with phase pure Form H crystals.

The crystallized eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

The seeding protocols described in this section and in the prior section relating to the preparation of Form H eplerenone also may allow for improved control of the particle size of the crystallized eplerenone.

### 5. Crystallization of Form L Directly From Solution

Form L eplerenone also can be prepared by the direct crystallization of eplerenone from a suitable solvent or mixture of solvents without the formation of an intermediate solvate and the accompanying need for desolvation. Typically, (i) the solvent has a molecular size that is incompatible with the available channel space in the solvate crystal lattice, (ii) the eplerenone and any impurities are soluble in the solvent at elevated temperatures, and (iii) upon cooling, results in the crystallization of the non-solvated Form L eplerenone. The solubility of eplerenone in the solvent or mixture of solvents generally is about 5 to about 200 mg/mL at room temperature. The solvent or mixture of solvents preferably comprises one or more solvents selected from the group consisting of methanol, ethyl acetate, isopropyl acetate, acetonitrile, nitrobenzene, water and ethyl benzene.

To crystallize Form L eplerenone directly from solution, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

To ensure the preparation of a product that comprises substantially phase pure Form L, the eplerenone starting material generally is a high purity eplerenone. The eplerenone starting material preferably is at least 65% pure, more preferably at least 90% pure, still more preferably at least 98% pure, and still more preferably at least 99% pure.

After the eplerenone starting material has completely solubilized in the solvent, the solution typically is cooled slowly to crystallize the solvated crystalline form of eplerenone. For most of the solvents described herein, for example, the solution is cooled at a rate slower than about 1.0°C/minute, preferably at a rate of about 0.2°C/minute or slower, and more preferably at a rate between about 5°C/minute and about 0.1°C/minute.

The endpoint temperature at which the Form L crystals are harvested will depend upon the solubility curve of the solvent or mixture of solvents. For most of the solvents described herein, for example, the endpoint temperature typically is less than about 25°C, preferably less than about 5°C, and more preferably less than about -5°C.

Alternatively, other techniques may be used to prepare the Form L crystals. Examples of such techniques include, but are not limited to, (i) dissolving the eplerenone starting material in one solvent and adding a co-solvent to aid in the crystallization of Form L eplerenone, (ii) vapor diffusion growth of Form L eplerenone, (iii) isolation of Form L eplerenone by evaporation, such as rotary evaporation, and (iv) slurry conversion.

The crystals of the solvated crystalline form prepared as described above can be separated from the solvent by any suitable conventional means such as by filtration or centrifugation.

In addition, Form L eplerenone also can be prepared by digesting (as described below) a slurry of high purity eplerenone in methyl ethyl ketone and filtering the digested eplerenone at the boiling point of the slurry.

### 6. Preparation of Form H Directly From Solution

It is hypothesized that if the crystallization is performed above the enantiotropic transition temperature (Tₜ) for Form H and Form L, particularly if Form H growth promoters or Form L growth inhibitors are present or the solvent is seeded with phase pure Form H crystals, Form H should crystallize directly from solution since Form H is more stable at these higher temperatures. The solvent system used preferably comprises a high boiling solvent such as nitrobenzene. Suitable Form H growth promoters would include, but would not be limited to, the diepoxide and the 11,12-olefin.

### 7. Digestion of Eplerenone With A Solvent

The solvated crystalline forms, Form H and Form L of eplerenone also can be prepared by digestion of an eplerenone starting material in a suitable solvent or mixture of solvents. In the digestion process, a slurry of eplerenone is heated at the boiling point of the solvent or mixture of solvents. For example, an amount of eplerenone starting material is combined with a volume of solvent or mixture of solvents, heated to reflux, and the distillate is removed while an additional amount of the solvent is added simultaneously with the removal of the distillate. Alternatively, the distillate can be condensed and recycled without the addition of more solvent during the digestion process. Typically, once the original volume of solvent has been removed or condensed and recycled, the slurry is cooled and solvated crystals form. The solvated crystals can be separated from the solvent by any suitable conventional means such as by filtration or centrifugation. Desolvation of the solvate as previously described yields either Form H or Form L eplerenone depending upon the presence or absence of the selected impurities in the solvated crystals. A suitable solvent or mixture of solvents generally comprises one or more of the solvents previously disclosed herein. The solvent may be selected, for example, from the group consisting of methyl ethyl ketone and ethanol.

The amount of eplerenone starting material added to the solvent used in the digestion process generally is sufficient to maintain a slurry (i.e., the eplerenone in the solvent or mixture of solvents is not completely solubilized) at the boiling point of the solvent or mixture of solvents. Illustrative values include, but are not limited to, about one gram of eplerenone per four mL methyl ethyl ketone and about one gram of eplerenone per eight mL ethanol.

The solution generally is cooled slowly once solvent turnover is complete to crystallize the solvated crystalline form of eplerenone. For the solvents tested, for example, the solution is cooled at a rate slower than about 20°C/minute, preferably about 10°C/minute or slower, more preferably about 5°C/minute or slower, and still more preferably about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C.

If a product comprising primarily or exclusively Form L is desired, a high purity eplerenone starting material typically is digested. The high purity eplerenone starting material preferably is at least 98% pure, more preferably at least 99% pure, and still more preferably at least 99.5% pure. The digested eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

If a product comprising primarily or exclusively Form H is desired, a low purity eplerenone starting material typically is digested. The low purity eplerenone starting material generally contains only as much Form H growth promoter and/or Form L growth inhibitor as is needed to yield Form H. Preferably, the low purity eplerenone starting material is at least 65% pure, more preferably at least 75% pure, and still more preferably at least 80% pure. The digested eplerenone product prepared in this manner generally comprises at least 10% Form H, preferably at least 50% Form H, more preferably at least 75% Form H, more preferably at least 90% Form H, still more preferably at least about 95% Form H, and still more preferably substantially phase pure Form H.

### 8. Preparation of Amorphous Eplerenone

Amorphous eplerenone can be prepared in small quantities by suitable comminution of solid eplerenone, such as by crushing, grinding and/or micronizing. Phase pure amorphous eplerenone can be prepared, for example, by lyophilizing a solution of eplerenone, particularly an aqueous solution of eplerenone. These processes are illustrated in Examples C-13 and C-18 below.

### Dosages and Treatment Regimen

The amount of the aldosterone antagonist that is administered and the dosage regimen for the uses of this invention depend on a variety of factors, including the age, weight, sex and medical condition of the subject, the severity of the pathogenic effect, the route and frequency of administration, and the particular aldosterone antagonist employed, and thus may vary widely. A daily dose administered to a subject of 0.001 to 30 mg/kg body weight, preferably between 0.005 and 20 mg/kg body weight, more preferably between 0.01 and 15 mg/kg body weight, still more preferably between 0.05 and 10 mg/kg body weight, and most preferably between 0.01 to 5 mg/kg body weight, may be appropriate. The amount of aldosterone antagonist that is administered to a human subject typically will range from 0.1 to 2000 mg, preferably from 0.5 to 500 mg, more preferably from 0.75 to 250 mg, and still more preferably from 1 to 100 mg. A daily dose of the aldosterone antagonist that produces no substantial diuretic or anti-hypertensive effect in a subject is specifically embraced by the present method. The daily dose can be administered in one to four doses per day.

Dosing of the aldosterone antagonist can be determined and adjusted based on measurement of blood pressure or appropriate surrogate markers (such as natriuretic peptides, endothelins, and other surrogate markers discussed below). Blood pressure and/or surrogate marker levels after administration of the aldosterone antagonist can be compared against the corresponding baseline levels prior to administration of the aldosterone antagonist to determine efficacy of the present method and titrated as needed. The primary surrogate markers useful in the method are surrogate markers for renal and cardiovascular disease.

In general, the degree of pathogenicity of aldosterone in individuals with an increased level of intracellular sodium (particularly human subjects having salt sensitivity and/or a high sodium intake) and the determination of the appropriate dosing of aldosterone antagonists according to the present invention initially will depend upon the presence of the then-existing pathology. Accordingly, individuals are first evaluated for hypertension, microvascular dysfunction, and pathologies associated with microvascular dysfunction. Such associated pathologies include renal and cardiac pathology, neuropathy, and retinopathy.

### Prophylatic Dosing

It is beneficial to administer the aldosterone antagonist prophylatically, particularly where the subject is susceptible to one or more pathogenic effects mediated by aldosterone in the presence of elevated sodium levels, prior to a diagnosis of said pathogenic effects and to continue administration of the aldosterone antagonist during the period of time the subject is susceptible to the pathogenic effects. Individuals with no remarkable clinical presentation but that are nonetheless susceptible to pathologic effects therefore can be placed upon a prophylatic dose of aldosterone antagonist. Such prophylactic doses of the aldosterone antagonist may, but need not, be lower than the doses used to treat the specific pathogenic effect of interest.

### Renal Pathology Dosing

Dosing to treat pathologies of renal function can be determined and adjusted based on measurement of proteinuria, microalbuminuria, decreased glomerular filtration rate (GFR), or decreased creatinine clearance. Proteinuria is identified by the presence of greater than 0.3 g of urinary protein in a 24 hour urine collection. Microalbuminuria is identified by an increase in immunoassayable urinary albumin. Based upon such measurements, dosing of the aldosterone antagonist can be adjusted to reduce the renal pathologic effect.

### Dosing Based on Plasma Renin or Serum Aldosterone Levels

In general, subjects treated according to the present invention are initially dosed with an amount of one or more aldosterone antagonists during an initial evaluation period (i.e. the period during which a subject receives one or more aldosterone antagonists at an initial daily dose). The initial evaluation period may be about one to four weeks, preferably about one to two weeks, in duration. After the initial evaluation period, blood and urine samples are obtained for routine evaluation (i.e., commonly known as blood and urine chemistries). If there are no contraindications to a dose increase (e.g. hyperkalemia), the daily dose of one or more aldosterone antagonists will be increased, if necessary.

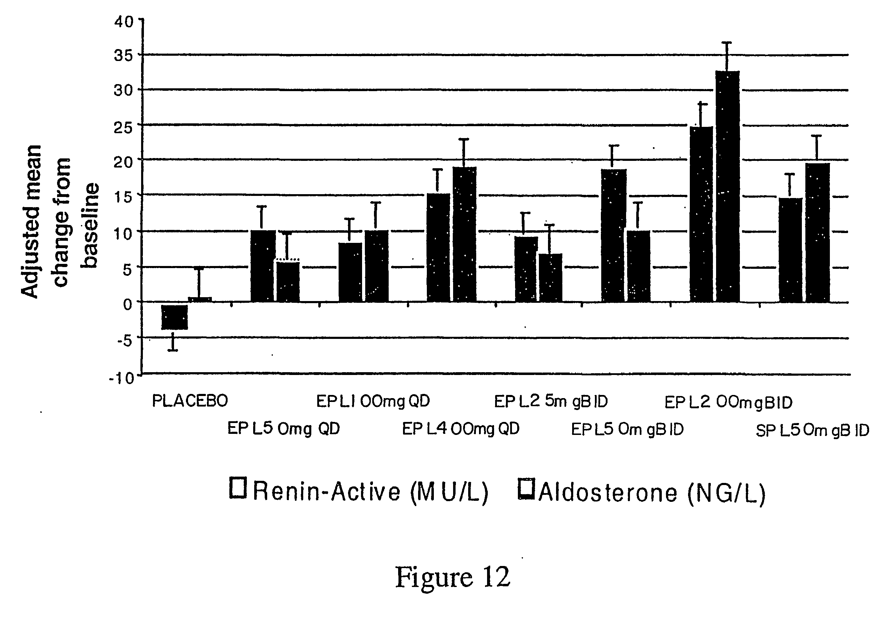

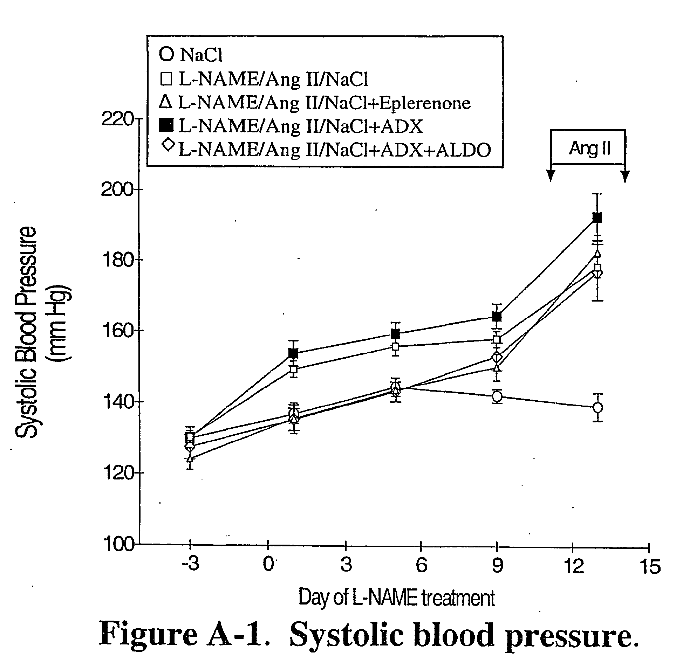

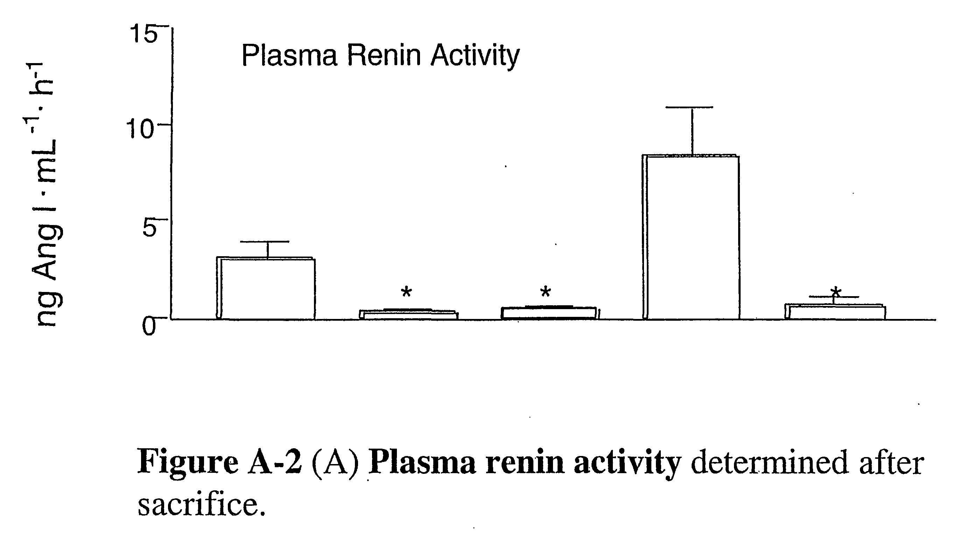

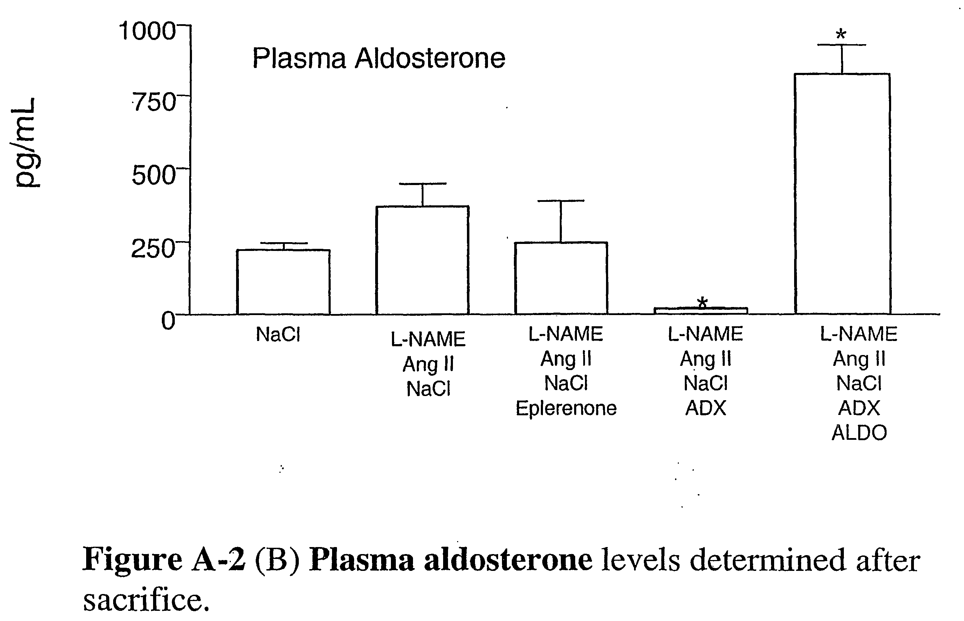

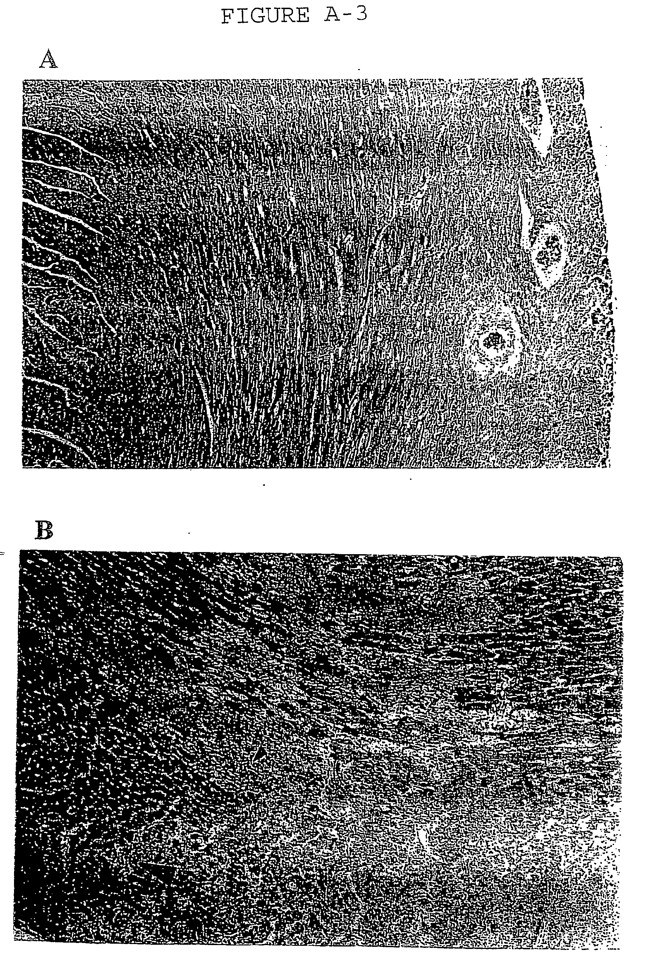

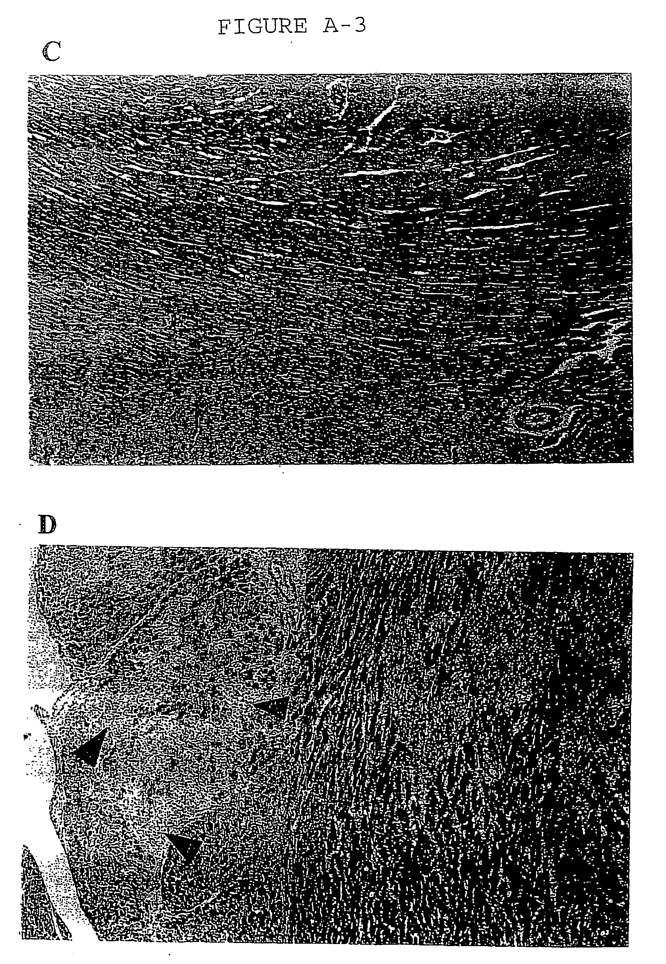

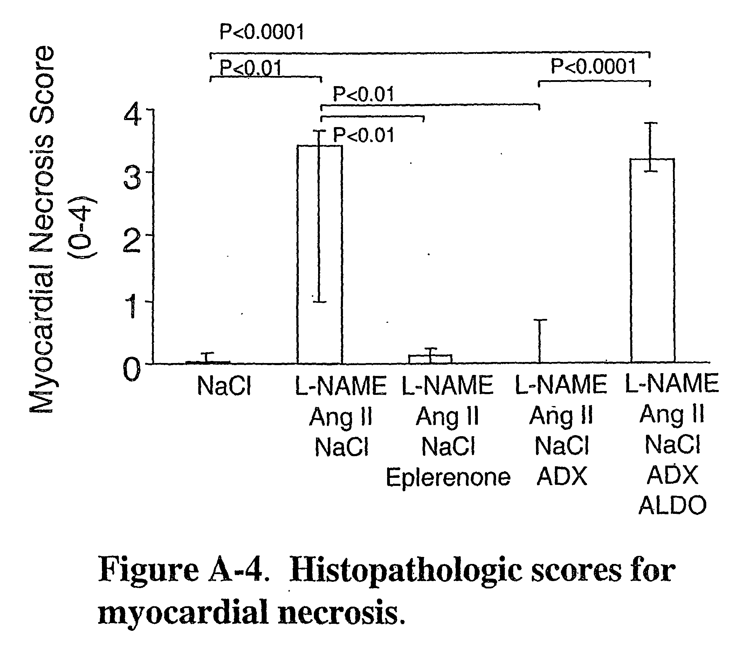

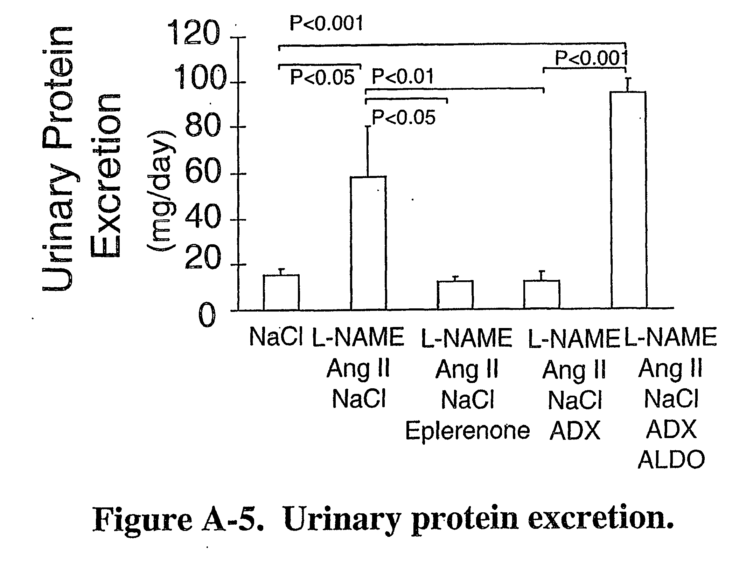

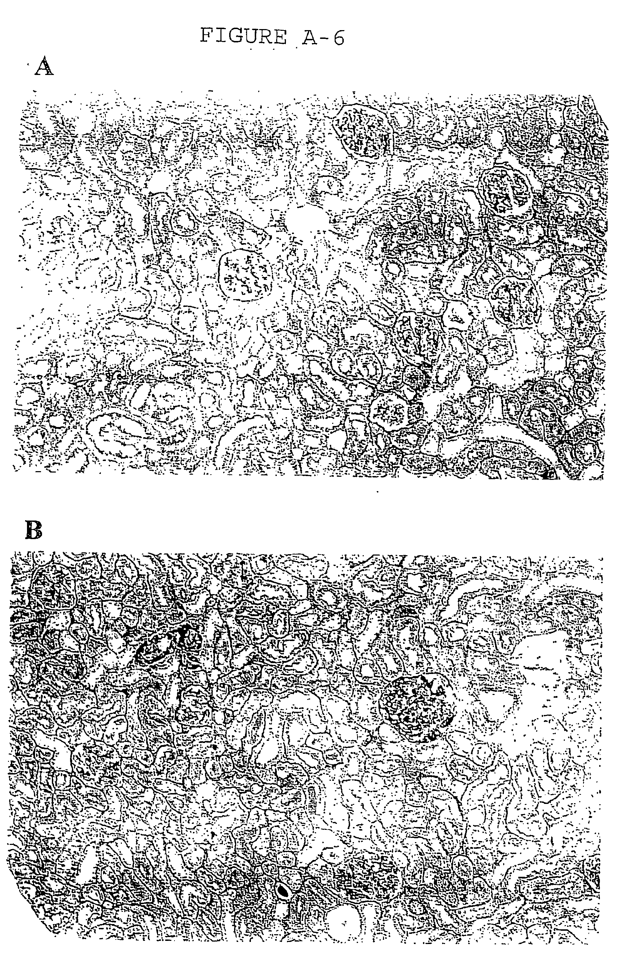

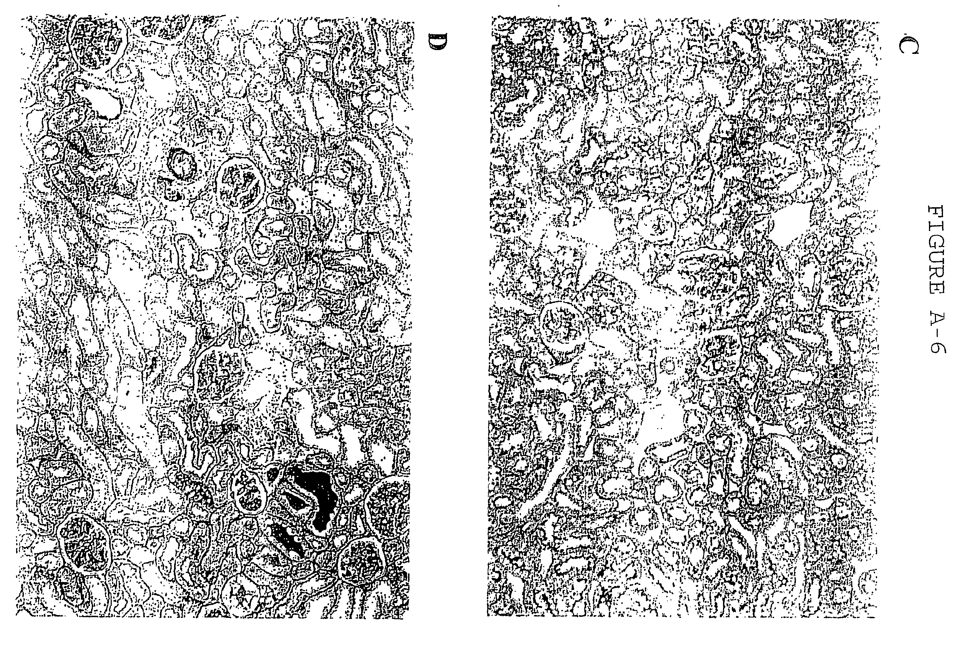

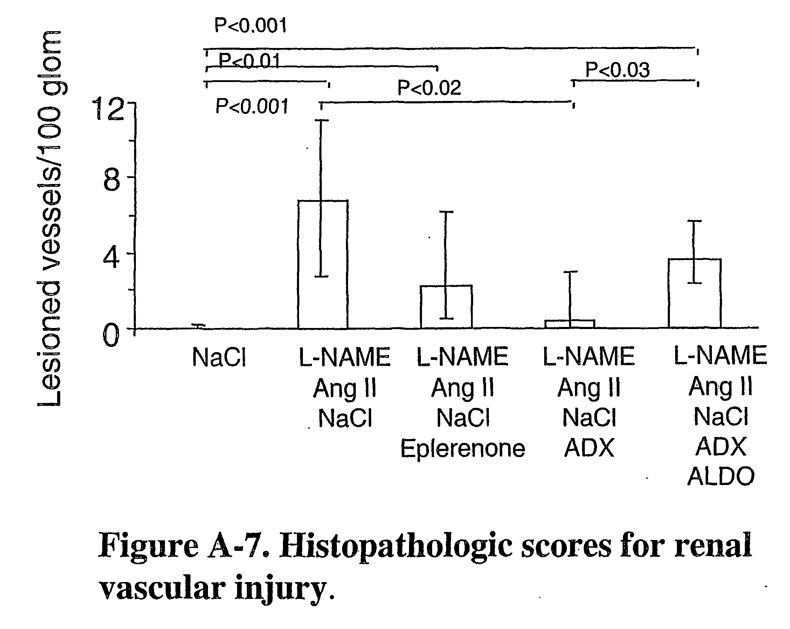

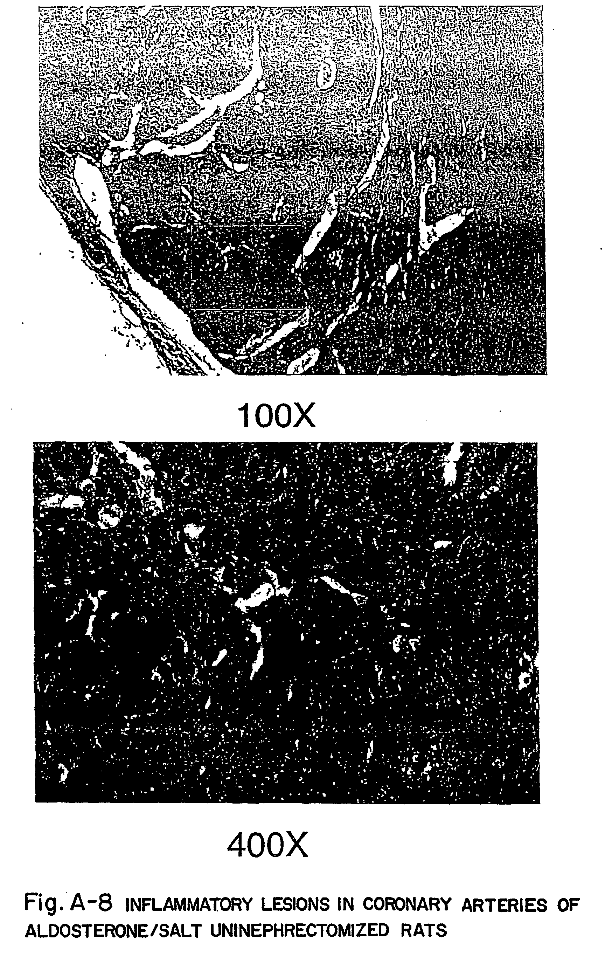

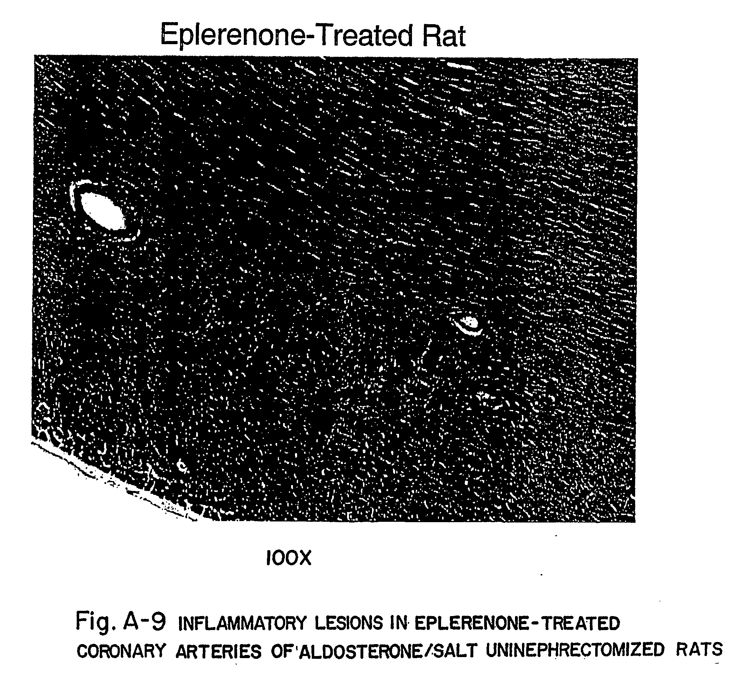

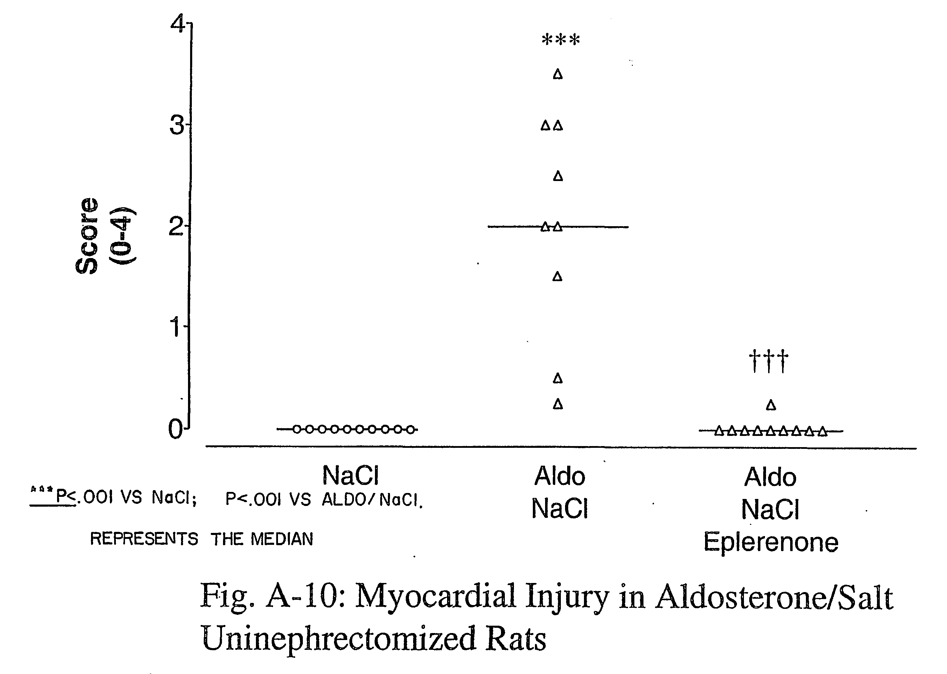

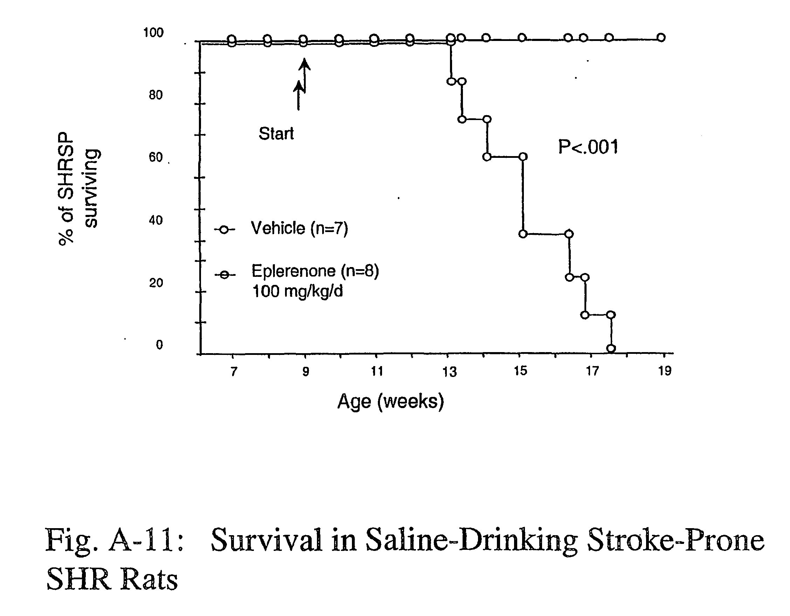

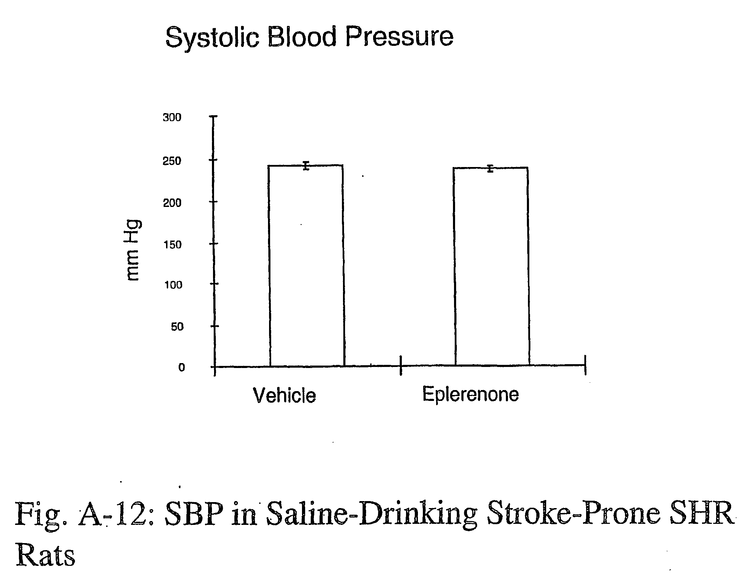

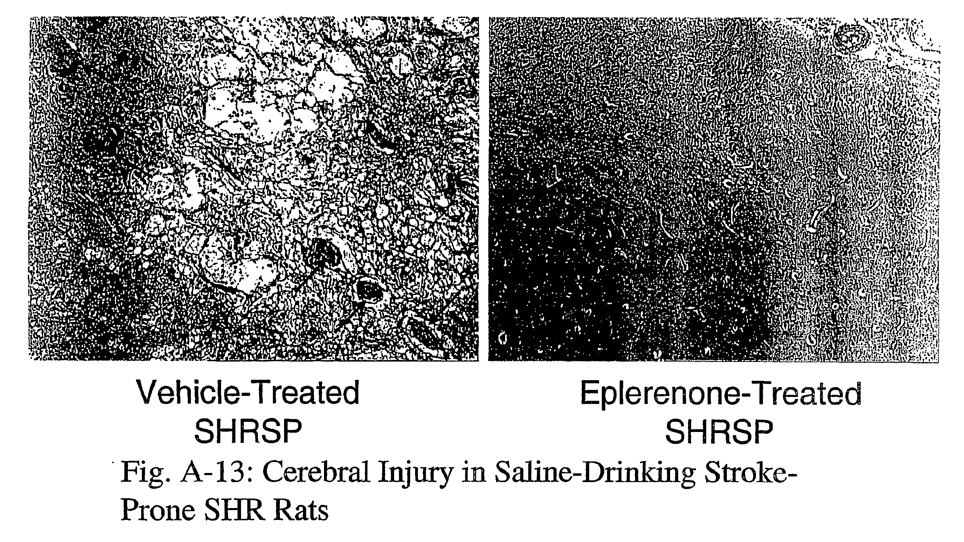

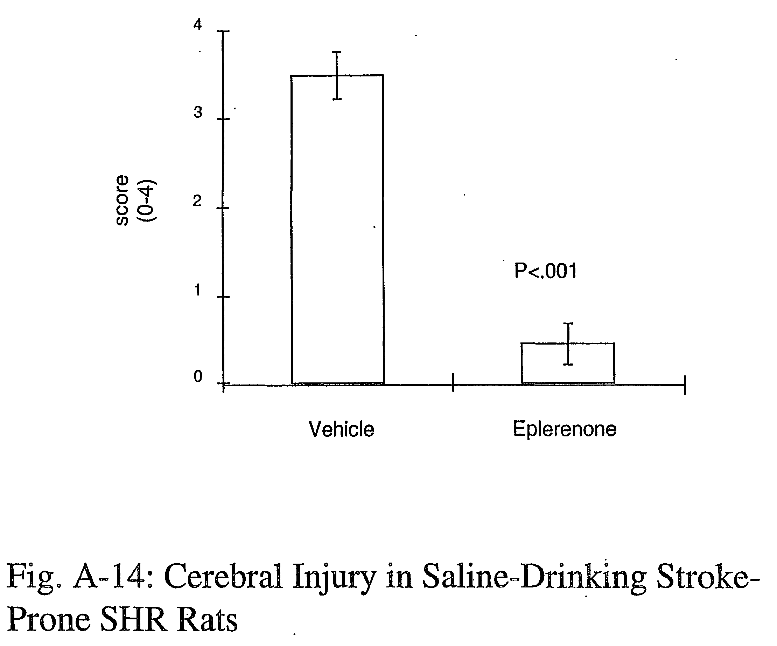

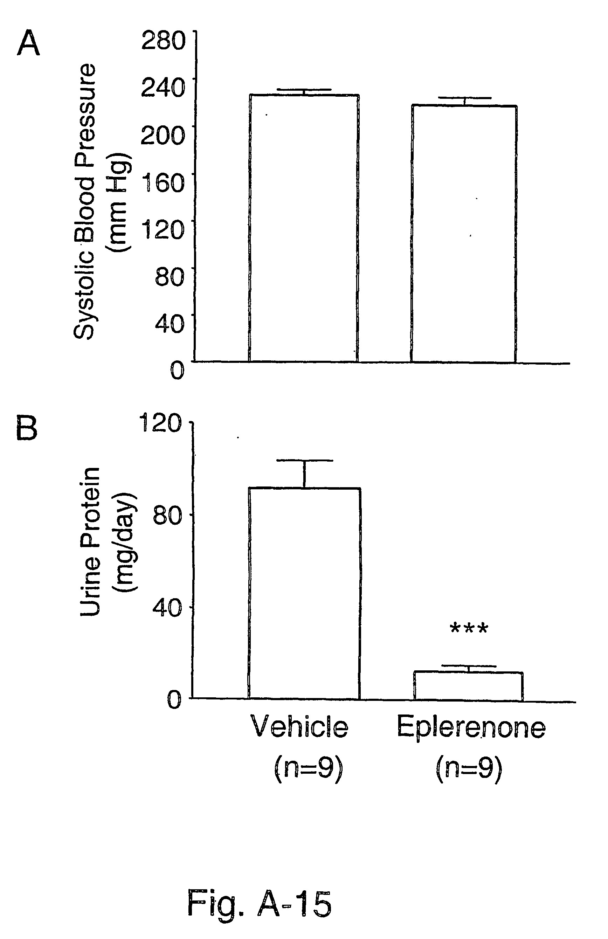

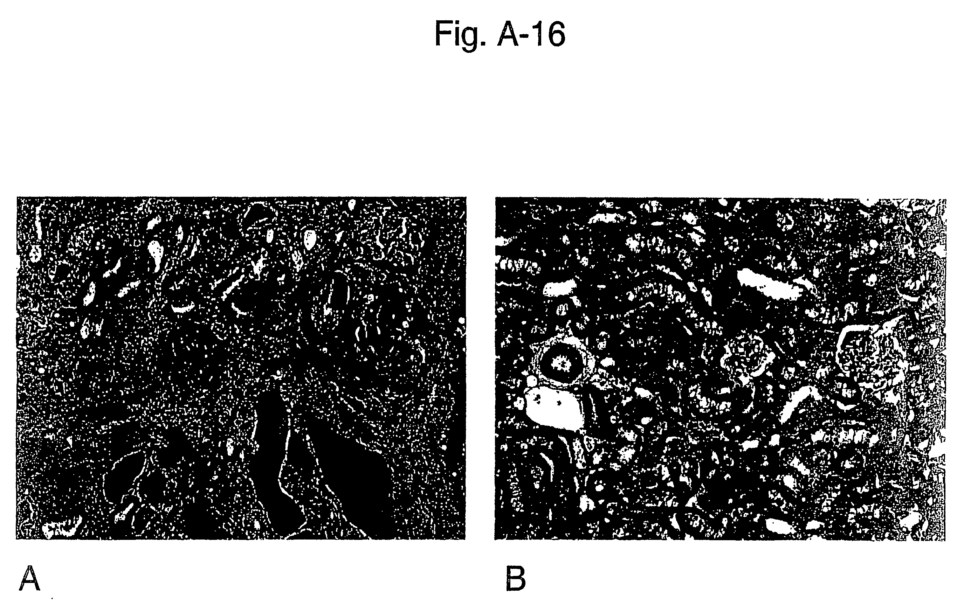

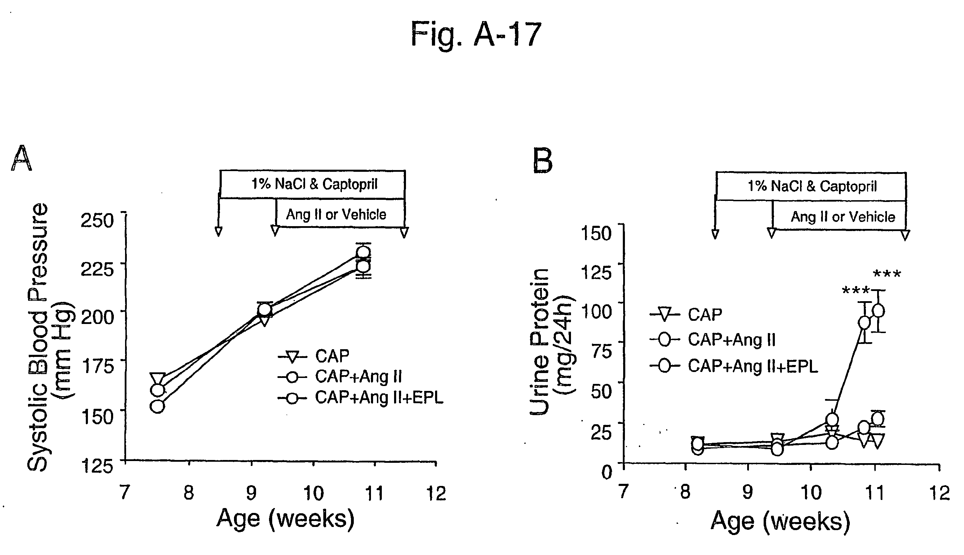

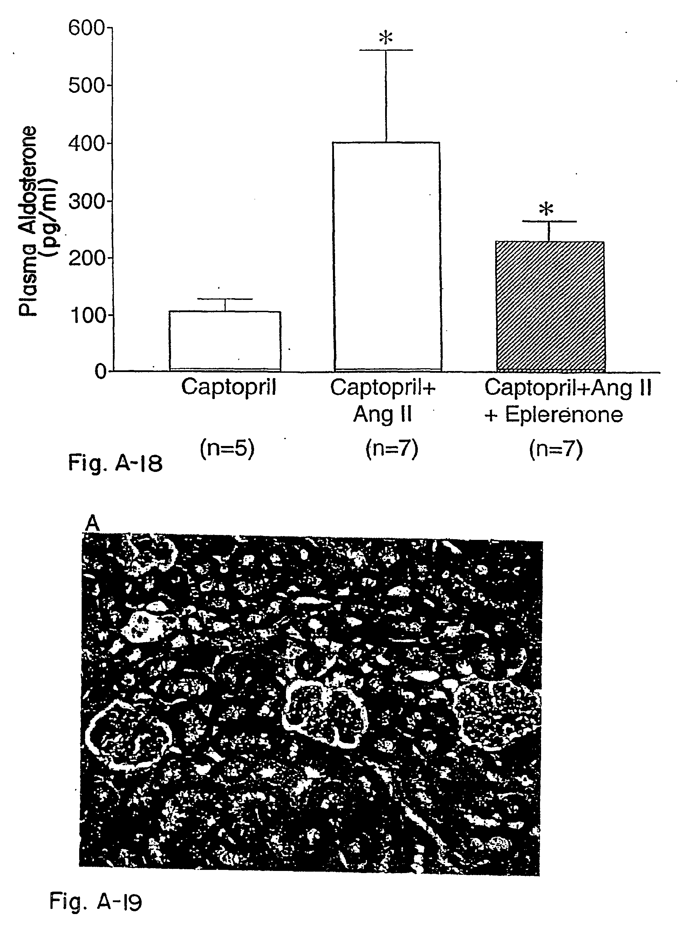

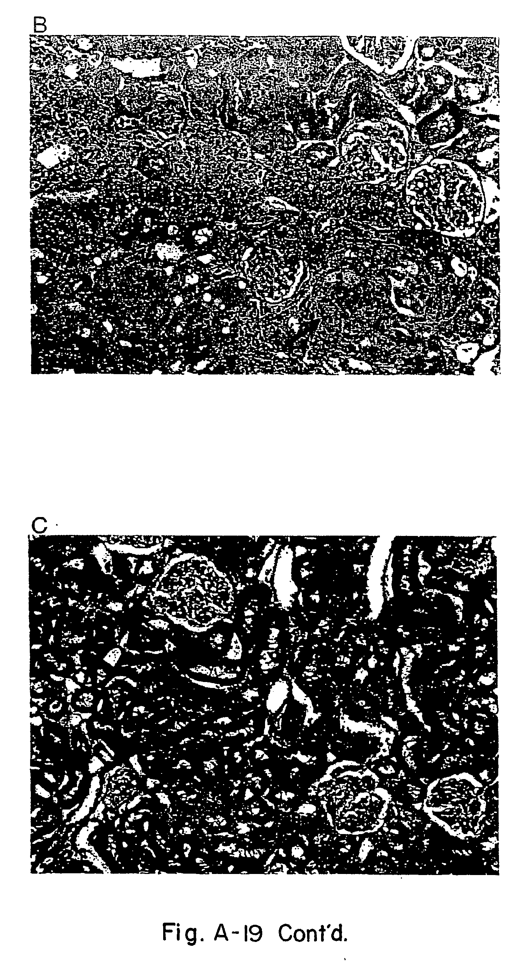

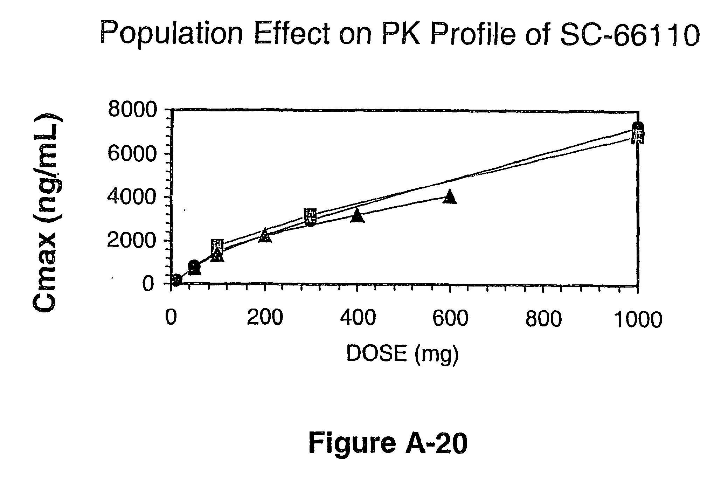

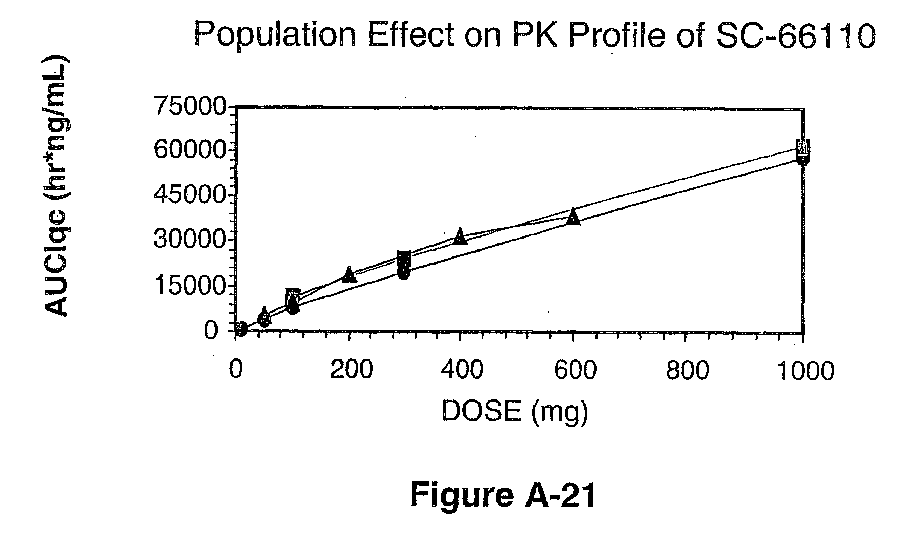

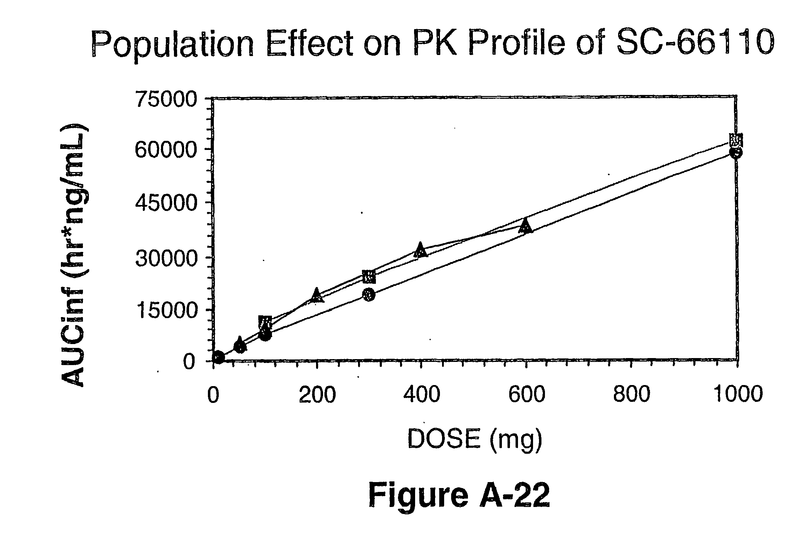

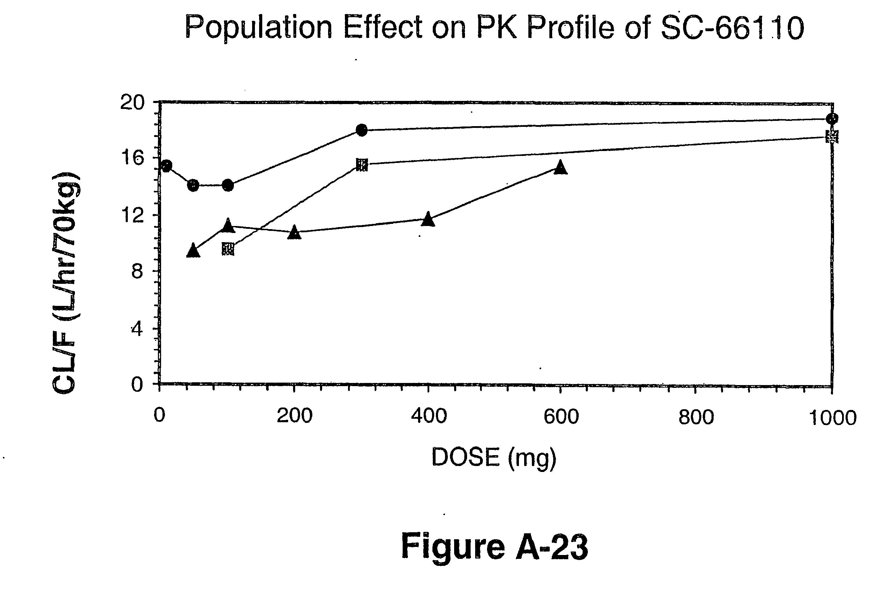

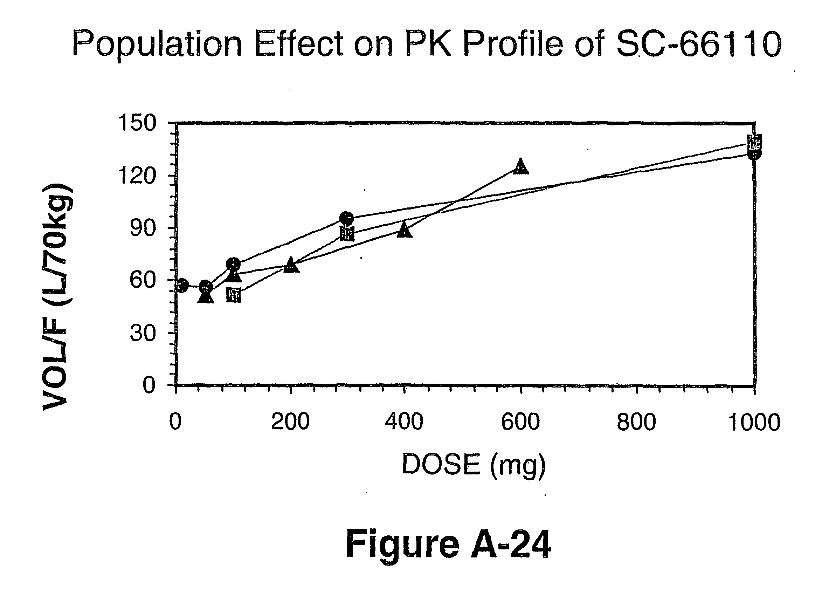

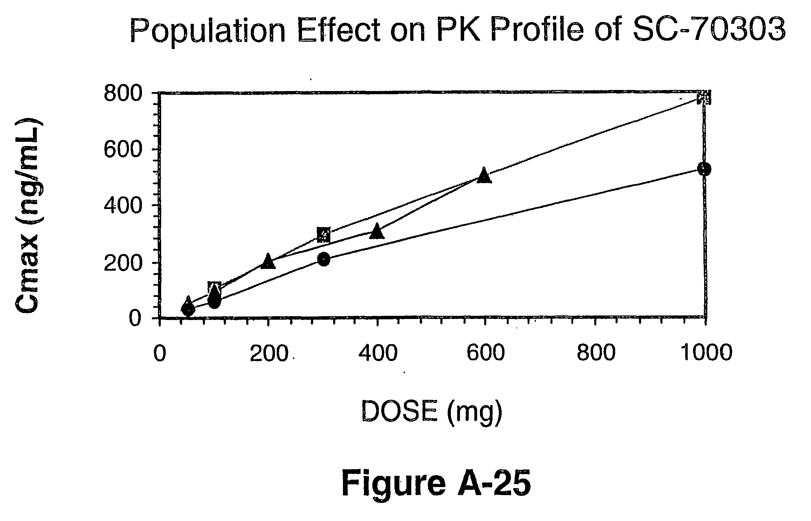

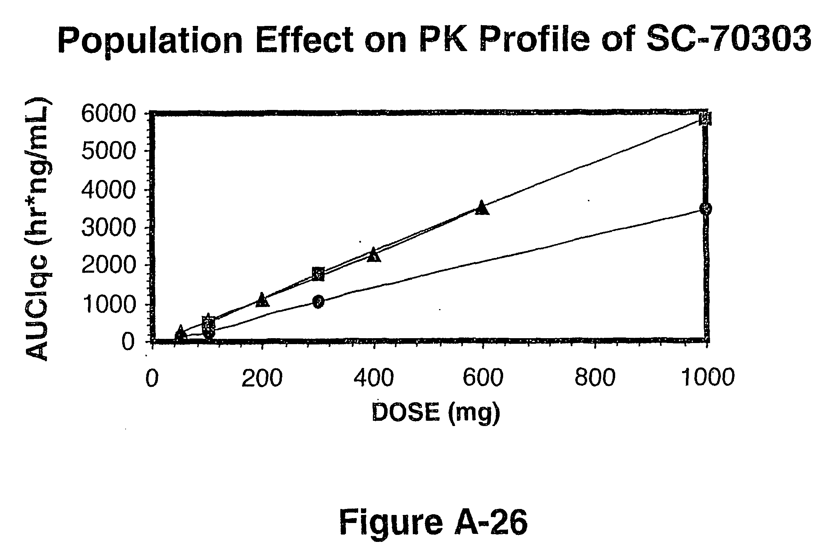

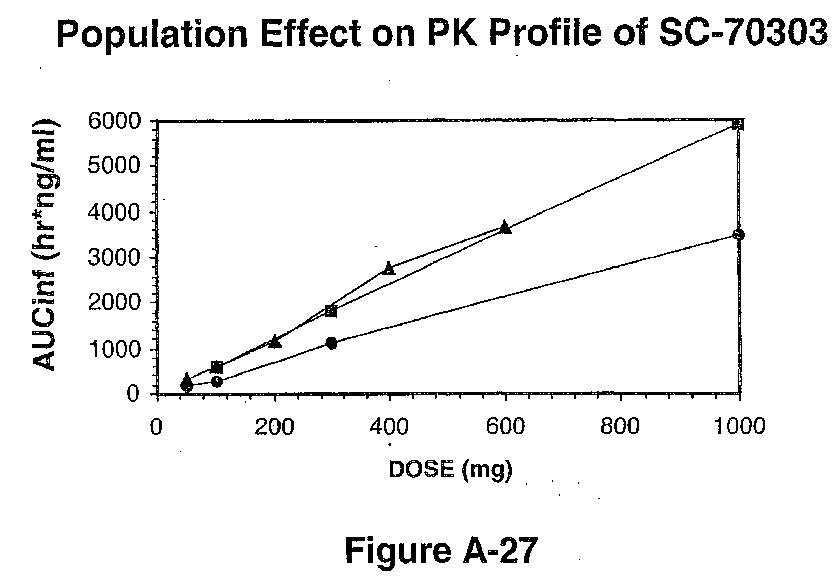

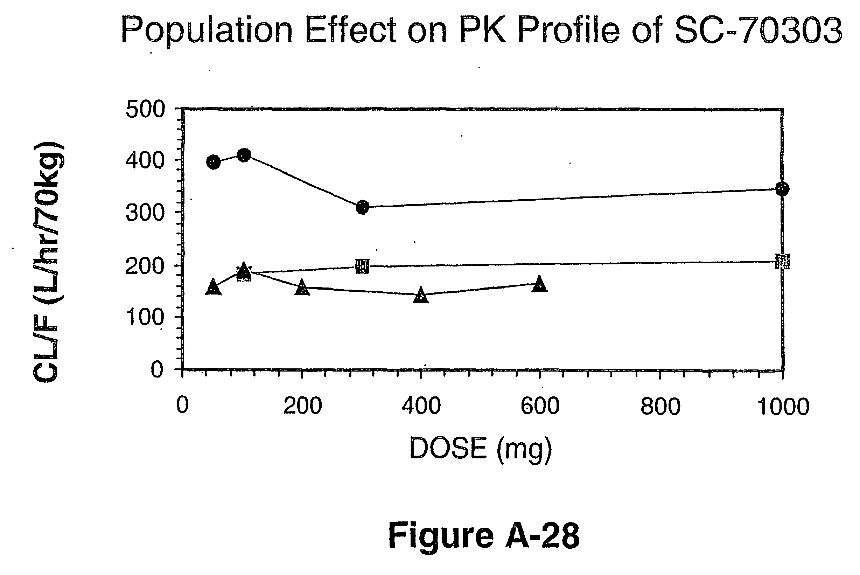

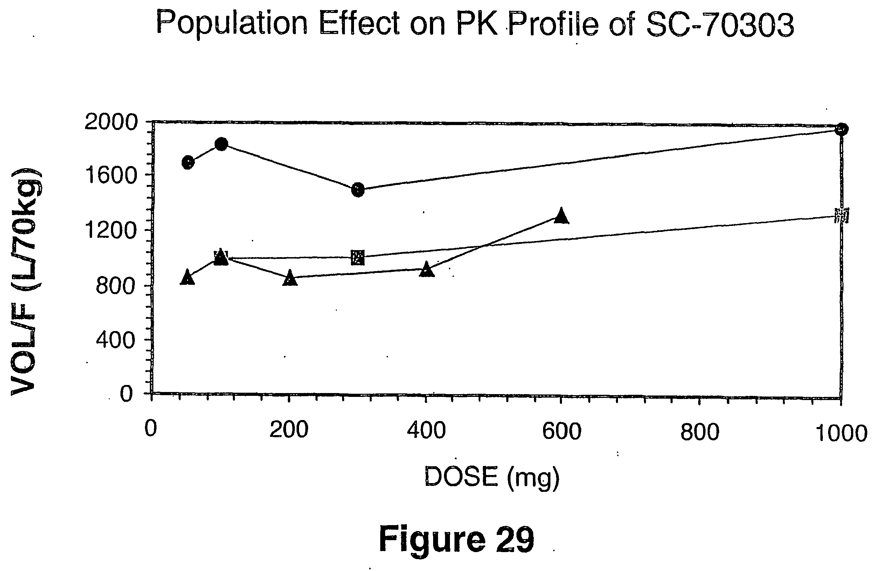

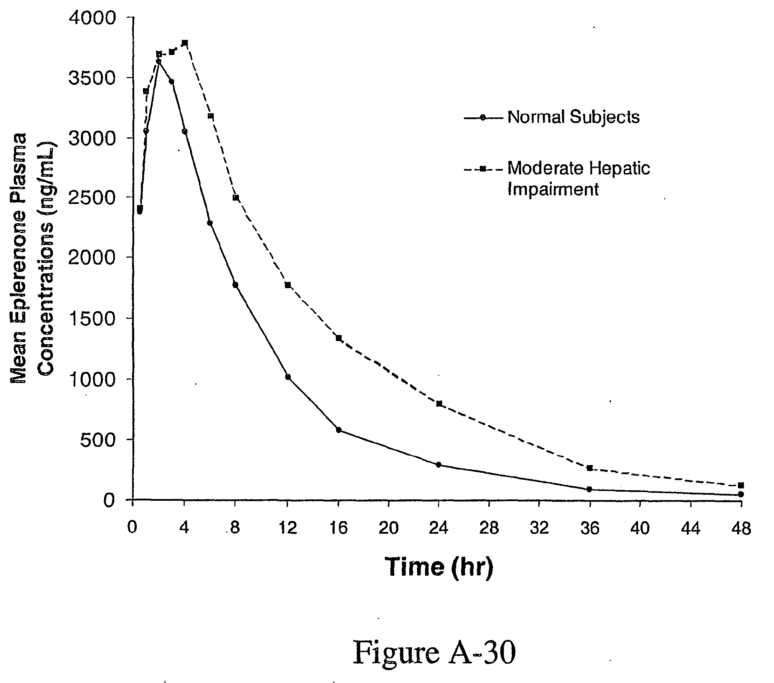

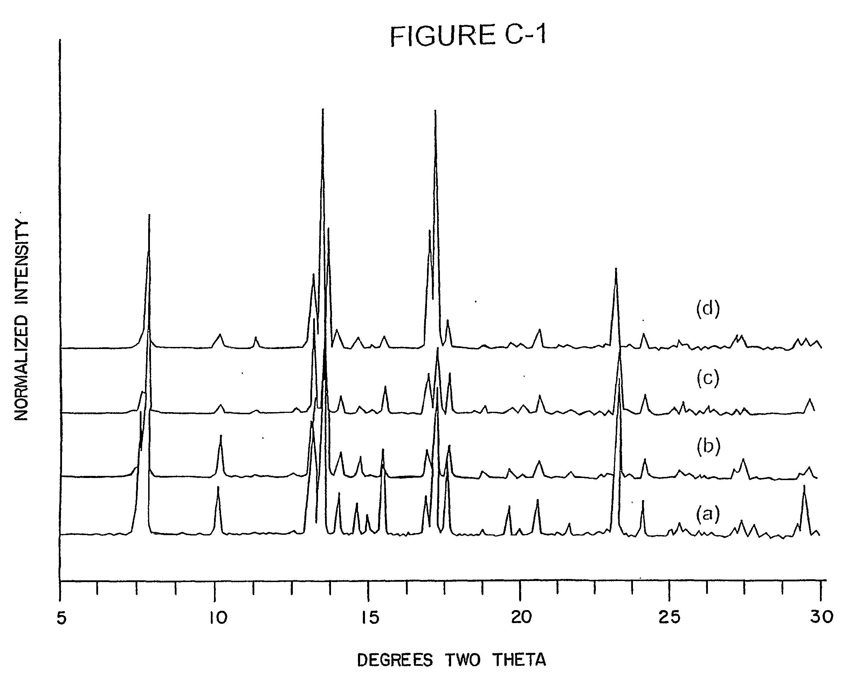

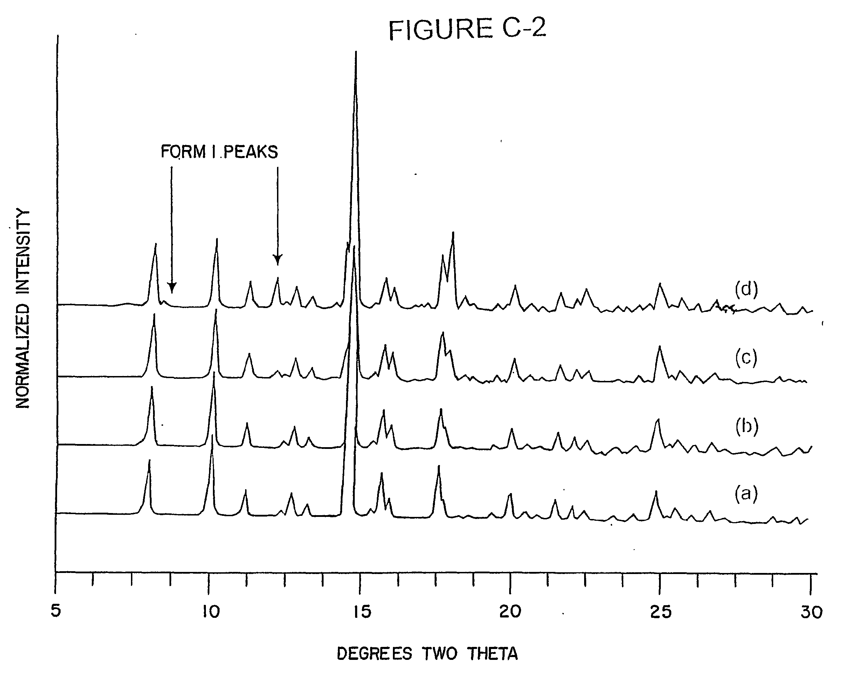

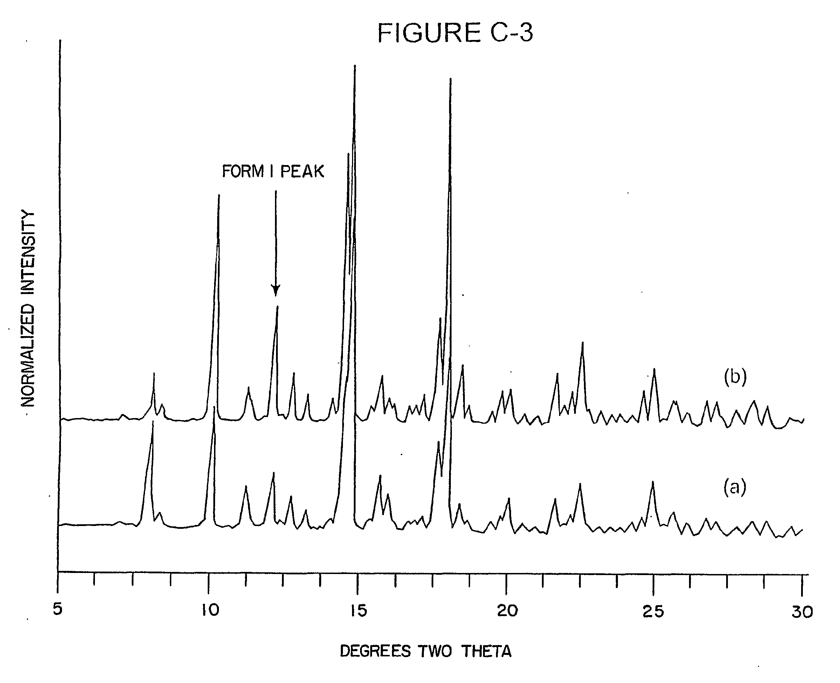

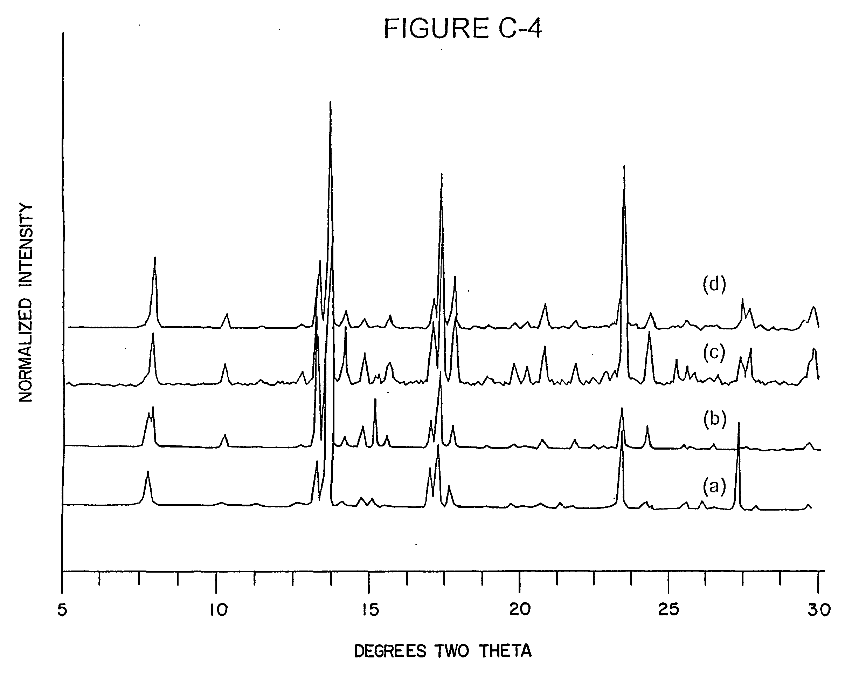

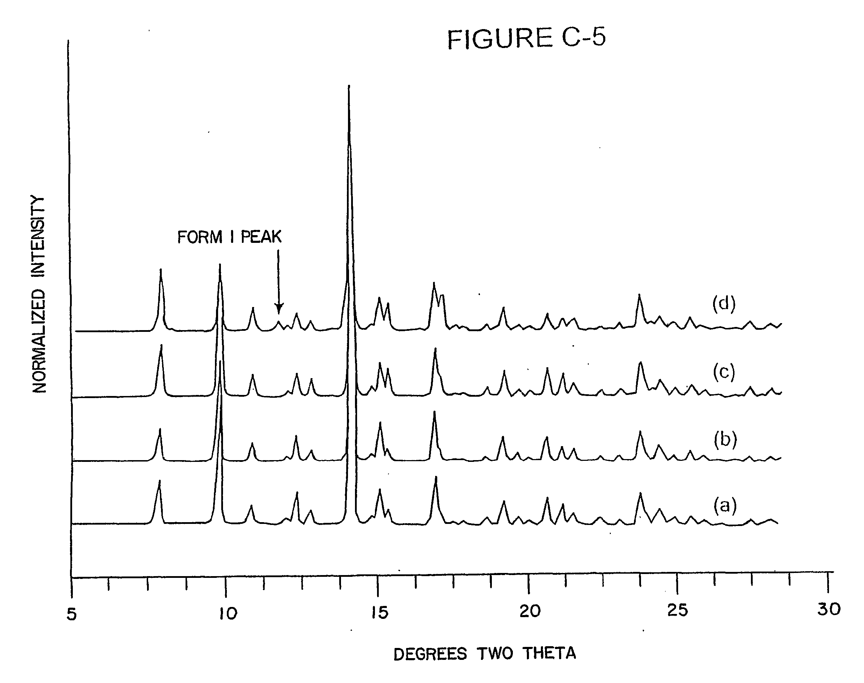

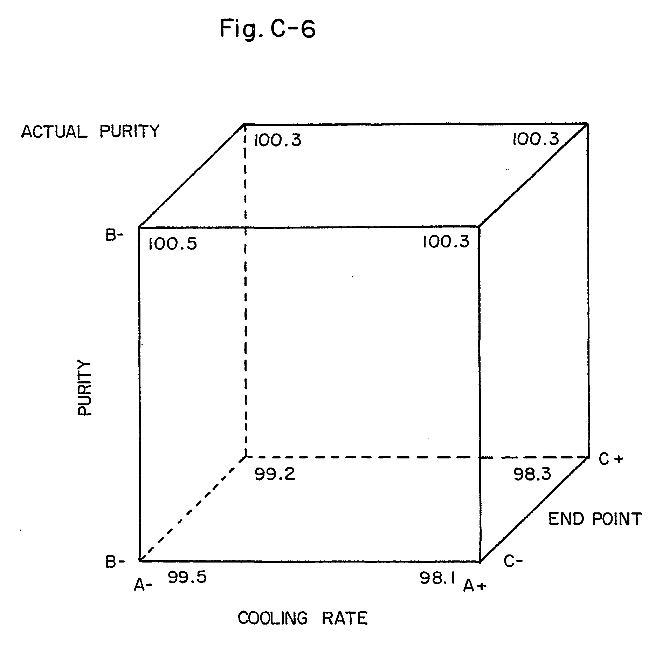

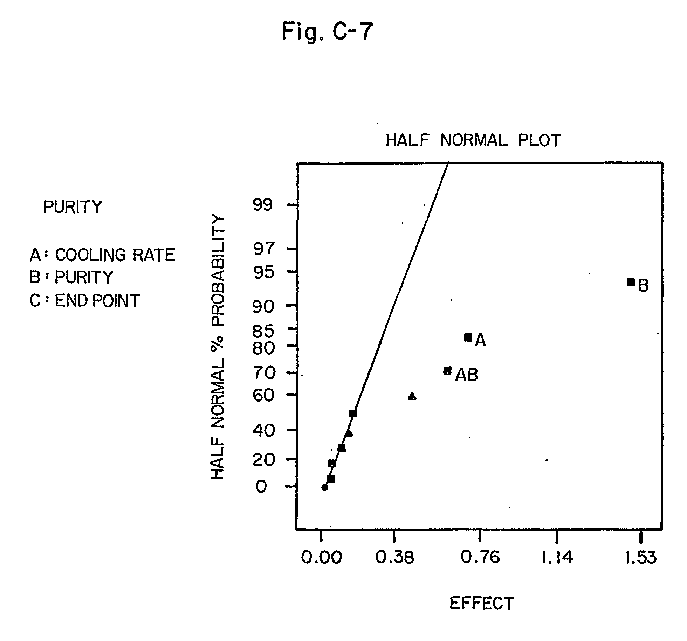

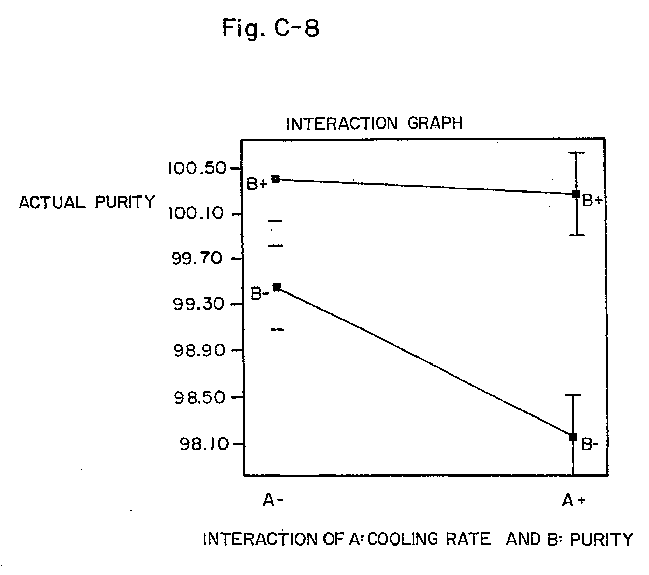

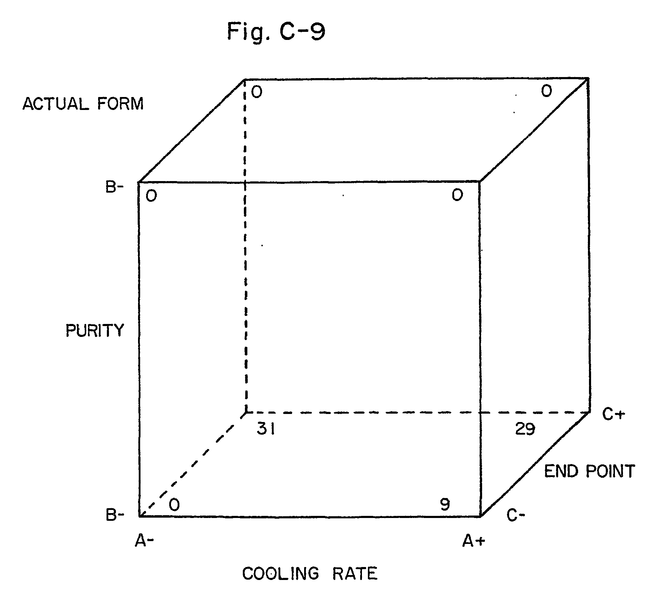

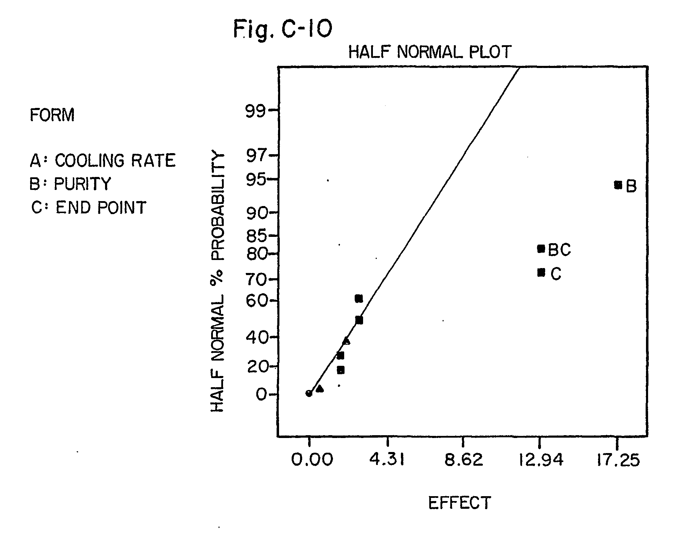

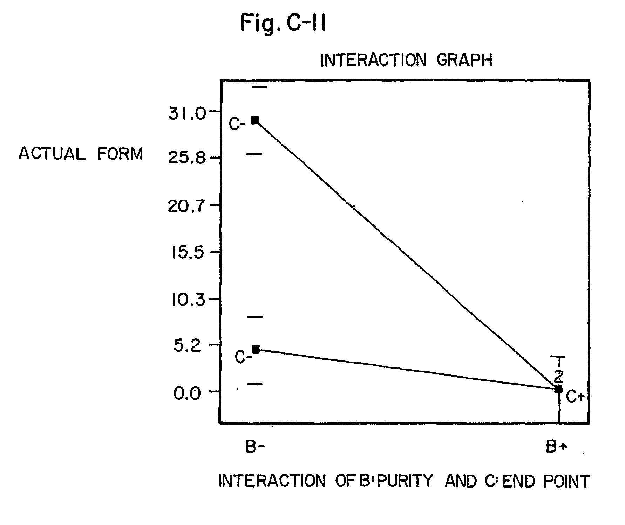

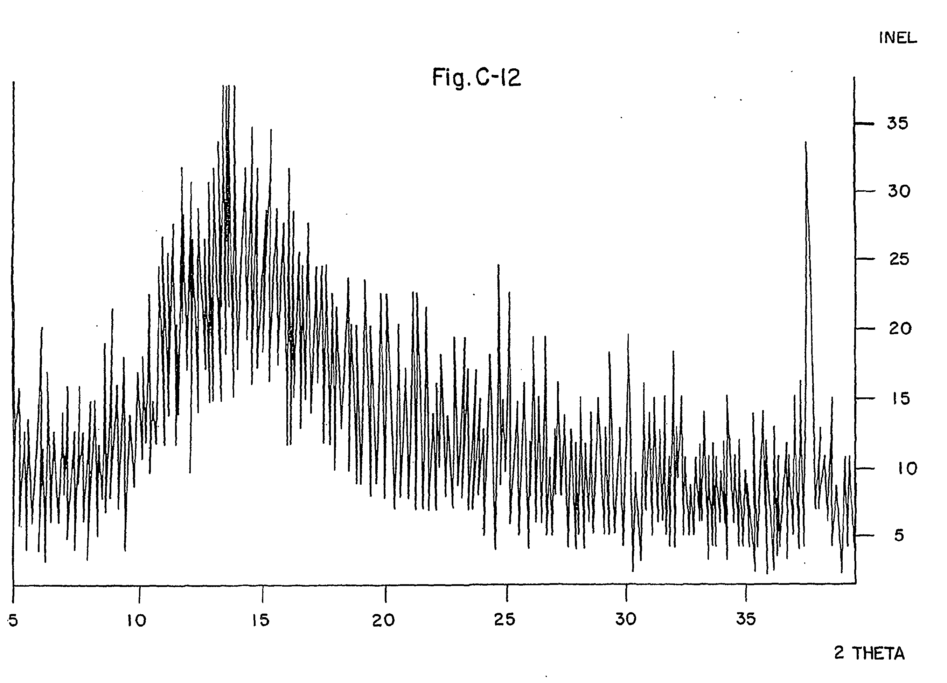

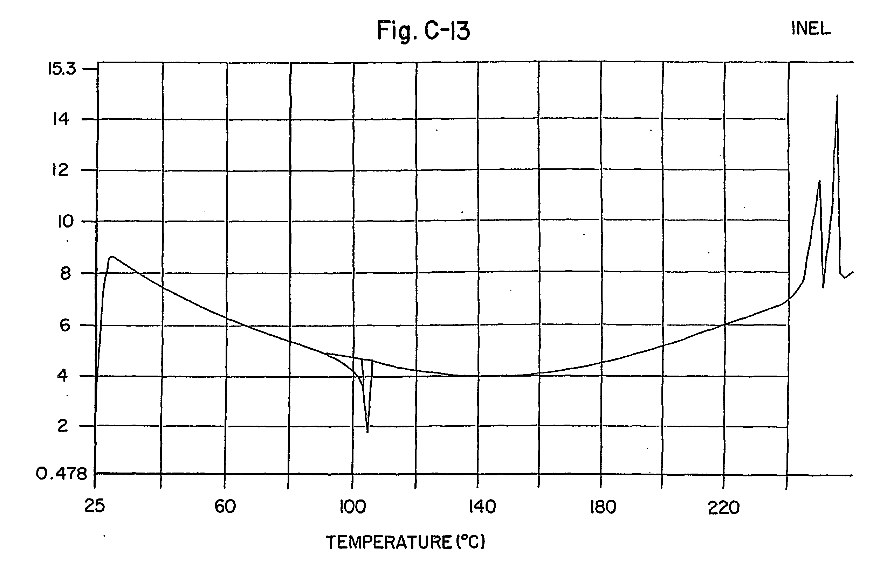

Appropriate dosing can also be determined by monitoring plasma renin activity. As shown in Figure 12, increasing doses of eplerenone result in increased levels of plasma renin activity. Accordingly, subjects can be treated with doses of one or more aldosterone antagonists according to the present invention by increasing doses of such compounds in a step-wise manner until the desired level of plasma renin activity is achieved while, at the same time, maintaining serum levels of potassium within the normal range.

Appropriate dosing can also be determined by monitoring serum aldosterone levels. As shown in Figure 12, increasing doses of eplerenone result in increased levels of serum aldosterone. Accordingly, subjects can be treated with doses of one or more aldosterone antagonists according to the present invention by increasing doses of such compounds in a step-wise manner until the desired level of serum aldosterone is achieved while, at the same time, also maintaining serum levels of potassium within the normal range.

### Pharmaceutical Compositions

Administration may be accomplished by any appropriate route such as oral administration, or administration by intravenous, intramuscular or subcutaneous injections.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. A suitable daily dose for a mammal may vary widely depending on the condition of the patient and other factors.

Similarly, the active ingredients may be administered by injection as a composition wherein, for example, saline, dextrose or water may be used as a suitable carrier. The formulation may be in the form of a bolus, or in the form of aqueous or non-aqueous isotonic sterile injection solutions or suspensions. These solution and suspensions may be prepared from sterile powders or granules having one or more pharmaceutically-acceptable carriers or diluents, or a binder such as gelatin or hydroxypropyl-methyl cellulose, together with one or more of a lubricant, preservative, surface-active or dispersing agent.

The term "pharmaceutically acceptable" is used adjectivally herein to mean that the modified noun is appropriate for use in a pharmaceutical product. Pharmaceutically acceptable cations include metallic ions and organic ions. More preferred metallic ions include, but are not limited to appropriate alkali metal salts, alkaline earth metal salts and other physiologically acceptable metal ions. Exemplary ions include aluminum, calcium, lithium, magnesium, potassium, sodium and zinc in their usual valences. Preferred organic ions include protonated tertiary amines and quaternary ammonium cations, including in part, trimethylamine, diethylamine, N,N'-dibenzy 1 ethylenediamine, chloroprocaine, choline, diethanolamine, ethylenediamine, meglumine (N-methylglucamine) and procaine. Exemplary pharmaceutically acceptable acids include without limitation hydrochloric acid, hydrobromic acid, phosphoric acid, sulfuric acid, methanesulfonic acid, acetic acid, formic acid, tartaric acid, maleic acid, malic acid, citric acid, isocitric acid, succinic acid, lactic acid, gluconic acid, glucuronic acid, pyruvic acid, oxalacetic acid, fumaric acid, propionic acid, aspartic acid, glutamic acid, benzoic acid, and the like.

For therapeutic purposes, the active component of this invention is ordinarily combined with one or more adjuvants appropriate to the indicated route of administration. If administered per os, the components may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The components may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

### Combination Therapies

The uses of the present invention may further comprise the administration of other active ingredients or therapies in combination with the administration of the aldosterone antagonist.

For example, the aldosterone antagonist can be administered to the subject in combination with other active drugs used in the treatment of hypertension and associated cardiovascular and renal conditions and disorders. The active drugs administered with the aldosterone antagonist can include, for example, the drugs selected from the group consisting of renin inhibitors, angiotensin II antagonists, ACE inhibitors, diuretics having no substantial aldosterone antagonist effect, and retinoic acid. The phrase "combination therapy" (or "co-therapy"), when used with respect to drug combinations, is intended to embrace the administration of each agent in a sequential manner in a regimen that will provide beneficial effects of the drug combination, and is intended as well to embrace co-administration of these agents in a substantially simultaneous manner, such as in a single capsule or injection having a fixed ratio of these active agents or in multiple, separate capsules or injections for each agent.

The phrase "angiotensin II antagonist" includes, for examples, those angiotensin II antagonists described in WO96/40257.

The phrase "angiotensin converting enzyme inhibitor" ("ACE inhibitor") includes an agent or compound, or a combination of two or more agents or compounds, having the ability to block, partially or completely, the enzymatic conversion of the decapeptide form of angiotensin ("angiotensin I") to the vasoconstrictive octapeptide form of angiotensin ("angiotensin II"). Blocking the formation of angiotensin II can affect the regulation of fluid and electrolyte balance, blood pressure and blood volume by removing the primary actions of angiotensin II. Included in these primary actions of angiotensin II are stimulation of the synthesis and secretion of aldosterone receptor by the adrenal cortex and raising blood pressure by direct constriction of the smooth muscle of the arterioles.

Examples of ACE inhibitors that can be used in the combination therapy include, but are not limited to, the following compounds: AB-103, ancovenin, benazeprilat, BRL-36378, BW-A575C, CGS-13928C, CL-242817, CV-5975, Equaten, EU-4865, EU-4867, EU-5476, foroxymithine, FPL 66564, FR-900456, Hoe-065,15B2, indolapril, ketomethylureas, KRI-1177, KRI-1230, L-681176, libenzapril, MCD, MDL-27088, MDL-27467A, moveltipril, MS-41, nicotianamine, pentopril, phenacein, pivopril, rentiapril, RG-5975, RG-6134, RG-6207, RGH-0399, ROO-911, RS-10085-197, RS-2039, RS 5139, RS 86127, RU-44403, S-8308, SA-291, spiraprilat, SQ-26900, SQ-28084, SQ-28370, SQ-28940, SQ-31440, Synecor, utibapril, WF-10129, Wy-44221, Wy-44655, Y-23785, Yissum P-0154, zabicipril, Asahi Brewery AB-47, alatriopril, BMS 182657, Asahi Chemical C-111, Asahi Chemical C-112, Dainippon DU-1777, mixanpril, Prentyl, zofenoprilat, 1-(-(1-carboxy-6-(4-piperidinyl)hexyl)amino)-1-oxopropyl octahydro-1H-indole-2-carboxylic acid, Bioproject BP1.137, Chiesi CHF 1514, Fisons FPL-66564, idrapril, Marion Merrell Dow MDL-100240, perindoprilat and Servier S-5590, alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

A group of ACE inhibitors of particular interest consists of alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

Many of these ACE inhibitors are commercially available. For example, a highly preferred ACE inhibitor, captopril, is sold by E.R. Squibb & Sons, Inc., Princeton, N.J., now part of Bristol-Myers-Squibb, under the trademark "CAPOTEN", in tablet dosage form at doses of 12.5 mg, 50 mg and 100 mg per tablet. Enalapril or Enalapril Maleate, and Lisinopril are two more highly preferred ACE inhibitors sold by Merck & Co, West Point, Pa. Enalapril is sold under the trademark "VASOTEC" in tablet dosage form at doses of 2.5 mg, 5 mg, 10 mg and 20 mg per tablet. Lisinopril is sold under the trademark "PRINIVIL" in tablet dosage form at doses of 5 mg, 10 mg, 20 mg and 40 mg per tablet.

The diuretic may be selected from several known classes, such as thiazides and related sulfonamides, potassium-sparing diuretics, loop diuretics and organic mercurial diuretics. Nonlimiting examples of thiazides are bendroflumethiazide, benzthiazide, chlorothiazide, cyclothiazide, hydrochlorothiazide, hydroflumethiazide, methylclothiazide, polythiazide and trichlormethiazide. Nonlimiting examples of sulfonamides related to thiazides are chlorthalidone, quinethazone and metolazone. Nonlimiting examples of potassium-sparing diuretics are triameterene and amiloride. Nonlimiting examples of loop diuretics, i.e., diuretics acting in the ascending limb of the loop of Henle of the kidney, are furosemide and ethynacrylic acid. Nonlimiting examples of organic mercurial diuretics are mercaptomerin sodium, merethoxylline, procaine and mersalyl with theophylline.

In one embodiment, the combination therapy comprises administering an ACE inhibitor, an aldosterone antagonist, and a loop diuretic having no substantial aldosterone antagonistic activity to a human subject having salt sensitivity and/or a high sodium intake, wherein the ACE inhibitor, the aldosterone antagonist, and the loop diuretic are administered at doses that in combination result in one or more of the following: (1) a statistically significant reduction in the death rate as compared to said combination therapy without the aldosterone antagonist; (2) a statistically significant reduction in the number of non-fatal hospitalizations as compared to said combination therapy without the aldosterone antagonist; (3) a statistically significant reduction in the death rate or the number of non-fatal hospitalizations as compared to said combination therapy without the aldosterone antagonist; (4) a statistically significant reduction in the rate of deaths resulting from sudden death in subjects afflicted with or susceptible to elevated heart rate variability as compared to said combination therapy without the aldosterone antagonist; (5) a statistically significant reduction in the death rate for deaths resulting from progression of heart failure as compared to said combination therapy without the aldosterone antagonist; (6) a statistically significant reduction in the death rate or the number of non-fatal hospitalizations in subjects having a left ventricular ejection fraction greater than about 26% as compared to said combination therapy without the aldosterone antagonist; (7) a statistically significant reduction in the death rate or the number of non-fatal hospitalizations in subjects having a left ventricular ejection fraction less than about 26% as compared to said combination therapy without the aldosterone antagonist; and/or (8) suppression of clinically significant cough due to elevated pulmonary arterial fibrosis or increased levels of pulmonary blood pressure in the subject as compared to said combination therapy without the aldosterone antagonist. Preferably, the subject receiving the combination therapy: (1) is susceptible to sudden death; (2) is classified in New York Heart Association class III or class IV prior to combination therapy; (3) has a left ventricular ejection fraction greater than about 26%; and/or (4) is susceptible to or suffering from clinically significant cough due to elevated pulmonary arterial fibrosis or low levels of pulmonary blood pressure.

Such combination therapy would be useful, for example, to reduce the death rate or the number of non-fatal hospitalizations or to prevent or retard, in subjects having salt sensitivity and/or an elevated dietary sodium intake, the development of heart failure that typically arises from essential hypertension or from heart conditions following myocardial infarct. A diuretic agent having no substantial aldosterone antagonistic activity may also be used in conjunction with an ACE inhibitor and the aldosterone antagonist.

Alternatively, the combination therapy may comprise administering a therapeutically-effective amount of an ACE inhibitor, a therapeutically-effective amount of an aldosterone antagonist, a therapeutically-effective amount of a loop diuretic having no substantial aldosterone antagonistic activity and a therapeutically-effective amount of digoxin to a human subject having salt sensitivity and/or a high sodium intake.

The following examples contain a detailed description of the methods of the present invention. This detailed description falls within the scope of, and serves to exemplify, the invention. This detailed description is presented for illustrative purposes only. The eplerenone starting material used in each working example primarily comprises or comprised the Form L polymorph and contains or contained less than about 10% of the Form H polymorph. For most examples, the eplerenone material used contains or contained no detectible amount of the Form H polymorph (i.e., less than about 3% Form H polymorph).

### Working Examples

### A. Biological Working Examples

### Example A-1. Use of Eplerenone To Block Myocardial Infarction And Renal Arteriopathy Independently of Blood Pressure

To demonstrate that eplerenone can prevent aldosterone/salt-mediated early cardiovascular injury in the heart, an experimental model in rats was used that combines elevated blood pressure, moderately high salt intake, an activated RAAS and suppressed nitric oxide production. This model involved chronic inhibition of nitric oxide synthase with N^{ω}-nitro-L-arginine methyl ester ("L-NAME") for 14 days in 1% NaCl-drinking rats combined with a 3 day infusion of angiotensin II on days 11-14. In this experiment, the early pathological effects of mineralocorticoids on the heart and kidney were determined by performing ablation/replacement experiments with aldosterone in the 14 day L-NAME/Angiotensin II/NaCI model of cardiac injury. Specifically, it was tested whether reduction of mineralocorticoids by either adrenalectomy or pharmacologic antagonism with eplerenone, a selective aldosterone receptor blocker, would prevent cardiac and renal damage in this model and whether aldosterone replacement in adrenalectomized rats would restore damage. In addition, it was determined what type of cardiac damage was induced by the L-NAME/Angiotensin II/NaCI treatment and compared these changes to those which occurred in the kidney.

Animals. Male Wistar rats (n=44), weighing 200 to 225 grams, and obtained from Charles River Laboratories (Wilmington, MA) were used in the study. All animals were housed in a room lighted 12 hours per day at an ambient temperature of 22±1°C. Animals were allowed one week to recover after arrival and had free access to Purina Lab Chow 5001 (Ralston Purina Inc, St. Louis, MO) and tap water until the initiation of the experiment. After initiation of the protocols, animals in all groups were placed in individual metabolic cages, handled and weighed daily. Twenty-four hour fluid intake, food intake and urine output were measured daily. Systolic blood pressure was measured three days before initiation of L-NAME treatment and on days 1, 5, 9, and 13. On day 14 of L-NAME treatment, animals were decapitated, trunk blood was collected into chilled tubes containing ethylene diamine tetraacetic acid and the heart and kidneys were removed, blotted dry and immediately weighed. The heart and the kidneys were stored in 10% phosphate-buffered formalin and later processed for light microscopic evaluation.

Drugs. Eplerenone was supplied by G.D. Searle Pharmaceuticals (St. Louis, MO), dissolved in 0.5% methylcellulose and administered twice a day by gavage. Dexamethasone was dissolved in sesame oil and administered as a single subcutaneous dose (12 µg/kg/day) every day. This dose of dexamethasone has been reported to maintain normal weight gain, glomerular filtration rate, and fasting plasma glucose and insulin levels in adrenalectomized rats. Stanton, B., Giebisch G et al. J. Clin. Invest 1985 75:1317-1326. The experiment was concluded on day 14 of L-NAME treatment. Angiotensin II and aldosterone were administered via Alzet osmotic minipumps (Models 2001 and 2002, respectively, Alza Co, Palo Alto, CA) which were implanted subcutaneously at the nape of the neck in animals anesthetized with isofluorane. The concentrations of Angiotensin II and aldosterone used to fill the pumps were calculated based on the mean pump rate provided by the manufacturer, the body weight of the animals on the day before implantation of the pumps, and the dose planned. Angiotensin II (human, 99% peptide purity) was purchased from American Peptide Inc. (Sunny Vale, CA) and administered at a dose of225 µg/kg/day as reported previously. (Hou J et al. J. Clin. Invest. 1995 96:2469-2477. The dose of aldosterone (40 µg/kg/day) is approximately 50% lower than the dose used previously in studies of aldosterone-induced cardiovascular injury. This lower dose induced lesions in stroke-prone spontaneously hypertensive rats. (Rocha R et al. Hypertension 1999 33:232-237. Dexamethasone, aldosterone and L-NAME were purchased from Sigma Chemical Co. (St. Louis, MO). The concentration of L-NAME in the drinking water was adjusted daily to provide a dose of 40 mg/kg/day based on the daily fluid intake and the body weight of the rats.

Surgical Procedure. Three days before initiation of L-NAME treatment, rats from groups 4 and 5 were anesthetized with sodium pentobarbital (Nembutal, Abbott Laboratories, North Chicago, IL; 60 mg/kg, i.p.). Bilateral adrenalectomy was performed using a dorsolumbar approach, making separate incisions on each side. Adrenalectomized animals received 1% NaCl *ad libitum* to drink following the surgical procedure. No post operative deaths occurred.

Assays and Analyses. Systolic blood pressure was measured in awake animals by tail-cuff plethysmography using a Natsume KN-210 manometer and tachometer (Peninsula Laboratories Inc, Belmont, CA). Rats were warmed at 37°C for 10 minutes and allowed to rest quietly in a Lucite chamber before measurement of blood pressure. Urinary protein concentration was determined in urine collected on the last day of the experiment using the sulfosalicylic acid turbidity method. Urinary protein excretion was calculated as the product of the urinary concentration times the urine output/24 hours. Plasma aldosterone concentration was determined using a standard radioimmunoassay-kit from Diagnostic Products Co. (Los Angeles, CA). Plasma renin activity ("PRA") was determined by radioimmunoassay detection of generated angiotensin I (DiaSorin Inc., Stillwater, MN).

Histology. Hearts were stained with collagen specific dye Sirius red for determination of fibrosis as reported elsewhere. (Young M et al. Am. J. Physiol 1995 269:E657-E662). Interstitial collagen was determined using an automated image analyzer. The hearts were also stained with hematoxylin and eosin for light microscopic analysis. Two or three slices of different sections of the heart containing both right and left ventricle were analyzed from each animal. A scale from 0 to 4 was used to score the level of myocardial injury with 0 representing no damage. A score of 1 represented the presence of myocytes demonstrating early necrotic changes such as nuclear pyknosis or karyolysis, non-contracting marginal wavy fibers and eosinophilic staining of the cytoplasm, associated with the presence of scattered neutrophilic infiltrates. A score of 2 was given when one clear area of necrosis (loss of myocardial cells with heavy neutrophilic infiltrates) was observed. When two or more separate areas of necrosis were found (implicating the presence of two different myocardial infarctions in the same heart), but the areas were localized and compromised less than 50% of the ventricular wall, the hearts received a score of 3. A score of 4 was assigned to hearts that demonstrated extensive areas of necrosis compromising more than 50% of either the left or the right ventricle.

Coronal sections of kidney were cut at 3 to 4 mm, and at least three to four of these were prepared as paraffin embedded blocks. Histologic sections (2-3 µm) were stained with periodic acid-Schiff reagent and examined by light microscopy at 10x and 40x by a pathologist who had no knowledge of the different experimental protocols. Glomerular damage, when present, was characterized as the presence of either segmental or global sclerosis with ischemic or thrombotic changes. Renal arterial and arteriolar damage was categorized as the presence of fibrinoid necrosis of the vascular wall. The renal arterial and arteriolar profiles presenting damage were counted and the number of injured vessels per section was divided by the number of glomeruli in the same section in order to normalize for the amount of tissue examined. Renal vascular lesions were expressed as the number of injured vessels per 100 glomeruli.

Statistical analysis. Data were tested for normality using the Kolmogorov-Smimov test. Systolic blood pressure was analyzed using repeated measures-analysis of variance for time and treatment group. One-way analysis of variance was used for normally distributed data with one grouping variable. Post-hoc analysis was performed using Newman Keuls multiple-comparison test. Data that were not normally distributed were analyzed with the Kruskal-Wallis test. Subsequently, selected pairwise comparisons were made using the exact Wilcoxon test. Data are reported as mean ± SE for normally distributed data and median with upper and lower quartile values for data that were not normally distributed.

Dosing Protocols. Wistar rats receiving 1 % NaCl to drink were placed on one of the following dosing protocols: 1) 1% NaCl alone for rats that were used as controls (*NaCl,* n=8); (2) *L-NAME*/*Angiotensin II*/*NaCl:* L-NAME treatment (40 mg/kg/day) in the drinking solution for 14 days (n=8). On day 11 of L-NAME treatment, an osmotic minipump containing Angiotensin II (0.225 ?g/kg/day) was implanted in each animal subcutaneously; (3) *L-NAME*/*Angiotensin II*/*NaCl+Eplerenone*: L-NAME/Angiotensin II/NaCl-treated rats received eplerenone (100 mg/kg/day, p.o.; n=8) in addition, from days 0 to 14. Two additional groups of NaCl-drinking rats were adrenalectomized three days before initiation of L-NAME/Angiotensin II treatment. Group 4 (*L-NAME*/*Angiotensin II*/*NaCl+ADX,* n=11) received glucocorticoid replacement with dexamethasone starting immediately after the surgery. Group 5 (*L-NAME*/*Angiotensin II*/*NaCl+ADX*/*ALDO*, n=9) received in addition to dexamethasone, aldosterone starting at day 0 simultaneously with L-NAME treatment.

### Results.

### Effect on blood pressure.

Baseline systolic blood pressure was similar in all treatment groups. All animals receiving L-NAME/Angiotensin II/NaCl treatment showed a progressive and significant increase in systolic blood pressure compared with the NaCl-drinking controls (P<0.01). The extent of hypertension observed at the end of the experiment was not appreciably influenced by eplerenone treatment or adrenalectomy (Figure A-1

### Plasma renin activity and aldosterone levels.

Data for PRA and circulating aldosterone levels are shown in Figure A-2. L-NAME/Angiotensin II/NaCl treatment significantly reduced PRA in intact animals compared with saline-drinking controls. The higher levels of PRA observed in L-NAME/Angiotensin II/NaCl-treated rats that were adrenalectomized was prevented by administration of aldosterone (Figure A-2A). Despite the marked inhibition of PRA observed in adrenal-intact animals treated with L-NAME/Angiotensin II/NaCl or L-NAME/Angiotensin II/NaCl plus eplerenone, plasma aldosterone was similar to that in saline-drinking controls (Figure A-2B). As anticipated, plasma aldosterone levels were reduced to undetectable levels in adrenalectomized rats while adrenalectomized, aldosterone-infused rats had elevated aldosterone levels.

### Role of aldosterone in cardiac injury.

Summarized in Table A-1A below are data obtained at the end of the experiment. Body weight was not different among the three groups of adrenal-intact animals. However, both groups of adrenalectomized rats demonstrated significantly lower body weight compared with adrenal-intact groups. The ratio between total heart weight and total body weight was used as an index of cardiac hypertrophy. The cardiac hypertrophy index was higher in all groups of animals receiving L-NAME/Angiotensin II/NaCl when compared with NaCl-drinking controls. Both eplerenone treatment and adrenalectomy significantly reduced cardiac hypertrophy compared to that observed in L-NAME/Angiotensin II/NaCl-treated rats. Infusion of aldosterone reversed the effect of adrenalectomy on cardiac hypertrophy index and restored it to a level that was not different from the L-NAME/Angiotensin II/NaCl group.

**Table A-1A**

| Group | n | BW g | SBP mmHg | HW mg | HW/BW mg/g |
|---|---|---|---|---|---|
| NaCl | 8 | 331 ± 4 | 139 ± 4 | 924 ± 2 | 2.79 ± .05 |
| L-NAME/AngII/NaCl | 8 | 311 ± 15 | 180 ± 5 | 1150 ± 4 | 3.87 ± .09 |
| L-NAME/AngII/NaCl/Epl | 8 | 330 ± 4 | 177 ± 8 | 1144 ± 4 | 3.46 ± .05 |
| L-NAME/AngII/NaCl/ADX | 11 | 278 ± 4 | 176 ± 3 | 880 ± 3 | 3.2 ± .13 |
| L-NAME/AngII/NaCl/ADX/ALDO | 9 | 267 ± 6 | 192 ± 7 | 951 ± 16 | 3.57 ± .06 |

Histological examination of the hearts revealed significant differences among the treatment groups, P<0.0001 (Figures A-3 and A-4). L-NAME/Angiotensin II/NaCl-treated rats developed vascular damage and myocardial necrosis. A representative photomicrograph of these lesions is shown in Figure A-3A. Myocardial necrosis was characterized by loss of cross striation of myofibers, homogenization of cytoplasm, loss of cellular membranes, pyknosis and eventually karyolysis of nuclei, and influx of inflammatory cells including polymorphonuclear cells and monocytes. Fibrinoid necrosis was present in small coronary arteries and arterioles (not shown). In contrast, cardiac injury in response to treatment with L-NAME/Angiotensin II/NaCl was markedly reduced in those animals in which eplerenone was chronically administered or adrenalectomy was performed (Figures A-3B, A-4). These two groups demonstrated levels of myocardial necrosis that were similar to those observed in the NaCl-drinking controls. The protective effect of adrenalectomy was completely reversed by the addition of an aldosterone infusion.

Staining with Sirius red (a collagen-specific dye) showed no increases in interstitial collagen volume fraction in the hearts in any of the groups receiving L-NAME/Angiotensin II/NaCl treatment (data not shown). Furthermore, collagen deposition was not increased in areas of myocardial necrosis (Figures A-3A and A-3C, staining of adjacent sections with hematoxilin eosin and Sirius red, respectively).

### Role of aldosterone in renal damage

Urinary protein excretion (24 hour) measured at the end of the two-week-treatment period was normal in the NaCl group (Figure A-5). Treatment with L-NAME/Angiotensin II/NaCl markedly increased urinary protein excretion. Eplerenone treatment and adrenalectomy prevented the development of proteinuria in animals receiving L-NAME/Angiotensin II/NaCl treatment. In contrast, administration of aldosterone to adrenalectomized rats completely restored the effects of L-NAME/Angiotensin II/NaCl treatment on proteinuria.

Histopathologic evaluation of the kidneys also demonstrated significant differences among the groups, P<0.001 (Figures A-6 and A-7). While renal arteriopathy was not found in kidneys from NaCl-drinking controls, animals receiving L-NAME/Angiotensin II/NaCl treatment demonstrated severe renal vascular damage involving primarily arcuate and interlobular arteries and arterioles (Figure A-6). These vessels demonstrated fibrinoid necrosis of the vascular wall with medial thickening and proliferation of the perivascular connective tissue. A few isolated glomeruli had areas of focal thrombosis. Proteinaceous casts at the level of the distal tubules, and reabsorption protein granules in proximal tubules frequently were observed in L-NAME/Angiotensin II/NaCl-treated rats. Renal arteriopathy tended to be reduced in animals receiving eplerenone treatment. However, this 60% reduction in damage compared to L-NAME/AngII/NaCl treated rats did not reach statistical significance upon analysis of the histopathologic scores (P=0.1). Adrenalectomy significantly reduced renal arteriopathy induced by L-NAME/Angiotensin II/NaCl treatment to levels that were not significantly different from NaCl-drinking controls (Figure A-7). As was observed in the heart, in the kidneys when aldosterone was infused into adrenalectomized, L-NAME/Angiotensin II/NaCl-treated rats, damage was significantly increased.

It was found that combined administration of Angiotensin II and L-NAME, an inhibitor of nitric oxide synthesis, to rats on a high sodium diet caused the development of hypertension, cardiac hypertrophy, myocardial necrosis, proteinuria and renal arteriopathy. In contrast, there was no evidence of myocardial fibrosis which is typically associated with chronic cardiovascular injury. Myocardial necrosis, proteinuria and vascular lesions were prevented by adrenalectomy, which eliminated the presence of aldosterone. The protective effect of adrenalectomy was lost when adrenalectomized rats were infused with aldosterone. Similarly, aldosterone antagonism with eplerenone decreased cardiovascular damage. Thus, epoxy-steroidal compounds prevent or reduce the development of the acute cardiovascular lesions in L-NAME/Angiotensin II/NaCl-treated rats.

This study shows that L-NAME/Angiotensin II/NaCl treatment is highly effective in inducing hypertension and end organ damage at the level of the heart and the kidney. Eplerenone is effective in preventing such an effect. Furthermore, since eplerenone did not appreciably alter systolic blood pressure, the therapeutic effect is independent of its effect on sodium retention, volume expansion and hypertension. Finally, the data suggest that the effect of eplerenone is not to prevent fibrosis but instead to reduce medial fibrinoid necrosis in small arteries and arterioles and subsequent tissue necrosis. Fibrosis may be a reparative process.

### Example A-2 Acute Effect On Blocking Aldosterone-Induced Salt Retention In Rats and Dogs

Methods: The antimineralocorticoid pharmacological activity was determined in saline-loaded adrenalectomized rats treated with aldosterone. Test compound was given by gavage 30 minutes before the aldosterone injection. Urinary sodium, potassium and water excretion were measured. The results are expressed as the ratio of excreted urinary sodium to potassium (Na/K)± SEM (Table A-2A).

The antimineralocorticoid pharmacological action was also evaluated in saline-hydrated conscious dogs (n=6), treated with aldosterone by intravenous infusion and given eplerenone orally. Excreted urinary sodium and potassium concentrations were measured. The results are expressed as changes in urinary sodium and potassium and the Na/K ratio and are compared to aldosterone treatment alone (Table A-2B).

Results: These results demonstrate the pharmacological reversal, by eplerenone, of aldosterone-induced sodium and water retention and potassium loss in vivo in two species. In these models, eplerenone produces a significant natriuresis following a single oral dose.

**Table A-2A.**

| Treatment | Urinary Na/K ± SEM |
|---|---|
| None | 5.15 ± 0.15* |
| Aldosterone | 1.68 ± 0.04 |
| Aldosterone & Eplerenone (1 mg/kg) | 2.53 ± 0.23* |
| Aldosterone & Eplerenone (3 mg/kg) | 4.08 ± 0.26* |
| Aldosterone & Eplerenone (10 mg/kg) | 4.11 ± 0.27* |
| Aldosterone & Spironolactone (1 mg/kg) | 2.26 ± 0.25* |
| Aldosterone & Spironolactone (3 mg/kg) | 2.90 ± 0.25* |
| Aldosterone & Spironolactone (10 mg/kg) | 4.38 ± 0.26* |
| * Significantly different from the aldosterone-treated group (p<0.05). | |

**Table A-2B**

| Treatment | Urinary Excretion (mmol/2 h) | | Na/K Ratio |
|---|---|---|---|
| | Sodium | Potassium | |
| Aldosterone | 1.0 | 10.5 | 0.09 |
| Eplerenone (10 mg/kg) | 24.4 ± 3 | 11.9 ± 1 | 2.1 |
| Spironolactone (10 mg/kg) | 12.6 ± 2 | 7.4 ± 0.7 | 1.7 |

### Example A-3: Comparison of Subcutaneous vs. Oral Administration of Eplerenone to Rats

Methods: The antimineralocorticoid pharmacological activity of eplerenone was determined in saline-loaded adrenalectomized rats treated with aldosterone. Eplerenone was given by oral gavage or subcutaneous injection 30 minutes before the aldosterone injection. Urinary sodium, potassium and water excretion were measured. The results are expressed as the ratio of excreted urinary sodium to potassium (Na/K) ± SEM.

Results: These results indicate the in vivo potency of eplerenone in antagonizing aldosterone-mediated renal effects (Table A-3A). Subcutaneous injection provided better efficacy than oral administration (Tables XVIII and XIX).

**Table A-3A**

| Group | Mean Na/K | SEM |
|---|---|---|
| Saline | 9.57 | 1.81 |
| Aldosterone | 1.68 | 0.68 |
| Aldosterone + Spironolactone (3) | 1.64 | 0.41 |
| Aldosterone + Spironolactone (10) | 1.56 | 0.32 |
| Aldosterone + Spironolactone (30) | 3.02 | 0.74 |
| Aldosterone + Spironolactone (100) | 4.02 | 1.50 |
| Aldosterone + Spironolactone (3) | 1.37 | 0.25 |
| Aldosterone + Eplerenone (10) | 2.17 | 0.61 |
| Aldosterone + Eplerenone (30) | 2.98 | 0.34 |
| SEM=standard error mean; numbers in parentheses represents the dose of antagonist administered (in mg/100g rat); number of animals for each group was between 6 and 9. | | |

**Table A-3B**

| Group | Mean Na/K | SEM |
|---|---|---|
| Saline | 10.07 | 1.72 |
| Aldosterone | 1.21 | 0.25 |
| Aldosterone + Eplerenone (3) | 0.90 | 0.13 |
| Aldosterone + Eplerenone (10) | 1.34 | 0.30 |
| Aldosterone + Eplerenone (30) | 1.18 | 0.27 |
| Aldosterone + Eplerenone (100) | 2.10 | 0.22 |
| Aldosterone + Eplerenone (300) | 4.79 | 1.24 |
| Aldosterone + Eplerenone (1000) | 5.70 | 1.04 |
| SEM=standard error mean; numbers in parentheses represent the dose of antagonist administered (in mg/100g rat); number of animals for each group was between 8 and 11. | | |

### (Reference) Example A-4: Hypertension Model: Volume Expanded Hypertensive Rats

Methods: Uninephrectomized rats were given 1% NaCl drinking water and infused s.c. with aldosterone (0.5 g/kg/hr) via an Alza osmotic pump, Model 2002. Test compound was administered by s.c. injection twice a day. Blood pressure and heart rate were evaluated continuously by telemetry via an implanted transmitter connected to a pressure transducer cannulated to the abdominal aorta.

Results: Eplerenone reduced blood pressure in this rodent model at both doses tested, when measured by continuous monitoring using telemetry (Table A-4A, data averaged for 24 hours after three weeks). Eplerenone did not produce a significant change in the heart rate.

**Table A-4A**

| Treatment | SAP | HR |
|---|---|---|
| Aldosterone & Vehicle | 202 ± 7 | 346 ± 6 |
| Aldosterone & Eplerenone (100 mg/kg) | 155 ± 7* | 352 ± 4 |
| Aldosterone & Eplerenone (200 mg/kg) | 174 ± 5* | 339 ± 4 |
| Aldosterone & Spironolactone (100 mg/kg) | 171 ± 5* | 338 ± 4 |

**Table A-4A**

| | | |
|---|---|---|
| Aldosterone & Spironolactone (200 mg/kg) | 170 ± 9* | 336 ± 7 |
| Significantly different from the vehicle-treated group (p<.05). SAP: Systolic arterial pressure (mmHg ± SEM). HR: Heart Rate (beats/min ± SEM) | | |

### (Reference) Example A-5: Effect of Eplerenone in Stroke Prone Spontaneously Hypertensive Rat (SHR-SP), a Genetic Model of Hypertension and Stroke

Effect of Eplerenone on Blood Pressure in Stroke Prone Spontaneously Hypertensive Rat (SHR-SP)

Methods: SHR-SP were bred in the animal facilities of New York Medical College and maintained on normal rat chow and non-saline drinking water (i.e., tap water). At the age of 13 weeks the animals (n=7-8) were administered either eplerenone (100 mg/kg/day, p.o. BID) or vehicle. Indirect measurements of systolic blood pressure were assessed by tail cuff plethysmography.

Results: The results are shown in Table A-5A. Both groups of animals had similar and elevated systolic blood pressure at the initiation of the study. The systolic blood pressure continued to increase during the three week duration of the experiment in animals treated with only vehicle. In contrast, systolic blood pressure of animals treated with eplerenone remained at pretreatment levels. These data demonstrate that eplerenone is an effective antihypertensive agent in this model of genetic hypertension and stroke.

**Table A-5A**

| | Week 13 | Week 14 | Week 15 | Week 16 |
|---|---|---|---|---|
| Vehicle | 203.8 ± 1.3 | 200.3 ± 3.3 | 224.6 ± 6.0 | 236.0 ± 4.8 |
| Eplerenone (100 mg/kg/day) | 201.4 ± 1.6 | 202.8 ± 1.8 | 208.0 ± 4.2* | 203.1 ± 6.2* |
| n=* Significantly different from the vehicle-treated group (p< 0.05), mmHg = millimeters of mercury. | | | | |

### (Reference) Example A-6: Aldosterone receptor antagonist to treat myocardial injury.

Sprague Dawley rats (250 g) were uninephrectomized and provided with 1% NaCl-solution as the only source of water. The rats were then implanted with Alzet mini pump that subcutaneously delivered either aldosterone (0.75ug/hr) or vehicle. These two treatment groups were further divided into those that received normal rat chow or or chow containing eplerenone (100 mg/k/d). Blood pressure was measured by radio telemetry units implanted in abdominal aorta. Rats were sacrificed for examination of the hearts.

As shown in Figure A-8, in this high salt/uninephrectomized rat model, aldosterone caused histopatholically-demonstratable cardiac lesions. After 2-4 of treatment, hearts characteristically showed perivascular inflammation, vascular wall hypertrophy, endothelial thickening (neo-intima),fibrinoid necrosis of coronary arteries. There was minimal cardiomyocyte changes and no apparent fibrosis. After 6-8 weeks of treatment, there was histologically apparent cardiomyocyte necrosis, reparative fibrosis (scars), and reactive interstitial fibrosis. In contrast, eplerenone supplementation in the rat chow prevents the histopathologic damage otherwise observed (Figure A-9).

As shown in Figure A-10, myocardial damage in uninephrectomized rats requires high salt intake and aldosterone. Sodium alone does not cause myocardial damage and aldosterone blockade by the administration of eplerenone prevents the aldosterone plus high sodium myocardial damage.

### (Reference) Example A-7: Use of eplerenone to prevent stroke and cerebrovascular damage.

Fifteen male, 9 weeks of age, saline-drinking SHRSP were included in the experiment. As shown in Figure A-11, Vehicle-treated SHRSP (n=8) all developed stroke signs and died at 15.2 ± 0.6 weeks of age whereas eplerenone-treated SHRSP (100 mg/kg/day, n=7) showed no signs of stroke up to 18.5 weeks of age (P<.005) when they were sacrificed for further evaluation. Eplerenone treatment also prevented the development of marked proteinuria (38 ± 18 v 136 ± 19 mg/day P<.005) but not severe hypertension (237 ± 3 v 242 ± 4 mmHg) (Figure A-12). As shown in Figure A-13, histopathologic analysis of the brains revealed the presence of liquofactive neorosis in all vehicle-treated SHRSP, associated with fibrinoid necrotic lesions in cerebral arteries and arterioles with focal hemorrhages. These lesions were markedly reduced by administration of eplerenone. Using a semiquantitative scoring system from 0-4 for cerebral injury (Figure A-14), a score of 3.5 ± 3 was observed in vehicle-treated rats vs a score of 0.5 ± 2 in the animals receiving eplerenone (P<0.001). Thus, eplerenone provides a vascular protective action in the brain in saline-drinking SHRSP, through mechanisms not associated with decreases in arterial blood pressure. The results indicate a heretofore unrecognized role for endogenous mineralocortoids (e.g. aldosterone) in sodium drinking rats in the development of stroke. Furthermore, eplerenone administration prevents stroke development resulting from aldosterone in rats with increased dietary sodium intake.

### Example A-8: Vascular protective effect eplerenone in stroke-prone spontaneously hypertensive rats.

In a first protocol, saline-drinking SHRSP (n = 9) were treated with oral eplerenone (100 mg/kg/d) for 5 to 6 weeks. Eplerenone prevented the development of proteinuria (16 ± 2 v 85 ± 11 mg/d, *P* < .001) and renal lesions (1 ± 1 v 40 ± 5%, *P* < .0005) but not severe hypertension (219 ± 6 v 227 ± 4 mm Hg) compared with vehicle (n = 9). In a second protocol, the contribution of mineralocorticoids to vascular lesion development was examined in captopril-treated SHRSP under conditions in which endogenous angiotensin II formation was suppressed and either vehicle or exogenous angiotensin II was chronically infused. Captopril-treated SHRSP received one of three treatment regimens: (*i*) an infusion of the vehicle used to dissolve Angiotensin II and no eplerenone (n=5); (*ii*) angiotensin II infusion (25 ng/min, subcutaneously; n = 7) plus 2 ml/kg/d of 0.5% methylcellulose by gavage; (*iii*) angiotensin II infusion (25 ng/min, subcutaneously; n = 7) plus eplerenone (100 mg/kg/d by gavage). After 2 weeks, systolic blood pressure in all three groups of SHRSP was comparable and severely elevated. In SHRSP that received captopril plus vehicle, plasma aldosterone levels were reduced and no renal pathology was noted. The addition of the angiotensin II infusion increased plasma aldosterone levels, and reversed captopril protection against proteinuria (96 ± 13 v 14 ± 1 mg/d, respectively) and renal injury. Despite continuous angiotensin II infusion, eplerenone treatment markedly attenuated proteinuria (28 ± 5 v 96 ± 13 mg/d, *P* < .001) and renal damage (3 ± 1 v 18 ± 4%, *P* < .0001) compared with vehicle. These findings indicate that endogenous mineralocorticoids mediate the progression of renal injury in saline-drinking SHRSP independent of angiotensin II and its effect on blood pressure.

### MATERIALS AND METHODS

Animals. Studies were conducted in accordance with institutional guidelines using male SHRSP/A3N (generations F-75 to F-78), n = 37, from our local colony. These animals were bred from NIH stock, derived from the SHRSP/A3N substrain described originally by Okamoto and coworkers (Okamoto K et al. Circ. Res. 34 and 35 (suppl I):I-143-I-153.). All animals were housed in a room maintained on a 12:12-h light:dark-cycle and an ambient temperature of 22 ± 1°C in the Animal Care Facility at New York Medical College. Rats were weaned at 4 weeks of age and allowed free access to Purina Lab Chow 5001 (Ralston Purina, St. Louis, MO) and tap water until the initiation of experimental protocols.

### Protocol 1: Effect of Eplerenone on Renal Pathologic Changes

Eighteen SHRSP were given 1% NaCl to drink and were fed Stroke-Prone Rodent Diet (#39-288, Zeigler Brothers, Inc., Gardners, PA) starting at 8.1 weeks of age. This diet is lower in potassium (0.7% v 1.2% by weight) and protein (17% v 22% by weight) than the standard diet and induces a higher incidence of stroke in SHRSP (Stier CT et al. Hypertension. 13:115-121) At 8.4 weeks of age, the animals were divided equally into two groups and chronic treatment with either eplerenone or vehicle was started. Eplerenone (SC-66110) was provided by G.D. Searle & Co., (St. Louis, MO). Eplerenone was suspended at 50 mg/ml in a solution of 0.5% methylcellulose (Sigma Chemical Co., St. Louis, MO) and was administered twice daily by gavage to provide a total daily dose of 100 mg/kg. Since eplerenone, unlike spironolactone, does not produce active metabolites, a higher dose was used. Vehicle-control littermates received 2 ml/kg/d of the 0.5% methylcellulose solution. Animals were housed individually in metabolic cages so that measurements of 24-h urine output and protein excretion could be made. Animals were examined each day for neurologic signs of stroke. Systolic arterial pressure and heart rate were measured each week in awake rats. The experiment was terminated after 5 weeks of treatment when the animals were 13.1 weeks of age. Trunk blood was collected into chilled EDTA tubes following rapid decapitation of the animals between 10:00 am and 12:00 pm. Blood was stored at - 20°C for later measurement of plasma aldosterone levels. The kidneys were rapidly removed, weighed, and then preserved in fixative for later histologic examination.

### Protocol 2: Effect of Eplerenone on Angiotensin II-Induced Renal Damage in SHRSP

In a second series of 19 SHRSP, the contribution of endogenous mineralocorticoids to the development of Angiotensin II-induced renal injury was evaluated. SHRSP were placed on Stroke-Prone Rodent Diet (#39-288, Zeigler Bros Inc., Gardners, PA) and 1% NaCl drinking solution *ad libitum* starting at 8.3 weeks of age. To provide a consistent background suppression of endogenous Angiotensin II levels among the animals, captopril (Sigma Chemical Co., St. Louis, MO) was added to the drinking solution of all animals to provide a dose of 50 mg/kg/d. It has previously been shown that this dose of captopril, in the absence of Angiotensin II infusion, will prevent the development of renal and cerebrovascular lesions in saline-drinking SHRSP (Rocha R et al. Hypertension. 33:232-237) At 9.3 weeks of age, Alzet osmotic minipumps, Model 2002 (Alza Co., Palo Alto, CA), containing Angiotensin II (human type, American Peptide Inc., Sunnyvale, CA) or its vehicle (sterile 0.9% NaCl) were implanted beneath the skin at the nape of the neck in SHRSP receiving inhalatory anesthesia with isofluorane (Ohmeda Caribe Inc., Guayama, PR). Rats were housed in individual metabolic cages and received one of three treatment regimens: (*i*) an infusion of the vehicle used to dissolve angiotensin II and no eplerenone (n=5); (*ii*) angiotensin II infusion (25 ng/min, subcutaneously; n = 7) plus 2 ml/kg/d of 0.5% methylcellulose by gavage; (*iii*) angiotensin II infusion (25 ng/min, subcutaneously; n = 7) plus eplerenone (100 mg/kg/d by gavage). In preliminary experiments, it was demonstrated that a dose of 25 ng/min of angiotensin II could reverse the vascular protective effect of ACE inhibitor treatment with enalapril in saline-drinking SHRSP. The dose of eplerenone selected was based on the results of the experiments in Protocol 1 that showed almost complete protection against proteinuria and renal injury in saline-drinking SHRSP through 13 weeks of age, independent of changes in systolic blood pressure. Animals were handled and weighed daily. Urine samples were collected for the assessment of proteinuria. Systolic blood pressure and heart rate were measured each week. After 2 weeks of treatment, animals were decapitated, trunk blood was collected into chilled EDTA tubes, and the kidneys were removed, blotted dry, and immediately weighed. Coronal sections of kidney were fixed and later processed for light microscopic evaluation.

Assays and Analyses. Systolic blood pressure and heart rate of awake animals were measured by tail-cuff plethysmography using a Natsume KN-210 manometer and tachometer (Peninsula Laboratories Inc., Belmont, CA). Rats were warmed at 37°C for 10 min and allowed to rest quietly in a Lucite chamber before measurement of blood pressure. Measurements of urine volume were made gravimetrically. Urinary protein concentration was determined by the sulfosalicylic acid turbidity method. Plasma aldosterone was measured by radioimmunoassay using ¹²⁵I-aldosterone as a tracer (Coat-a Count Aldosterone, Diagnostic Products Co., Los Angeles, CA).

Histology. Kidneys were preserved in 10% phosphate-buffered formalin. Coronal sections (2-3 µm) were stained with hematoxylin and eosin and examined by light microscopy in a blinded fashion as previously described (Stier CT et al. J Pharmacol Exp Ther (1992) 260:1410-1415). Glomerular damage was categorized as ischemic or thrombotic. Ischemic lesions were defined as retraction of glomerular capillary tufts with or without appreciable mesangiolysis. Glomerular thrombotic lesions were defined as any one of a combination of the following: segmental to global fibrinoid necrosis, focal thrombosis of glomerular capillaries, swelling and proliferation of intracapillary (endothelial and mesangial) and/or extracapillary cells (crescents), and expansion of reticulated mesangial matrix with or without significant hypercellularity. The number of glomeruli exhibiting lesions in either category was enumerated from each kidney and was expressed as a percentage of the total number of glomeruli present per mid-coronal section (mean ± SEM = 218 ± 7 glomeruli per animal; range = 162 to 261 glomeruli for Protocol 1 and 229 ± 7 glomeruli per animal; range = 196 to 290 glomeruli for Protocol 2). Vascular damage was assessed by counting the total number of arterial and arteriolar profiles that showed thrombotic and/or proliferative arteriopathy in the same mid-coronal section. Vascular thrombotic lesions were defined as any one or a combination of the following: mural fibrinoid necrosis, extravasation and fragmentation of red blood cells, and luminal and/or mural thrombosis. Proliferative arteriopathy was characterized by proliferation of markedly swollen myointimal cells with swollen round to ovoid vesicular nuclei surrounded by mucinous extracellular matrix ("onion skinning") often resulting in nodular thickening. Vascular damage was expressed as the number of arteries and arterioles with lesions per 100 glomeruli. The presence of casts and tubular (ischemic) retraction and simplification was assessed semiquantitatively.

Statistical Analysis. Significant effects with respect to treatment and time were determined by two-way analysis of variance. Data with only one grouping variable were analyzed statistically by Student's unpaired *t* tests. When more than two groups were compared, one-way analysis of variance was performed followed by the post-hoc Newman-Keul's multiple comparison test. Data were analyzed using version 2.01 of the GraphPad Prism statistical software package (GraphPad Software Inc., San Diego, CA). *P* < .05 was considered statistically significant. Data are reported as mean ± SEM.

### RESULTS

### Protocol 1

Figure A-15 shows the results for preterminal systolic blood pressure and urinary protein excretion in SHRSP chronically treated with eplerenone (100 mg/kg/d) or vehicle. This bar graphs showing preterminal (A) systolic arterial blood pressure (SBP) and (B) urinary protein excretion (UPE) in stroke-prone spontaneously hypertensive rats receiving chronic treatment with either eplerenone (100 mg/kg/d) or vehicle from 8.4 to 13.1 weeks of age. Animals were given 1% NaCl to drink and Stroke-Prone Rodent Diet *ad libitum* starting at 8.1 weeks of age. *** *P*<.001 compared with vehicle-treated littermates. Values are mean ± SEM.

Eplerenone prevented the development of proteinuria (85 ± 11 v 16 ± 2 mg/d, *P* < .001) but not severe hypertension (227 ± 4 v 219 ± 6 mm Hg) as compared with the littermate controls. Systolic blood pressure and heart rate also did not differ between the eplerenone- and vehicle-treated groups during the study.

Table A-8A summarizes the results from the histologic analysis for renal lesions. Kidneys from vehicle-treated, saline-drinking SHRSP exhibited ischemic or thrombotic damage in 35 ± 5% of glomeruli and showed extensive thrombotic and proliferative damage in small arteries and arterioles as illustrated in Figure A-16A. This Figure shows representative photomicrographs of hematoxylin and eosin-stained mid-coronal kidney sections from saline-drinking stroke-prone spontaneously hypertensive rats after 5 weeks of eplerenone or vehicle treatment starting at 8 weeks of age (original magnification, x 130). Renal cortex from animals treated with vehicle demonstrate typical findings of malignant nephrosclerosis such as ischemic retraction (small arrow), thrombonecrosis of capillary tufts (large arrow), and arteriolar fibrinoid necrosis with fragmented and extravasated erythrocytes, and concentric proliferative arteriopathy (arrowheads). Several smaller arteries and arterioles reveal marked mural thickening due to medial hypertrophy (double arrows). There is patchy ischemic retraction and simplification of tubules. Others are dilated with protein casts. In contrast, after 5 weeks of eplerenone treatment, age-matched littermates showed only rare instances of ischemic or thrombotic glomeruli (Figure A-16B; Renal cortex from an animal treated with eplerenone (100 mg/kg/d by gavage) reveals no significant pathology).

Protein casts were present in 10 ± 5% of tubules in vehicle-treated SHRSP and 0.11 ± 0.04% of tubules in eplerenone-treated SHRSP (*P* < .001). Similarly, 48 ± 7% of the tubules in vehicle-treated SHRSP displayed ischemic retraction and simplification compared with only 0.5 ± 0.2% of the tubules in eplerenone-treated SHRSP (*P* < .001). The plasma concentration of aldosterone did not differ significantly between the groups and averaged 305 ± 68 pg/m1 in vehicle-treated SHRSP and 315 ± 35 pg/ml in eplerenone-treated SHRSP. Six of the nine vehicle-treated SHRSP showed definite signs of stroke whereas none of eplerenone-treated SHRSP showed evidence of stroke (*P* < .01, Fisher's Exact Test).

Body weight was not affected by eplerenone through the first 3 weeks of treatment (data not shown). Thereafter, body weight declined in the vehicle-treated group but was maintained or increased in eplerenone-treated SHRSP. Absolute kidney weight at autopsy averaged 2.42 ± 0.11 g in vehicle-treated SHRSP and 2.58 ± 0.06 g in eplerenone-treated SHRSP and was not affected by the chronic administration of eplerenone. Because terminal body weight was lower in vehicle-treated SHRSP than in eplerenone-treated SHRSP (220 ± 7 g v 279 ± 7 g, *P* < .001), the kidney-weight to body-weight ratio was significantly higher in that group.

### Protocol 2

Figure A-17A shows the systolic blood pressure of captopril-treated, saline-drinking SHRSP that received an infusion of either vehicle (saline) or angiotensin II with or without concomitant oral administration of eplerenone. Figure A-17 contains a line graph showing (A) systolic arterial blood pressure and (B) urinary protein excretion in saline-drinking stroke-prone spontaneously hypertensive rats during treatment with captopril plus vehicle (CAP), captopril plus Angiotensin II (CAP + angiotensin II), or captopril plus angiotensin II plus eplerenone (CAP + angiotensin II + EPL). Captopril treatment (50 mg/kg/d) was started at 8.3 weeks of age. Alzet osmotic minipumps containing Angiotensin II (25 ng/min) were implanted subcutaneously at 9.3 weeks of age and concomitant treatment with vehicle (n = 5), Angiotensin II alone (n = 7), or angiotensin II plus EPL (100 mg/kg/d) (n = 7) was started. *** *P* < .001 compared with CAP or CAP + angiotensin II + EPL. Values are mean ± SEM. All SHRSP developed severe hypertension, which did not differ significantly among the groups. Preterminal systolic blood pressure averaged 224 ± 6 mm Hg in the captopril plus vehicle-treated group, 224 ± 4 mm Hg in the captopril plus angiotensin II plus vehicle-treated group, and 231 ± 5 mm Hg in captopril plus angiotensin II plus eplerenone-treated SHRSP. Results showed that adding angiotensin II to captopril did not further increase blood pressure over captopril plus vehicle. Additionally, blood pressure measurements were similar in captopril-treated SHRSP receiving angiotensin II plus eplerenone. Urinary protein excretion remained at low levels in all three groups through 9.5 weeks of age, which was 2 days after initiation of angiotensin II infusion (Figure A-17B). Thereafter, SHRSP treated with captopril plus vehicle did not develop proteinuria. However, SHRSP receiving captopril plus angiotensin II plus vehicle developed marked proteinuria. In contrast, proteinuria was prevented in SHRSP receiving captopril plus angiotensin II plus eplerenone. Urinary protein excretion averaged 14 ± 1 mg/d in the captopril group, 96 ± 13 mg/d in the captopril plus angiotensin II group, and 28 ± 5 mg/d in the captopril plus angiotensin II plus eplerenone-treated group at the end of the study (*P* < .001 for captopril or captopril plus angiotensin II plus eplerenone v captopril plus angiotensin II). The plasma aldosterone concentration averaged 404 ± 160 pg/ml in the group treated with captopril plus angiotensin II and 231 ± 37 pg/ml in the group receiving captopril plus angiotensin II plus eplerenone (*P* = NS; Figure A-18). Figure A-18 is a bar graph showing plasma aldosterone levels in stroke-prone spontaneously hypertensive rats that were started on captopril treatment (50 mg/kg/d) and 1% NaCl/Stroke-Prone Rodent Diet starting at 8.3 weeks of age. Alzet osmotic minipumps containing vehicle or angiotensin II (25 ng/min) were implanted subcutaneously at 9.3 weeks of age and concomitant treatment with or without eplerenone was started. Animals were sacrificed 2 weeks later (* *P* < .05 compared with animals treated with captopril alone). These values were significantly elevated relative to the group receiving captopril alone (107 ± 26 pg/ml; *P* < .05) and were similar to those observed in Protocol 1.

Consonant with the high levels of proteinuria, the kidneys of captopril-treated, saline-drinking SHRSP at the end of 2 weeks did not show any injury. Administration of angiotensin II plus vehicle to captopril-treated SHRSP induced the development of prominent thrombotic microangiopathic lesions of malignant nephrosclerosis affecting glomeruli and microvessels (Table A-8B). In contrast, nephrosclerotic lesion development in response to angiotensin II infusion was markedly diminished in captopril-treated animals that were concomitantly receiving eplerenone. Figure A-19 shows representative photomicrographs of the histologic changes in kidneys from saline-drinking, captopril-treated SHRSP (hematoxylin and eosin-stained renal cortex from saline-drinking stroke-prone spontaneously hypertensive rats (SHRSP) (original magnification, x 130). Captopril plus vehicle treatment prevented the development of renal pathology (Figure A-19A). Captopril-treated SHRSP infused with angiotensin II plus vehicle (Figure A-19B) showed renal lesions similar to those of saline-drinking SHRSP treated with vehicle (see Figure A-16A). In contrast, the incidence of renal vascular and glomerular injury was markedly reduced in animals receiving captopril plus angiotensin II plus eplerenone (Figure A-19C). The presence of tubular ischemia was assessed semiquantitatively as in Protocol 1. These changes were absent in SHRSP receiving captopril plus vehicle. Twenty-one ± 3% of the tubules in captopril plus angiotensin II plus vehicle-treated SHRSP revealed ischemic retraction and simplification whereas only 2.5 ± 0.9% of tubules displayed similar changes in captopril plus angiotensin II plus eplerenone-treated SHRSP (*P* < .0001).

Body weight showed no difference among the groups over the course of the study and was 258 ± 6 g in the captopril plus vehicle-treated group, 223 ± 7 g in the captopril plus angiotensin II plus vehicle-treated group, and 234 ± 5 g in the captopril plus angiotensin II plus eplerenone-treated group at the end of the experiment. Absolute kidney weight at autopsy was 2.56 ± 0.06 g in the captopril plus vehicle-treated group, 2.12 ± 0.06 g in captopril plus angiotensin II plus vehicle-treated group, and 2.00 ± 0.03 g in captopril plus angiotensin II plus eplerenone-treated SHRSP. Absolute kidney weight, or kidney weight expressed as a percentage of body weight, was not affected by treatment with eplerenone.

**TABLE A-8A**

| Lesion | | Vehicle | Eplerenone |
|---|---|---|---|
| | | (n=9) | (n = 9) |
| Glomerular damage | | | |
| (lesions/100 glomeruli) | | | |
| | Ischemic | 24 ± 2 | 1.0 ± 1.0 *** |
| | Thrombotic | 11 ± 4 | 0.1 ± 0.1 *** |
| | Total | 35 ± 5 | 1.0 ± 1.0 *** |
| | | | |
| Renal angiopathy | | | |
| (lesions/100 glomeruli) | | | |
| | Thrombotic | 25 ± 4 | 1.0 ± 1.0 *** |
| | Proliferative | 9 ± 1 | 0.1 ± 0.1 *** |
| | Total | 34 ± 4 | 1.0 ± 1.0 *** |

| | | | |
|---|---|---|---|
| Stroke-prone spontaneously hypertensive rats (SHRSP) were chronically treated with either eplerenone (100 mg/kg/d, by gavage, divided between two doses) or vehicle (0.5% methylcellulose, 2 ml/kg/d, by gavage) starting at 8.4 weeks of age. All animals were maintained on a 1% NaCl drinking solution and Stroke-Prone Rodent Diet starting at 8.1 weeks of age and the experiment was terminated 5 weeks later when rats were 13.1 weeks old. *** *P* < .001 compared with vehicle. Values are mean ± SEM. | | | |

**TABLE A-8B**

| Lesion | Captopril (CAP) + Vehicle (n = 5) | CAP, Angiotensin II + Vehicle (n = 7) | CAP, Angiotensin II + Eplerenone (n = 7) |
|---|---|---|---|
| Glomerular damage | | | |
| (lesions/100 glomeruli) | | | |
| Ischemic | 0 ± 0 | 13 ± 3 | 3.0 ± 1.0** |
| Thrombotic | 0±0 | 2 ± 1 | 1.0 + 0.3 |
| Total | 0±0 | 15 ± 3 | 3.0 ± 1.0** |
| Renal arteriopathy | | | |
| (lesions/100 glomeruli) | | | |
| Thrombotic | 0±0 | 15 ± | 4.0 ± 1.0*** |
| Proliferative | 0±0 | 1 ± 1 | 0.1 ± 0.1 |
| Total | 0±0 | 16 ± 2 | 4.0 ± 1.0*** |

Saline-drinking stroke-prone spontaneously hypertensive rats (SHRSP) were treated with captopril (50 mg/kg/d) starting at 8.3 weeks of age. At 9.3 weeks, osmotic minipumps containing angiotensin II (25 ng/min) were implanted subcutaneously in all of the animals and treatment with eplerenone (100 mg/kg/d) or vehicle was started. All animals were maintained on a 1% NaCl drinking solution and Stroke-Prone Rodent Diet and were sacrificed 2 weeks later. ** *P* < .01; *** *P* <.001 v captopril plus angiotensin II plus vehicle. Values are mean ± SEM.

Saline-drinking SHRSP treated with eplerenone showed markedly diminished proteinuria and exhibited almost complete prevention of glomerular and renal vascular lesions. Patchy, possibly ischemic, contraction (retraction) and simplification of tubules observed in the vehicle-treated animals also was largely prevented by eplerenone treatment. Consonant with the ability of eplerenone to protect against renal lesion development in saline-drinking SHRSP is the close parallelism between malignant nephrosclerosis that develops in these rats and those with mineralocorticoid-salt hypertension, a low-renin model of severe hypertension.

The beneficial effects of eplerenone were independent of blood pressure lowering. In rats with mineralocorticoid-induced hypertension, it is unclear whether the pathology (thrombotic microangiopathic lesions) can be attributed to direct mechanical damage due to severe hypertension, a direct effect of the mineralocorticoid independent of hypertension, a combination of both, or other factors. Increased arterial pressure is a prerequisite for the development of thrombotic microangiopathic lesions in response to aldosterone. Our results indicate that hypertension alone is not sufficient for the production of vascular injury and that endogenous mineralocorticoids mediate the development of malignant nephrosclerosis in SHRSP. The onset of severe renal vascular lesions occurs very early in salt-loaded SHRSP and indicates the salt-sensitive nature of lesion development in these animals. Likewise, high-salt intake is required for the induction of renal lesions in mineralocorticoid-treated (deoxycorticosterone acetate-salt) rats. Our observations indicate that high-salt intake, like hypertension, may serve as a prerequisite for aldosterone-induced lesion formation but may not be sufficient for lesion development.

Although mortality, which is typically the consequence of stroke in SHRSP, was not an end point in this study, a very definite protective effect of eplerenone against the development of neurological signs of stroke was observed. Eplerenone had no effect on plasma aldosterone levels (315 ± 35 pg/ml in eplerenone-treated SHRSP and 305 ± 68 pg/ml in vehicle-treated SHRSP), which is consistent with inhibition of aldosterone action rather than aldosterone synthesis. Together, the results of the present study show that the SARA causes vascular protection in saline-drinking SHRSP.

Under these conditions, the levels of angiotensin II in both of these groups should be similar, as endogenous angiotensin II formation is suppressed and exogenous angiotensin II infusion is constant. Infusion of a low dose of angiotensin II (25 ng/min) was effective in inducing proteinuria and the development of thrombotic and proliferative arteriopathy associated with ischemic as well as thrombotic lesions of glomeruli despite concomitant captopril treatment. In preliminary studies, low doses of angiotensin II (ie, 25 ng/min) did not elevate blood pressure in SHRSP. Similar to our findings in saline-drinking SHRSP that were not treated with captopril (Protocol 1), it was observed a marked protective effect of eplerenone against the development of angiotensin II-induced renal vascular injury in captopril-treated, saline-drinking SHRSP, which was not associated with blood pressure lowering. These observations are consistent with the notion that the vascular protective effects of this ACE inhibitor relate specifically to interference with the endogenous RAAS and identify endogenous mineralocorticoids as playing a critical role in the progression of angiotensin II-induced renal injury.

Angiotensin II has an established role as a pathogenic factor in hypertensive vascular disease. Angiotensin II acts not only as a potent vasoconstrictor, but also as a facilitator of adrenergic responses. The beneficial effects of ACE inhibitors have typically been attributed to reductions in the vascular actions of angiotensin II; however, these agents also can suppress plasma aldosterone levels. SHRSP receiving angiotensin II with or without eplerenone maintained plasma aldosterone levels that were significantly elevated relative to captopril-treated SHRSP and were similar to those observed in vehicle-treated SHRSP from Protocol 1. In addition, angiotensin II infusion was associated with renal lesion development, an effect that was largely attenuated by MR antagonism with eplerenone. Our studies were performed in SHRSP, which typically show an impaired inhibition of the RAAS when fed a high-salt diet. This phenomenon could account for the differences observed in our experiment. The present studies reveal a critical contribution of endogenous mineralocorticoids to the progression of renal injury in SHRSP induced by the infusion of Angiotensin II at a low dose. These findings suggest that mineralocorticoids are important mediators of the vascular toxic effects of angiotensin II in SHRSP and are consistent with our previous observation that aldosterone infusion reverses captopril prevention of vascular injury under conditions in which angiotensin II is not infused. Taken together, these findings demonstrate that eplerenone administration is an effective treatment to prevent lesion formation in SHRSP, which may occur secondary to activation of the RAAS.

In summary, chronic administration of eplerenone is efficacious in reducing proteinuria, renal lesions of malignant nephrosclerosis, and stroke signs in saline-drinking SHRSP. Furthermore, chronic exogenous administration of a low dose of Angiotensin II (25 ng/min) reversed the known ability of captopril treatment to reduce plasma aldosterone levels and to prevent the development of renal vascular injury in saline-drinking SHRSP. However, the ability of angiotensin II to induce renal lesions was largely attenuated by selective aldosterone blockade with eplerenone. These effects were independent of major changes in blood pressure.

### Example A-9: Mechanism and Specificity of Action: Mineralocorticoid Receptor Binding In Vivo.

### Pharmacokinetics and Absorption

The pharmacokinetics and metabolism of eplerenone were investigated in the male and female rat after IV and oral administration of [¹⁴C]eplerenone as an aqueous solution at a dose of 15 mg/kg. Plasma, urine and fecal samples were analyzed for total radioactivity. The concentrations of eplerenone and the open lactone-ring form of eplerenone in unacidified pooled plasma were analyzed by an LC/MS/MS procedure. In addition, concentrations of "total eplerenone" (the lactone ring closed form plus the open form) in acidified pooled plasma were analyzed by a separate LC/MS/MS procedure.

After IV administration of [¹⁴C]eplerenone, the elimination half-lives of total radioactivity were 1.9 and 1.6 hours in the male and female rat, respectively. After oral administration of [¹⁴C]eplerenone as an aqueous solution, the mean peak plasma concentrations (Cₘₐₓ) of total radioactivity were reached at 1.1 and 0.8 hours postdose in the male and female, respectively, indicating that the rate of absorption of the radioactive dose was rapid. The mean Cₘₐₓ values of total radioactivity in male and female rats were 7.64 and 7.67 µg equivalents/mL, respectively. The systemic availabilities of total radioactivity after oral administration of [¹⁴C]eplerenone were 59.6% and 66.4% in the male and female rat, respectively, indicating good absorption of eplerenone.

The elimination half-lives of eplerenone after IV administration were 0.803 and 1.14 hours in the male and female rat, respectively. The corresponding values for total eplerenone were 1.01 and 1.14 hour, respectively. The plasma clearance (CL) values of eplerenone were 1.22 and 1.20 L/kg/hr in the male and female rat, respectively. The corresponding values for total eplerenone were 0.983 and 0.694 L/kg/hr, respectively.

After oral administration, there was a noticeable sex difference in plasma concentrations of the parent drug with the higher values in females. Eplerenone was rapidly absorbed achieving Cₘₐₓ of 1.71 µg/mL at 0.5 hours in male rats and 3.54 µg/mL at one hour in the female rats. The systemic availabilities of eplerenone were 25.6% and 66.1% in the male and female rat, respectively. The Cₘₐₓ of total eplerenone were 3.20 and 6.35 µg/mL in the male and female rat, respectively. The systemic availabilities of total eplerenone were 29.4% and 74.2% in the male and female rat, respectively

### Distribution

A rat tissue distribution study was conducted after oral administration of [¹⁴C]eplerenone to the pigmented male rat (Long-Evans Hooded) at a dose of 20 mg/kg as an aqueous solution. The rate of tissue uptake of the radioactive dose was rapid, with most of the tissues reaching Cₘₐₓ at 0.5 hours. The mean Cₘₐₓ in blood and plasma were 4.90 and 8.64 µg equivalents/g, respectively. The tissues with the highest mean Cₘₐₓ values, excluding the gastrointestinal tract tissues, were liver, pancreas, and kidneys, with concentrations of 41.1, 12.1, and 10.1µ equivalents/g, respectively. The tissues with the lowest Cₘₐₓ values were eye (minus lens), brain and spinal cord with concentrations of 0.045, 0.516 and 0.630 µg equivalents/g, respectively. By 96 hours postdose, concentrations of radioactivity were below the limit of detection in all tissues except cecum, kidneys, large intestine and liver, which all showed values of less than 0.024 µg equivalents/g.

### (Reference) Example A-10: Selective Aldosterone Receptor Blockade Improves Endothelial Function in Diet Induced Atherosclerosis:

The efficacy of eplerenone in improving nitric oxide bio-availability was tested to determine whether eplerenone improves or prevents endothelial dysfunction that occurs with atherosclerosis.

*Methods:* New Zealand white rabbits were randomized to four treatment groups. 32 Rabbits were placed on normal (NC) or 1 % cholesterol chow (HC) for a duration of 8 weeks. After the first 2 weeks 16 rabbits were randomized to receive either saline (S) or eplerenone (E, 50 mg/kg twice daily) by gavage feeding for an additional 6 weeks. Rabbits were euthanized at the end of 8 weeks and the aortas extracted for isometric tension studies and estimation of superoxide (O₂⁻) generation in vessel segments by lucigenin chemiluminescence (250 µM). Vessels were preconstricted with phenylephrine (3 x 10⁻⁷) to approximately 50% of peak constriction and dose responses to acetylcholine (Ach) and nitroglycerin (NTG) tested.

*Results:* The peak relaxations to Ach, NTG, ED₅₀ (M) values and O₂⁻ counts (per mg of dry weight) are depicted below in Table A-10:

**Table A-10**

| Groups | %Ach | ED₅₀ Ach | % NTG | ED₅₀ NTG | O₂ counts |
|---|---|---|---|---|---|
| NC-S | 97±2 | 2.5 x 10⁻⁸ | 104±2 | 7.1 x 10⁻⁹ | 1478±352 |
| NC-E | 97±3 | 3.2 x 10⁻⁸ | 107±2 | 5.7 x 10⁻⁹ | 1110±373 |
| HC-S | 61±4# | 1.2 x 10⁻⁷# | 104±3 | 1.3 x 10⁻⁸ | 3445±863# |
| HC-E | 82±6* | 6.8 x 10⁻⁸ | 112±4 | 1.0 x 10⁻⁸ | 1400±504* |

| | | | | | |
|---|---|---|---|---|---|
| *= *p≤0.01 vs. HC-S, #* = *p<0.05 vs. NC-S and NC-E* | | | | | |

*Conclusion:* Eplerenone improves endothelial function and reduces O₂⁻ generation in diet induced atherosclerosis. These data provide evidence that Eplerenone will provide an additional therapeutic strategy for conditions where endothelial function is compromised.

Abbreviations Used for presentation of Pharmacokinetic Data:
- ANCOVA: Analysis of Covariance
- AUC: Area Under the Plasma Concentration-Time Curve
- Cₘₐₓ: Maximum Plasma Concentration
- Cₘᵢₙ: Minimum Plasma Concentration
- CI: Confidence Interval
- CL/F: Apparent Plasma Clearance
- CRF: Case Report Form
- CV: Coefficient of Variation
- Kₑₗ: Terminal Phase Elimination Rate Constant
- lqc: Last Quantifiable Concentration
- T_{1/2}: Plasma Elimination Half-Life
- Tₘₐₓ: Time to Maximum Plasma/Blood Concentration
- XU: Total Amount of Analyte Collected in Urine

### Example A-11: Pharmacokinetic Studies

Five studies (including single and multiple dose tolerability and food effect trials conducted in Japanese (Japan) and European (Ex-Japan) subjects) were conducted to obtain pharmacokinetic data for eplerenone. A total of 76 European subjects and 38 Japanese subjects participated in the single and multiple dose tolerability and food effect trials. Three of the studies were Ex-Japan studies and included doses ranging from 10 mg to 1000 mg. The other two studies were conducted in Japan and included doses ranging from 50 mg to 600 mg.

The Ex-Japan trials included (i) a single dose tolerability trial, and (ii) a multiple dose tolerability trial, and (iii) a food effect study. The single dose Ex-Japan trial investigated the administration of 10, 50, 100, 300 or 1000 mg doses of eplerenone. A single oral dose of eplerenone was given to 40 healthy male subjects. The Ex-Japan multiple dose tolerability trial investigated the multiple dose effects of eplerenone given for 11 days at doses of 100, 300 and 1000 mg. Eplerenone was given to 24 healthy males administered in a dose escalating fashion. The ex-Japan food effect study was conducted in 12 healthy males who received single oral 100 mg doses of eplerenone on two separate occasions in a crossover fashion, either following an overnight fast or immediately after consuming a high fat (75 g) meal.

Both single dose and multiple dose tolerability trials were conducted in Japan utilizing healthy male Japanese subjects. The Japanese single dose tolerability trial investigated the administration of single oral eplerenone doses of 50, 100, 200, 400 and 600 mg in 32 subjects. Six subjects who received the 100 mg dose also participated in the food effect treatment arm of the single dose tolerability trial. These six subjects received a single 100 mg oral dose of eplerenone on two separate occasions in a crossover fashion; either following an overnight fast or immediately after consuming a fat meal. The multiple dose tolerability trial (JE3-99-06-401) was conducted in 6 healthy male subjects who received 7 once-daily doses of eplerenone 400mg.

Combining single dose fasting pharmacokinetic data from all five studies, an analysis of variance with factors for country (Japan, Ex-Japan), study nested within country, and dose were performed on the dose-normalized Cₘₐₓ and AUC values to assess the effect of country (population). Dose normalization was done to the lowest dose (10 mg for single dose, 100 mg for multiple dose). In the absence of intravenous data, the distribution volume (V/F) and clearance (CL/F) parameters are represented as the apparent values and are normalized to 70 kg of body weight. There were no statistically significant differences between the Japan and Ex-Japan trials in any of the pharmacokinetic parameters analyzed. Formal statistical analyses and comparison of the multiple dose data was not performed due to having only the 400 mg eplerenone dose in the Japanese trial as compared to the 3 doses in the Ex-Japan trial.

Figures A-20 through A-24 and A-25 through A-29 display graphically derived single dose pharmacokinetic parameters versus dose for eplerenone and the inactive open lactone ring form of eplerenone, respectively.

Tables A-11A and A-11B present the least squares means of the single dose pharmacokinetic parameters of eplerenone and its inactive open lactone ring in the Ex-Japan studies and Japan studies. No statistically significant differences were noted between the Japan and ex-Japan population for any of the derived parameters evaluated.

Table A-11C contains the geometric least squares means for the fed and fasting regimens, the ratios of means and the corresponding 95% confidence intervals (CI) for the ratios of the means for the Japan and Ex-Japan food effect trials. An analysis of variance (ANOVA) model with effects for sequence, subject nested within sequence, period and regimen was used to analyze Cₘₐₓ and AUC parameters, and consequently to obtain the geometric least squares means, the ratios of the means and the 95% CI for the ratio of the means.

Tables A-11D and A-11E present the arithmetic mean of the dose normalized pharmacokinetic parameters for the Ex-Japan 100, 300 and 1000 mg eplerenone doses and the Japan 400 mg eplerenone dose trials. No formal statistical analyses were performed and the actual derived data is presented for comparison purposes only.

Comparison of the Japan and Ex-Japan single dose pharmacokinetic data shows no statistically significant differences between the derived pharmacokinetic parameters. The food effect observed in the Japan data for total exposure as reflected in the AUC parameters is similar to that observed in the ex-Japan trial. The mean multiple dose pharmacokinetic parameters for the Japan trial (400 mg) align reasonably well with the data obtained from the Ex-Japan trial.

**Table A-11A. Least Squares Means for Single Dose Eplerenone Pharmacokinetic Parameters**

| | Japan | Ex-Japan | P-value^{a} |
|---|---|---|---|
| Dose Normalized Cₘₐₓ(ng/mL) | 120.0 | 131.0 | 0.412 |
| Dose Normalized AUC (0-lqc) hr•ng/mL | 779.0 | 790.0 | 0.932 |
| Dose Normalized AUC (0-inf) hr•ng/mL | 826.0 | 792.0 | 0.782 |
| CL/F(L/hr) | 13.79 | 13.69 | 0.955 |
| CL/F (L/hr/70 kg) | 13.52 | 14.44 | 0.639 |
| Vss/F(L) | 80.11 | 80.58 | 0.944 |
| Vss/F (L/70 kg) | 81.42 | 83.83 | 0.796 |

| | | | |
|---|---|---|---|
| a-F-test, P-value for the comparison between Japan and Ex-Japan from an ANOVA model | | | |

**Table A-11B. Least Squares Means for Single Dose SC-70303 Pharmacokinetic Parameters**

| | Japan | Ex-Japan | P-value^{a} |
|---|---|---|---|
| Dose Normalized Cₘₐₓ (ng/mL) | 9.71 | 8.93 | 0.518 |
| Dose Normalized AUC (0-Iqc) hr•ng/mL | 49.8 | 40.6 | 0.295 |
| Dose Normalized AUC (0-inf) hr•ng/mL | 52.4 | 48.5 | 0.669 |

| | | | |
|---|---|---|---|
| a-F-test, P-value for the comparison between Japan and Ex-Japan from an ANOVA model | | | |

**Table A-11C. Least Squares Means for Eplerenone Pharmacokinetic Parameters in Fed and Fasted State**

| | | | Mean Ratio | |
|---|---|---|---|---|
| Ex-Japan Food Effect Study | Fed | Fasted | (95% Confidence interval) | P-value^{a} |
| | | | 0.814 | |
| Cₘₐₓ(ng/mL) | 1318 | 2619 | (0.745-0.889) | 0.0004 |
| | | | 1.079 | |
| AUC (0-lqc) hr•ng/mL | 9647 | 8938 | (0.915-1.273) | 0.328 |
| | | | 1.079 | |
| AUC (0-inf) hr•ng/mL | 9781 | 9062 | (0.916-1.272) | 0.324 |
| | | | | |

| Japan Food Effect Study | | | Mean Ratio | |
|---|---|---|---|---|
| | Fed | Fasted | (95% Confidence Interval) | P-value^{a} |
| Cₘₐₓ(ng/mL) | | | 1.157 | |
| | 1517 | 1311 | (1.070-1.252) | 0.0066 |
| | | | 1.218 | |
| AUC (0-lqc) hr•ng/mL | 10818 | 8880 | (0.958-1.550) | 0.085 |
| | | | 1.218 | |
| AUC (0-inf) hr•ng/mL | 10929 | 8971 | (0.956-1.553) | 0.087 |

| | | | | |
|---|---|---|---|---|
| a-F-test, P-value for the comparison between fed and fasting regimen from an ANOVA model | | | | |

**Table A-11D. Arithmetic Means for Multiple Dose Eplerenone Pharmacokinetic Parameters**

| Ex-Japan Multiple Dose Study | 100 mg | 300 mg | 1000 mg |
|---|---|---|---|
| Dose Normalized Cₘₐₓ (ng/mL) | 1903.6 | 1194.1 | 739.4 |
| Dose Normalized AUC (0-24) hr•ng/mL | 11771.99 | 8838.0 | 6324.9 |
| CL/F (L/hr/70 kg) | 9.38 | 15.8 | 17.0 |
| | | | |

| Japan Multiple Dose Study | 400 mg | | |
|---|---|---|---|
| Dose Normalized Cₘₐₓ(ng/mL) | 1030.8 | | |
| Dose Normalized AUC (0-24) hr•ng/mL | 6380.0 | | |
| CL/F (L/hr/70 kg) | 16.2 | | |

**Table A-11E. Arithmetic Means for Multiple Dose SC-70303 Pharmacokinetic Parameters**

| Ex-Japan Multiple Dose Study | 100 mg | 300 mg | 1000 mg |
|---|---|---|---|
| Dose Normalized Cₘₐₓ (ng/mL) | 128.5 | 121.4 | 83.03 |
| Dose Normalized AUC (0-24) hr•ng/mL | 663.4 | 733.4 | 631.0 |
| | | | |

| Japan Multiple Dose Study | 400 mg | | |
|---|---|---|---|
| Dose Normalized Cₘₐₓ (ng/mL) | 84.68 | | |
| Dose Normalized AUC (0-24) hr•ng/mL | 467.5 | | |

This study was a single-blind, randomized, placebo-controlled, rising oral dose, sequential panel study in which 40 healthy Japanese male subjects were exposed to one of five panels of eplerenone doses (50, 100, 200, 400 and 600 mg). Each panel consisted of six subjects who were given eplerenone and two subjects who were given placebo. The effect of food was also evaluated in the same study in six subjects in a crossover design by administering a single dose of 100 mg eplerenone in the fasted and fed states. Serial blood and urine samples were collected over a 48-hour period to evaluate the pharmacokinetics of eplerenone.

No clinically significant abnormalities were found in any of the clinical lab parameters measured and no adverse events were reported from any of the volunteers who participated in the study.

### Example A-12: Multiple Dose Japanese Pharmacokinetic Study

A multiple-dose safety and pharmacokinetic study among Japanese subjects was conducted in Japan. This was a single-blind, randomized, placebo-controlled study. A total of eight Japanese male subjects participated in this study (six of them received a single daily dose of 400 mg of eplerenone and two of them received placebo for seven consecutive days). Serial blood and urine samples were collected throughout the study for measurement of pharmacokinetic, renal, and hormonal parameters after a single dose on Day 1 and after seven days of dosing.

One subject who received eplerenone experienced mild fatigue on Day 2 but no other adverse events were reported from any of the other seven subjects.

### (Reference) Example A-13: Hepatic impairment

This was an open-label, multiple dose study conducted in 16 normal healthy subjects and 16 subjects with moderate hepatic impairment. The degree of the subject's impairment was Class B (with ascites) as based on the Child-Pugh Classification System. These hepatically-impaired individuals were matched with normal healthy volunteers based on sex, age, weight and smoking status.

All study participants received a single 400 mg dose of eplerenone on the morning of Day 1. They did not receive study drug on Day 2. Subjects received a single 400 mg dose of eplerenone on Days 3-7. Blood and urine samples for pharmacokinetic analyses were collected at predose and following dosing on Days 1 and 7.

For subjects with hepatic impairment, medications related to the subject's liver disease or its complications were allowed. No medication that had the potential of influencing the outcome of the study was allowed. The dosing of any concomitant medication was at a consistent regimen throughout the study.

The subjects were maintained on a controlled salt diet (50 mEq sodium and 80 mEq potassium per day) from five days prior to the first dose of study drug until the end of the dosing period. Creatinine clearance rate and sodium excretion rate were determined at baseline and for each 24-hour period during study drug administration.

The eplerenone bioavailability in moderately hepatic-impaired subjects was compared with that in healthy subjects mainly based on ratio of geometric means and 95% confidence intervals for the ratio of means for those log-normal pharmacokinetic response variables. The ratio of geometric means and 95% confidence intervals for the ratio of means were derived according to the following steps: using LSMEANS option of SAS GLM procedure to obtain the least differences and their corresponding standard error estimate obtained from the ANCOVA to construct 95% confidence intervals for mean difference; anti-log transforming the mean differences and the endpoints of the confidence intervals for the mean differences to obtain ratios of geometric means and the 95% confidence intervals for ratio of means.

Trough plasma concentration (Cₘᵢₙ) data were summarized for eplerenone on Days 4-8 for the two hepatic groups separately. Repeated measures analyses were performed on Cₘᵢₙ data for eplerenone on Days 6-8 to assess whether steady-state for eplerenone had been achieved by the fourth day of eplerenone 400 mg QD multiple dosing.

Linear kinetics were evaluated based on the comparison of the multiple-dose AUC₀₋₂₄ with the single-dose AUC_{0-∞} using ratio of geometric means between multiple-dose AUC₀₋₂₄ and single-dose AUC_{0-∞}, and 95% confidence intervals for the ratio of means derived from a paired t-test using the log-transformed AUC data.

As normal subjects were matched with those with hepatic impairment, there were no statistically significant differences between subjects with hepatic impairment and matched normal subjects with respect to gender, weight, and age. In each of the hepatic function and matched normal groups, subjects were primarily Caucasian (≥ 82%). Subjects ranged in age from 32 to 64 years, with the mean age in each group being approximately 46-51 years. The majority of the subjects in each group were male (≥ 65%).

The etiology of hepatic impairment was determined to be primarily alcoholic cirrhosis (56%), followed by infectious cirrhosis (17%), chronic active hepatitis (6%), and other (17%). Subjects with moderate hepatic impairment had been diagnosed for a mean of 6.6 years. Smoking was reported for 28% (5/18) of the subjects with moderate impairment compared to 35% (6/17) of the matched normal subjects.

**Table A-13A Summary Statistics for Eplerenone Following Single and Multiple Dosing**

| | | Eplerenone | | | |
|---|---|---|---|---|---|
| | | Single Dose | | Multiple Dose | |
| Parameter (a) | | Normal | Moderate | Normal | Moderate |
| AUC (ng/mL*hr) (b) | | | | | |
| | n | 16 | 17 | 17 | 16 |
| | Mean | 31015.37 | 51438.98 | 33784.48 | 48821.25 |
| | %CV | (38.73) | (46.50) | (46.94) | (32.87) |
| Cmax (ng/mL) | | | | | |
| | n | 17 | 18 | 17 | 18 |
| | Mean | 3840.23 | 3787.53 | 3883.18 | 4168.10 |
| | %CV | (28.30) | (35.69) | (33.17) | (26.95) |
| Tmax (hr) | | | | | |
| | n | 17 | 18 | 17 | 18 |
| | Mean | 2.03 | 2.56 | 2.12 | 2.81 |
| | %CV | (49.64) | (42.38) | (49.05) | (50.83) |
| T_{1/2} (hr) | | | | | |
| | n | 16 | 17 | 17 | 18 |
| | Mean | 6.31 | 6.78 | 7.61 | 8.07 |
| | %CV | (37.20) | (25.49) | (26.67) | (26.58) |
| CL/F (L/hr) (c) | | | | | |
| | n | 16 | 17 | 17 | 16 |
| | Mean | 15.21 | 10.51 | 13.82 | 8.96 |
| | %CV | (46.33) | (81.02) | (36.01) | (29.33) |
| XU₀₋₄₈ (µg) | | | | | |
| | n | 16 | 14 | 16 | 10 |
| | Mean | 8.78 | 10.27 | 9.12 | 16.92 |
| | %CV | (64.97) | (75.25) | (86.79) | (71.42) |

The geometric least squares means, 95% CIs and corresponding p-values for Eplerenone pharmacokinetic parameters are presented in Tables A-13B.

**Table A-13B Bioavailability Assessments**

| Parameter | | Least Squares Means | | | | |
|---|---|---|---|---|---|---|
| | | Moderate Impairment | Normal | Ratio of Means Moderate/Normal | 95% CI for Ratio of Means | p-Valuc |
| EplerenoneSingle Dose | | | | | | |
| | AUC (0-∞) (ng/mL*hr) | 43578.3 | 29114.1 | 1.497 | (1.056, 2.121) | 0.025 * |
| | AUC (0-lqc) (ng/mL*hr) | 42766.8 | 30276.5 | 1.413 | (1.004, 1.988) | 0.048 * |
| | CL/.F (L/hr) | 9.2 | 13.7 | 0.668 | (0.471, 0.947) | 0.025 * |
| | Cmax (ng/mL) | 3406.8 | 3812.5 | 0.894 | (0.723, 1.105) | 0.288 |
| | Tmax(hr) | 2.7 | 2.1 | -- | -- | 0.084 |
| | XU(0-48)(mg) | 7.9 | 7.8 | 1.016 | (0.540, 1.915) | 0.958 |
| EplerenoneMultiple Dose | | | | | | |
| | AUC (0-24) (ng/mL*hr) | 43765.9 | 30066.4 | 1.456 | (1.163, 1.821) | 0.002 ** |
| | CL/F(L/hr) | 9.1 | 13.3 | 0.687 | (0.549,0.860) | 0.002 ** |
| | Cmax (ng/mL) | 3808.8 | 3674.6 | 1.037 | (0.863,1.245) | 0.693 |
| | Tmax (hr) | 2.9 | 2.1 | -- | -- | 0.091 |
| | XU (0-48) (mg) | 14.9 | 7.4 | 2.007 | (1.007, 3.999) | 0.047 * |
| * p < 0.05; ** p <0.01; *** p < 0.001 | | | | | | |

**Table A-13C Linear Kinetics Assessment**

| Parameter | Least Squares Means | | | |
|---|---|---|---|---|
| | Single Dose AUC(0-co) | Multiple Dose AUC (0-24) | Ratio of Means AUC (0-24)/AUC0-co) | 95% CI for Ratio of Means |
| Eplerenone | | | | |
| Moderate Hepatic Impairment | 49815.47 | 47239.78 | 0.95 | (0.84, 1.07) |
| Normal | 28713.97 | 29318.67 | 1.02 | (0.93, 1.12) |
| | | | | |

The results of the repeated measures analysis on trough plasma concentrations on Days 6 through 8 indicate that steady-state concentrations of eplerenone were achieved by the fourth day of eplerenone 400 mg QD dosing (p-values for day effect were 0.0761 for normal subjects and 0.1118 for subjects with moderate hepatic impairment).

Eplerenone plasma concentrations were consistently greater among subjects with moderate hepatic impairment than among normal subjects after three hours following single dosing and at all time points following multiple dosing (Figure A-30)

This study showed that the metabolism of eplerenone was not adversely affected by moderate hepatic impairment. Following single-dose (400 mg) and multiple dose (400 mg QD) eplerenone administration, the exposures of eplerenone in plasma (as represented by AUC) were statistically significantly greater among subjects with moderate hepatic impairment than matched normal subjects (41-50% greater for eplerenone). Steady-state plasma concentrations of eplerenone were reached by the fourth day following multiple 400 mg QD dosing of Eplerenone.

Subjects with moderate hepatic impairment experienced statistically significant increases in AUC values compared to matched normal subjects following both single (AUC_{0-∞}, 43578.3 vs. 29114.1 ng/mL hr) and multiple (AUC₀₋₂₄, 43765.9 vs. 30066.4 ng/mL hr) dosing. Among subjects with moderate impairment, mean eplerenone AUC values were 41-50% higher following single dosing and 46% higher following multiple dosing compared to matched normal subjects. However, mean peak eplerenone plasma concentrations (Cₘₐₓ) and the time to reach eplerenone Cₘₐₓ (Tₘₐₓ) were not statistically significant different between the subjects groups. Linear kinetics following multiple dosing of Eplerenone 400 mg QD were established for eplerenone. The 95% confidence intervals for the ratios of the mean AUC values (AUC₀₋₂₄/AUC_{0-∞}) included the equality point, 1.0, for both analytes. Repeated measures analysis on trough plasma concentrations on Days 6-8 indicated that steady-state concentrations of eplerenone was achieved by the fourth day of eplerenone 400 mg QD dosing in both subject groups (p-value for day effect ≥0.0761).

Although the overall exposure among subjects with moderate impairment was greater than that among normal subjects, the study medication appeared to be equally well tolerated among the subject groups. Adverse events were reported for 35% of the subjects with normal hepatic function and 44% of the subjects with moderate hepatic impairment. Adverse events were primarily mild to moderate in severity and had an uncertain or probable relationship to study medication. No adverse events causing withdrawal or serious adverse events were reported.

Eplerenone administration, the exposures of eplerenone in plasma (as represented by AUC) were statistically significantly greater among subjects with moderate hepatic impairment than matched normal subjects (41-50% greater for Eplerenone. Steady-state plasma concentrations of eplerenone were reached by the fourth day following multiple 400 mg QD dosing of eplerenone. Although the exposure of eplerenone among subjects with moderate impairment was greater than that among normal subjects, the study medication appeared to be equally well tolerated in the two subject groups.

### (Reference) Example A-14: Clinical Efficacy of Eplerenone in Treating Hypertension

A multicenter, randomized, double-blind, parallel group study was conducted in patients with hypertension. The study was designed to compare the effects on blood pressure (BP), tolerability and safety of a range of doses of eplerenone (EPL) to placebo (PL). Spironolactone (SPL) was administered as an active control. The study consisted of a two-week pretreatment period for completion of screening procedures and discontinuation of any current antihypertensive medication(s). The screening period was followed by a four-week single-blind placebo lead-in treatment period. Randomization was followed by an eight-week double-blind active or placebo treatment period.

The primary efficacy measure was the change from baseline in cuff diastolic blood pressure measured at trough as compared to placebo. Secondary efficacy measures included changes in cuff assessed trough systolic blood pressure and changes in average 24-hour diastolic and systolic blood pressure assessed by ambulatory blood pressure monitoring. Safety was assessed by conducting routine clinical lab tests, physical exams, ECGs, and monitoring for adverse events.

A total of 417 patients were randomly allocated to receive one of three daily doses: (1) eplerenone (50,100, or 400 mg, administered QD or in divided doses), (2) spironolactone (100 mg/day administered in divided doses), or (3) placebo. The adjusted mean change in blood pressure (mm Hg) from baseline to final visit is described in Table A-14A.

**Table A-14A: Blood Pressure Changes During Double-Blind Treatment with Eplerenone, Spironolactone, and Placebo**

| | | EPL | | | | | | SPL | PL |
|---|---|---|---|---|---|---|---|---|---|
| mg/day | | 50 mg | 50 mg | 100 mg | 100 mg | 400 mg | 400 mg | 100 mg | |
| (regimen) | | (25 BID) | (50 QD) | (50 BID) | (100 QD) | (200 BID) | (400 QD) | (50 BID) | |
| Cuff | DBP | -4.4 | -4.5 | -7.8 | -4.4 | -8.9 | -8.7 | -9.5 | -1.1 |
| | SBP | -8.1 | -4.4 | -11.7 | -7.9 | -14.8 | -15.0 | -16.7 | 1.6 |
| ABPM | DBP | -4.1 | -5.1 | -6.6 | -5.6 | -9.0 | -7.7 | -8.7 | 0.4 |
| | SBP | -7.5 | -6.2 | -11.6 | -9.6 | -16.1 | -13.7 | -15.8 | 0.0 |
| ABPM = Ambulatory Blood Pressure Monitoring QD = Once daily BID = Twice daily | | | | | | | | | |

These responses differed significantly from placebo (p ±0.05). Reductions in 24-hour ABPM DBP and SBP at trough (last four hours) were similar whether EPL was given QD or BID. Peak to trough ratios for QD and BID regimens were similar for all doses (1.04 - 1.10), indicating sustained antihypertensive efficacy over 24 hours with once-a-day administration of eplerenone. Eplerenone was well tolerated and the incidence of adverse events was similar to placebo with no reports of gynecomastia.

Subgroup analyses of the primary and secondary efficacy measures can be performed with respect to subgroups based on, for example, baseline recordings of such factors as race (black, non-black, Japanese, etc.), sex, age, plasma renin levels, aldosterone/renin activities ratio, urinary sodium to potassium ratio, presence of diabetes, history of hypertension, history of heart failure, history of renal dysfunction, and the like. Subgroups based on continuous measures such as age can be dichotomized at the median value.

### (Reference) Example A-15: Comparison Study of the Efficacy and Safety of Eplerenone and Enalapril Alone and in Combination With Each Other in Patients With Left Ventricular Hypertrophy and Essential Hypertension.

A clinical study is conducted to evaluate the effect of enalapril and eplerenone, given alone and in combination with each other, following nine months of treatment on change in blood pressure (BP) and on change in left ventricular mass (LVM) as measured by magnetic resonance imaging (MRI) in patients with left ventricular hypertrophy (LVH) and with essential hypertension. The study is a multicenter, randomized, double-blind, placebo run-in, parallel group trial involving a minimum of 150 completed patients with LVH and essential hypertension and consisting of a one- to two-week pretreatment screening period followed by a two-week single-blind placebo run-in period and a nine-month double-blind treatment period.

Patients who will enter the single-blind placebo run-in period (1) will have a prior electrocardiogram that shows LVH (a) by the Sokolow Lyon voltage criteria (Sokolow M et al. Am Heart J 1949;37:161), or (b) by the Devereux criteria (LVMI =134 g/m² for males and =110 g/m² for females; see Neaton JD et al. JAMA 1993;27:713-724); and (2) will have a seated blood pressure that as follows: (a) seDBP <110 mmHg and seSBP =180 mmHg if currently treated with antihypertensive medication, or (b) seDBP =85 mmHg and <114 mmHg and seSBP >140 mmHg and =200 mmHg if not currently treated with antihypertensive medication.

During the single-blind placebo run-in period at Visit 2, all patients must have an echocardiogram that demonstrates LVH per the Devereux criteria. After completing the two-week single-blind placebo run-in period, and after an MRI has been received, and approved as acceptable by the core laboratory, patients will be randomized to one of three groups: eplerenone, enalapril, or eplerenone plus enalapril 10 mg. For the first two weeks of double-blind treatment patients will receive (1) eplerenone 50 mg plus placebo, (2) enalapril 10 mg plus placebo, or (3) eplerenone 50 mg plus enalapril 10 mg. The dose of study medication will be force-titrated for all patients at Week 2 to (1) eplerenone 100 mg plus placebo, (2) enalapril 20 mg plus placebo, or (3) eplerenone 100 mg plus enalapril 10 mg. At Week 4 the dose of study medication will be force-titrated for all patients to (1) eplerenone 200 mg plus placebo, (1) enalapril 40 mg plus placebo, or (3) eplerenone 200 mg plus enalapril 10 mg). Table A-15A illustrates forth the above-described dosing scheme.

**Table A-15A. Study Medication Dose Levels**

| Dose Levels | Randomized Study Medication | | | Number of Tablets/Capsules |
|---|---|---|---|---|
| | Eplerenone | Enalapril | Eple + Enalp | |
| Placebo Run-In | Placebo | Placebo | Placebo | 1 tablet/1 capsule |
| Dose 1 | 50 mg | 10 mg | (50 + 10) mg | 1 tablet/1 capsule |
| Dose 2 | 100 mg | 20 mg | (100 +10) mg | 1 tablet/1 capsule |
| Dose 3 | 200 mg | 40 mg | (200 +10) mg | 2 tablets/2 capsules |

| | | | | |
|---|---|---|---|---|
| Hydrochlorothiazide 12.5 Amlodipine 10 mg | | | | |

If BP is not controlled (DBP =90 mmHg or SBP >180 mmHg) at Week 8, open-label hydrochlorothiazide (HCTZ) 12.5 mg will be added. If BP is uncontrolled at Week 10, (1) the HCTZ dose will be increased to 25 mg if HCTZ was started at Week 8, or (2) HCTZ 12.5 mg will be added if not done so at Week 8. If BP is not controlled at Week 12, (1) open-label HCTZ 12.5 mg will be added if not previously done so at Weeks 8 or 10, or (2) the HCTZ dose will be increased to 25 mg if not done so at Week 10, or (3) amlodipine 10 mg will be added if the patient is receiving HCTZ 25 mg. If at Week 16 or at any subsequent visit, the patient exhibits sustained uncontrolled DBP (i.e., seDBP =90 mmHg or seSBP >180 mmHg which persists at two consecutive visits, 3-10 days apart), the patient will be withdrawn from study participation.

If a patient is taking double-blind treatment alone and experiences symptomatic hypotension at any time during the trial, the patient will be withdrawn. Those patients taking open-label medications will have the open-label medications down-titrated in the reverse sequence as they were added until hypotension is resolved. If after all open-label medications are discontinued symptomatic hypotension is still present, the patient will be withdrawn from the trial. At any time during the study, if serum potassium level is elevated (>5.5 mEq/L) on repeat measurement (with BUN and creatinine levels drawn as well, sample split and sent to local and central laboratories, treatment decision based on local value) at two consecutive visits 1-3 days apart, the patient will be withdrawn. NOTE: If BUN and/or creatinine levels are significantly elevated over baseline (creatinine =2.0 mg/dL or =1.5x baseline value or BUN =35 mg/dL or =2x baseline value), the patient should be followed under medical treatment until resolved.

Patients will return to the clinic for evaluations at Weeks 0, 2, 4, 6, 8, 10, 12, 16, and monthly thereafter for a total of nine months. Heart rate, BP, serum potassium levels, and adverse events will be assessed at each visit. BUN and creatinine levels will be determined at Weeks 2 and 6. Additional laboratory assessments of blood for clinical safety will be done monthly. Routine urinalysis will be done every three months. A neurohormone profile (plasma renin [total and active], serum aldosterone, and plasma cortisol) and special studies (PIIINP, PAI, microalbuminuria, and tPA) will be done at Weeks 0, 12, and at Months 6 and 9. A blood sample for genotyping will be collected at Week 0. At screening and at Month 9, a 12-lead ECG and physical examination will be done. An MRI to assess changes in LV mass, a blood sample for storage retention, a blood sample for thyroid stimulating hormone (TSH), and a 24-hour urine collection for albumin, potassium, sodium, and creatinine will be done at Week 0 and at Month 9. A 24-hour urine collection for urinary aldosterone will be done at Weeks 0,12 and at Months 6 and 9. In case of early termination, an MRI and blood sample for TSH will be done for those patients who have received double-blind treatment for at least three months. At Weeks 0, 12 and Months 6 and 9, pharmacoeconomic data will be collected on all patients.

The schematic below illustrates the study protocol:

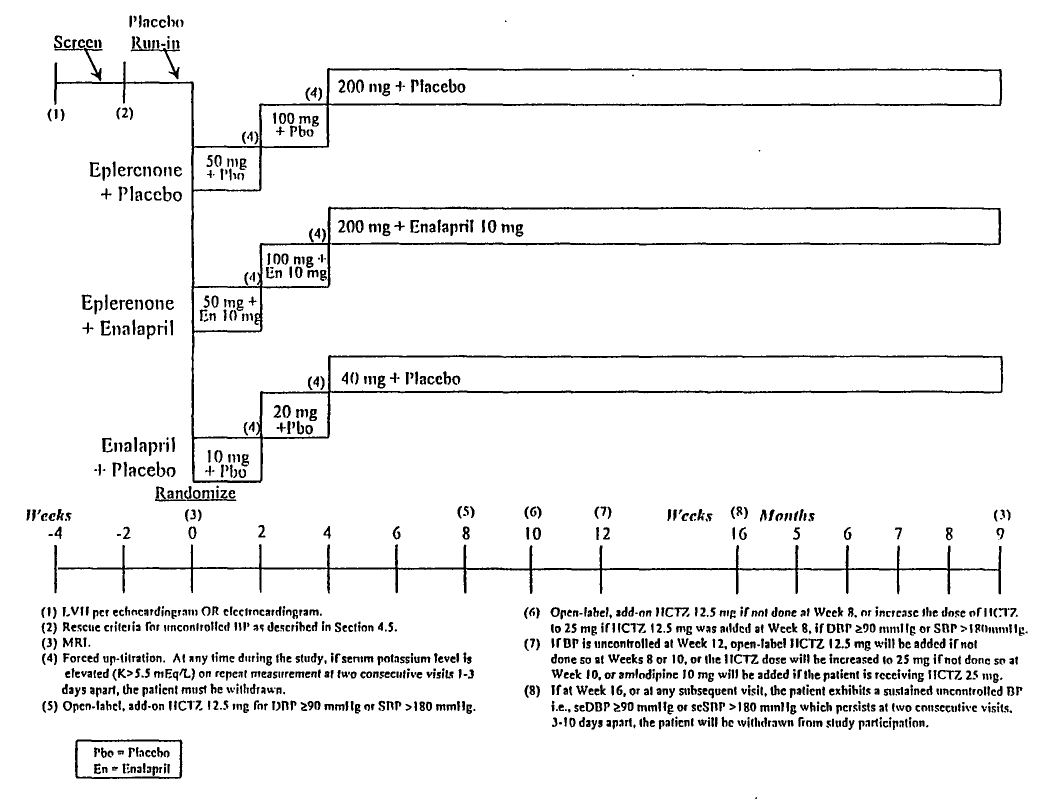

The primary measure of efficacy is the change from baseline in LVM, as assessed by MRI, in the eplerenone group versus the enalapril group. Additionally, efficacy will be evaluated with respect to the patient's degree of salt sensitivity by tertile (wherein tertiles are empirically determined by the increment of blood pressure response to salt challenge).

Secondary measures of efficacy will be the following: (1) the change from baseline in LVM among the three treatment groups; (2) the change from baseline of seated trough cuff DBP (seDBP) and SBP (seSBP) in each of the three treatment groups; (3) aortic compliance and ventricular filling parameters; and (4) special studies (PIIINP, microalbuminuria, PAI, and tPA). Additionally, the long-term safety and tolerability of the three treatment groups will be compared.

The primary objective of the study is to compare the effect of enalapril versus eplerenone on change in left ventricular mass (LVM) in patients with LVH and with essential hypertension. The secondary objectives of the study are the following: (1) to compare the change from baseline in LVM among the three treatment groups; (2) to compare the antihypertensive effect among the three treatment groups as measured by seated trough cuff DBP and SBP; (3) to compare the effect of the three treatment groups on aortic compliance and ventricular filling parameters as measured by MRI; (4) to compare the effect of the three treatment groups on plasma markers of fibrosis by measuring the aminoterminal propeptide of Type III procollagen (PIIINP), on renal glomerular function by measuring microalbuminuria, and on fibrinolytic balance by measuring plasminogen activator inhibitor (PAI) and tissue plasminogen activator (tPA); and (5) to compare the long-term safety and tolerability of the three treatment groups.

Subgroup analyses of the primary and secondary efficacy measures can be performed with respect to other subgroups based on, for example, baseline recordings of such factors as sex, age, plasma renin levels, aldosterone/renin activities ratio, urinary sodium to potassium ratio, presence of diabetes, history of hypertension, history of heart failure, history of renal dysfunction, and the like. Subgroups based on continuous measures such as age can be dichotomized at the median value.

### (Reference) Example A-16: Comparison Study of Eplerenone and Losartan in Patients With Low Renin Hypertension

A clinical study is conducted to compare the antihypertensive effects of eplerenone and losartan in patients with low renin hypertension. The study is a multicenter, double-blind, randomized, placebo run-in, parallel group trial involving a minimum of 150 completed patients. Each patient will be tested for salt sensitivity by salt challenge-unidirectional testing. The trial will also consist of a one- to three-week pretreatment screening period followed by a two- to three-week single-blind placebo run-in period and a 16-week double-blind treatment period. After completing the placebo run-in period and meeting the entry blood pressure (BP) criteria (mean seated diastolic BP [seDBP] =90 mmHg and =115 mmHg and mean seated systolic BP [seSBP] <200 mmHg) on two consecutive run-in visits, patients will be randomized to receive either eplerenone or losartan. Patients will receive eplerenone 100 mg or losartan 50 mg for the first four weeks. If BP is uncontrolled (DBP =90 mmHg) at Week 4, the dose of study medication will be increased to eplerenone 200 mg or losartan 100 mg. If BP is uncontrolled at Week 8, hydrochlorothiazide (HCTZ) 12.5 mg will be added. If BP is uncontrolled at Week 12, HCTZ 12.5 mg will be added if not done so at Week 8, or the HCTZ dose will be increased to 25 mg if it had been started at Week 8. Patients will continue on therapy until Week 16. Table A-16A illustrates the above-described dosing scheme.

**Table A-16A. Study Medication Dose Levels**

| Dose Level | Randomized Study Medication | | Number of Daily Eplerenone Tablets/ Losartan Capsules* |
|---|---|---|---|
| | Eplerenone | Losartan | |
| Placebo Run-in | Placebo | Placebo | 1 tablet/1 capsule |
| Level 1 | 100 mg | 50 mg | 1 tablet/1 capsule |
| Level 2 | 200 mg | 100 mg | 2 tablets/2 capsules |
| * "Eplerenone tablets" refers to active eplerenone or its matching placebo, and "Losartan capsules" refers to active losartan or its matching placebo | | | |

Heart rate, BP, serum potassium levels, and adverse events will be assessed at every visit (Weeks 0, 2, 4, 6, 8, 10, 12, 14, and 16). At Week 0, a blood sample for genotyping and storage retention, and 24-hour urine samples for assessment of aldosterone, potassium, sodium, creatinine, and creatinine clearance will be collected; an aliquot of the Week 0 24-hour urine collection will be retained for storage. Plasma renin will be determined at screening; serum aldosterone, plasma renin, and plasma cortisol will be determined at Weeks 0, 8, and 16. Additional laboratory assessments for clinical safety (hematology, blood chemistry, and urinalysis) will be done at Weeks 0, 4, 8, 12, and 16. A 12-lead electrocardiogram will be done at Weeks 0 and 16. Physical examinations will be completed at screening and at Week 16.

The schematic below illustrates the study protocol:

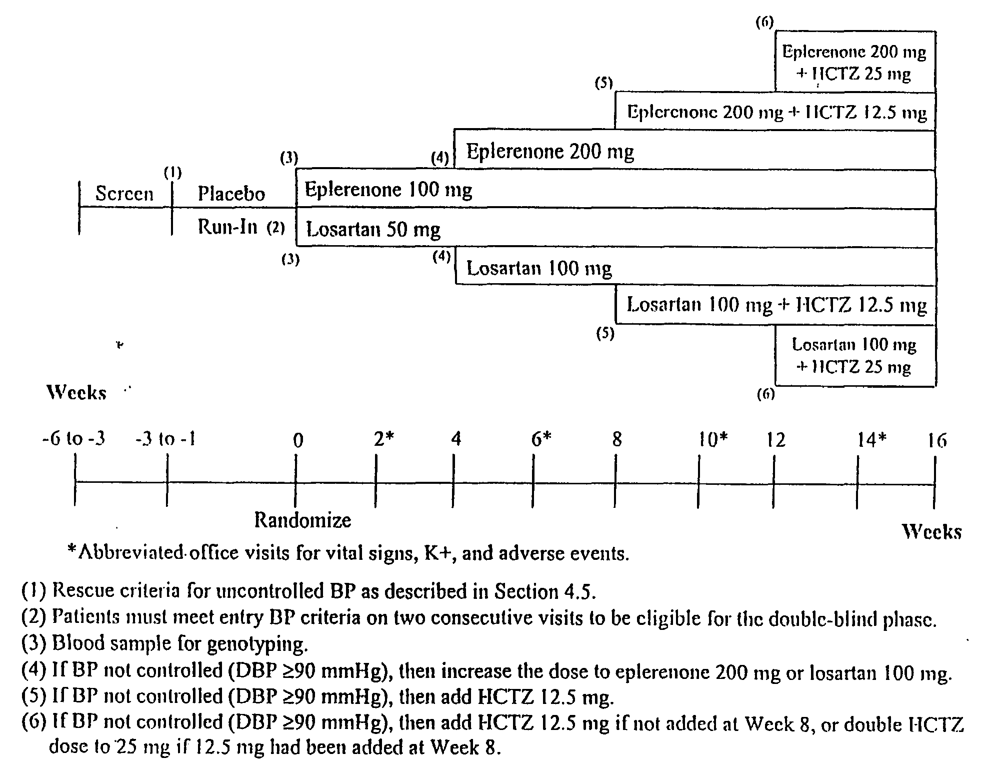

The primary measure of efficacy will be the mean change from baseline in trough cuff seDBP between eplerenone and losartan at Weeks 8 and 16. Additionally, efficacy will be evaluated with respect to the patient's degree of salt sensitivity by tertile (wherein tertiles are empirically determined by the increment of blood pressure response to salt challenge).

Secondary measures of efficacy will be the following: (1) mean change from baseline in trough cuff seSBP at Weeks 8 and 16; (2) percent of patients requiring add-on HCTZ; and (3) percent of patients meeting the goal BP (DBP <90 mmHg) or a reduction of =10 mmHg in DBP. Other secondary measures can be used to evaluate the association between the change from baseline in seDBP and pre-treatment aldosterone/renin ratios and 24-hour urine tests (urinary aldosterone, potassium, sodium, and creatinine, and creatinine clearance) in eplerenone and losartan treated patients, and to evaluate the safety and tolerability of eplerenone and losartan as assessed by reported adverse events, clinical laboratory assessments, physical examination, and electrocardiogram.

In this study of patients with low-renin hypertension, the effectiveness of eplerenone is compared to that of a selective angiotensin II receptor antagonist, losartan. At Weeks 8 and 12, hydrochlorothiazide (HCTZ) can be added for patients with uncontrolled hypertension in both treatment groups. The HCTZ will activate the RAAS, thus illustrating the role of activation in the success of either agent. The primary objective of this study is to compare the mean change from baseline in seated trough cuff diastolic BP (seDBP) of eplerenone vs losartan in patients with low renin hypertension at Weeks 8 and 16. The secondary objectives of this study are the following: (1) to compare the mean change from baseline in seated cuff systolic BP (seSBP) at Weeks 8 and 16, the percent of patients requiring add-on HCTZ, and the percent of patients meeting the goal BP (DBP <90 mmHg or a reduction of =10 mmHg in DBP) in eplerenone versus losartan treated patients; (2) to evaluate the association between the change from baseline in seDBP and pre-treatment aldosterone/renin ratios, and pre-treatment 24-hour urine tests (urinary aldosterone, potassium, sodium, and creatinine levels and creatinine clearance) in eplerenone- and losartan-treated patients; and (3) to evaluate the safety and tolerability of eplerenone and losartan as assessed by reported adverse events, clinical laboratory assessments, physical examination, and electrocardiogram.

Subgroup analyses of the primary and secondary efficacy measures can be performed with respect to subgroups based on, for example, baseline recordings of such factors as race (black, non-black, Japanese, etc.), sex, age, urinary sodium to potassium ratio, presence of diabetes, history of hypertension, history of heart failure, history of renal dysfunction, and the like. Subgroups based on continuous measures such as age can be dichotomized at the median value.

### (Reference) Example A-17: Comparison Study of the Antihypertensive Effect and Safety of Eplerenone Versus Placebo and Versus Losartan in Black and White Hypertensive Patients

A clinical study is conducted to compare the antihypertensive effect and the safety and tolerability of eplerenone versus placebo and versus losartan in hypertensive black patients and in hypertensive white patients. In this study, hypertension is defined as a seated diastolic blood pressure (DBP) = 95 mmHg and < 110 mmHg and seated systolic blood pressure (SBP) < 180 mmHg. The study is a multicenter, randomized, double-blind, placebo- and active-controlled, placebo run-in, parallel group trial involving a minimum of 500 randomized black and white patients with mild to moderate hypertension. Each patient will be tested for salt sensitivity by salt challenge-unidirectional testing. The trial will further consist of a one- to two-week pretreatment screening period followed by a two- to four-week single-blind placebo run-in period and a 16-week double-blind treatment period. Black patients and white patients will be entered into the study in approximately a 2:1 ratio balanced and pre-stratified within each study center. After completing the single-blind placebo run-in period, eligible patients will be randomized to receive either eplerenone, placebo, or losartan.

For the first four weeks of double-blind treatment patients will receive eplerenone 50 mg, losartan 50 mg, or matching placebo. If blood pressure (BP) is uncontrolled (DBP = 90 mmHg or SBP = 140 mmHg) at Weeks 4, 8, or 12, the dose of study medication will be increased to eplerenone 100 mg, losartan 100 mg, or matching placebo. The dose will not be changed for patients with adequate BP control. If the BP is uncontrolled (DBP = 90 mmHg or SBP = 140 mmHg) at Weeks 8 or 12 and the patient is on eplerenone 100 mg, losartan 100 mg, or matching placebo, the dose will be increased to eplerenone 200 mg, losartan 100 mg, or matching placebo. At Weeks 2, 6, 10, and 14, the dose of study drug will not be titrated upward to allow time for the full effect of the current dose. Table A-17A illustrates the above-described dosing scheme.

**Table A-17A. Study Medication Dose Levels**

| Dose Level | Randomized Study Medication | | | Number of Tablets/Capsules |
|---|---|---|---|---|
| | Eplerenone | Losartan | Placebo | |
| Placebo Lead-in | matching placebo | matching placebo | matching placebo | 1 tablet/1 capsule |
| Dose 1 | 50 mg | 50 mg | matching placebo | 1 tablet/1 capsule |
| Dose 2 | 100 mg | 100 mg | matching placebo | 2 tablets/2 capsules |
| Dose 3 | 200 mg | 100 mg | matching placebo | 2 tablets/2 capsules |

If BP is uncontrolled (DBP = 95 mmHg or SBP = 150 mmHg) during Week 12 or thereafter and the patient is on the highest tolerated dose of study drug, he/she is withdrawn from the study. If symptomatic hypotension (i.e., lightheadedness, dizziness, or syncope associated with low BP) occurs at any time during the study, the study drug may be down titrated to the next lower dose; however, if the patient is on the minimum dose of study drug (eplerenone 50 mg, losartan 50 mg, or matching placebo), or if DBP is = 110 mmHg or SBP is = 180 mmHg at two consecutive visits, one to three days apart, any time during the trial, the patient is withdrawn. Patients will receive study medication for a total of 16 weeks.

Patients will return to the clinic for evaluations at Weeks 0, 2, 4, 6, 8, 10, 12, 14, 16, and 17. Heart rate, BP, serum potassium levels, and adverse events will be assessed at each visit. Hematology and biochemistry evaluations and urinalysis for safety will be taken at Weeks 0, 16, and 17, or at Final Visit. Additionally, a neurohormone profile (plasma renin [total and active], serum aldosterone, and plasma cortisol) will be collected at Weeks 0 and 16, or at Final Visit. A spot urine for measurement of microalbuminuria will be done at Weeks 0 and 16 or at Final Visit. A 12-lead electrocardiogram and physical examination will be done at screening and at Week 17, or at Final Visit.

As part of this trial, a substudy of 100 patients per arm (300 patients total) determining population pharmacokinetics will be performed. Visits for plasma concentrations of eplerenone will occur on Day 0, and Visits 3A and 5A. Two 7 mL blood samples will be collected on Day 0, and at Visits 3A and 5A. On Day 0, the first blood sample will be collected at 0 hour (predose) and the second blood sample will be collected one hour after the initial dose of study drug; for Visits 3A and 5A, the patient will take the study drug in the morning at his/her regularly scheduled time prior to returning to the clinic, and two blood samples will be collected one hour apart. At Visits 3A and 5A, patients will be scheduled for either one morning or one afternoon visit.

The schematic below illustrates the study protocol:

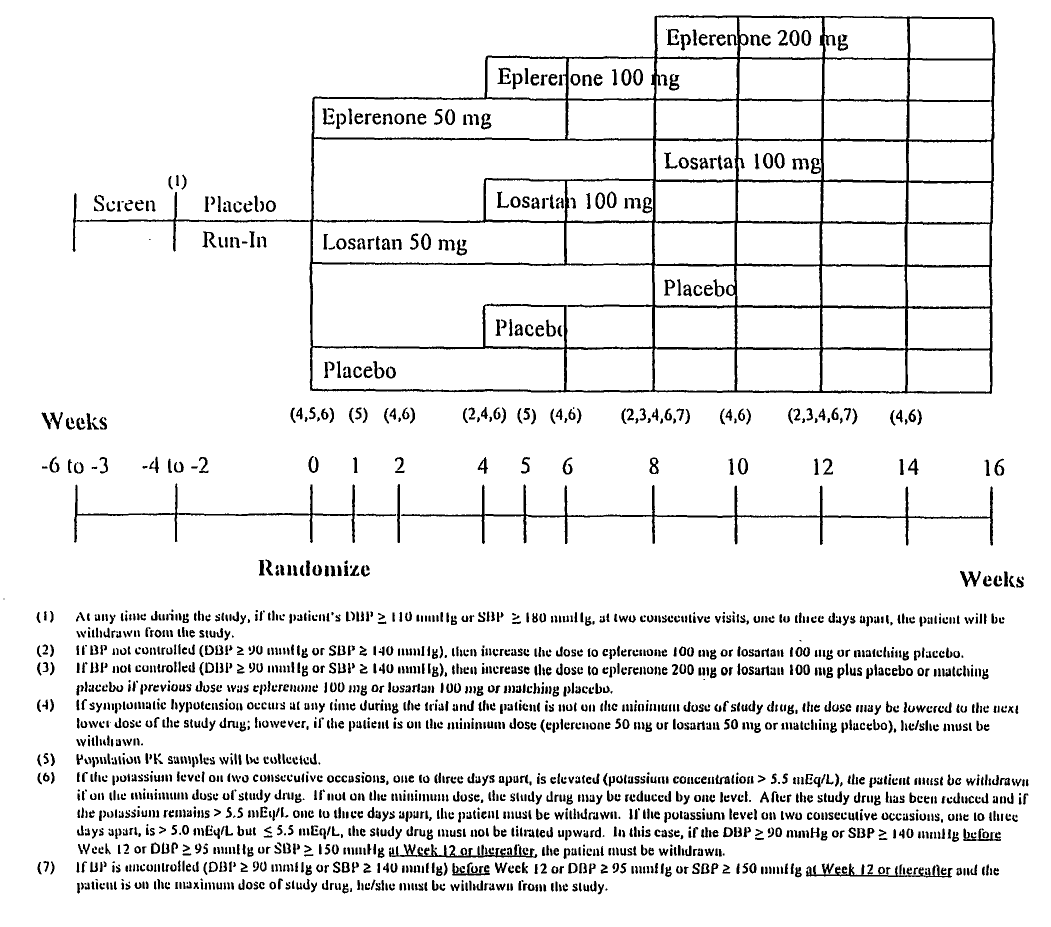

The primary measure of efficacy will be the mean change from baseline in seated trough cuff DBP between eplerenone versus placebo at Week 16 in all patients (black and white). Additionally, efficacy will be evaluated with respect to the patient's degree of salt sensitivity by tertile (wherein tertiles are empirically determined by the increment of blood pressure response to salt challenge). The primary safety endpoints are serious and non-serious adverse events, withdrawals, and laboratory abnormalities over the 16-week period.

Secondary endpoints will be the following: (1) The mean change from baseline in seated trough cuff DBP between eplerenone versus placebo within and between racial groups at Week 16; (2) The mean change from baseline in seated trough cuff DBP between eplerenone versus losartan in all patients and within and between racial groups at Week 16; (3) The mean change from baseline in seated trough cuff SBP between eplerenone versus placebo or losartan in all patients and within and between racial groups at Week 16; (4) The mean change from baseline in microalbuminuria as measured by urinary albumin to creatinine ratio between eplerenone versus placebo or losartan in all patients and within and between racial groups at Week 16; (6) The mean change from baseline in serum potassium, magnesium, sodium, albumin, and creatinine between eplerenone versus placebo or losartan in all patients and within and between racial groups at Week 16; (7) The mean change from baseline in neurohormone profile (plasma renin [total and active], serum aldosterone, and plasma cortisol) between eplerenone versus placebo or losartan in all patients and within and between racial groups at Week 16; (8) The mean change from baseline between eplerenone and placebo and losartan in all patients and within and between racial groups on neurohormonal, metabolic, renal and antihypertensive effects in selected subpopulations, e.g., women, elderly (= 65 years of age), obese (body mass,index = 30 kg/m2), microalbuminurics (urinary albumin to creatinine ratio> .041, mg albumin/mg creatinine), and specific hypertensive patients (SBP =160 mmHg); and (9) The plasma concentration-time profile of eplerenone relative to patient factors (covariates) which affect the apparent clearance of the drug.

The primary objectives of this study are (1) to compare the antihypertensive effect of eplerenone versus placebo in all patients (black and white) with mild to moderate hypertension as measured by seated diastolic blood pressure (DBP) at Week 16; and (2) to compare the safety and tolerability of eplerenone versus placebo in all patients during 16 weeks of therapy.

The secondary objectives of this study are (1) to compare the antihypertensive effect of eplerenone versus placebo within and between black and white hypertensive patients as measured by DBP; (2) to compare the antihypertensive effect of eplerenone versus losartan in all patients and within and between black and white hypertensive patients as measured by DBP; (3) to compare the antihypertensive effect of eplerenone versus placebo or losartan in all patients and within and between black and white hypertensive patients as measured by seated systolic blood pressure (SBP); (4) to compare the effect of eplerenone versus placebo or losartan in all patients and within and between black and white hypertensive patients on renal effects, as measured by a spot urine for measurement of microalbuminuria; (5) to compare the effect of eplerenone versus placebo or losartan in all patients and within and between black and white hypertensive patients on renal effects, as measured by serum potassium, magnesium, sodium, albumin, and creatinine levels; (6) to compare the effect of eplerenone versus placebo or losartan in all patients and within and between black and white hypertensive patients on neurohormone profiles (plasma renin [total and active], serum aldosterone, and plasma cortisol); (8) to compare the effect of eplerenone versus placebo or losartan in all patients and within and between black and white hypertensive patients on neurohormonal, metabolic, renal, and antihypertensive effects in selected subpopulations, e.g., a) women; b) elderly (= 65 years of age); c) obese (body mass index = 30 kg/m²); d) microalbuminurics (microalbuminuria > .041 mg albumin/mg creatinine); and e) specific hypertensive patients (SBP =160 mmHg); and (9) to model the plasma concentration-time profile of eplerenone and identify patient factors (covariates) which affect the apparent clearance of the drug.

Subgroup analyses of the primary and secondary efficacy measures can be performed with respect to subgroups based on, for example, baseline recordings of such factors as sex, age, plasma renin levels, aldosterone/renin activities ratio, urinary sodium to potassium ratio, presence of diabetes, history of hypertension, history of heart failure, history of renal dysfunction, and the like. Subgroups based on continuous measures such as age can be dichotomized at the median value.

### Example A-18: Comparison Study of the Antihypertensive. Renal, and Metabolic Effects of Eplerenone Versus Enalapril in Patients With Type 2 Diabetes Mellitus, Albuminuria, and Hypertension.

A clinical study is conducted to compare the antihypertensive, renal, and metabolic effects of eplerenone and enalapril, and the combination, in patients with Type 2 diabetes mellitus, albuminuria, and hypertension. The study is a multicenter, randomized, double-blind, active-controlled, placebo run-in, parallel group trial involving a minimum of 200 randomized patients with Type 2 diabetes mellitus, albuminuria, and hypertension. Each patient will be tested for salt sensitivity by salt challenge-unidirectional testing. The trial will further consist of a one- to two-week pretreatment screening period followed by a two- to four-week single-blind placebo run-in period and a 24-week double-blind treatment period. After completing the single-blind placebo run-in period, eligible patients will be randomized to one of three groups: eplerenone plus placebo, enalapril plus placebo, or eplerenone plus enalapril. For the first two weeks of double-blind treatment patients will receive eplerenone 50 mg plus placebo, enalapril 10 mg plus placebo, or eplerenone 50 mg plus enalapril 10 mg. At Week 2, the study medication dose will be force titrated to eplerenone 100 mg plus placebo, enalapril 20 mg plus placebo, or eplerenone 100 mg plus enalapril 10 mg. At Week 4, the dose will be force titrated to eplerenone 200 mg plus placebo, enalapril 40 mg plus placebo, or eplerenone 200 mg plus enalapril 10 mg. Table A-18A illustrates the above-described dosing scheme.

**Table A-18A. Study Medication Dose Levels**

| Dose Levels | Randomized Study Medication | | | Number of Tablets/Capsules |
|---|---|---|---|---|
| | Eplerenone | Enalapril | Eple + Enalp | |
| Placebo Run-In | Placebo | Placebo | Placebo | 1 tablet/1 capsule |
| Dose 1 | 50 mg | 10 mg | (50 + 10) mg | 1 tablet/1 capsule |
| Dose 2 | 100 mg | 20 mg | (100 + 10) mg | 1 tablet/1 capsule |
| Dose 3 | 200 mg | 40 mg | (200 + 10) mg | 2 tablets/2 capsules |

| | | | | |
|---|---|---|---|---|
| Hydrochlorothiazide 12.5 mg Amlodipine 10 mg | | | | |

If blood pressure (BP) is not controlled (diastolic BP [DBP] =90 mmHg) at Week 8, amlodipine 10 mg will be added as the first open-label drug. If DBP is not controlled at Week 10, amlodipine 10 mg will be added if not done so previously, or if amlodipine 10 mg was added previously, hydrochlorothiazide (HCTZ) 12.5 mg will be added as the second open-label drug. If BP is uncontrolled at Week 12 or thereafter, amlodipine 10 mg will be added if not done so previously, or if amlodipine 10 mg was added previously and HCTZ 12.5 mg was not added previously, HCTZ 12.5 mg will be added, or if HCTZ 12.5 mg was added previously, the dose of HCTZ will be increased to 25 mg. If at Week 15 or at any subsequent visit, the patient exhibits sustained uncontrolled DBP =95 mmHg which persists at two consecutive visits 3-10 days apart and the patient had received all add-on open-label medication as described above, the patient will be withdrawn from the study.

If symptomatic hypotension occurs and the patient is receiving open-label add-on medication, the add-on medication may be withdrawn in the reverse sequence as it was added until hypotension resolves. If the patient is not taking open-label medication, he/she must be withdrawn from the study.

At any time during the study, if DBP =110 or systolic BP [SBP] =180 mmHg persists at two consecutive visits 3-10 days apart, or if serum potassium level is elevated (>5.5 mEq/L) on repeat measurement (sample split and sent to local and central laboratories), the patient will be withdrawn.

Patients will return to the clinic for evaluations at Weeks 0, 2, 4, 6, 8, 10, 12, 15, 18, 21, 24, and 25. Heart rate, BP, body weight, serum potassium, and adverse events will be assessed at each visit. Hematology and biochemistry evaluations and urinalysis for safety will be at Weeks 0, 4, 6, 8, 10, 15, 21, 24, and 25. Collagen markers (aminoterminal propeptide of Type III procollagen [PIIINP], 7S domain of Type IV collagen [7SIVC], and Type I collagen telopeptide [ICTP]), fibrinolytic balance (plasminogen activator inhibitor [PAI-1] and tissue plasminogen activator [t-PA]), insulin, and glycosylated hemoglobin will be measured at Weeks 0, 8, 15, and 24. Pharmacoeconomic data will be collected at Weeks 0, 8, 15, and 24. Albuminuria by 24-hour urine collection will be measured at Weeks 0, 8, and 24. A 12 lead electrocardiogram and physical examination will be done at screening and at Week 25. Genotype, waist circumference, plasma renin (total and active), and serum aldosterone will be measured at Week 0.

The schematic below demonstrates the study protocol:

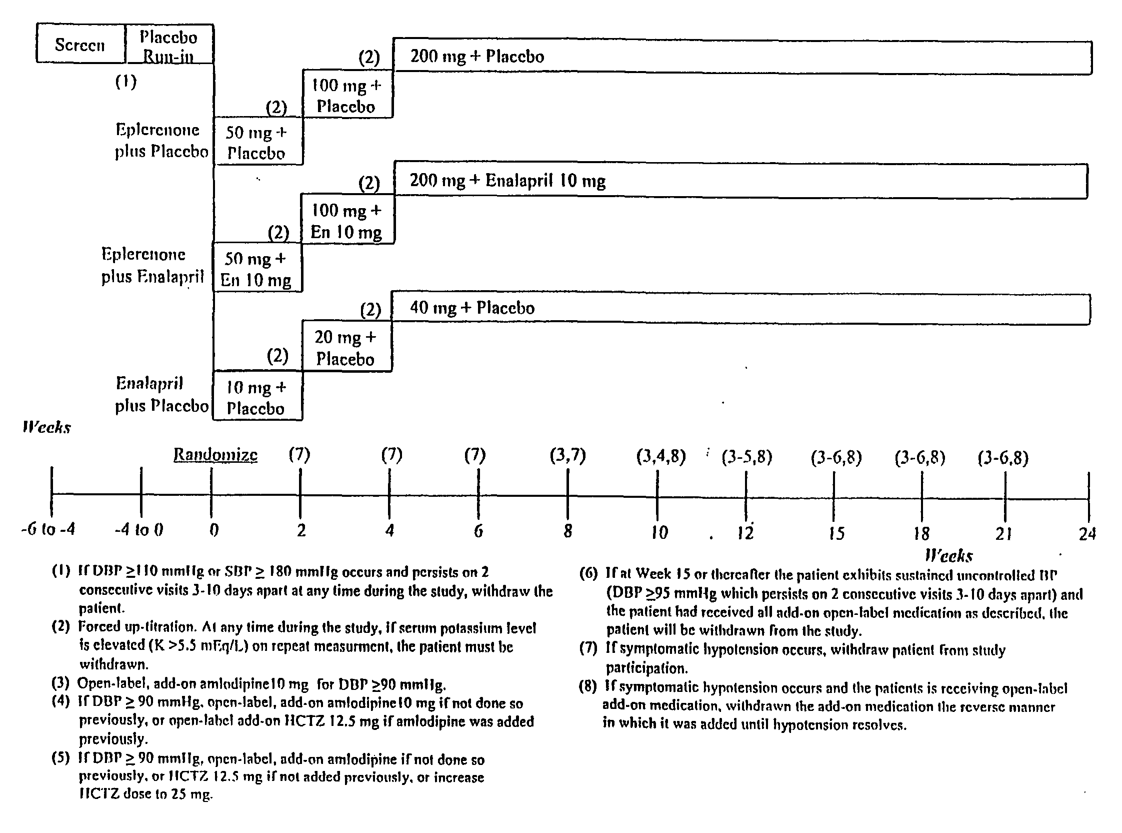

The primary measure of efficacy will be the change from baseline in urinary albumin excretion between eplerenone and enalapril, or the combination, at Week 24. Additionally, efficacy will be evaluated with respect to the patient's degree of salt sensitivity by tertile (wherein tertiles are empirically determined by the increment of blood pressure response to salt challenge).

Secondary measures of efficacy will be the following: (1) the mean change from baseline in seated trough cuff DBP ("seDBP") and SBP (seSBP) between eplerenone and enalapril, or the combination, at Weeks 8 and 24; (2) the mean change from baseline in collagen markers (PIIINP, 7SIVC, and ICTP), fibrinolytic balance (PAI-1 and t-PA), and metabolic effects (insulin, glycosylated hemoglobin, fasting serum glucose, and lipids [triglycerides, total cholesterol, and HDL cholesterol]) between eplerenone and enalapril, or the combination, at Week 24; (3) the mean change from baseline in antihypertensive, metabolic, or urinary albumin excretion response between eplerenone and enalapril, or the combination, due to genotype, baseline truncal obesity, baseline plasma renin level (total and active), or baseline serum aldosterone level; and (4) Safety and tolerability will be assessed by adverse events, clinical laboratory values, physical examination, vital signs, and electrocardiogram.

This double-blind, active-controlled study is designed to determine the net effect of eplerenone on the insulin resistance, glycemic control, renal function, and lipid profile of hypertensive patients with NIDDM and albuminuria as compared to enalapril. The primary objective of this study is to compare the mean change from baseline in urinary albumin excretion in patients treated with eplerenone versus enalapril or the combination at Week 24. The secondary objectives of this study are to (1) compare the effect on mean change from baseline of trough cuff seDBP and seSBP of eplerenone versus enalapril or the combination at Weeks 8 and 24; (2) compare the effects of eplerenone versus enalapril, or the combination, as measured by mean change from baseline of collagen markers (aminoterminal propeptide of Type III procollagen [PIIINP], 7S domain of Type IV collagen [7SIVC], and Type I collagen telopeptide [ICTP]); fibrinolytic balance (plasminogen activation inhibitor [PAI-1], tissue plasminogen activator [t-PA]), and metabolic effects (insulin, glycosylated hemoglobin, fasting serum glucose, and lipids [triglycerides, total cholesterol, and HDL cholesterol]) at Week 24; (3) measure any difference in mean change from baseline in antihypertensive, metabolic, or urinary albumin excretion response of eplerenone versus enalapril or the combination due to genotype, baseline truncal obesity (waist circumference), baseline plasma renin level (total and active), or baseline serum aldosterone level; and compare the safety and tolerability of eplerenone versus enalapril, or the combination, as assessed by reported adverse events, clinical laboratory values, physical examination, vital signs, and electrocardiogram.

Subgroup analyses of the primary and secondary efficacy measures can be performed with respect to other subgroups based on, for example, baseline recordings of such factors as race (black, non-black, Japanese, etc.), sex, age, plasma renin levels, aldosterone/renin activities ratio, urinary sodium to potassium ratio, history of heart failure, and the like. Subgroups based on continuous measures such as age can be dichotomized at the median value.

### (Reference) Example A-19: Comparison Study of the Antihypertensive Effect of Eplerenone Versus Amlodipine in Patients With Elevated Systolic Blood Pressure.

A clinical study is conducted to compare the effect of eplerenone versus amlodipine on systolic blood pressure in patients with systolic hypertension. This is a multicenter, randomized, double-blind, active-controlled, placebo run-in, parallel group trial involving a minimum of 200 randomized patients with systolic hypertension defined as: 1) seated systolic blood pressure (SBP)==150 mmHg and < 165 mmHg, and pulse pressure (PP) = 70 mmHg, or 2) SBP = 165 mmHg and < 200 mmHg, and seated diastolic blood pressure (DBP) < 95 mmHg. Each patient will be tested for salt sensitivity by salt challenge- unidirectional testing. The trial will further consist of a one- to two-week pretreatment screening period followed by a two- to four-week single-blind placebo run-in period and a 24-week double-blind treatment period. After completing the single-blind placebo run-in period, eligible patients will be randomized to receive either eplerenone or amlodipine. Patients will receive eplerenone 50 mg or amlodipine 2.5 mg for the first two weeks of double-blind treatment. At Week 2, if SBP is uncontrolled (SBP = 140 mmHg), the dose of study medication will be increased by one dose level to eplerenone 100 mg or amlodipine 5 mg; the dose will not be changed for patients with adequate BP control. If SBP is uncontrolled (SBP = 140 mmHg) at Week 6 or thereafter, the dose of study medication will be increased by one dose level to eplerenone 100 mg or amlodipine 5 mg if not done at Week 2, or to eplerenone 200 mg or amlodipine 10 mg if already increased at Week 2. The dose will not be changed for patients with adequate SBP control. At Week 10 or thereafter, if SBP is = 170 mmHg on two consecutive visits, one to three days apart, and the patient is on the maximum dose of study drug, he/she must be withdrawn. Patients will receive double-blind study medication for a total of 24 weeks. Table A-19A illustrates the above-described dosing scheme.

**Table A-19A. Study Medication Dose Levels**

| Dose Level | Randomized Study Medication | | Number of Tablets/Capsules |
|---|---|---|---|
| | Eplerenone | Amlodipine | |
| Placebo Lead-In | matching placebo | matching placebo | 1 tablet/1 capsule |
| Dose 1 | 50 mg | 2.5 mg | 1 tablet/1 capsule |
| Dose 2 | 100 mg | 5 mg | 1 tablet/1 capsule |
| Dose 3 | 200 mg | 10 mg | 2 tablets/2 capsules |

If symptomatic hypotension (SH) (i.e., lightheadedness, dizziness, or syncope associated with low BP) occurs at any time during the study and the patient is not on the minimum dose of study drug, the patient may be down titrated. If SH occurs on the minimum dose of study drug, the patient must be withdrawn.

At any time during this study, if the SBP = 200 mmHg or DBP is = 110 mmHg at two consecutive visits, one to three days apart, the patient must be withdrawn. Patients will receive double-blind study medication for a total of 24 weeks.

Patients will return to the clinic for evaluations at Weeks 0, 2, 6, 10, 14, 19, 24, and 25. Heart rate, BP, serum potassium levels, concomitant medications and adverse events will be assessed at each visit. Hematology and biochemistry evaluations and urinalysis for safety will be performed at screening, Weeks 0, 24, and 25. Special studies: collagen markers [aminoterminal propeptide of type III procollagen (PIIINP), 7S domain of type IV collagen (7SIVC), and type I collagen telopeptide (ICTP)], plasma activator inhibitor (PAI-1), tissue plasminogen activator (t-PA), microalbuminuria, and at selected sites, Ambulatory Blood Pressure Monitoring (ABPM) and arterial compliance will be done at Weeks 0, 14, and 24 or at Final Visit. Quality of Life Questionnaires will be done at visit 2A (beginning of single blind) and Weeks 0, 14, and 24 or at Final Visit. DNA genotyping will be performed at baseline. A 12-lead electrocardiogram and physical examination will be done at screening and at Week 25 or at Final Visit.

The schematic below demonstrates the study protocol:

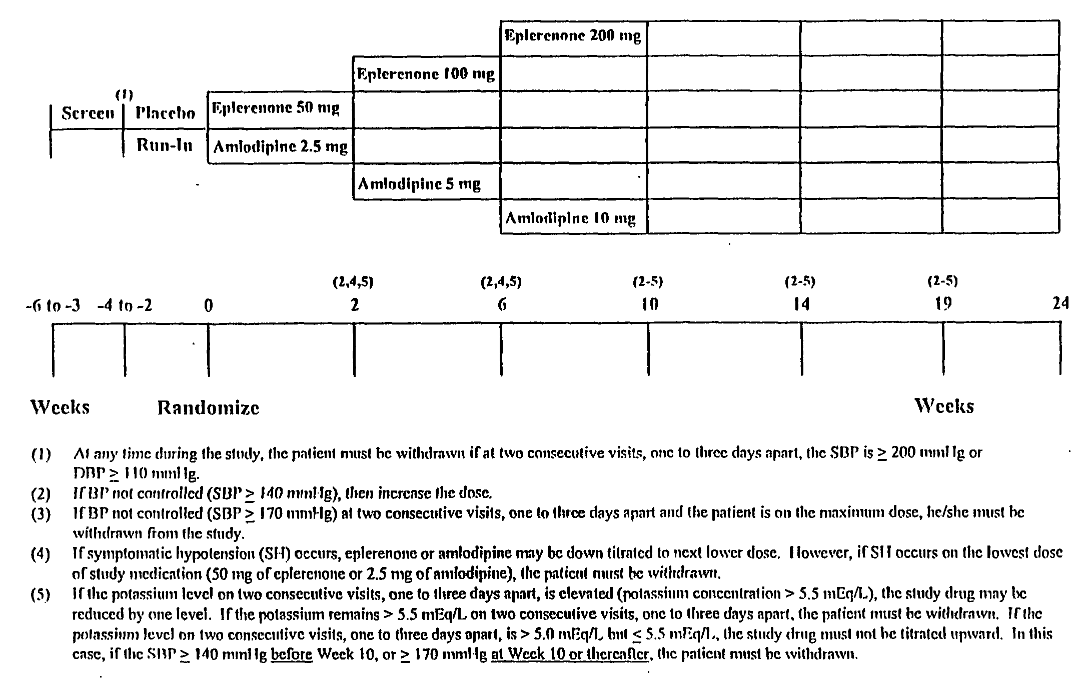

Primary measure of efficacy will be the mean change from baseline in seated trough cuff SBP between eplerenone versus amlodipine at Week 24. Secondary endpoints will be the following: (1) mean change from baseline in pulse pressure (SBP-DBP) between eplerenone versus amlodipine at Week 24; (2) mean change from baseline in seated trough cuff diastolic BP (DBP) between eplerenone versus amlodipine at Week 24; (3) mean change from baseline of heart rate (HR), PP, SBP, and DBP between eplerenone versus amlodipine as measured by ABPM recordings at Week 24; (4) mean change from baseline in arterial compliance between eplerenone versus amlodipine at Week 24; (5) mean change from baseline in PAI-1, t-PA, and collagen markers between eplerenone versus amlodipine at Week 24; (6) mean change from baseline in microalbuminuria as measured by urinary albumin to creatinine ratio, between eplerenone versus amlodipine at Week 24; (7) the response to eplerenone and amlodipine relative to genotype; (8) safety and tolerability of eplerenone versus amlodipine; and (9) mean change from baseline in quality of life evaluation between eplerenone versus amlodipine at Weeks 14 and 24.

Additionally, efficacy (items 1 - 9, above) will be evaluated with respect to the patient's degree of salt sensitivity by tertile (wherein tertiles are empirically determined by the increment of blood pressure response to salt challenge).

This trial in a population with systolic hypertension is designed to determine the antihypertensive effect of eplerenone relative to amlodipine treatment, to compare the quality of life on eplerenone treatment versus amlodipine, to compare the side effect profile of eplerenone versus amlodipine in the elderly, and to assess arterial compliance, plasma activator inhibitor (PAI-1), and markers of collagen metabolism in the two groups. The primary objective of this study is to compare the effect of eplerenone versus amlodipine on SBP as measured by mean change from baseline in seated trough cuff SBP at Week 24. The secondary objectives of this study are to: (1) compare the effect of eplerenone versus amlodipine on mean change from baseline in pulse pressure (SBP-DBP) at Week 24; (2) compare the antihypertensive effect of eplerenone versus amlodipine as measured by mean change from baseline in seated trough cuff DBP at Week 24; (3) compare from ABPM recordings, the mean change from baseline of HR, PP, SBP, and DBP between eplerenone versus amlodipine at Week 24; (4) compare the effect of eplerenone versus amlodipine on arterial compliance as measured by mean change from baseline at Week 24; (5) compare the effect of eplerenone versus amlodipine on PAI-1, tissue plasminogen activator (t-PA), and collagen markers [aminoterminal propeptide of type III procollagen (PIIINP), 7S domain of type IV collagen (7SIVC), and type I collagen telopeptide (ICTP)], as measured by mean change from baseline at Week 24; (6) compare the effect of eplerenone versus amlodipine on microalbuminuria expressed as urinary albumin to creatinine ratio, as measured by mean change from baseline at Week 24; (7) compare the response to eplerenone versus amlodipine relative to genotype; (8) compare the safety and tolerability of eplerenone versus amlodipine as assessed by reported adverse events, clinical laboratory values, physical examination, vital signs, and electrocardiogram during 24 weeks of therapy; and (9) compare the effect of eplerenone versus amlodipine on patients' health related quality of life as measured by mean change from baseline at Weeks 14 and 24.

Subgroup analyses of the primary and secondary efficacy measures can be performed with respect to other subgroups based on, for example, baseline recordings of such factors as race (black, non-black, Japanese, etc.), sex, age, plasma renin levels, aldosterone/renin activities ratio, urinary sodium to potassium ratio, presence of diabetes, history of heart failure, history of renal dysfunction, and the like. Subgroups based on continuous measures such as age can be dichotomized at the median value.

### (Reference) Example A-20: Dose-Ranging Study of Eplerenone Vs. Placebo in Patients With Symptomatic Heart Failure

A clinical study is conducted to evaluate the safety and tolerability of a range of doses of eplerenone, to assess their effect on neurohormonal function, and to examine their potential for improving signs and symptoms in patients with heart failure concurrently treated with an ACE inhibitor and a loop diuretic. Additionally, each of the above paramteres will be evaluated with respect to the patient's degree of salt sensitivity by tertile (wherein tertiles are empirically determined by the increment of blood pressure response to salt challenge). The study is a randomized, double-blind, multicenter, placebo-controlled parallel group trial evaluating three different daily doses of eplerenone vs. placebo. The study will enroll at least 100 patients. Each patient will be tested for salt sensitivity by salt challenge-unidirectional testing.

The study population will be patients with symptomatic heart failure who have an ejection fraction = 40% and are New York Heart Association (NYHA) Functional Class II - IV on entry. Patients eligible for the trial will receive one of the following treatments: eplerenone 25 mg QD, 50 mg QD, 100 mg QD, or placebo, for 12 weeks. The measures for evaluation of neurohormones will be determinations of N-terminal atrial natriuretic peptide (N-terminal ANP), brain natriuretic peptide (BNP and pro-BNP), plasma renin (total and active), and plasma and urine aldosterone. Assessment of patients' signs and symptoms will be made using the NYHA Functional Class. Safety will be evaluated by the assessment of incidence of hyperkalemia and symptomatic hypotension, other adverse experiences, and clinical laboratory abnormalities. The study is structured to detect differences between eplerenone and placebo treatment in the neurohormone levels and in major changes in clinical signs and symptoms.

The primary objectives of this study are (1) to evaluate the safety and tolerability of a range of doses of eplerenone in patients with HF concurrently treated with an ACE inhibitor and a loop diuretic; (2) to evaluate the effect of a range of doses of eplerenone given measurements of neurohormonal function [N-terminal atrial natriuretic peptide (ANP), brain natriuretic peptide (BNP) and its pro-form (pro-BNP), serum and urine aldosterone, and plasma renin (total and active)] in patients with HF concurrently treated with an ACE inhibitor and a loop diuretic; and (3) to evaluate the efficacy of a range of doses of eplerenone given over 12 weeks in improving the signs and symptoms of HF as assessed by change from baseline in NYHA Functional Classification. The secondary objectives of this study are (1) to evaluate the effect of a range of doses of eplerenone co-administered with an ACE inhibitor and a loop diuretic on heart rate (HR), BP, and body weight; and (2) to evaluate the effect of eplerenone on the changes in dosing of ACE inhibitors and diuretics when they are given concurrently with eplerenone.

The schematic below demonstrates the study protocol:

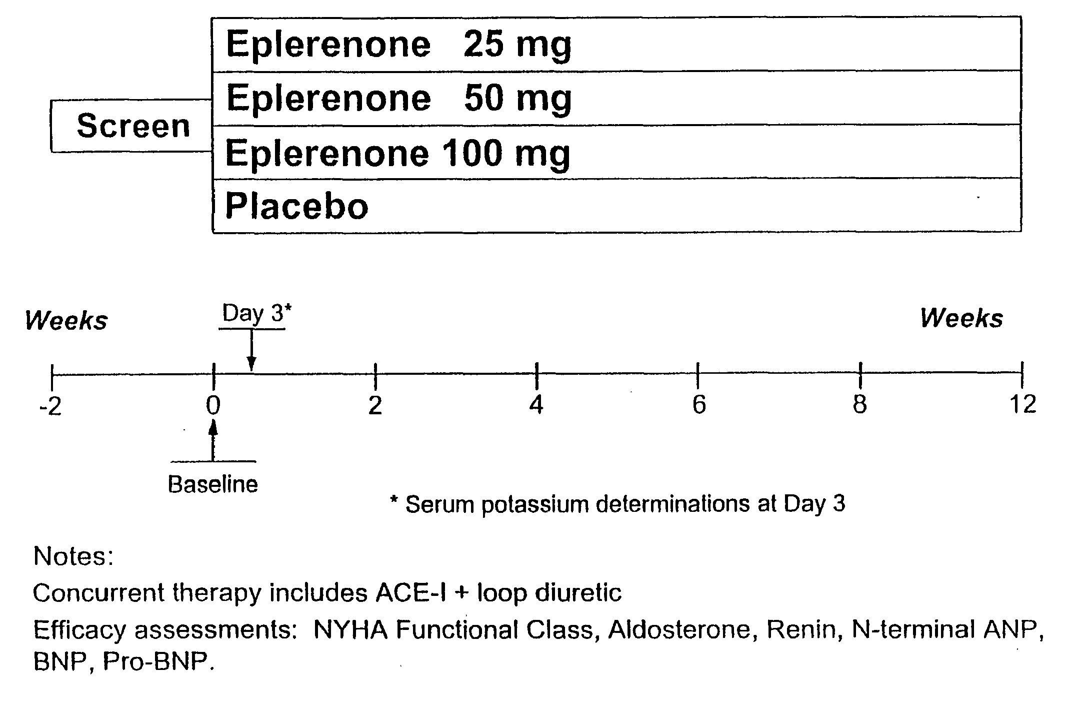

If the patient becomes intolerant of study medication, alterations in the dose of concomitant medications (e.g., potassium supplements, ACE-I, etc.) should be considered prior to dose adjustment of study medication. If at any time during the study the serum potassium level is = 6.0 mEq/L, study medication is to be temporarily withheld. If serum potassium level is persistently = 6.0 mEq/L, the patient is to discontinue study medication. If elevated potassium levels are observed < 6.0 mEq/L, potassium supplements, if any, should be stopped and the patient should continue to receive study medication. If study medication is stopped, concurrent medications should be reviewed and the doses adjusted if possible according to good clinical practice.

Table A-20A summarizes necessary dosing changes for serum potassium levels. Serum potassium will be determined within one week following initiation of treatment and within one week following any dose change. If at any time during the study the serum potassium is > 5.5 mEq/L, the dose of study drug will be reduced to the next lower dose level, i.e., 1 tablet QD to 1 tablet QOD or 1 tablet QOD to temporarily stopped. Study medication is to be restarted at 1 tablet QOD when the serum potassium level is < 5.5 mEq/L and increased according to the scheme presented in Table A-20B. The potassium level may be repeated if the potassium increase is thought to be spurious (i.e., due to hemolysis or recent dosing with a potassium supplement).

**Table A-20A. Study Medication Dosing Adjustment for Serum Potassium Levels**

| If Serum Potassium | And Current | Dose | Number of Tablets: |
|---|---|---|---|
| Level Is: | Dose Is: | Change: | |
| <5.0mEq/L | Withhold | Increase | 1 tablet QOD |
| < 5.0 mEq/L | 1 tablet QOD | Increase | 1 tablet QD |
| < 5.0 mEq/L | 1 tablet QD | No change | 1 tablet QD |
| = 5.0 and < 5.5 mEq/L | Withhold | Increase | 1 tablet QOD |
| = 5.0 and < 5.5 mEq/L | 1 tablet QOD | No change | 1 tablet QOD |
| = 5.0 and < 5.5 mEq/L | 1 tablet QD | No change | 1 tablet QD |
| = 5.5 and < 6.0 mEq/L | Withhold | No change | None |
| = 5.5 and < 6.0 mEq/L | 1 tablet QOD | Decrease | None |
| = 5.5 and < 6.0 mEq/L | 1 tablet QD | Decrease | 1 tablet QOD |
| = 6.0 mEq/L | Any dose | * | None |

| | | | |
|---|---|---|---|
| *If persistent elevation, discontinue medication. If single elevation, withhold dosing. | | | |

Subgroup analyses of the primary and secondary efficacy measures can be performed with respect to other subgroups based on, for example, baseline recordings of such factors as sex, age, plasma renin levels, aldosterone/renin activities ratio, urinary sodium to potassium ratio, presence of diabetes, history of hypertension, history of renal dysfunction, and the like. Subgroups based on continuous measures such as age can be dichotomized at the median value.

### (Reference) Example A-21: Efficacy and Safety Evaluation of a Range of Doses of Eplerenone in the Treatment of Mild to Moderate Hypertension

A multicenter, randomized, double-blind, placebo-controlled, parallel group trial is conducted to evaluate the safety and efficacy of three different total daily doses (50,100, and 200 mg) of eplerenone to placebo. The study consists of a two-week pretreatment period for washout of any current antihypertensive medication(s) and for completion of screening procedures. Each patient will be tested for salt sensitivity by salt challenge- unidirectional testing after washout. Salt sensitivity testing is followed by a four-week, single-blind, placebo lead-in treatment, prior to randomization to an eight-week, double-blind, active versus placebo treatment period. Table A-21A illustrates the above-described dosing scheme.

**Table A-21A. Dose Regimen Packaging**

| | Tablets | |
|---|---|---|
| Dose Regimen | 50 mg | 100mg |
| Placebo Lead-in & Treatment Period | (1) matching placebo for 50 mg | (1) matching placebo for 100 mg + |
| | | (1) matching placebo for 100 mg |
| Eplerenone | (1) 50 mg Active | (1) matching placebo for 100 mg + |
| 50 mg | | (1) matching placebo for 100 mg |
| Eplerenone | (1) matching placebo for 50 mg | (1) 100 mg Active + |
| 100 mg | | (1) matching placebo for 100 mg |
| Eplerenone | (1) matching placebo for 50 mg | (1) 100 mg Active + |
| 200 mg | | (1) 100 mg Active |

If symptomatic hypotension (SH), i.e., lightheadedness, dizziness, or syncope associated with low blood pressure (BP), occurs at any time during the study, the patient must be withdrawn. At any time during the double-blind treatment period, if the systolic blood pressure (SBP) =180 mmHg or diastolic blood pressure (DBP) = 110 mmHg at two consecutive visits, one to three days apart, the patient must be withdrawn. At any time during the study, if serum potassium level is elevated > 5.5 mmol/L on repeat measurement (sample split and sent to local and central laboratories, treatment decision based on local value) at two consecutive visits, one to three days apart, the patient must be withdrawn.

The primary efficacy measure will be the change from baseline in cuff DBP measured at trough as compared to placebo. Secondary efficacy measures will include changes in cuff assessed trough SBP, and changes in average 24-hour DBP and SBP assessed by ambulatory BP monitoring (performed at selected investigational sites only). Changes in neurohormones (plasma renin, serum aldosterone) after eight weeks of dosing will also be a secondary efficacy measure. Efficacy will be also evaluated with respect to the patient's degree of salt sensitivity by tertile (wherein tertiles are empirically determined by the increment of blood pressure response to salt challenge).

The primary objective of this study is to evaluate the antihypertensive effect of once daily doses of 50,100, and 200 mg of eplerenone as compared to placebo, when administered for eight weeks to patients with mild to moderate hypertension using change from baseline in trough cuff DBP measurements. After eight weeks treatment of eplerenone as compared to placebo the secondary objectives of this study are (1) to evaluate change from baseline in trough cuff SBP; (2) to determine the 24-hour antihypertensive effect of eplerenone relative to placebo using Ambulatory Blood Pressure Monitoring (ABPM); (3) to evaluate change in plasma renin and serum aldosterone levels; and (4) to establish the safety and tolerability of eplerenone for antihypertensive treatment as assessed by reported adverse events, clinical laboratory values, physical examination, vital signs, and electrocardiogram.

The schematic below demonstrates the study protocol:

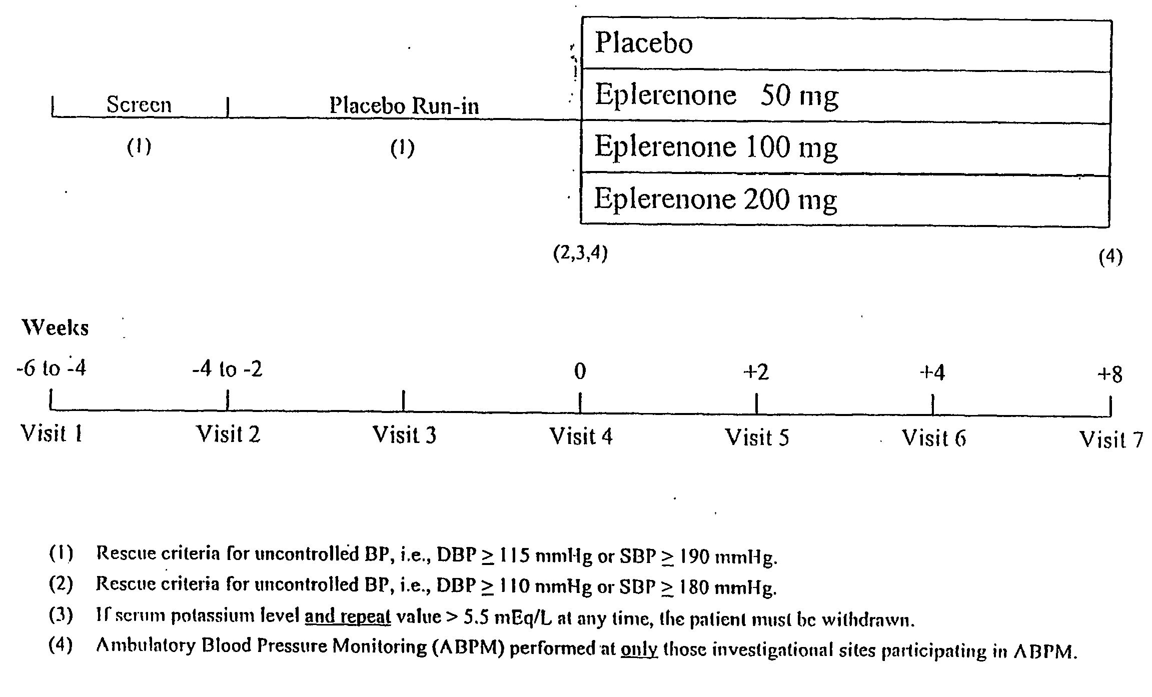

Subgroup analyses of the primary and secondary efficacy measures can be performed with respect to other subgroups based on, for example, baseline recordings of such factors as sex, age, plasma renin levels, aldosterone/renin activities ratio, urinary sodium to potassium ratio, presence of diabetes, history of heart failure, history of renal dysfunction, and the like. Subgroups based on continuous measures such as age can be dichotomized at the median value.

### (Reference) Example A-22: Safety And Efficacy Of Eplerenone In Patients With Heart Failure Following Acute Myocardial Infarction.

A clinical trial is conducted to compare the effect of eplerenone plus standard therapy versus placebo plus standard therapy on the rate of all cause mortality in patients with heart failure (HF) after an acute myocardial infarction (AMI). Secondary endpoints include cardiovascular morbidity and mortality. The study is a multicenter, randomized, double-blind, placebo-controlled, two-arm, parallel group trial will continue until 1,012 deaths occur, which is estimated to require approximately 6,200 randomized patients followed for an average of approximately 2.5 years.

Patients eligible for this study will have (1) AMI (the index event) documented by (a) abnormal cardiac enzymes (creatine phosphokinase [CPK] >2 x upper limit of the normal range [ULN] and/or CPK-MB >10% of total CPK), and (b) an evolving electrocardiogram (ECG) diagnostic of MI (progressive changes in ST segment and T wave compatible with AMI with or without presence of pathological Q waves); and (2) left ventricular (LV) dysfunction, demonstrated by LV ejection fraction (LVEF) =40% determined following AMI and before randomization; and (3) clinical evidence of HF documented by at least one of the following: (a) pulmonary edema (bilateral posttussive crackles extending at least 1/3 of the way up the lung fields in the absence of significant chronic pulmonary disease); or (b) chest x-ray showing pulmonary venous congestion with interstitial or alveolar edema; or (c) auscultatory evidence of a third heart sound (S₃) with persistent tachycardia (>100 beats per minute). Eligible patients may be identified for inclusion at any time following emergency room evaluation and presumptive diagnosis of AMI with HF. Patients who qualify for this study will be randomized between 3 (>48 hours) and 10 days post-AMI if their clinical status is stable, e.g., no vasopressors, inotropes, intra-aortic balloon pump, hypotension (systolic blood pressure [SBP]<90 mmHg), or recurrent chest pain likely to lead to acute coronary arteriography. Patients with implanted cardiac defibrillators are excluded.

Patients will receive standard therapy which may include ACE inhibitors, diuretics, nitrates, and β-blockers, and may have received anticoagulants and antiplatelet agents, and may have received thrombolytics or emergency angioplasty. Patients will be randomized to receive eplerenone 25 mg QD (once daily) or placebo. At four weeks, the dose of study drug will be increased to 50 mg QD (two tablets) if serum potassium <5.0 mEq/L. If at any time during the study the serum potassium is >5.5 mEq/L but <6.0 mEq/L, the dose of study drug will be reduced to the next lower dose level, i.e., 50 mg QD to 25 mg QD (one tablet), 25 mg QD to 25 mg QOD (every other day), or 25 mg QOD to temporarily withheld. If at any time during the study the serum potassium is =6.0 mEq/L, study medication should be temporarily withheld, and may be restarted at 25 mg QOD when serum potassium is <5.5 mEq/L. If at any time during the study the serum potassium is persistently =6.0 mEq/L, study medication should be permanently discontinued. If the patient becomes intolerant of study medication, alterations in the dose of concomitant medications should be considered prior to dose adjustment of study medication. Serum potassium will be determined at 48 hours after initiation of treatment, at 1 and 5 weeks, at all other scheduled study visits, and within one week following any dose change.

Study visits will occur at screening, baseline (randomization), 1 and 4 weeks, 3 months, and every 3 months thereafter until the study is terminated. Medical history, cardiac enzymes, Killip class, time to reperfusion (if applicable), documentation of AMI and of HF, determination of LVEF, and a serum pregnancy test for women of childbearing potential will be done at screening. A physical examination and 12-lead ECG will be done at screening and at the final visit (cessation of study drug). Hematology and biochemistry evaluations and urinalysis for safety will be done at screening, Week 4, Months 3 and 6, and every 6 months thereafter until the study is terminated. An additional blood sample for DNA analysis will be collected during screening. Vital signs (seated heart rate and BP), New York Heart Association (NYHA) functional class, adverse events, and selected concurrent medications will be recorded at every visit. Quality of Life assessments will be completed during screening, at Week 4, Months 3, 6, and 12, and at the final visit. All randomized patients will be followed for endpoints every 3 months until the study is terminated.

The schematic below demonstrates the study protocol:

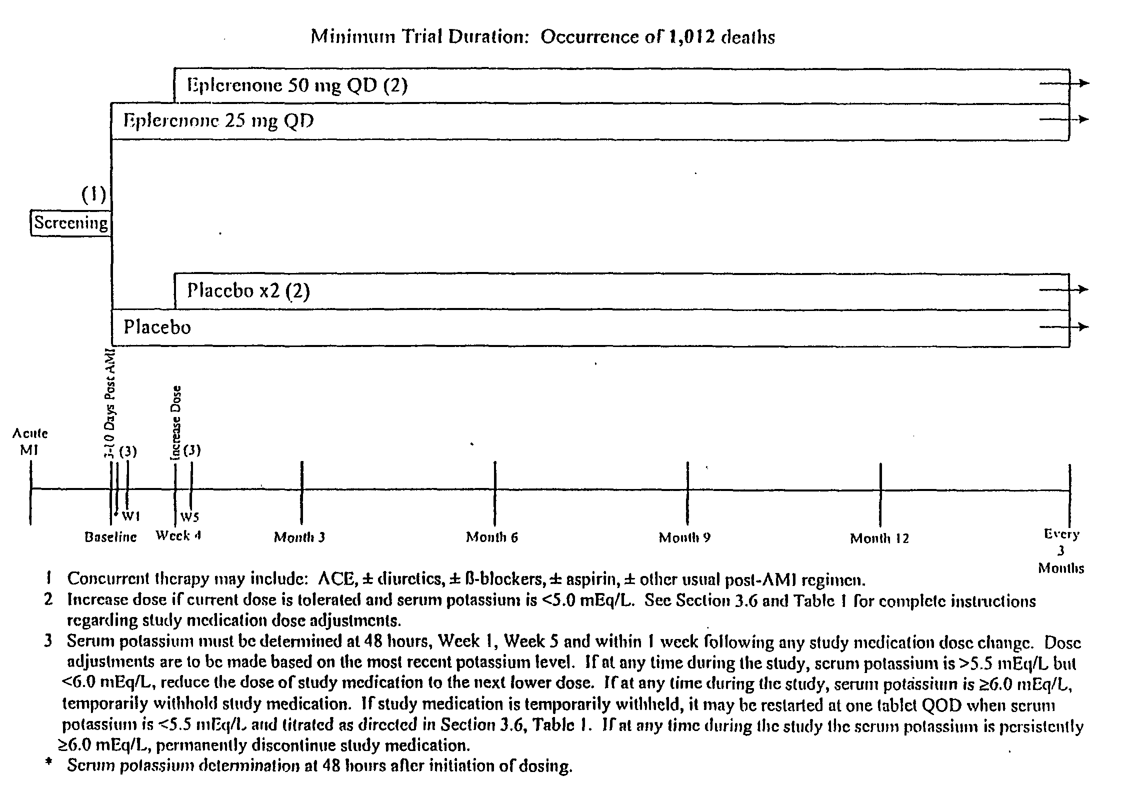

The primary endpoint is all cause mortality. The trial is structured to detect an 18.5% reduction in all cause mortality, and requires 1,012 deaths before terminating the study. Secondary endpoints include (1) cardiovascular mortality; (2) sudden cardiac death; (3) death due to progressive heart failure; (4) all cause hospitalizations; (5) cardiovascular hospitalizations; (6) heart failure hospitalizations; (7) all cause mortality plus all cause hospitalizations; (8) cardiovascular mortality plus cardiovascular hospitalizations; (9) cardiovascular mortality plus heart failure hospitalizations; (10) new diagnosis of atrial fibrillation; (11) hospitalization for recurrent non-fatal AMI and fatal AMI; (12) hospitalization for stroke; and (13) quality of life.

The primary objective of this study is to compare the effect of eplerenone plus standard therapy versus placebo plus standard therapy on the rate of all cause mortality in patients with heart failure after AMI. The secondary objectives of this study are to compare the two treatment groups for include (1) cardiovascular mortality; (2) sudden cardiac death; (3) death due to progressive heart failure; (4) all cause hospitalizations; (5) cardiovascular hospitalizations; (6) heart failure hospitalizations; (7) all cause mortality plus all cause hospitalizations; (8) cardiovascular mortality plus cardiovascular hospitalizations; (9) cardiovascular mortality plus heart failure hospitalizations; (10) new diagnosis of atrial fibrillation; (11) hospitalization for recurrent non-fatal AMI and fatal AMI; (12) hospitalization for stroke; and (13) quality of life.

Patients will receive eplerenone 25 mg QD or placebo (one tablet) for the first four weeks of treatment. At four weeks, the dose of study drug will be increased to 50 mg QD (two tablets) if serum potassium <5.0 mEq/L. If the serum potassium is =5.0 mEq/L at Week 4 but <5.0 mEq/L at Week 5, the dose of study drug will be increased to 50 mg QD (two tablets). In this case, serum potassium is to be checked at Week 6.

Table A-22A summarizes mandated dosing changes for serum potassium levels. Serum potassium will be determined at 48 hours after initiation of treatment, at 1 and 5 weeks, and within one week following any dose change. If at any time during the study the serum potassium is >5.5 mEq/L, the dose of study drug will be reduced to the next lower dose level, i.e., 50 mg QD to 25 mg QD, 25 mg QD to 25 mg QOD, or 25 mg QOD to temporarily stopped. Study medication is to be restarted at 25 mg QOD when the serum potassium level is <5.5 mEq/L and increased according to the scheme presented in Table A-22A. The potassium level may be repeated if the potassium increase is thought to be spurious (i.e., due to hemolysis or recent dosing with a potassium supplement).

If the patient becomes intolerant of study medication, alterations in the dose of concomitant medications (e.g., potassium supplements, ACE-I, etc.) should be considered prior to dose adjustment of study medication. If at any time during the study the serum potassium level is =6.0 mEq/L, study medication is to be temporarily withheld. If serum potassium level is persistently =6.0 mEq/L, the patient is to discontinue study medication. If elevated potassium levels are observed <6.0 mEq/L, potassium supplements, if any, should be stopped and the patient should continue to receive study medication. If study medication is stopped, concurrent medications should be reviewed and the doses adjusted if possible according to good clinical practice.

**Table A-22A. Study Medication Dosing Adjustment for Serum Potassium Levels**

| If Serum Potassium | And Current | Dose | Number of |
|---|---|---|---|
| Level Is: | Dose Is: | Change: | Tablets: |
| <5.0 mEq/L | Withhold | Increase | 1 tablet QOD |
| <5.0 mEq/L | 1 tablet QOD | Increase | 1 tablet QD |
| <5.0 mEq/L | 1 tablet QD | Increase | 2 tablets QD |
| <5.0 mEq/L | 2 tablets QD | No change | 2 tablets QD |
| =5.0 and <5.5 mEq/L | Withhold | Increase | 1 tablet QOD |
| =5.0 and <5.5 mEq/L | 1 tablet QOD | No change | 1 tablet QOD |
| =5.0 and <5.5 mEq/L | 1 tablet QD | No change | 1 tablet QD |
| =5.0 and <5.5 mEq/L | 2 tablets QD | No change | 2 tablets QD |
| =5.5 and <6.0 mEq/L | Withhold | No change | None |
| =5.5 and <6.0 mEq/L | 1 tablet QOD | Decrease | None |
| =5.5 and <6.0 mEq/L | 1 tablet QD | Decrease | 1 tablet QOD |
| =5.5 and <6.0 mEq/L | 2 tablets QD | Decrease | 1 tablet QD |
| =6.0 mEq/L | Any dose | * | None |

| | | | |
|---|---|---|---|
| *If persistent elevation, discontinue medication. If single elevation, withhold dosing. | | | |

Subgroup analyses of the primary and secondary endpoints will be performed. Subgroups will be based on baseline recordings of race (black, non-black), sex, age, presence of diabetes, ejection fraction, serum potassium, serum creatinine, use of β-blockers, use of digoxin, use of potassium supplements, first versus subsequent AMI, Killip class, reperfusion status, history of hypertension, history of HF, history of smoking, history of angina, time from index AMI to randomization, and geographic region. Subgroups based on continuous measures such as age, ejection fraction, serum potassium, and serum creatinine will be dichotomized at the median value.

### (Reference) Example A-22: Eplerenone to Prevent or Treat Endothelial Dysfunction:

After 20 minutes of supine rest, the nondominant brachial artery is cannulated under local anesthesia. After 30 minutes of saline infusion, baseline forearm blood flow is measured by forearm venous-occlusion plethysmography. Drugs are then infused into the study arm with a constant rate infuser. Forearm blood flow is measured at each baseline and during the last two minutes of each drug infusion. Blood pressure is measured in the non-infused (control) arm at regular intervals throughout the study.
Drug infusions. First, acetylcholine (endothelium-dependant vasodilator) is infused at 25, 50, and 100 mmol/minute, each for five minutes. This is followed by sodium nitroprusside (endothelium independent vasodilator) at 4.2, 12.6, and 37.8 nmol/min, each for 5 minutes, and then N-monoethyl-L-arginine (L-NMMA; competitive NO synthase inhibitor) at 1, 2, and 4 µmol/min for 5 minutes each. This is followed by angiotensin I (vasoconstrictor only through conversion to angiotensin II) at 64, 256, and 1024 pmol/min for 7 minutes each. Between the different drugs, the drug infusion is flushed with saline for 20 to 30 minutes to allow sufficient time for the forearm blood flow to return to baseline values
Results. It is expected that eplerenone will significantly increased the forearm blood flow response to acetylcholine (percentage change in forearm blood flow), with an associated increase in vasoconstriction due to L-NMMA. It is further expected that the angiotensin I response is also significantly reduced with eplerenone, with angiotensin II responses unaltered. This study will further indicate that aldosterone is associated with endothelial dysfunction and decreased NO bioactivity in patients with heart failure. Furthermore, eplerenone is expected to prevent such dysfunctions and other consequential pathologic changes.

### B. Composition Working Examples

The following examples illustrate aspects of the present invention. The experimental procedures used to generate the data shown are discussed in more detail below. The symbols and conventions used in these examples are consistent with those used in the contemporary pharmacological literature. Unless otherwise stated, (i) all percentages recited in these examples are weight percents based on total composition weight, (ii) total composition weight for capsules is the total capsule fill weight and does not include the weight of the actual capsule employed, and (iii) coated tablets are coated with a conventional coating material such as Opadry White YS-1-18027A and the weight fraction of the coating is about 3% of the total weight of the coated tablet.

### Example B-1

An oral dosage may be prepared by screening and then mixing together the following list of ingredients in the amounts indicated. The dosage may then be placed in a hard gelatin capsule.

| Ingredients | Amounts |
|---|---|
| eplerenone | 12.5 mg |
| magnesium stearate | 10 mg |
| lactose | 100 mg |

### Example B-2

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| eplerenone | 12.5 mg |
| calcium sulfate dihydrate | 100 mg |
| sucrose | 15 mg |
| starch | 8 mg |
| talc | 4 mg |
| stearic acid | 2 mg |

### Example B-3

An oral dosage may be prepared by screening and then mixing together the following list of ingredients in the amounts indicated. The dosage may then be placed in a hard gelatin capsule.

| Ingredients | Amounts |
|---|---|
| eplerenone | 12.5 mg |
| magnesium stearate | 10 mg |
| lactose | 100 mg |

### Example B-4

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| eplerenone | 12.5 mg |
| calcium sulfate dihydrate | 100 mg |
| sucrose | 15 mg |
| starch | 8 mg |
| talc | 4 mg |
| stearic acid | 2 mg |

### Example B-5: 25 Mg Dose Immediate Release Tablet

A 25 mg dose immediate release tablet (tablet diameter of 7/32") was prepared having the following composition:

| INGREDIENT | WEIGHT % OF TABLET | Amount (mg) |
|---|---|---|
| Eplerenone | 29.41 | 25.00 |
| Lactose Monohydrate (#310, NF) | 42.00 | 35.70 |
| Microcrystalline Cellulose (NF, Avicel PH101) | 18.09 (7.50% intragranular plus 10.59% extragranular) | 15.38 |
| Croscarmellose Sodium (NF, Ac-Di-So1) | 5.00 | 4.25 |
| Hydroxypropyl Methylcellulose (#2910, USP, Pharmacoat 603) | 3.00 | 2.55 |
| Sodium Lauryl Sulfate (NF) | 1.00 | 0.85 |
| Talc (USP) | 1.00 | 0.85 |
| Magnesium Stearate (NF) | 0.50 | 0.42 |
| Total | 100 | 85 |
| Opadry White YS-1-18027A | 3.00 | 2.55 |

### Example B-6: 50 Mg Dose Immediate Release Tablet

A 50 mg dose immediate release tablet (tablet diameter of 9/32") was prepared having the following composition:

| INGREDIENT | WEIGHT % OF TABLET | Amount (mg) |
|---|---|---|
| Eplerenone | 29.41 | 50.00 |
| Lactose Monohydrate (#310, NF) | 42.00 | 71.40 |
| Microcrystalline Cellulose (NF, Avicel PH101) | 18.09 (7.50% intragranular plus 10.59% extragranular) | 30.75 |
| Croscarmellose Sodium (NF, Ac-Di-Sol) | 5.00 | 8.50 |
| Hydroxypropyl Methylcellulose (#2910, USP, Pharmacoat 603) | 3.00 | 5.10 |
| Sodium Lauryl Sulfate (NF) | 1.00 | 1.70 |
| Talc (USP) | 1.00 | 1.70 |
| Magnesium Stearate (NF) | 0.50 | 0.85 |
| Total | 100 | 170 |
| Opadry White YS-1-18027A | 3.00 | 5.10 |

### Example B-7: 100 Mg Dose Immediate Release Tablet

A 100 mg dose immediate release tablet formulation (tablet diameter of 12/32") was prepared having the following composition:

| INGREDIENT | WEIGHT % OF TABLET | Amount (mg) |
|---|---|---|
| Eplerenone | 29.41 | 100.00 |
| Lactose Monohydrate (#310, NF) | 42.00 | 142.80 |
| Microcrystalline Cellulose (NF, Avicel PH101) | 18.09 (7.50% intragranular plus 10.59% extragranular) | 61.50 |
| Croscarmellose Sodium (NF, Ac-Di-Sol) | 5.00 | 17.00 |
| Hydroxypropyl Methylcellulose (#2910, USP, Pharmacoat 603) | 3.00 | 10.20 |
| Sodium Lauryl Sulfate (NF) | 1.00 | 3.40 |
| Talc (USP) | 1.00 | 3.40 |
| Magnesium Stearate (NF) | 0.50 | 1.70 |
| Total | 100 | 340 |
| | | |

| INGREDIENT | WEIGHT % OF TABLET | Amount (mg) |
|---|---|---|
| Opadry White YS-1-18027A | 3.00 | 10.20 |

### Example B-8: 10 mg Dose Immediate Release Capsule

A 10 mg dose immediate release capsule formulation was prepared having the following composition:

| INGREDIENT | AMOUNT (mg) | REPRESENTATIVE BATCH AMOUNT (kg) |
|---|---|---|
| Eplerenone | 10.0 | 1.00 |
| Lactose, Hydrous NF | 306.8 | 30.68 |
| Microcrystalline Cellulose, NF | 60.0 | 6.00 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example B-9: 25 mg Dose Immediate Release Capsule

A 25 mg dose immediate release capsule formulation was prepared having the following composition:

| INGREDIENT | AMOUNT (mg) | REPRESENTATIVE BATCH AMOUNT (kg) |
|---|---|---|
| Eplerenone | 25.0 | 2.50 |
| Lactose, Hydrous NF | 294.1 | 29.41 |
| Microcrystalline Cellulose, NF | 57.7 | 5.77 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example B-10: 50 mg Dose Immediate Release Capsule

A 50 mg dose immediate release capsule formulation was prepared having the following composition:

| INGREDIENT | AMOUNT (mg) | REPRESENTATIVE BATCH AMOUNT (kg) |
|---|---|---|
| Eplerenone | 50.0 | 5.00 |
| Lactose, Hydrous NF | 273.2 | 27.32 |
| Microcrystalline Cellulose, NF | 53.6 | 5.36 |
| Talc, USP | 10.0 | 1.00 |
| Croscarmellose Sodium, NF | 8.0 | 0.80 |
| Sodium Lauryl Sulfate, NF | 2.0 | 0.20 |

| INGREDIENT | AMOUNT (mg) | REPRESENTATIVE BATCH AMOUNT (kg) |
|---|---|---|
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example B-11: 100 mg Dose Immediate Release Capsule

A 100 mg dose immediate release capsule formulation was prepared having the following composition:

| INGREDIENT | AMOUNT (mg) | REPRESENTATIVE BATCH AMOUNT (kg) |
|---|---|---|
| Eplerenone | 100.0 | 10.00 |
| Lactose, Hydrous NF | 231.4 | 23.14 |
| Microcrystalline Cellulose, NF | 45.4 | 4.54 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example B-12: 200 mg Dose Immediate Release Capsule

A 200 mg dose immediate release capsule formulation was prepared having the following composition:

| INGREDIENT | AMOUNT (mg) | REPRESENTATIVE BATCH AMOUNT (kg) |
|---|---|---|
| Eplerenone | 200.0 | 20.00 |
| Lactose, Hydrous NF | 147.8 | 14.78 |
| Microcrystalline Cellulose, NF | 29.0 | 2.90 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### C. Solid State Working Examples

The following examples contain detailed descriptions of the methods of preparation of the various solid state forms of eplerenone described in this application. These detailed descriptions fall within the scope, and serve to exemplify the invention. These detailed descriptions are presented for illustrative purposes only. All parts are by weight and temperatures are in degrees Centigrade unless otherwise indicated. The eplerenone starting material used in each of the following examples was prepared in accordance with scheme 1 set forth in Ng et al., WO98/25948.

### Example C-1: Preparation of (a) methyl ethyl ketone solvate from high purity eplerenone starting material and (b) Form L crystalline eplerenone from resulting solvate.

### A. Preparation of Methyl Ethyl Ketone Solvate:

High purity eplerenone (437 mg; greater than 99% purity with less than 0.2% diepoxide and 11,12 epoxide present) was dissolved in 10 mL of methyl ethyl ketone by heating to boiling on a hot plate with magnetic stirring at 900 rpm. The resulting solution was allowed to cool to room temperature with continuous magnetic stirring. Once at room temperature, the solution was transferred to a 1°C bath with maintenance of the stirring for one hour. After one hour, the solid methyl ethyl ketone solvate was collected by vacuum filtration.

### B. Preparation of Form L crystalline eplerenone:

The solid methyl ethyl ketone solvate prepared in Step A above was dried in an oven at 100°C for four hours at ambient pressure. The dried solid was determined to be pure Form L by DSC and XPRD analysis.

### Example C-2: Preparation of additional solvates from high purity eplerenone starting material

Additional solvated crystalline forms were prepared by replacing methyl ethyl ketone with one of the following solvents: n-propanol, 2-pentanone, acetic acid, acetone, butyl acetate, chloroform, ethanol, isobutanol, isobutyl acetate, isopropanol, methyl acetate, ethyl propionate, n-butanol, n-octanol, propyl acetate, propylene glycol, t-butanol, tetrahydrofuran, and toluene and carrying out the crystallization substantially as described above in Step A of Example C-1. Form L eplerenone was formed from each of the solvates substantially as described in Step B of Example C-1.

### Example C-3: Preparation of methyl Ethyl Ketone Solvate by Vapor Diffusion Growth

Eplerenone (400 mg; greater than 99.9% purity) was dissolved in 20 mL of methyl ethyl ketone by warming on a hot plate to form a stock solution. An 8 mL amount of the stock solution was transferred to a first 20 mL scintillation vial and diluted to 10 mL with methyl ethyl ketone (80%). A 10 mL amount of the stock solution was transferred to a second 20 mL scintillation vial and diluted to 10 mL with methyl ethyl ketone (40%). The final 2 mL of the stock solution was diluted to 10 mL with methyl ethyl ketone (20%). The four vials containing the dilutions were transferred to a dessicator jar containing a small amount of hexane as an anti-solvent. The dessicator jar was sealed and the hexane vapor allowed to diffuse into the methyl ethyl ketone solutions. Methyl ethyl ketone solvate crystals grew in the 80% dilution sample by the next day.

### Example C-4: Preparation of Methyl Ethyl Ketone Solvate by Rotary Evaporation

About 400 mg of eplerenone (greater than 99.9% purity) is weighed into a 250 mL round bottom flask. Solvent (150 mL) is added to the flask and, if necessary, the solution is heated gently until the solid is dissolved. The resulting clear solution is placed on a Buchi rotary evaporator with a bath temperature of about 85°C and the solvent is removed under vacuum. Solvent removal is stopped when approximately 10 mL of solvent remain in the round bottom flask. The resulting solids are analyzed by appropriate method (XPRD, DSC, TGA, microscopy, etc.) for determination of form.

### Example C-5: Slurry Conversion

Approximately 150 mg of Form L eplerenone and 150 mg of Form H eplerenone were added to 5 mL of ethyl acetate. The resulting slurry was allowed to stir at 300 rpm (magnetic stirring) overnight. The next day a sample of the solid was collected by filtration. Analysis of the sample by XPRD indicated that the sample was entirely composed of Form L eplerenone.

### Example C-6: Preparation of (a) solvate from low purity eplerenone starting material and (b) Form H crystalline eplerenone from resulting solvate

Samples containing varying amounts of the impurity 7-methyl hydrogen 4α, 5α:9α,11α-diepoxy-17-hydroxy-3-oxo-17α-pregnane-7α,21-dicarboxylate, γ-lactone (the "diepoxide") or the impurity 7-methyl hydrogen 1 1α,12α-epoxy-17-hydroxy-3-oxo-17α-pregn-4-ene-7α,21-dicarboxylate, γ-lactone (the "11,12-epoxide") were prepared by adding the desired amount of the impurity to a 7 mL scintillation vial together with an amount of eplerenone sufficient to provide a total sample mass of 100 mg. The weight percent of the diepoxide or 11,12-epoxide in each sample is given in Tables C-6A and C-6B, respectively. A micro-flea magnetic stirrer was added to each scintillation vial along with 1 mL of methyl ethyl ketone. The vials were loosely capped and the solid dissolved by heating to reflux on a hot plate with magnetic stirring. Once the solids were dissolved, the solutions were allowed to cool to room temperature on the hot plate. Magnetic stirring was maintained during the cooling period. After the solutions reached room temperature, the solids were collected by vacuum filtration and immediately analyzed by X-ray powder diffraction (XPRD). The solids were then placed in a 100°C oven and dried for one hour at ambient pressure. The dried solids were analyzed by XPRD for Form H content by monitoring the area of the Form H diffraction peak at about 12.1 degrees two theta. All XPRD diffraction patterns were recorded using an Inel Multipurpose Diffractometer.

**Table C-6A**

| Weight Percent Diepoxide | Weight Eplerenone (mg) | Weight Diepoxide (mg) |
|---|---|---|
| 0% | 100.44 | -- |
| 1% | 99.08 | 1.24 |
| 2% | 98.09 | 2.24 |
| 3% | 97.08 | 3.04 |
| 5% | 95.09 | 5.04 |

**Table C-6B**

| Weight Percent 11,12-Epoxide | Weight Eplerenone (mg) | Weight 11,12-Epoxide (mg) |
|---|---|---|
| 0% | 101.38 | 0 |
| 1% | 99.23 | 1.10 |
| 5% | 94.97 | 5.36 |
| 10% | 90.13 | 10.86 |

### A. Diepoxide Results

Fig. C-1 shows the X-ray powder diffraction patterns for the wet cake (methyl ethyl ketone solvate) obtained from the (a) 0%, (b) 1%, (c) 3%, and (d) 5% diepoxide-doped methyl ethyl ketone crystallizations. The peak intensities have been normalized for ease of comparison. No peaks characteristic of Form H or the diepoxide are present in the diffraction patterns. The patterns are characteristic of the methyl ethyl ketone solvate of eplerenone.

Fig. C-2 shows the X-ray powder diffraction patterns for the dried solids obtained from the (a) 0%, (b) 1%, (c) 3%, and (d) 5% diepoxide-doped methyl ethyl ketone crystallizations. The peak intensities have been normalized for ease of comparison. No Form H was detected for the dried samples corresponding to the methyl ethyl ketone crystallizations performed at doping levels of 0 and 1%. Form H was detected in the dried samples corresponding to the methyl ethyl ketone crystallizations performed at doping levels of 3 and 5%. The area for the Form H diffraction peak at about 12.1 degrees two theta and the estimated Form H content for each sample are given in Table C-6C below.

**Table C-6C**

| Weight Percent of Diepoxide in Starting Eplerenone Mixture | Weight Percent of Diepoxide in Resulting Crystals (HPLC) | Form H Peak Area 12° Two Theta Peak | Estimated Weight Percent of Form H |
|---|---|---|---|
| 0% | --- | None Detected | None Detected |
| 1% | 0.29% | None Detected | None Detected |
| 3% | 0.58% | 1168 | 10% |
| 5% | 1.05% | 4175 | 30% |

The results reported in Table C-6C confirm that the presence of the diepoxide affects the formation of Form H during the desolvation. These results indicate that the diepoxide is effective in inducing the formation of Form H eplerenone when it is incorporated into and/or adsorbed onto the methyl ethyl ketone solvate crystals.

The 3% diepoxide doping experiment was repeated to analyze the impact of the route of preparation on the amount of Form H formed during the desolvation. In this experiment, the methyl ethyl ketone solvate obtained from the doped crystallization was divided into two portions. The first portion was left untreated while the second portion was lightly ground in a mortar and pestle to induce a higher level of crystal defects. The two portions were both dried at 100 °C for one hour at ambient pressure. The dried solids were analyzed by XPRD. The XPRD patterns are given in Fig. C-3 for the dried solids from the methyl ethyl ketone crystallization with 3% doping of diepoxide (a) without grinding of the solvate prior to drying, and (b) with grinding of the solvate prior to drying. The XPRD patterns indicated a greater amount of Form H in the ground sample relative to the unground sample. These results suggest that the conditions under which the methyl ethyl ketone solvate is isolated and handled can affect the crystal form that results from the desolvation.

### B. 11,12-Epoxide Results

Fig. C-4 shows the X-ray powder diffraction patterns for the wet cake (methyl ethyl ketone solvate) obtained from the (a) 0%, (b) 1%, (c) 5%, and (d) 10% 11,12-epoxide-doped methyl ethyl ketone crystallizations. The peak intensities have been normalized for ease of comparison. No peaks characteristic of Form H or the 11,12-epoxide are present in the diffraction patterns. The patterns are characteristic of the methyl ethyl ketone solvate of eplerenone.

Fig. C-5 shows the X-ray powder diffraction patterns for the dried solids obtained from the (a) 0%, (b) 1%, (c) 5%, and (d) 10% 11,12-epoxide-doped methyl ethyl ketone crystallizations. The peak intensities have been normalized for ease of comparison. No Form H was detected for the dried samples corresponding to the methyl ethyl ketone crystallizations performed at doping levels of 0, 1% and 5%. Form H was detected in the dried samples corresponding to the methyl ethyl ketone crystallization performed at a doping level of 10%. The area for the Form H diffraction peak at 12.1 degrees two theta and estimated Form H content for each sample are given in Table C-6D.

**Table C-6D**

| Weight Percent 11,12-Epoxide in Starting Eplerenone Mixture | Weight Percent 11,12-Epoxide in Resulting Crystals (HPLC) | Form H Peak Area 12° Two Theta Peak | Estimated Weight Percent of Form H |
|---|---|---|---|
| 0% | Not Available | None Detected | None Detected |
| 1% | Not Available | None Detected | None Detected |
| 5% | Not Available | None Detected | None Detected |
| 10% | Not Available | 1541 | 10-15% |

The results reported in Table C-6D confirm that the presence of the 11,12-epoxide impacts the formation of Form H during the desolvation. The percentage of impurity in the methyl ethyl ketone crystallization required to induce the formation of Form H eplerenone appears to be greater for the 11,12-epoxide than for the diepoxide.

### Example C-7: Effect of Crystallization and Drying on Final Crystal Form

The following four experiments analyzing the effect of crystallization and drying on the final crystal form were conducted: (i) methyl ethyl ketone crystallization of eplerenone (2³+3 statistical design of experiment), (ii) crystallization of poor quality mother liquor residue, (iii) crystallization of high purity eplerenone with Form H seeding, and (iv) crystallization of low purity eplerenone with Form L seeding. Variables in the design of the experiments included cooling rate, starting material purity level, and end point temperature of crystallization. For purposes of this Example, high purity eplerenone was defined as ultra-pure milled eplerenone (HPLC analysis showed this material to be 100.8% pure) and low purity eplerenone was defined as 89% pure eplerenone. To prepare the low purity eplerenone, stripped-down mother liquors from the process for the preparation of eplerenone were analyzed and blended to yield a material that was 61.1% eplerenone, 12.8% diepoxide and 7.6% 11,12-epoxide. This material was then blended with a sufficient amount of high purity eplerenone to yield the 89% eplerenone.

### Methyl Ethyl Ketone Crystallization

In the methyl ethyl ketone crystallization experiment, all runs were performed using 60 g of high purity eplerenone. High endpoint was defined as 45° C and low endpoint was defined as 5°C. High cooling rate was defined as 3° C/minute cooling and low cooling rate was defined as 0.1 °C/minute cooling. Center points were 1.5°C/minute cooling, 94.5% pure eplerenone, and a 25°C endpoint.

After a background reading was taken with the FTIR, 250 mL of methyl ethyl ketone was charged to a 1L Mettler RC-1, MP10 reactor and stirred at 100 rpm. After several scans, eplerenone was charged to the reactor followed by an additional 470 mL of methyl ethyl ketone. Agitation was increased to 500 rpm to suspend solids and the batch temperature was increased to 80°C. The batch temperature was held at 80°C to ensure dissolution of the eplerenone. Black or white specks generally were visible in the resulting transparent solution. The batch temperature was then ramp cooled at the desired rate to the desired endpoint, where it was maintained for one hour before being pulled into a transfer flask and filtered. The vacuum was reactor, transfer flask and cake were then washed with 120 mL methyl ethyl ketone. Once the wash was pulled through the cake, the stopped. About 10 grams of each wet cake were dried in a vacuum oven under nominal conditions of 75°C with a light nitrogen bleed. For the "high, high, high" and "low, low, low" experiments described below, fluid bed drying was operated under high and low conditions. High fluid bed drying was defined as 100°C with a blower setting of "4" while low fluid bed drying was defined as 40°C with a blower setting of "1".

### Crystallization of Poor Quality Mother Liquor Residue

In the crystallization of poor quality mother liquor residue experiment, 60 g of the 61.1% pure material and 720 mL methyl ethyl ketone were charged directly to a 1L Mettler RC-1, MP10 reactor. The 61.1% pure material was not blended with high purity eplerenone prior to being charged to the reactor. The resulting mixture was heated to 80°C and was an opaque slurry at that temperature. The crystallization continued and the mixture was filtered at 45°C under fast cooling conditions.

### Form H Seeding

In the Form H seeding experiment, 60 g of pure (100.8%) eplerenone and 720 mL of methyl ethyl ketone were charged to a 1L Mettler RC-1, MP10 reactor. The mixture was heated to 80°C and then cooled to 25°C at a rate of 1.5°C/minute. When the solution had cooled to 62°C, it was seeded with 3 g of phase pure Form H crystals to initiate crystallization. The Form H seed crystals were prepared by the digestion process described in Example C-9 below.

### Form L Seeding

In the Form L seeding experiment, 66.6 g of 89.3% eplerenone (prepared by mixing 48.3 g of 100% eplerenone with 18.3 g of 61.1% eplerenone) and 720 mL of methyl ethyl ketone were charged to a 1L Mettler RC-1, MP10 reactor. The mixture was heated to 80°C and then cooled to 25°C at a rate of 1.5°C/minute. When the solution had cooled to 63°C, it was seeded with 3 g of phase pure Form L crystals to initiate crystallization. The Form L seed crystals were prepared by the crystallization and desolvation process described in Example C-1 above.

Results from the experiments are reported in Table C-7A. In the n+1 crystallization experiment, Form H was detected only in the experiments employing low purity eplerenone where the product contained the diepoxide. Elevated levels of the diepoxide in the final product were also observed with higher cooling rates.

The crystallization of poor quality mother liquor residue experiment yielded poor quality material that appeared to be a mixture of the diepoxide and Form H when analyzed by X-ray powder diffraction.

The Form H seeding experiment (where high purity eplerenone was seeded with Form H) yielded a product that was 77% Form H based on X-ray powder diffraction analysis, but entirely Form H based on DSC. The X-ray powder diffraction model, however, had not been tested for linearity beyond about 15% Form H. This experiment was the only one of the four experiments of this Example where Form H was created in the absence of the diepoxide.

The Form L seeding experiment (where low purity eplerenone was seeded with Form L) yielded a product that was entirely Form L.

The data obtained for the high fluid bed drying of eplerenone appeared to correspond to the data obtained for the vacuum oven drying. The low fluid bed dryings yielded results that differed from those of the vacuum oven dryings.

**Table C-7A**

| Cooling Rate¹ | Cooling Endpoint² | Impurity Level³ | Nucleation Temperature (°C) | Weight Percent 11,12-Epoxide⁴ | Weight Percent Diepoxide⁴ | Assay For Desolvated Crystal | Percent Yield | Weight Percent Form H (XPRD) |
|---|---|---|---|---|---|---|---|---|
| + | + | - | 57.0 | ND | ND | 100.3 | 66.1 | ND |
| + | - | - | 54.9 | ND | ND | 100.3 | 98.1 | ND |
| - | + | - | 60.9 | ND | ND | 100.3 | | ND |
| - | - | - | 63.4 | ND | ND | 100.5 | 79.3 | ND |
| + | + | ++ | N/A | 4.8 | 36.6 | 43.3 | 27 | 100⁵ |
| + | + | + | 52.2 | 0.49 | 0.88 | 98.3 | 62 | 29 |
| + | - | + | 53.3 | 0.56 | 1.0 | 98.1 | 87 | 9 |
| 0 | 0 | 0 | 59.0 | 0.18 | 0.36 | 99.4 | 75 | 5 |
| - | + | + | 63.3 | 0.20 | 0.44 | 99.4 | 36 | 31 |
| - | - | + | 61.4 | 0.18 | 0.40 | 99.5 | 87 | ND |
| 0 | 0 | 0 | 60.6 | 0.18 | 0.36 | 99.5 | 79.2 | ND |
| 0 | 0 | 0 | 55.9 | 0.38 | 0.80 | 98.6 | 80.5 | <3% |
| 0 | 0 | 100.8% eplerenone seeded with Form H | | 0.03 | ND | 100.4 | 82.2 | 77/100⁶ |
| 0 | 0 | 89.3% eplerenone seeded with Form L | | 0.33 | 0.50 | 97.5 | 80.2 | ND |

| | | | | | | | | |
|---|---|---|---|---|---|---|---|---|
| ¹Cooling Rate: (+) = 3°C/min.; (0) = 1.5 °C/min.; and (-) = 0.1 °C/min. ²Cooling Endpoint: (+) = 45°C; (0) = 25 °C; and (-) = 5°C. ³Impurity Level: : (+) = 89.3% purity eplerenone starting material; (++) = 61.1% purity eplerenone starting material; (0) = 100.8% purity eplerenone starting material; and (-) = 94.5% purity eplerenone starting material. ⁴Weight percent after drying solvate in a vacuum oven at 75°C. ⁵Appears to be mixture of Form II and diepoxide when analyzed by XPRD. ⁶Appears to be 77% Form II when analyzed by XPRD and 100% Form H when analyzed by DSC. | | | | | | | | |

### A. Material Purity

A cube plot of product purity, starting material purity, cooling rate and endpoint temperature based on the data reported in Table C-7A is shown in Fig. C-6. The cube plot suggests that the use of a higher purity material at the start of crystallization will yield a higher purity product. The endpoint temperature of crystallization does not appear to greatly affect the product purity. The cooling rate, however, appears to have an effect with slightly less pure product resulting from a faster cooling rate. In fact, the level of diepoxide generally was higher with faster cooling rates.

Fig. C-7 shows a half normal plot that was prepared using the results of cube plot to determine which variables, if any, had a statistically significant effect on the product purity. Starting material purity had the greatest statistically significant effect on product purity, although cooling rate and the interaction between cooling rate and starting material purity were also seen as statistically significant effects.

Fig. C-8 is an interaction graph based on these results and showing the interaction between starting material purity and cooling rate on product purity. With the high purity eplerenone (100.8% eplerenone starting material) the cooling rate appears to have little or no effect on final purity. With the low purity eplerenone (89.3% eplerenone starting material), however, the product purity decreases as cooling rate increases. This result suggests that more impurities crystallize out in eplerenone crystallizations conducted at higher cooling rates.

### Form H Content

A cube plot of Form H weight fraction, starting material product purity, cooling rate and endpoint temperature based on the data reported in Table C-7A is shown in Fig. C-9. The cube plot suggests that the use of a higher purity eplerenone at the start of crystallization will yield a lower amount of Form H. The endpoint temperature of crystallization also appears to have an effect on the form of the final product. The cooling rate does not appear to greatly affect the formation of Form H although some Form H may result from faster cooling at the low endpoint temperature in the presence of impurities.

Fig. C-10 shows a half normal plot that was prepared using the results of the cube plot to determine which variables, if any, had a statistically significant effect on the amount of Form H in the final material. Starting material purity, endpoint temperature of the crystallization and the interaction between the two variables were seen as statistically significant effects.

Fig. C-11 is an interaction graph based on these results and showing the interaction between starting material purity and endpoint temperature on final Form H content. With the high purity eplerenone (100.8% eplerenone starting material), endpoint temperature appears to have little effect on Form H content. No Form H resulted in either case with pure eplerenone. With low purity eplerenone (89.3% eplerenone starting material), however, Form H was present in both cases, with significantly more Form H at higher endpoint temperatures.

Table C-7B reports the weight fraction of Form H measured in materials dried using either a fluid bed (LAB-LINE/P.R.L. Hi-Speed Fluid Bed Dryer, Lab-Line Instruments, Inc.) or a vacuum oven (Baxter Scientific Products Vacuum Drying Oven, Model DP-32). Similar Form H content was observed for comparable materials dried in either the high fluid bed or the vacuum oven. A difference was observed, however, for comparable materials dried in the low fluid bed relative to the vacuum oven.

**Table C-7B**

| Cooling Rate | End Point | Impurity Level | Drying Type | Weight Percent Form H |
|---|---|---|---|---|
| High | High | High | Vacuum Oven | 29% |
| High | High | High | High Fluid Bed | 25% |
| High | High | High | Low Fluid Bed | 4.7% |
| Low | Low | Low | Vacuum Oven | ND |
| Low | Low | Low | High Fluid Bed | ND |
| Low | Low | Low | Low Fluid Bed | 5.5% |

### Example C-8: Crystallization of a Mixture of Form H and Form L From Methyl Ethyl Ketone To Prepare a Solvate, and (b) Desolvation of the Solvate to Prepare Form L

Form H eplerenone (10 g) was combined with 80 mL of methyl ethyl ketone. The mixture was heated to reflux (79°C) and stirred at this temperature for about 30 minutes. The resulting slurry was then cooled with a stepwise, holdpoint protocol by maintaining the slurry at 65°C, 50°C, 35°C and 25°C for about 90 minutes at each temperature. The slurry was filtered and rinsed with about 20 mL methyl ethyl ketone. The isolated solid was initially dried on the filter and then in a vacuum oven at 40-50°C. The drying was completed in the vacuum oven at 90-100°C. The desolvated solid was obtained with an 82% recovery. XPRD, MIR and DSC confirmed that the solid had a Form L crystalline structure.

### Example C-9: Digestion of Low Purity Eplerenone Starting Material With a Solvent to Prepare Form H

### Digestion With Ethanol Solvent:

Low purity eplerenone (24.6 g; 64% by weight assay via HPLC) was combined with 126 mL of ethanol 3A. The slurry was heated to reflux and the distillate removed. An additional 126 mL of ethanol 3A was simultaneously added as 126 ml of solvent was removed via atmospheric distillation. Upon completion of the solvent turnover, the mixture was cooled to 25°C and stirred for one hour. The solid was filtered and rinsed with ethanol 3A. The solid was air-dried to give the ethanol solvate. The solvate was further dried in a vacuum oven at 90-100°C for six hours to obtain 14.9 g of Form H eplerenone.

### Digestion With Methyl Ethyl Ketone Solvent

In an alternative digestion process, 1 gram of low purity eplerenone (about 65% pure) was digested in 4 mL of methyl ethyl ketone for two hours. After the two hours, the mixture was allowed to cool to room temperature. Once cooled, the solid was collected by vacuum filtration and determined to be the methyl ethyl ketone solvate by XPRD analysis. The solid was dried at 100°C for 30 to 60 minutes. The dried solids were determined to be pure Form H by XPRD.

### Example C-10: Digestion of High Purity Eplerenone Starting Material With a Solvent to Prepare Form L

### Digestion With Ethanol Solvent:

High purity eplerenone (1 gram) was digested in 8 mL of ethanol for approximately two hours. The solution was then allowed to cool to room temperature and the solids were collected by vacuum filtration. Analysis of the solids by XPRD immediately after filtration indicated that the solids were a solvate (presumably the ethanol solvate). The solids were subsequently dried at 100°C at atmospheric pressure for 30 minutes. The dried solid was analyzed by XPRD and determined to be predominately Form L (no Form H detected).

### Digestion with Methyl Ethyl Ketone Solvent:

High purity eplerenone (1 gram) was digested in 4 mL of methyl ethyl ketone for two hours. After the two hours, the solution was allowed to cool to room temperature and the solids collected by vacuum filtration. The solid was immediately analyzed by XPRD and determined to be a solvate of eplerenone (presumably the methyl ethyl ketone solvate). The solvate was subsequently dried at 100°C at ambient pressure for 30 to 60 minutes. The dried solids were analyzed by XPRD and determined to be primarily Form L with no diffraction peaks for Form H present.

### Example C-11: Crystallization of Form L Directly From Solution

Procedure A: Eplerenone (2.5 g) was dissolved in ethyl acetate by heating to 75°C. Once the eplerenone dissolved, the solution was held at 75°C for 30 minutes to ensure complete dissolution. The solution was then cooled at 1 °C/min to 13°C. Once at 13°C, the slurry was allowed to stir for two hours at 750 rpm with an overhead stirrer. The crystals were collected by vacuum filtration and dried in a vacuum oven at 40°C for one hour. The XPRD pattern and DSC thermogram of the solid were characteristic of Form L eplerenone. Thermal gravimetric analysis (TGA) of the solid indicated no weight loss from the solid up to 200°C.

Procedure B: In an alternative procedure, 2 g of eplerenone was dissolved in 350 mL of 15/85% acetonitrile/water by heating on a hot plate with magnetic stirring. Once the eplerenone was dissolved, the solution was allowed to cool to room temperature overnight with magnetic stirring. The resulting solid was collected by vacuum filtration. The crystals were birefringent and had a triangular, plate-like crystal habit. The solid had an XPRD and DSC characteristic of Form L. eplerenone. TGA indicated no weight loss up to 200°C.

Procedure C: In an alternative procedure, 640 mg of eplerenone was placed in a 50 mL flask with 20 mL of ethyl benzene. The resulting slurry was heated to 116°C and became a clear solution. The clear solution was cooled to 25°C over 30 minutes. Nucleation began at 84°C during the cooling period. The resulting solids were filtered from the solution and air-dried to give 530 mg of solids (83% recovery). Hot-stage microscopy and XPRD confirmed that the solids were Form L crystals.

Procedure D: In an alternative procedure, 1.55 g of eplerenone was added to 2.0 mL of nitrobenzene and heated to 200°C. The resulting slurry was stirred overnight at 200°C. The solution was allowed to cool to room temperature (natural air convection) the following day and the solid was isolated. The solid was determined to be Form L eplerenone by XPRD and polarized light microscopy.

Procedure E: In an alternative procedure, 5.0 g of eplerenone (purity greater than 99%) was added to 82 g of methanol (104 mL). Under stirring action (210 rpm), the solution was heated to 60°C and held at that temperature for 20 minutes to ensure complete dissolution. The solution was then cooled to -5°C at a rate of 0.16°C/minute under stirring. The crystals were collected by filtration and dried in a vacuum oven at 40°C for 20 hours. The dried solids were determined to be pure Form L eplerenone by DSC and XPRD analysis.

Procedure F: In an alternative procedure, 6.0 g of eplerenone (ethanol solvate containing 9% ethanol and having a corrected purity of 95.2%) was added to 82 g of methanol (104 mL). Under stirring action (210 rpm), the solution was heated to 60°C and held at that temperature for 20 minutes to ensure complete dissolution. The solution was then cooled to 50°C at a rate of 0.14°C/minute and then held at that temperature for about 2.5 hours. The solution was then cooled to - 5°C at a rate of 0.13°Clminute under stirring. The crystals were collected by filtration and dried in a vacuum oven at 40°C for 16 hours. The dried solids were determined to be pure Form L eplerenone by DSC and XPRD analysis.

### Example C-12: Crystallization of Form H Directly From Solution

150.5 mg of the diepoxide and 2.85 g of eplerenone were added to 1.5 mL of nitrobenzene. The mixture was magnetically stirred at 200°C for several hours. The slurry was then allowed to cool to room temperature by natural air convection. The sample was dried and analyzed by polarized light microscopy and XPRD. The XPRD indicated that the sample was a mixture of Form H and Form L. The crystals were translucent by microscopy, indicating that desolvation (and conversion to either Form H or Form L) did not occur.

### Example C-13: Preparation of Amorphous Eplerenone By Comminution

Approximately one-half of a steel Wig-L-Bug container was filled with about 60 g of eplerenone (greater than 99.9% purity). A steel ball and cap were placed on the sample container and agitated for 30 seconds by the Wig-L-Bug apparatus. The eplerenone was scraped off the surface of the Wig-L-Bug container and the container agitated for an additional 30 seconds. The resulting solid was analyzed by XPRD and DSC and determined to be a mixture of amorphous eplerenone and Form L crystalline eplerenone.

### Example C-14: Preparation of Amorphous By Lyophilization

Approximately 100 mg of crude eplerenone was weighed into a beaker containing 400 mL of water. The solution was heated slightly for five minutes, and then sonicated and heated with stirring for an additional five minutes. Approximately 350 mL of the eplerenone solution was filtered into a 1000 mL round bottom flask containing 50 mL of HPLC water. The solution was flashed frozen in a dry ice/acetone bath over a time period of one to two minutes. The flask was attached to a Labconco Freezone 4.5 freeze dryer and dried overnight. The solids in the flask were transferred to a small brown bottle. A small aliquot was observed under polarized light microscopy at 10X, 1.25X optivar in cargille oil (1.404) and observed to be at least 95% amorphous eplerenone. Figures C-12 and C-13 show the XPRD pattern and DSC thermogram obtained for the amorphous eplerenone. The peak observed at 39 degrees two theta in Figure C-12 is attributable to the aluminum sample container.

### Example C-15: Eplerenone Polymorph Composition

Tablets containing 25 mg, 50 mg, 100 mg and 200 mg doses of Form L eplerenone are prepared and have the following composition:

| Ingredient | Weight % of Tablet |
|---|---|
| Form L Eplerenone | 29.41 |
| Form H Eplerenone | Not Detected |
| Lactose Monohydrate (#310, NF) | 42.00 |
| Microcrystalline Cellulose (NF, Avicel PH101) | 18.09 |
| Croscarmellose Sodium (NF, Ac-Di-Sol) | 5.00 |
| Hydroxypropyl Methylcellulose (#2910, USP, Pharmacoat 603) | 3.00 |
| Sodium Lauryl Sulfate (NF) | 1.00 |
| Talc (USP) | 1.00 |
| Magnesium Stearate (NF) | 0.5 |
| Total | 100.00 |

### Example C-16: Eplerenone Polymorph Composition

Capsules (hard gelatin capsule, #0) are prepared containing a 100 mg dose of eplerenone and have the following composition:

| Ingredient | Amount (mg) |
|---|---|
| Form L Eplerenone | 90.0 |
| Form H Eplerenone | 10.0 |
| Lactose, Hydrous, NF | 231.4 |
| Microcrystalline Cellulose, NF | 45.4 |
| Talc, USP | 10.0 |
| Croscarmellose Sodium, NF | 8.0 |
| Sodium Lauryl Sulfate, NF | 2.0 |
| Colloidal Silicon Dioxide, NF | 2.0 |
| Magnesium Stearate, NF | 1.2 |
| Total Capsule Fill Weight | 400.0 |

### Example C-17: Eplerenone Polymorph Composition

Capsules (hard gelatin capsule, size #0) are prepared containing a 200 mg dose of eplerenone and have the following composition:

| Ingredient | Amount (mg) |
|---|---|
| Form L Eplerenone | 190.0 |
| Form H Eplerenone | 10.0 |
| Lactose, Hydrous, NF | 147.8 |
| Microcrystalline Cellulose, NF | 29.0 |
| Talc, USP | 10.0 |
| Croscarmellose Sodium, NF | 8.0 |
| Sodium Lauryl Sulfate, NF | 2.0 |
| Colloidal Silicon Dioxide, NF | 2.0 |
| Magnesium Stearate, NF | 1.2 |
| Total Capsule Fill Weight | 400.0 |

### Example C-18: Preparation of Milled Eplerenone

Dried methyl ethyl ketone solvate is first delumped by passing the solvate through a 20 mesh screen on a Fitzmill. The delumped solid is then pin milled using an Alpine Hosakawa stud disk pin mill operating under liquid nitrogen cooling at a feed rate of approximately 250 kilograms/hour. Pin milling produces milled eplerenone with a D₉₀ size of approximately 65-100 microns.

## Claims

1. The use of eplerenone for the manufacture of a medicament for the treatment or prophylaxis of renal dysfunction in a human being suffering from or susceptible to renal dysfunction, wherein said renal dysfunction is diabetic nephropathy or end-stage renal disease and the subject has one or more conditions selected from a sub-normal endogenous aldosterone level, salt sensitivity and an elevated dietary sodium intake.

2. Use according to Claim 1, wherein the subject has a sub-normal endogenous aldosterone level.

3. Use according to Claim 2, wherein the aldosterone level is less than 3 ng/dL.

4. Use according to Claim 1, wherein the subject has salt sensitivity.

5. Use according to Claim 1, wherein the subject has an elevated dietary sodium intake.

6. Use according to Claim 5, wherein the subject has an average daily intake of sodium of at least 50 milliequivalents.

7. Use according to Claim 5, wherein the subject has an average daily intake of sodium of at least 200 milliequivalents.

8. Use according to any of Claims 1 to 4, wherein the subject has an activities ratio of plasma aldosterone (ng/dL) to plasma renin (ng/mL/hr) of greater than 30.

9. Use according to any of Claims 1 to 4, wherein the subject has a morning plasma renin level of less than or equal to 1.0 ng/dL/hr.

10. Use according to any of Claims 1 to 4, wherein the subject has a urinary sodium to potassium ratio of less than 6.

11. Use according to any of Claims 1 to 4, wherein the subject is, in whole or in part, a member of the Black ethnic group.

## Patentansprüche

1. Verwendung von Eplerenon zur Herstellung eines Medikaments zur Behandlung oder Prophylaxe von Nierendysfunktion bei einem Menschen, leidend an oder anfällig für Nierendysfunktion, wobei die Nierendysfunktion diabetische Nephropathie oder terminale Niereninsuffizienz ist und das Subjekt einen oder mehrere Zustände, ausgewählt aus subnormalem endogenen Aldosteronspiegel, Salzempfindlichkeit und einer erhöhten Diät-/Nahrungsnatriumaufnahme, aufweist.

2. Verwendung nach Anspruch 1, wobei das Subjekt einen subnormalen endogenen Aldosteronspiegel aufweist.

3. Verwendung nach Anspruch 2, wobei der Aldosteronspiegel weniger als 3 ng/dL ist.

4. Verwendung nach Anspruch 1, wobei das Subjekt Salzempfindlichkeit aufweist.

5. Verwendung nach Anspruch 1, wobei das Subjekt eine erhöhte Diät-/Nahrungsnatriumaufnahme aufweist.

6. Verwendung nach Anspruch 5, wobei das Subjekt eine durchschnittliche tägliche Aufnahme von Natrium von wenigstens 50 Milliäquivalenten aufweist.

7. Verwendung nach Anspruch 5, wobei das Subjekt eine durchschnittliche tägliche Aufnahme von Natrium von wenigstens 200 Milliäquivalenten aufweist.

8. Verwendung nach einem der Ansprüche 1 bis 4, wobei das Subjekt ein Aktivitätenverhältnis von Plasmaaldosteron (ng/dL) zu Plasmarenin (ng/mL/h) von mehr als 30 aufweist.

9. Verwendung nach einem der Ansprüche 1 bis 4, wobei das Subjekt einen morgendlichen Plasmareninspiegel von weniger als oder gleich 1,0 ng/dL/h aufweist.

10. Verwendung nach einem der Ansprüche 1 bis 4, wobei das Subjekt ein Natrium-zu-Kalium-Verhältnis im Harn von weniger als 6 aufweist.

11. Verwendung nach einem der Ansprüche 1 bis 4, wobei das Subjekt vollständig oder teilweise ein Mitglied der schwarzen Bevölkerungsgruppe ist.

## Revendications

1. Utilisation de l'éplérénone pour la fabrication d'un médicament pour le traitement ou la prophylaxie de la dysfonction rénale d'un être humain souffrant, ou susceptible de souffrir, d'une dysfonction rénale, dans laquelle ladite dysfonction rénale est une néphropathie diabétique ou une maladie rénale au stade terminal et le sujet a un ou plusieurs états sélectionnés entre un niveau d'aldostérone endogène sub-normal, une sensibilité au sel et une capture élevée du sodium alimentaire.

2. Utilisation selon la revendication 1, dans laquelle le sujet a niveau d'aldostérone endogène sub-normal.

3. Utilisation selon la revendication 2, dans laquelle le niveau d'aldostérone est inférieur à 3 ng/dl.

4. Utilisation selon la revendication 1, dans laquelle le sujet a une sensibilité au sel.

5. Utilisation selon la revendication 1, dans laquelle le sujet a une capture élevée du sodium alimentaire.

6. Utilisation selon la revendication 5, dans laquelle le sujet a une capture moyenne quotidienne de sodium d'au moins 50 milliéquivalents.

7. Utilisation selon la revendication 5, dans laquelle le sujet a une capture moyenne quotidienne de sodium d'au moins 200 milliéquivalents.

8. Utilisation selon l'une quelconque des revendications 1 à 4, dans laquelle le sujet a un rapport d'activité entre l'aldostérone plasmatique (ng/dl) et la rénine plasmatique (ng.ml/h) supérieure à 30.

9. Utilisation selon l'une quelconque des revendications 1 à 4, dans laquelle le sujet a un niveau matinal de rénine plasmatique inférieure ou égale à 1,0 ng.dl/h.

10. Utilisation selon l'une quelconque des revendications 1 à 4, dans laquelle le sujet a un rapport sodium urinaire au potassium urinaire inférieur à 6.

11. Utilisation selon l'une quelconque des revendications 1 à 4, dans laquelle le sujet est, en tout ou partie, membre du groupe ethnique noir.
